(19) Europäisches Patentamt
European Patent Office
Office européen des brevets

(11) EP 4 647 434 A1

(12) **EUROPEAN PATENT APPLICATION**
published in accordance with Art. 153(4) EPC

(43) Date of publication:
**12.11.2025 Bulletin 2025/46**

(21) Application number: **24738558.6**

(22) Date of filing: **05.01.2024**

(51) International Patent Classification (IPC):
***C07F 9/44** (2006.01)* ***A61K 31/664** (2006.01)*
***A61P 35/00** (2006.01)* ***A61P 31/00** (2006.01)*

(52) Cooperative Patent Classification (CPC):
**A61K 31/664; A61P 31/00; A61P 35/00; C07F 9/44**

(86) International application number:
**PCT/CN2024/070780**

(87) International publication number:
**WO 2024/146627 (11.07.2024 Gazette 2024/28)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **06.01.2023 CN 202310017707**

(71) Applicant: **Unicet Biotech Co., LLC**
**Beijing 102206 (CN)**

(72) Inventors:
- **MA, Weiwei**
  **Beijing 102206 (CN)**
- **XIE, Yonghua**
  **Beijing 102206 (CN)**
- **SUI, Yinqiang**
  **Beijing 102206 (CN)**
- **ZHANG, Hui**
  **Beijing 102206 (CN)**
- **HAO, Rui**
  **Beijing 102206 (CN)**

(74) Representative: **EIP**
**Fairfax House**
**15 Fulwood Place**
**London WC1V 6HU (GB)**

(54) **MODULATOR FOR PROMOTING BINDING OF BUTYROPHILIN 3A1/2A1**

(57) A compound of formula (I) or a pharmaceutically acceptable salt, enantiomer, diastereoisomer, solvate, hydrate, polymorph or an isotopic variant thereof. The compound can promote the binding of butyrophilin 3A1/2A1, and the definition of each group in formula (I) is as detailed in the description and the claims. The present invention further relates to a pharmaceutical composition containing the compound of formula (I) and the use thereof in clinical treatments.

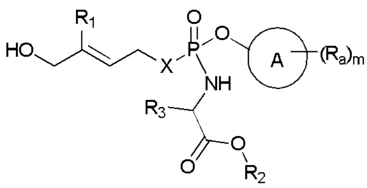

(I)

EP 4 647 434 A1

## Description

## TECHNICAL FIELD

The present disclosure relates to a small molecule modulator that promotes binding of butyrophilin 3A1 (BTN3A1) and butyrophilin 2A1 (BTN2A1) in vivo, thereby exerting their biological functions, including applications in the treatment of tumor and infectious disease.

## BACKGROUND

Butyrophilin (BTN) is a class of transmembrane proteins that generally exert their specific biological functions by forming heteropolymers. Both butyrophilin 3A1 (BTN3A1) and butyrophilin 2A1 (BTN2A1) proteins include two extracellular domains (IgV and IgC), a transmembrane domain (TM), a proximal coiled-coil domain (JM), and an intracellular B30.2 domain, wherein the intracellular B30.2 domain of BTN2A1 has a homology of up to 50 % with the intracellular B30.2 domain of BTN3A1. However, in the intracellular B30.2 domain of BTN3A1 and its ligand binding pocket, BTN2A1 lacks a key basic residue amino acid, and therefore, only when the B30.2 domains of the BTN3A1 and BTN2A1 are bound, the target cells will be recognized by immune cells such as αβ T cells and γδ T cells.

For example, Vγ9Vδ2 T cells are a common subtype in γδ T cells, an important type of immune cell, and an important bridge for connecting natural immunity and adaptive immunity. Vγ9Vδ2 T cells can generate a killing effect on tumor cells and multiple pathogens after activation. Vγ9Vδ2 T cells play an important role in anti-infection and anti-tumor fields. Although BTN3A1 plays an important role in identifying and activating Vγ9Vδ2 T cells, small molecules that bind to BTN3A1 alone do not have biological functions. Only when small molecules can promote binding of the intracellular segments of BTN3A1 to BTN2A1, the small molecules can activate Vγ9Vδ2 T cells.

HMBPP is a natural small molecule produced in cells after the cells have been infected by an infectious agent. Its activity in activating BTN3A1/BTN2A1 is thousands of times higher than the activity of endogenous molecules such as IPP and DMAPP. HMBPP carries two negative charges under physiological conditions and is difficult to enter cells by itself. Its activity is weak in the case of short-term incubation (2h pulse stimulation), and at the same time, HMBPP has poor plasma stability and is difficult in drugability. The activity of C-HMBPP has a certain improvement compared with that of HMBPP, but its short-term stimulation activity is still weak.

Two patents, WO2020008189 and WO2019182904, provide some new molecules. In addition to the in vitro activity data, the in vivo activity and subsequent clinical information of these molecules have not yet been disclosed.

This disclosure describes a small molecule compound that can effectively promote binding of the intracellular B30.2 domains of BTN3A1-BTN2A1, and use thereof in the treatment of a disease. The molecule has high activity and good drugability.

## SUMMARY

The present disclosure proves that the BTN3A1 B30.2 domain and the BTN2A1 B30.2 domain do not bind in the absence of a modulator by using Size Exclusion Chromatography - Multi-Angle Light Scattering (SEC-MALS) and Sedimentation Velocity Analytical Ultracentrifugation (SV-AUC) tests. Moreover, after the addition of small molecules, they cannot lead to the activation of Vγ9Vδ2 T cells if they can only bind to the BTN3A1 B30.2 domain; only when the B30.2 domains of these two proteins can be adhered together by the small molecules (such as those disclosed in the present application), can the activation of Vγ9Vδ2 T cells be promoted.

The compound provided by the present disclosure has the ability to promote binding of 3A1-2A1, and can activate Vγ9Vδ2 T cells. Further, some compounds provided by the present disclosure have good stability, low clearance rate, and low binding rate with plasma proteins, which is conducive to producing more free active drugs in the blood. Furthermore, some compounds provided by the present disclosure also have good cell killing activity, as well as longer human liver microsome stability, lower plasma protein binding rate, and other comprehensive properties suitable for drugability.

**In** one aspect, the present disclosure provides a compound of formula (I), or a pharmaceutically acceptable salt, an enantiomer, a diastereomer, a solvate, a hydrate, a polymorph or an isotopic variant thereof:

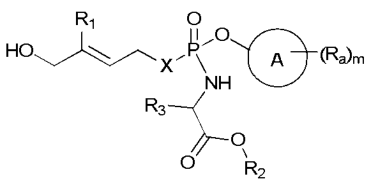

(I)

wherein,

ring A is $C_{6-10}$ aryl or 5- to 10-membered heteroaryl;
$R_a$ is selected from H, halogen, $C_{1-6}$ alkyl, $C_{1-6}$ haloalkyl, -OR", NR"R", $C_{2-6}$ alkenyl and $C_{2-6}$ alkynyl;
m=1, 2, 3, 4, or 5;
X is O or CRR';
$R_1$ is selected from H, F, Cl, CN and methyl, wherein the methyl is optionally substituted with 1 to 3 substituents independently selected from halogen, CN, OH and $NH_2$;
$R_2$ is selected from $C_{1-6}$ hydroxyalkyl, $C_{5-6}$ alkyl, $C_{5-6}$ haloalkyl, $C_{5-6}$ alkenyl, $C_{5-6}$ alkynyl, $C_{0-6}$ alkylene-$C_{3-12}$ cycloalkyl, $C_{0-6}$ alkylene-4- to 12-membered heterocyclyl and $C_{0-6}$ alkylene-5- to 10-membered heteroaryl, wherein the $C_{1-6}$ hydroxyalkyl, $C_{5-6}$ alkyl, $C_{5-6}$ haloalkyl, $C_{5-6}$ alkenyl, $C_{5-6}$ alkynyl, $C_{0-6}$ alkylene-$C_{3-12}$ cycloalkyl, $C_{0-6}$ alkylene-4- to 12-membered heterocyclyl and $C_{0-6}$ alkylene-5- to 10-membered heteroaryl are optionally substituted with one or more substituents selected from halogen, $C_{1-6}$ alkyl, $C_{1-6}$ haloalkyl, $C_{1-6}$ hydroxyalkyl, $C_{2-6}$ alkenyl, $C_{2-6}$ alkynyl, 3- to 10-membered cycloalkyl, 4- to 10-membered heterocycloalkyl, $C_{6-10}$ aryl, 5- to 10-membered heteroaryl, -C(O)-$C_{1-6}$ alkyl, -C(O)-$C_{1-6}$ hydroxyalkyl, -C(O)-$C_{1-6}$ haloalkyl, -C(O)-$C_{2-6}$ alkenyl, -C(O)-$C_{2-6}$ alkynyl, -C(O)-3- to 10-membered cycloalkyl, -C(O)-4-to 10-membered heterocycloalkyl, -C(O)-$C_{6-10}$ aryl, -C(O)-5- to 10-membered heteroaryl, -CN and oxo group;
$R_3$ is selected from $C_{1-6}$ alkyl, $C_{1-6}$ haloalkyl, $C_{2-6}$ alkenyl and $C_{2-6}$ alkynyl;
wherein,
R and R' are independently selected from H and halogen;
R" is independently selected from H, $C_{1-6}$ alkyl, $C_{1-6}$ haloalkyl, $C_{2-6}$ alkenyl, $C_{2-6}$ alkynyl, $C_{3-7}$ cycloalkyl, 3- to 7-membered heterocyclyl, $C_{6-10}$ aryl and 5- to 10-membered heteroaryl.

In one aspect, the present disclosure provides a compound of formula (I), or a pharmaceutically acceptable salt, an enantiomer, a diastereomer, a solvate, a hydrate, a polymorph or an isotopic variant thereof:

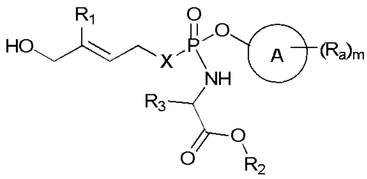
(I)

wherein,

ring A is $C_{6-10}$ aryl or 5- to 10-membered heteroaryl;
$R_a$ is selected from H, halogen, $C_{1-6}$ alkyl, $C_{1-6}$ haloalkyl, -OR", NR"R", $C_{2-6}$ alkenyl and $C_{2-6}$ alkynyl;
m=1, 2, 3, 4, or 5;
X is O or CRR';
$R_1$ is selected from H, F, Cl, CN and methyl, wherein the methyl is optionally substituted with 1 to 3 substituents independently selected from halogen, CN, OH and $NH_2$;
$R_2$ is selected from $C_{5-6}$ alkyl, $C_{5-6}$ haloalkyl, $C_{5-6}$ alkenyl, $C_{5-6}$ alkynyl, $C_{0-6}$ alkylene-$C_{3-7}$ cycloalkyl, $C_{0-6}$ alkylene-4- to 7-membered heterocyclyl, $C_{0-6}$ alkylene-$C_{6-10}$ aryl and $C_{0-6}$ alkylene-5- to 10-membered heteroaryl;
$R_3$ is selected from $C_{1-6}$ alkyl, $C_{1-6}$ haloalkyl, $C_{2-6}$ alkenyl and $C_{2-6}$ alkynyl;
wherein,
R and R' are independently selected from H and halogen;
R" is independently selected from H, $C_{1-6}$ alkyl, $C_{1-6}$ haloalkyl, $C_{2-6}$ alkenyl, $C_{2-6}$ alkynyl, $C_{3-7}$ cycloalkyl, 3- to 7-membered heterocyclyl, $C_{6-10}$ aryl and 5- to 10-membered heteroaryl.

In another aspect, the present disclosure provides a compound of formula (I), or a pharmaceutically acceptable salt, an enantiomer, a diastereomer, a solvate, a hydrate, a polymorph or an isotopic variant thereof:

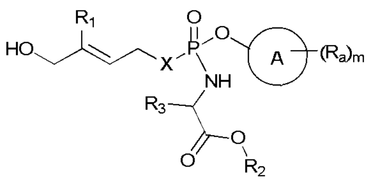
(I)

wherein,

ring A is $C_{6-10}$ aryl or 5- to 10-membered heteroaryl;
$R_a$ is selected from H, halogen, $C_{1-6}$ alkyl, $C_{1-6}$ haloalkyl, -OR", NR"R", $C_{2-6}$ alkenyl and $C_{2-6}$ alkynyl;
m=1, 2, 3, 4, or 5;
X is O or CRR';
$R_1$ is selected from H, F, Cl, CN and methyl, wherein the methyl is substituted with 1 to 3 substituents independently selected from halogen, CN, OH and $NH_2$;
$R_2$ is selected from $C_{1-6}$ alkyl, $C_{1-6}$ haloalkyl, $C_{2-6}$ alkenyl, $C_{2-6}$ alkynyl, $C_{0-6}$ alkylene-$C_{3-7}$ cycloalkyl, $C_{0-6}$ alkylene-3- to 7-membered heterocyclyl, $C_{0-6}$ alkylene-$C_{6-10}$ aryl and $C_{0-6}$ alkylene-5- to 10-membered heteroaryl;
$R_3$ is selected from $C_{1-6}$ alkyl, $C_{1-6}$ haloalkyl, $C_{2-6}$ alkenyl and $C_{2-6}$ alkynyl;
wherein,
R and R' are independently selected from H and halogen;
R" is independently selected from H, $C_{1-6}$ alkyl, $C_{1-6}$ haloalkyl, $C_{2-6}$ alkenyl, $C_{2-6}$ alkynyl, $C_{3-7}$ cycloalkyl, 3- to 7-membered heterocyclyl, $C_{6-10}$ aryl and 5- to 10-membered heteroaryl.

In another aspect, the present disclosure provides a pharmaceutical composition comprising a compound of the present disclosure or a pharmaceutically acceptable salt, an enantiomer, a diastereomer, a solvate, a hydrate, a polymorph or an isotopic variant thereof, and a pharmaceutically acceptable excipient.

In another aspect, the present disclosure provides a pharmaceutical composition comprising a compound of the present disclosure or a pharmaceutically acceptable salt, an enantiomer, a diastereomer, a solvate, a hydrate, a polymorph or an isotopic variant thereof, and a pharmaceutically acceptable excipient, further comprising an additional therapeutic agent.

In another aspect, the present disclosure provides a kit comprising a compound of the present disclosure or a pharmaceutically acceptable salt, an enantiomer, a diastereomer, a solvate, a hydrate, a polymorph or an isotopic variant thereof, and an additional therapeutic agent, and a pharmaceutically acceptable carrier, adjuvant or vehicle.

In another aspect, the present disclosure provides use of a compound of the present disclosure or a pharmaceutically acceptable salt, an enantiomer, a diastereomer, a solvate, a hydrate, a polymorph or an isotopic variant thereof in the manufacture of a medicament for treating and/or preventing a proliferative disease.

In another aspect, the present disclosure provides a method for treating and/or preventing a proliferative disease in a subject, comprising administering to the subject a compound of the present disclosure or a pharmaceutically acceptable salt, an enantiomer, a diastereomer, a solvate, a hydrate, a polymorph or an isotopic variant thereof, or a composition of the present disclosure.

In another aspect, the present disclosure provides a compound of the present disclosure or a pharmaceutically acceptable salt, an enantiomer, a diastereomer, a solvate, a hydrate, a polymorph or an isotopic variant thereof, or a composition of the present disclosure for use in treating and/or preventing a proliferative disease.

In a specific embodiment, the proliferative diseases described herein include, but are not limited to, cancer, cardiovascular disorders, infectious diseases, chronic inflammatory diseases, autoimmune disorders, and other cell proliferation disorders. More specifically, the cancer includes, but is not limited to, solid tumors and hematological malignancies, such as breast cancer, neuroblastoma, malignant rhabdomyomas, well-differentiated and dedifferentiated liposarcoma, glioma, lung cancer, colorectal cancer, gastric cancer, gastrointestinal stromal tumor (GIST), hepatocellular carcinoma, prostate tumor, sarcoma, ovarian cancer, cervical cancer, pancreatic cancer, melanoma, thyroid cancer, bile duct cancer, endometrial cancer, renal cancer, mesothelioma, lymphoma, leukemia, non-Hodgkin's lymphoma, mantle cell lymphoma, anaplastic large cell lymphoma, acute myeloid leukemia (AML), multiple myeloma.

In another aspect, the present disclosure provides use of a compound of the present disclosure or a pharmaceutically acceptable salt, an enantiomer, a diastereomer, a solvate, a hydrate, a polymorph or an isotopic variant thereof, or a composition of the present disclosure in the manufacture of a medicament for promoting binding of butyrophilin 3A1/2A1.

In another aspect, the present disclosure provides a method for promoting binding of butyrophilin 3A1/2A1 in a subject, comprising administering to the subject a compound of the present disclosure or a pharmaceutically acceptable salt, an enantiomer, a diastereomer, a solvate, a hydrate, a polymorph or an isotopic variant thereof, or a composition of the present disclosure.

In another aspect, the present disclosure provides a compound of the present disclosure or a pharmaceutically acceptable salt, an enantiomer, a diastereomer, a solvate, a hydrate, a polymorph or an isotopic variant thereof, or a composition of the present disclosure for use in promoting binding of butyrophilin 3A1/2A1.

Other objects and advantages of the present disclosure will be apparent to those skilled in the art from the following detailed description, examples and claims.

## BRIEF DESCRIPTION OF DRAWINGS

**Fig. 1.** The binding mode of the small molecule compound (**compound 7**) with BTN3A1 in vivo, showing that the R1 group of the general formula molecule is substantially exposed to the solvent region, has no effect on the binding of the small molecule compound with BTN3A1, and may be derivatized in various ways.

**Fig. 2.** The binding mode of the small molecule compound (**compound 7**) with BTN3A1 and BTN2A1 at the same time in vivo, showing that the R1 structure of the general formula molecule is limited by the specific cavity, and only R1 with a specific space size can bind to BTN3A1 and BTN2A1 at the same time to produce corresponding biological effects.

Figs. 1 and 2 show that the requirements for small molecule ligands designed based on the model of BTN3A1 (Fig. 1) are entirely different from the requirements for small molecule ligands designed based on the model of BTNA1-BTN2A1 (Fig. 2). Only the model shown in Fig. 2, that is, the small molecule binds to the cavity formed by BTN3A1 and BTN2A1 at the same time, can it exert its biological function.

## DETAILED DESCRIPTION

Definition

Chemical definitions

Definitions of specific functional groups and chemical terms are described in more detail below.

When a range of values is listed, it is intended to encompass each value and sub-range within the range. For example, "$C_{1-6}$ alkyl" is intended to include $C_1$, $C_2$, $C_3$, $C_4$, $C_5$, $C_6$, $C_{1-6}$, $C_{1-5}$, $C_{1-4}$, $C_{1-3}$, $C_{1-2}$, $C_{2-6}$, $C_{2-5}$, $C_{2-4}$, $C_{2-3}$, $C_{3-6}$, $C_{3-5}$, $C_{3-4}$, $C_{4-6}$, $C_{4-5}$ and $C_{5-6}$ alkyl.

It should be understood that when described herein, any of the groups defined below may be substituted with a number of substituents and that the corresponding definitions are within their scope listed below, including substituted groups. Unless otherwise stated, the term "substituted" is as defined below.

"$C_{1-10}$ alkyl" refers to a radical of a straight or branched, saturated hydrocarbon group having 1 to 10 carbon atoms. In some embodiments, $C_{1-8}$ alkyl is alternative. In some embodiments, $C_{1-6}$ alkyl, which is also referred to as "lower alkyl", is alternative. In some embodiments, $C_{1-4}$ alkyl is yet alternative. Examples of the alkyl group include, but are not limited to, methyl ($C_1$), ethyl ($C_2$), propyl ($C_3$), butyl ($C_4$), pentyl ($C_5$), hexyl ($C_6$), heptyl ($C_7$), octyl ($C_8$), nonyl ($C_9$) and decyl ($C_{10}$). The alkyl group also includes any isomers of the above groups, for example, propyl ($C_3$) includes n-propyl ($C_3$) and isopropyl ($C_3$), butyl ($C_4$) includes n-butyl ($C_4$), tert-butyl (C4), sec-butyl ($C_4$) and isobutyl (C4), pentyl ($C_5$) includes n-pentyl ($C_5$), 3-pentyl ($C_5$), pentyl ($C_5$), neopentyl ($C_5$), 3-methyl-2-butyl ($C_5$) and tert-pentyl ($C_5$), and the like. Unless otherwise specified, each of the alkyl groups is independently optionally substituted, i.e., unsubstituted ("unsubstituted alkyl") or substituted with one or more substituents ("substituted alkyl"); for example, 1 to 5 substituents, 1 to 3 substituents, or 1 substituent. In some embodiments, the alkyl group is an unsubstituted $C_{1-6}$ alkyl group (e.g., $-CH_3$). In some embodiments, the alkyl group is a substituted $C_{1-6}$ alkyl group.

"$C_{2-10}$ alkenyl" refers to a radical of a straight or branched hydrocarbon group having 2 to 10 carbon atoms and one or more carbon-carbon double bonds (e.g., 1, 2 or 3 carbon-carbon double bonds). In some embodiments, $C_{2-6}$ alkenyl is alternative. In some embodiments, $C_{2-4}$ alkenyl is alternative. One or more carbon-carbon double bonds may be inside (e.g., in 2-butenyl) or at the terminal (e.g., in 1-butenyl). Examples of the alkenyl group include, but are not limited to, vinyl (C2), propenyl (C3), butenyl (C4), butadienyl ($C_4$), pentenyl ($C_5$), pentadienyl ($C_5$), hexenyl (C6), and the like. The alkenyl group also includes any isomers of the above groups, for example, propenyl ($C_3$) includes 1-propenyl ($C_3$), 2-propenyl ($C_3$) and 1-propen-2-yl ($C_3$), butenyl ($C_4$) includes 1-butenyl ($C_4$) and 2-butenyl ($C_4$), $C_{10}$ alkenyl includes, for example, 1-decenyl ($C_{10}$), 2-decenyl ($C_{10}$), 3-decenyl ($C_{10}$), 4-decenyl ($C_{10}$), 1,3-decenediyl ($C_{10}$), 1,4-decenediyl ($C_{10}$), 1,5-decenediyl ($C_{10}$), 3,7-dimethyloct-2,6-dien-1-yl ($C_{10}$), and the like. Unless otherwise specified, each of the alkenyl groups is independently optionally substituted, i.e., unsubstituted ("unsubstituted alkenyl") or substituted with one or more substituents ("substituted alkenyl"); for example, 1 to 5 substituents, 1 to 3 substituents, or 1 substituent. In some embodiments, the alkenyl group is an unsubstituted $C_{2-6}$ alkenyl. In some embodiments, the alkenyl group is a substituted $C_{2-6}$ alkenyl.

"$C_{2-10}$ alkynyl" refers to a radical of a straight or branched hydrocarbon group having 2 to 10 carbon atoms, one or more carbon-carbon triple bonds (e.g., 1, 2, or 3 carbon-carbon triple bonds), and optionally one or more carbon-carbon double bonds (e.g., 1, 2, or 3 carbon-carbon double bonds). In some embodiments, $C_{2-6}$ alkynyl is alternative. In some embodiments, $C_{2-4}$ alkynyl is alternative. In some embodiments, the alkynyl group does not contain any double bonds. The one or more carbon-carbon triple bonds may be inside (e.g., in 2-butynyl) or at the terminal (e.g., in 1-butynyl). Examples of

the alkynyl group include, but are not limited to, ethynyl ($C_2$), 1-propynyl ($C_3$), 2-propynyl ($C_3$), 1-butynyl ($C_4$), 2-butynyl ($C_4$), pentynyl ($C_5$), 3-methylbut-1-ynyl ($C_5$), hexynyl ($C_6$), and the like. Unless otherwise specified, each of the alkynyl groups is independently optionally substituted, i.e., unsubstituted ("unsubstituted alkynyl") or substituted with one or more substituents ("substituted alkynyl"); for example, 1 to 5 substituents, 1 to 3 substituents, or 1 substituent. In some embodiments, the alkynyl group is an unsubstituted $C_{2-6}$ alkynyl. In some embodiments, the alkynyl group is a substituted $C_{2-6}$ alkynyl.

"Halogen" refers to fluorine (F), chlorine (Cl), bromine (Br), and iodine (I). In some embodiments, the halogen is -F, -Cl, or -Br. In some embodiments, the halogen is -F or -Cl.

Thus, "$C_{1-10}$ haloalkyl" refers to the above "$C_{1-10}$ alkyl", which is substituted with one or more halogens. In some embodiments, $C_{1-6}$ haloalkyl or $C_{1-4}$ haloalkyl is alternative, and still alternatively $C_{1-2}$ haloalkyl. Exemplary haloalkyl groups include, but are not limited to: $-CF_3$, $-CH_2F$, $-CHF_2$, $-CHFCH_2F$, $-CH_2CHF_2$, $-CF_2CF_3$, $-CCl_3$, $-CH_2Cl$, $-CHCl_2$, 2,2,2-trifluoro-1,1-dimethyl-ethyl, and the like.

"$C_{3-12}$ cycloalkyl" refers to a radical of a non-aromatic cyclic hydrocarbon group having 3 to 12 ring carbon atoms and zero heteroatoms, including fused ring, bridged ring, spiro ring, etc. In some embodiments, $C_{3-7}$ cycloalkyl is alternative, $C_{3-6}$ cycloalkyl is alternative, and still alternatively $C_{5-6}$ cycloalkyl. Exemplary cycloalkyl groups include, but are not limited to, cyclopropyl ($C_3$), cyclopropenyl ($C_3$), cyclobutyl ($C_4$), cyclobutenyl ($C_4$), cyclopentyl ($C_5$), cyclopentenyl ($C_5$), cyclohexyl ($C_6$), cyclohexenyl ($C_6$), cyclohexadienyl ($C_6$), cycloheptyl ($C_7$), cycloheptenyl ($C_7$), cycloheptadienyl ($C_7$), cycloheptatrienyl ($C_7$), cyclooctyl (C8), cyclooctenyl (C8), cyclononyl (C9), cyclononenyl (C9), cyclodecyl (C10), cyclodecenyl (C10), cycloundecyl (C11), cycloundecenyl (C11), cyclododecyl (C12), cyclododecenyl (C12), cyclotridecyl (C13), cyclotridecenyl (C13), adamantyl, and the like.

"3- to 12-membered heterocyclyl" refers to a 3- to 12-membered non-aromatic ring system having ring carbon atoms and 1 to 4 ring heteroatoms, including fused ring, bridged ring, spiro ring, etc. In some embodiments, 3- to 7-membered heterocyclyl, 3- to 6-membered heterocyclyl are alternative, in which the 3-to 6-membered heterocyclyl is a radical of 3- to 6-membered non-aromatic ring systems having ring carbon atoms and 1 to 3 ring heteroatoms. In some embodiments, 4- to 7-membered heterocyclyl is alternative, which is a radical of 4- to 7-membered non-aromatic ring system having ring carbon atoms and 1 to 3 ring heteroatoms. In some embodiments, 5- to 6-membered heterocyclyl is alternative, which is a radical of 5- to 6-membered non-aromatic ring system having ring carbon atoms and 1 to 3 ring heteroatoms. Exemplary 3-membered heterocyclyl groups containing one heteroatom include, but are not limited to, aziridinyl, oxiranyl and thiorenyl. Exemplary 4-membered heterocyclyl groups containing one heteroatom include, but are not limited to, azetidinyl, oxetanyl and thietanyl. Exemplary 5-membered heterocyclyl groups containing one heteroatom include, but are not limited to, tetrahydrofuranyl, dihydrofuranyl, tetrahydrothiophenyl, dihydrothienyl, pyrrolidinyl, dihydropyrrolyl and pyrrolyl-2,5-dione. Exemplary 5-membered heterocyclyl groups containing two heteroatoms include, but are not limited to, dioxolanyl, oxasulfuranyl, disulfuranyl, and oxazolidin-2-one. Exemplary 5-membered heterocyclyl groups containing three heteroatoms include, but are not limited to, triazolinyl, oxadiazolinyl, and thiadiazolinyl. Exemplary 6-membered heterocyclyl groups containing one heteroatom include, but are not limited to, piperidyl, tetrahydropyranyl, dihydropyridyl and thianyl. Exemplary 6-membered heterocyclyl groups containing two heteroatoms include, but are not limited to, piperazinyl, morpholinyl, dithianyl and dioxanyl. Exemplary 6-membered heterocyclyl groups containing three heteroatoms include, but are not limited to, triazinanyl. Exemplary 7-membered heterocycly groups containing one heteroatom include, but are not limited to, azepanyl, oxepanyl and thiepanyl. In some embodiments, the 3- to 12-membered heterocyclyl of the present disclosure includes:

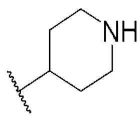 ,

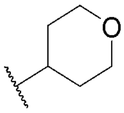 , 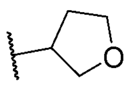 , 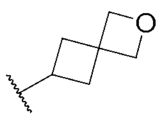 , 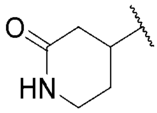 , 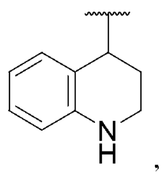 , 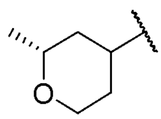 , 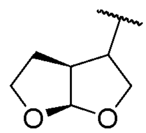 ,

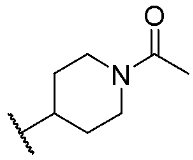, 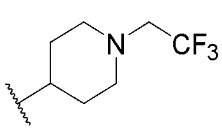, 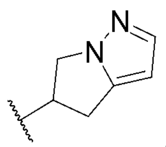, 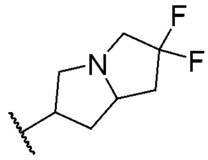, 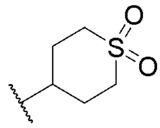, 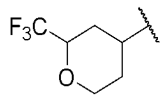,

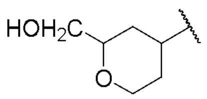 and 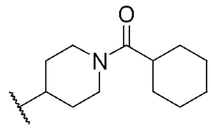.

"$C_{6-10}$ aryl" refers to a radical of a monocyclic or bicyclic 4n+2 aromatic ring system (e.g., having 6 or 10 shared $\pi$ electrons in a cyclic array) having 6-10 ring carbon atoms and zero heteroatoms provided in the aromatic ring system. In some embodiments, the aryl group has six ring carbon atoms ("$C_6$ aryl"; e.g., phenyl). In some embodiments, the aryl group has ten ring carbon atoms ("$C_{10}$ aryl"; e.g., naphthyl, e.g., 1-naphthyl and 2-naphthyl). Unless otherwise specified, each of the aryl groups is independently optionally substituted, i.e., unsubstituted ("unsubstituted aryl") or substituted with one or more substituents ("substituted aryl").

"5- to 10-membered heteroaryl" refers to a radical of 5- to 10-membered monocyclic or bicyclic 4n+2 aromatic ring system (e.g., having 6 or 10 shared $\pi$ electrons in a cyclic array) having ring carbon atoms and 1 to 4 ring heteroatoms, wherein each heteroatom is independently selected from nitrogen, oxygen and sulfur. In some embodiments, a 5- to 6-membered heteroaryl is alternative, which is a radical of a 5- to 6-membered monocyclic 4n+2 aromatic ring system (e.g., having 6 shared $\pi$ electrons in a cyclic array) having ring carbon atoms and 1 to 4 ring heteroatoms, wherein each heteroatom is independently selected from nitrogen, oxygen and sulfur; in some embodiments, a 5-membered heteroaryl is alternative, which is a radical of a 5-membered monocyclic 4n+2 aromatic ring system (e.g., having 6 shared $\pi$ electrons in a cyclic array) having ring carbon atoms and 1 to 4 ring heteroatoms, wherein each heteroatom is independently selected from nitrogen, oxygen and sulfur. In some embodiments, a 6-membered heteroaryl is alternative, which is a radical of a 6-membered monocyclic 4n+2 aromatic ring system (e.g., having 6 shared $\pi$ electrons in a cyclic array) having ring carbon atoms and 1 to 4 ring heteroatoms, wherein each heteroatom is independently selected from nitrogen, oxygen, and sulfur. Unless otherwise specified, each of the heteroaryl groups is independently optionally substituted, i.e., unsubstituted ("unsubstituted heteroaryl") or substituted with one or more substituents ("substituted heteroaryl"). Exemplary 5-membered heteroaryl groups containing one heteroatom include, but are not limited to, pyrrolyl, furyl and thienyl. Exemplary 5-membered heteroaryl groups containing two heteroatoms include, but are not limited to, imidazolyl, pyrazolyl, oxazolyl, isoxazolyl, thiazolyl, and isothiazolyl. Exemplary 5-membered heteroaryl groups containing three heteroatoms include, but are not limited to, triazolyl, oxadiazolyl, and thiadiazolyl. Exemplary 5-membered heteroaryl groups containing four heteroatoms include, but are not limited to, tetrazolyl. Exemplary 6-membered heteroaryl groups containing one heteroatom include, but are not limited to, pyridyl. Exemplary 6-membered heteroaryl groups containing two heteroatoms include, but are not limited to, pyridazinyl, pyrimidinyl, and pyrazinyl. Exemplary 6-membered heteroaryl groups containing three or four heteroatoms include, but are not limited to, triazinyl and tetrazinyl, respectively.

"Heteroatom" refers to non-metal atoms other than carbon atoms; oxygen, nitrogen, phosphorus, sulfur, silicon and boron atoms are alternative, and still alternatively oxygen, nitrogen, phosphorus and sulfur atoms.

"-$C_{1-6}$ alkylene-" refers to a divalent group of the" $C_{1-6}$ alkyl" defined above. Specifically, it refers to a divalent group formed by removing another hydrogen of the $C_{1-6}$ alkyl, and can be a substituted or unsubstituted alkylene group. In some embodiments, a $C_{1-4}$ alkylene is yet alternative. The unsubstituted alkylene groups include, but are not limited to, methylene ($-CH_2-$), ethylene ($-CH_2CH_2-$), propylene ($-CH_2CH_2CH_2-$), butylene ($-CH_2CH_2CH_2CH_2-$), pentylene ($-CH_2CH_2CH_2CH_2CH_2-$), hexylene ($-CH_2CH_2CH_2CH_2CH_2CH_2-$), etc. Examples of substituted alkylene group, such as those substituted with one or more alkyl (methyl) groups, include, but are not limited to, substituted methylene ($-CH(CH_3)-$, $-C(CH_3)_2-$), substituted ethylene ($-CH(CH_3)CH_2-$, $-CH_2CH(CH_3)-$, $-C(CH_3)_2CH_2-$, $-CH_2C(CH_3)_2-$), substituted propylene ($-CH(CH_3)CH_2CH_2-$, $-CH_2CH(CH_3)CH_2-$, $-CH_2CH_2CH(CH_3)-$, $-C(CH_3)_2CH_2CH_2-$, $-CH_2C(CH_3)_2CH_2-$, $-CH_2CH_2C(CH_3)_2-$), etc.

The "$C_{0-6}$ alkylene group" refers to a chemical bond and the "$C_{1-6}$ alkylene group" as defined above.

When a chemical bond "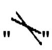" is used at a double bond position in a compound, it means that the cis-isomer and the trans-isomer of the compound are co-existed in any ratio, that is, it can be a cis-isomer, a trans- isomer or a mixture thereof.

Alkyl, alkenyl, alkynyl, aryl and heteroaryl, etc., as defined herein, are optionally substituted groups, whether or not there is a preceding "optionally substituted". In general, the term "substituted", whether or not there is a preceding term "optionally", refers to at least one hydrogen present on a group (e.g., on a carbon or nitrogen atom) being replaced by a permissible substituent, for example, a substituent that produces a stable compound upon substitution, for example, a

compound that does not spontaneously undergo a transformation (e.g., by rearrangement, cyclization, elimination or other reactions). Unless otherwise specified, a "substituted" group has a substituent at one or more substitutable positions of the group, and when more than one position in any given structure is substituted, the substituent at each position is the same or different. The term "substituted" includes substitutions with all permissible substituents of an organic compound (any substituent described herein resulting in the formation of a stable compound). For the present disclosure, heteroatoms such as nitrogen may have hydrogen substituents and/or any suitable substituents described herein that satisfy the valence of the heteroatoms and result in the formation of a stable part.

Exemplary substituents on carbon atoms include, but are not limited to, halogen, -CN, $-NO_2$, $-N_3$, $-SO_2H$, $-SO_3H$, -OH, $-OR^{aa}$, $-ON(R^{bb})_2$, $-N(R^{bb})_2$, $-N(R^{bb})_3^+X^-$, $-N(OR^{cc})R^{bb}$, -SH, $-SR^{aa}$, $-SSR^{cc}$, $-C(=O)R^{aa}$, $-CO_2H$, -CHO, $-C(OR^{cc})_2$, $-CO_2R^{aa}$, $-OC(=O)R^{aa}$, $-OCO_2R^{aa}$, $-C(=O)N(R^{bb})_2$, $-OC(=O)N(R^{bb})_2$, $-NR^{bb}C(=O)R^{aa}$, $-NR^{bb}CO_2R^{aa}$, $-NR^{bb}C(=O)N(R^{bb})_2$, $-C(=NR^{bb})R^{aa}$, $-C(=NR^{bb})OR^{aa}$, $-OC(=NR^{bb})R^{aa}$, $-OC(=NR^{bb})OR^{aa}$, $-C(=NR^{bb})N(R^{bb})_2$, $-OC(=NR^{bb})N(R^{bb})_2$, $-NR^{bb}C(=NR^{bb})N(R^{bb})_2$, $-C(=O)NR^{bb}SO_2R^{aa}$, $-NR^{bb}SO_2R^{aa}$, $-SO_2N(R^{bb})_2$, $-SO_2R^{aa}$, $-SO_2OR^{aa}$, $-OSO_2R^{aa}$, $-S(=O)R^{aa}$, $-OS(=O)R^{aa}$, $-Si(R^{aa})_3$, $-OSi(R^{aa})_3$, $-C(=S)N(R^{bb})_2$, $-C(=O)SR^{aa}$, $-C(=S)SR^{aa}$, $-SC(=S)SR^{aa}$, $-SC(=O)SR^{aa}$, $-OC(=O)SR^{aa}$, $-SC(=O)OR^{aa}$, $-SC(=O)R^{aa}$, $-P(=O)_2R^{aa}$, $-OP(=O)_2R^{aa}$, $-P(=O)(R^{aa})_2$, $-OP(=O)(R^{aa})_2$, $-OP(=O)(OR^{cc})_2$, $-P(=O)_2N(R^{bb})_2$, $-OP(=O)_2N(R^{bb})_2$, $-P(=O)(NR^{bb})_2$, $-OP(=O)(NR^{bb})_2$, $-NR^{bb}P(=O)(OR^{cc})_2$, $-NR^{bb}P(=O)(NR^{bb})_2$, $-P(R^{cc})_2$, $-P(R^{cc})_3$, $-OP(R^{cc})_2$, $-OP(R^{cc})_3$, $-B(R^{aa})_2$, $-B(OR^{cc})_2$, $-BR^{aa}(OR^{cc})$, alkyl, haloalkyl, alkenyl, alkynyl, carbocyclyl, heterocyclyl, aryl and heteroaryl, wherein each of the alkyl, alkenyl, alkynyl, carbocyclyl, heterocyclyl, aryl and heteroaryl is independently substituted with 0, 1, 2, 3, 4 or 5 $R^{dd}$ groups;

or two geminal hydrogens on a carbon atom are replaced with =O, =S, $=NN(R^{bb})_2$, $=NNR^{bb}C(=O)R^{aa}$, $=NNR^{bb}C(=O)OR^{aa}$, $=NNR^{bb}S(=O)_2R^{aa}$, $=NR^{bb}$ or $=NOR^{cc}$ groups;

each of the $R^{aa}$ is independently selected from alkyl, haloalkyl, alkenyl, alkynyl, carbocyclyl, heterocyclyl, aryl and heteroaryl, or two of the $R^{aa}$ groups are combined to form a heterocyclyl or heteroaryl ring, wherein each of the alkyl, alkenyl, alkynyl, carbocyclyl, heterocyclyl, aryl and heteroaryl is independently substituted with 0, 1, 2, 3, 4 or 5 $R^{dd}$ groups;

each of the $R^{bb}$ is independently selected from hydrogen, -OH, $-OR^{aa}$, $-N(R^{cc})_2$, -CN, $-C(=O)R^{aa}$, $-C(=O)N(R^{cc})_2$, $-CO_2R^{aa}$, $-SO_2R^{aa}$, $-C(=NR^{cc})OR^{aa}$, $-C(=NR^{cc})N(R^{cc})_2$, $-SO_2N(R^{cc})_2$, $-SO_2R^{cc}$, $-SO_2OR^{cc}$, $-SOR^{aa}$, $-C(=S)N(R^{cc})_2$, $-C(=O)SR^{cc}$, $-C(=S)SR^{cc}$, $-P(=O)_2R^{aa}$, $-P(=O)(R^{aa})_2$, $-P(=O)_2N(R^{cc})_2$, $-P(=O)(NR^{cc})_2$, alkyl, haloalkyl, alkenyl, alkynyl, carbocyclyl, heterocyclyl, aryl and heteroaryl, or two $R^{bb}$ groups are combined to form a heterocyclyl or a heteroaryl ring, wherein each of the alkyl, alkenyl, alkynyl, carbocyclyl, heterocyclyl, aryl and heteroaryl is independently substituted with 0, 1, 2, 3, 4 or 5 $R^{dd}$ groups;

each of the $R^{cc}$ is independently selected from hydrogen, alkyl, haloalkyl, alkenyl, alkynyl, carbocyclyl, heterocyclyl, aryl and heteroaryl, or two $R^{cc}$ groups are combined to form a heterocyclyl or a heteroaryl ring, wherein each of the alkyl, alkenyl, alkynyl, carbocyclyl, heterocyclyl, aryl and heteroaryl is independently substituted with 0, 1, 2, 3, 4 or 5 $R^{dd}$ groups;

each of the $R^{dd}$ is independently selected from halogen, -CN, $-NO_2$, $-N_3$, $-SO_2H$, $-SO_3H$, -OH, $-OR^{ee}$, $-ON(R^{ff})_2$, $-N(R^{ff})_2$, $-N(R^{ff})_3^+X^-$, $-N(OR^{ee})R^{ff}$, -SH, $-SR^{ee}$, $-SSR^{ee}$, $-C(=O)R^{ee}$, $-CO_2H$, $-CO_2R^{ee}$, $-OC(=O)R^{ee}$, $-OCO_2R^{ee}$, $-C(=O)N(R^{ff})_2$, $-OC(=O)N(R^{ff})_2$, $-NR^{ff}C(=O)R^{ee}$, $-NR^{ff}CO_2R^{ee}$, $-NR^{ff}C(=O)N(R^{ff})_2$, $-C(=NR^{ff})OR^{ee}$, $-OC(=NR^{ff})R^{ee}$, $-OC(=NR^{ff})OR^{ee}$, $-C(=NR^{ff})N(R^{ff})_2$, $-OC(=NR^{ff})N(R^{ff})_2$, $-NR^{ff}C(=NR^{ff})N(R^{ff})_2$, $-NR^{ff}SO_2R^{ee}$, $-SO_2N(R^{ff})_2$, $-SO_2R^{ee}$, $-SO_2OR^{ee}$, $-OSO_2R^{ee}$, $-S(=O)R^{ee}$, $-Si(R^{ee})_3$, $-OSi(R^{ee})_3$, $-C(=S)N(R^{ff})_2$, $-C(=O)SR^{ee}$, $-C(=S)SR^{ee}$, $-SC(=S)SR^{ee}$, $-P(=O)_2R^{ee}$, $-P(=O)(R^{ee})_2$, $-OP(=O)(R^{ee})_2$, $-OP(=O)(OR^{ee})_2$, alkyl, haloalkyl, alkenyl, alkynyl, carbocyclyl, heterocyclyl, aryl, and heteroaryl, wherein, each of the alkyl, alkenyl, alkynyl, carbocyclyl, heterocyclyl, aryl and heteroaryl is independently substituted with 0, 1, 2, 3, 4 or 5 $R^{gg}$ groups, or two geminal $R^{dd}$ substituents can be combined to form=O or =S;

each of the $R^{ee}$ is independently selected from alkyl, haloalkyl, alkenyl, alkynyl, carbocyclyl, aryl, heterocyclyl, and heteroaryl, wherein, each of the alkyl, alkenyl, alkynyl, carbocyclyl, heterocyclyl, aryl and heteroaryl is independently substituted with 0, 1, 2, 3, 4 or 5 $R^{gg}$ groups;

each of the $R^{ff}$ is independently selected from hydrogen, alkyl, haloalkyl, alkenyl, alkynyl, carbocyclyl, heterocyclyl, aryl and heteroaryl, or two $R^{ff}$ groups are combined to form a heterocyclyl or a heteroaryl ring, wherein each of the alkyl, alkenyl, alkynyl, carbocyclyl, heterocyclyl, aryl and heteroaryl is independently substituted with 0, 1, 2, 3, 4 or 5 $R^{gg}$ groups;

each of the $R^{gg}$ is independently halogen, -CN, $-NO_2$, $-N_3$, $-SO_2H$, $-SO_3H$, -OH, $-OC_{1-6}$ alkyl, $-ON(C_{1-6}$ alkyl$)_2$, $-N(C_{1-6}$ alkyl$)_2$, $-N(C_{1-6}$ alkyl$)_3^+X^-$, $-NH(C_{1-6}$ alkyl$)_2^+X^-$, $-NH_2(C_{1-6}$ alkyl$)^+X^-$, $-NH_3^+X^-$, $-N(OC_{1-6}$ alkyl)($C_{1-6}$ alkyl), $-N(OH)(C_{1-6}$ alkyl), -NH(OH), -SH, $-SC_{1-6}$ alkyl, $-SS(C_{1-6}$ alkyl), $-C(=O)(C_{1-6}$ alkyl), $-CO_2H$, $-CO_2(C_{1-6}$ alkyl), $-OC(=O)(C_{1-6}$ alkyl), $-OCO_2(C_{1-6}$ alkyl), $-C(=O)NH_2$, $-C(=O)N(C_{1-6}$ alkyl$)_2$, $-OC(=O)NH(C_{1-6}$ alkyl), $-NHC(=O)(C_{1-6}$ alkyl), $-N(C_{1-6}$ alkyl)$C(=O)(C_{1-6}$ alkyl), $-NHCO_2(C_{1-6}$ alkyl), $-NHC(=O)N(C_{1-6}$ alkyl$)_2$, $-NHC(=O)NH(C_{1-6}$ alkyl), $-NHC(=O)NH_2$, $-C(=NH)O(C_{1-6}$ alkyl), $-OC(=NH)(C_{1-6}$ alkyl), $-OC(=NH)OC_{1-6}$ alkyl, $-C(=NH)N(C_{1-6}$ alkyl$)_2$, $-C(=NH)NH(C_{1-6}$ alkyl), $-C(=NH)NH_2$, $-OC(=NH)N(C_{1-6}$ alkyl$)_2$, $-OC(NH)NH(C_{1-6}$ alkyl), $-OC(NH)NH_2$, $-NHC(NH)N(C_{1-6}$ alkyl$)_2$, $-NHC(=NH)NH_2$,

$-NHSO_2(C_{1-6}$ alkyl), $-SO_2N(C_{1-6}$ alkyl$)_2$, $-SO_2NH(C_{1-6}$ alkyl), $-SO_2NH_2$, $-SO_2C_{1-6}$ alkyl, $-SO_2OC_{1-6}$ alkyl, $-OSO_2C_{1-6}$ alkyl, $-SOC_{1-6}$ alkyl, $-Si(C_{1-6}$ alkyl$)_3$, $-OSi(C_{1-6}$ alkyl$)_3$, $-C(=S)N(C_{1-6}$ alkyl$)_2$, $C(=S)NH(C_{1-6}$ alkyl), $C(=S)NH_2$, -C(=O) $S(C_{1-6}$ alkyl), $-C(=S)SC_{1-6}$ alkyl, $-SC(=S)SC_{1-6}$ alkyl, $-P(=O)_2(C_{1-6}$ alkyl), $-P(=O)(C_{1-6}$ alkyl$)_2$, $-OP(=O)(C_{1-6}$ alkyl$)_2$, $-OP(=O)(OC_{1-6}$ alkyl$)_2$, $C_{1-6}$ alkyl, $C_{1-6}$ perhaloalkyl, $C_{2-6}$ alkenyl, $C_{2-6}$ alkynyl, $C_{3-10}$ carbocyclyl, $C_{6-10}$ aryl, 3- to 10-membered heterocyclyl, 5- to 10-membered heteroaryl; or two geminal $R^{gg}$ substituents may combine to form =O or =S; wherein, $X^-$ is a counter-ion.

Exemplary substituents on nitrogen atoms include, but are not limited to, hydrogen, -OH, $-OR^{aa}$, $-N(R^{cc})_2$, -CN, $-C(=O)R^{aa}$, $-C(=O)N(R^{cc})_2$, $-CO_2R^{aa}$, $-SO_2R^{aa}$, $-C(=NR^{bb})R^{aa}$, $-C(=NR^{cc})OR^{aa}$, $-C(=NR^{cc})N(R^{cc})_2$, $-SO_2N(R^{cc})_2$, $-SO_2R^{cc}$, $-SO_2OR^{cc}$, $-SOR^{aa}$, $-C(=S)N(R^{cc})_2$, $-C(=O)SR^{cc}$, $-C(=S)SR^{cc}$, $-P(=O)_2R^{aa}$, $-P(=O)(R^{aa})_2$, $-P(=O)_2N(R^{cc})_2$, $-P(=O)(NR^{cc})_2$, alkyl, haloalkyl, alkenyl, alkynyl, carbocyclyl, heterocyclyl, aryl and heteroaryl, or two $R^{cc}$ groups attached to a nitrogen atom combine to form a heterocyclyl or a heteroaryl ring, wherein each of the alkyl, alkenyl, alkynyl, carbocyclyl, heterocyclyl, aryl and heteroaryl is independently substituted with 0, 1, 2, 3, 4 or 5 $R^{dd}$ groups, and wherein $R^{aa}$, $R^{bb}$, $R^{cc}$ and $R^{dd}$ are as described herein.

When a chemical structural formula of a compound contains chiral atoms, such as chiral carbon atoms and chiral phosphorus atoms, the present disclosure covers all isomers related to the chiral atoms, including R isomer, S isomer, (+) isomer, (-) isomer, mixtures and racemates thereof. At the same time, when linked to a chiral atom by a chemical bond "-" rather than a wedge bond, it only represents the linking relationship of 2 atoms and represents that the compound does not specify a particular configuration, i.e. all isomers, mixtures or racemates thereof may be included, and should not be construed as representing the racemates only.

When a chemical structural formula of a compound contains a double bond, it only represents that two atoms are connected by the double bond and that the compound has no specific configuration, that is, (E) isomers, (Z) isomers and mixtures thereof may be included.

Other definitions

The term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. For example, Berge et al, describes pharmaceutically acceptable salts in detail in J. Pharmaceutical Sciences (1977) 66: 1-19. Pharmaceutically acceptable salts of the compounds of the present disclosure include those derived from suitable inorganic and organic acids and bases. Examples of pharmaceutically acceptable, nontoxic acid addition salts are salts of an amino group formed with inorganic acids such as hydrochloric acid, hydrobromic acid, phosphoric acid, sulfuric acid and perchloric acid, or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2- hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Pharmaceutically acceptable salts derived from appropriate bases include alkali metal, alkaline earth metal, ammonium and $N^+(C_{1-4}$ alkyl$)_4$ salts. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, lower alkyl sulfonate, and aryl sulfonate.

A "subject" to which administration is contemplated includes, but is not limited to, humans (i.e., a male or female of any age group, e.g., a pediatric subject (e.g., infant, child, adolescent) or adult subject (e.g., young adult, middle-aged adult or senior adult)) and/or a non-human animal, e.g., a mammal such as primates (e.g., cynomolgus monkeys, rhesus monkeys), cattle, pigs, horses, sheep, goats, rodents, cats, and/or dogs. In certain embodiments, the subject is a human. In certain embodiments, the subject is a non-human animal.

As used herein, and unless otherwise specified, the terms "treat," "treating" and "treatment" contemplate an action that occurs while a subject is suffering from the specified disease, disorder or condition, which reduces the severity of the disease, disorder or condition, or retards or slows the progression of the disease, disorder or condition ("therapeutic treatment"), and also contemplates an action that occurs before a subject begins to suffer from the specified disease, disorder or condition ("prophylactic treatment").

**In** general, the "effective amount" of a compound refers to an amount sufficient to elicit the desired biological

response. As will be appreciated by those of ordinary skill in this art, the effective amount of a compound of the disclosure may vary depending on such factors as the desired biological endpoint, the pharmacokinetics of the compound, the disease being treated, the mode of administration, and the age, health, and condition of the subject. An effective amount includes both therapeutic and prophylactic treatment effective amounts.

As used herein, and unless otherwise specified, a "therapeutically effective amount" of a compound is an amount sufficient to provide a therapeutic benefit in the treatment of a disease, disorder or condition, or to delay or minimize one or more symptoms associated with the disease, disorder or condition. A therapeutically effective amount of a compound means an amount of therapeutic agent, alone or in combination with other therapies, which provides a therapeutic benefit in the treatment of the disease, disorder or condition. The term "therapeutically effective amount" can encompass an amount that improves overall therapy, reduces or avoids symptoms or causes of disease or condition, or enhances the therapeutic efficacy of another therapeutic agent.

As used herein, and unless otherwise specified, a "prophylactically effective amount" of a compound is an amount sufficient to prevent a disease, disorder or condition, or one or more symptoms associated with the disease, disorder or condition, or prevent its recurrence. A prophylactically effective amount of a compound means an amount of a therapeutic agent, alone or in combination with other agents, which provides a prophylactic benefit in the prevention of the disease, disorder or condition. The term "prophylactically effective amount" can encompass an amount that improves overall prophylaxis or enhances the prophylactic efficacy of another prophylactic agent.

Compound

As used herein, "compounds of the present disclosure" refers to the compounds of the following general formula or its sub-formula, or a pharmaceutically acceptable salt, an enantiomer, a diastereomer, a solvate, a hydrate, a polymorph or an isotopic variant thereof.

**In** one embodiment, the present disclosure relates to a compound of formula (I), or a pharmaceutically acceptable salt, an enantiomer, a diastereomer, a solvate, a hydrate, a polymorph or an isotopic variant thereof:

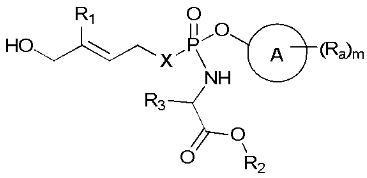

(I)

wherein,

ring A is $C_{6-10}$ aryl or 5- to 10-membered heteroaryl;
$R_a$ is selected from H, halogen, $C_{1-6}$ alkyl, $C_{1-6}$ haloalkyl, -OR", NR"R", $C_{2-6}$ alkenyl and $C_{2-6}$ alkynyl;
m=1, 2, 3, 4, or 5;
X is O or CRR';
$R_1$ is selected from H, F, Cl, CN and methyl, wherein the methyl is optionally substituted with 1 to 3 substituents independently selected from halogen, CN, OH and $NH_2$;
$R_2$ is selected from $C_{1-6}$ hydroxyalkyl, $C_{5-6}$ alkyl, $C_{5-6}$ haloalkyl, $C_{5-6}$ alkenyl, $C_{5-6}$ alkynyl, $C_{0-6}$ alkylene-$C_{3-12}$ cycloalkyl and $C_{0-6}$ alkylene-4- to 12-membered heterocyclyl, wherein the $C_{1-6}$ hydroxyalkyl, $C_{5-6}$ alkyl, $C_{5-6}$ haloalkyl, $C_{5-6}$ alkenyl, $C_{5-6}$ alkynyl, $C_{0-6}$ alkylene-$C_{3-12}$ cycloalkyl and $C_{0-6}$ alkylene-4- to 12-membered heterocyclyl are optionally substituted with one or more substituents selected from halogen, $C_{1-6}$ alkyl, $C_{1-6}$ haloalkyl, $C_{1-6}$ hydroxyalkyl, $C_{2-6}$ alkenyl, $C_{2-6}$ alkynyl, 3- to 10-membered cycloalkyl, 4- to 10-membered heterocycloalkyl, $C_{6-10}$ aryl, 5- to 10-membered heteroaryl, -C(O)-$C_{1-6}$ alkyl, -C(O)-$C_{1-6}$ hydroxyalkyl, -C(O)-$C_{1-6}$ haloalkyl, -C(O)-$C_{2-6}$ alkenyl, -C(O)-$C_{2-6}$ alkynyl, -C(O)-3- to 10-membered cycloalkyl, -C(O)-4- to 10-membered heterocycloalkyl, -C(O)-$C_{6-10}$ aryl, -C(O)-5- to 10-membered heteroaryl, -CN and oxo group;
$R_3$ is selected from $C_{1-6}$ alkyl, $C_{1-6}$ haloalkyl, $C_{2-6}$ alkenyl and $C_{2-6}$ alkynyl;
wherein,
R and R' are independently selected from H and halogen;
R" is independently selected from H, $C_{1-6}$ alkyl, $C_{1-6}$ haloalkyl, $C_{2-6}$ alkenyl, $C_{2-6}$ alkynyl, $C_{3-7}$ cycloalkyl, 3- to 7-membered heterocyclyl, $C_{6-10}$ aryl and 5- to 10-membered heteroaryl.

In one embodiment, the present disclosure relates to a compound of formula (I), or a pharmaceutically acceptable salt, an enantiomer, a diastereomer, a solvate, a hydrate, a polymorph or an isotopic variant thereof:

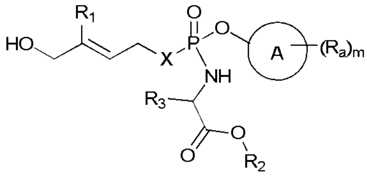

(I)

wherein,

ring A is $C_{6-10}$ aryl or 5- to 10-membered heteroaryl;
$R_a$ is selected from H, halogen, $C_{1-6}$ alkyl, $C_{1-6}$ haloalkyl, -OR", NR"R", $C_{2-6}$ alkenyl and $C_{2-6}$ alkynyl;
m=1, 2, 3, 4, or 5;
X is O or CRR';
$R_1$ is selected from H, F, Cl, CN and methyl, wherein the methyl is optionally substituted with 1 to 3 substituents independently selected from halogen, CN, OH and $NH_2$;
$R_2$ is selected from $C_{5-6}$ alkyl, $C_{5-6}$ haloalkyl, $C_{5-6}$ alkenyl, $C_{5-6}$ alkynyl, $C_{0-6}$ alkylene-$C_{3-7}$ cycloalkyl, $C_{0-6}$ alkylene-4- to 7-membered heterocyclyl, $C_{0-6}$ alkylene-$C_{6-10}$ aryl and $C_{0-6}$ alkylene-5- to 10-membered heteroaryl;
$R_3$ is selected from $C_{1-6}$ alkyl, $C_{1-6}$ haloalkyl, $C_{2-6}$ alkenyl and $C_{2-6}$ alkynyl;
wherein,
R and R' are independently selected from H and halogen;
R" is independently selected from H, $C_{1-6}$ alkyl, $C_{1-6}$ haloalkyl, $C_{2-6}$ alkenyl, $C_{2-6}$ alkynyl, $C_{3-7}$ cycloalkyl, 3- to 7-membered heterocyclyl, $C_{6-10}$ aryl and 5- to 10-membered heteroaryl.

In another embodiment, the present disclosure is directed to a compound of formula (I), or a pharmaceutically acceptable salt, an enantiomer, a diastereomer, a solvate, a hydrate, a polymorph or an isotopic variant thereof:

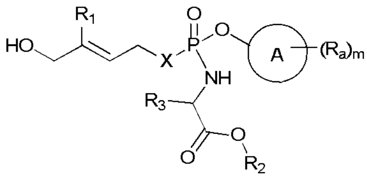

(I)

wherein,

ring A is $C_{6-10}$ aryl or 5- to 10-membered heteroaryl;
$R_a$ is selected from H, halogen, $C_{1-6}$ alkyl, $C_{1-6}$ haloalkyl, -OR", NR"R", $C_{2-6}$ alkenyl and $C_{2-6}$ alkynyl;
m=1, 2, 3, 4, or 5;
X is O or CRR';
$R_1$ is selected from H, F, Cl, CN and methyl, wherein the methyl is substituted with 1 to 3 substituents independently selected from halogen, CN, OH and $NH_2$;
$R_2$ is selected from $C_{1-6}$ alkyl, $C_{1-6}$ haloalkyl, $C_{2-6}$ alkenyl, $C_{2-6}$ alkynyl, $C_{0-6}$ alkylene-$C_{3-7}$ cycloalkyl, $C_{0-6}$ alkylene-3- to 7-membered heterocyclyl, $C_{0-6}$ alkylene-$C_{6-10}$ aryl and $C_{0-6}$ alkylene-5- to 10-membered heteroaryl;
$R_3$ is selected from $C_{1-6}$ alkyl, $C_{1-6}$ haloalkyl, $C_{2-6}$ alkenyl and $C_{2-6}$ alkynyl;
wherein,
R and R' are independently selected from H and halogen;
R" is independently selected from H, $C_{1-6}$ alkyl, $C_{1-6}$ haloalkyl, $C_{2-6}$ alkenyl, $C_{2-6}$ alkynyl, $C_{3-7}$ cycloalkyl, 3- to 7-membered heterocyclyl, $C_{6-10}$ aryl and 5- to 10-membered heteroaryl.

Ring A

In a specific embodiment, ring A is $C_{6-10}$ aryl, phenyl is alternative; in another specific embodiment, ring A is 5- to 10-membered heteroaryl.

$R_a$

In a specific embodiment, $R_a$ is H; in another specific embodiment, $R_a$ is halogen; in another specific embodiment, $R_a$ is $C_{1-6}$ alkyl; in another specific embodiment, $R_a$ is $C_{1-6}$ haloalkyl; in another specific embodiment, $R_a$ is -OR"; in

another specific embodiment, $R_a$ is NR"R"; in another specific embodiment, $R_a$ is $C_{2-6}$ alkenyl; in another specific embodiment, $R_a$ is $C_{2-6}$ alkynyl.

m

In a specific embodiment, m=1; in another specific embodiment, m=2; in another specific embodiment, m=3; in another specific embodiment, m=4; in another specific embodiment, m=5.

X

In a specific embodiment, X is O; in another specific embodiment, X is CRR'; in another specific embodiment, X is $CH_2$ or $CF_2$.

$R_1$

In a specific embodiment, $R_1$ is H; in another specific embodiment, $R_1$ is F; in another specific embodiment, $R_1$ is Cl; in another specific embodiment, $R_1$ is CN; in another specific embodiment, $R_1$ is methyl substituted with 1 to 3 substituents independently selected from halogen, CN, OH and $NH_2$, for example, $R_1$ is $CF_3$ or $CH_2OH$; in another specific embodiment, $R_1$ is methyl, wherein the methyl is optionally substituted with 1-3 substituents independently selected from halogen, CN, OH and $NH_2$.

In a more specific embodiment, $R_1$ is H; in another more specific embodiment, $R_1$ is selected from $CF_3$ and $CH_2OH$; in another more specific embodiment, $R_1$ is selected from H and $CF_3$; in another more specific embodiment, $R_1$ is $CF_3$; in another more specific embodiment, $R_1$ is selected from H, F, Cl, CN, $CH_3$, $CF_3$ and $CH_2OH$; in another more specific embodiment, $R_1$ is selected from H, $CH_3$ and $CF_3$; in another more specific embodiment, $R_1$ is selected from $CH_3$ and $CF_3$; in another more specific embodiment, $R_1$ is $CH_3$.

$R_2$

In a specific embodiment, $R_2$ is $C_{1-6}$ alkyl, alternatively $C_{1-4}$ alkyl, alternatively $C_{5-6}$ alkyl, alternatively isopropyl or 2-ethylbutyl; in another specific embodiment, $R_2$ is $C_{1-6}$ haloalkyl, alternatively $C_{1-4}$ haloalkyl, alternatively $C_{5-6}$ haloalkyl; in another specific embodiment, $R_2$ is $C_{2-6}$ alkenyl, alternatively $C_{2-4}$ alkenyl, alternatively $C_{5-6}$ alkenyl; in another specific embodiment, $R_2$ is $C_{2-6}$ alkynyl, alternatively $C_{2-4}$ alkynyl, alternatively $C_{5-6}$ alkynyl; in another specific embodiment, $R_2$ is $C_{0-6}$ alkylene-$C_{3-7}$ cycloalkyl, alternatively $C_{0-6}$ alkylene-$C_{5-6}$ cycloalkyl; in another specific embodiment, $R_2$ is $C_{0-6}$ alkylene-3- to 7-membered heterocyclyl, alternatively $C_{0-6}$ alkylene-4- to 7-membered heterocyclyl, $C_{0-6}$ alkylene-5- to 6-membered heterocyclyl, alternatively tetrahydropyranyl; in another specific embodiment, $R_2$ is $C_{0-6}$ alkylene-$C_{6-10}$ aryl; in another specific embodiment, $R_2$ is $C_{0-6}$ alkylene-5- to 10-membered heteroaryl.

In a more specific embodiment, $R_2$ is $C_{1-6}$ alkyl, $C_{0-6}$ alkylene-$C_{3-7}$ cycloalkyl, $C_{0-6}$ alkylene-3- to 7-membered heterocyclyl, $C_{0-6}$ alkylene-$C_{6-10}$ aryl or $C_{0-6}$ alkylene-5- to 10-membered heteroaryl; in another more specific embodiment, $R_2$ is isopropyl, 2-ethylbutyl, cyclopentyl, cyclohexyl, tetrahydrofuranyl, tetrahydrothiophenyl, pyrrolidinyl, tetrahydropyranyl, tetrahydrothiopyranyl, tetrahydropyridinyl or benzyl; in another more specific embodiment, $R_2$ is 2-ethylbutyl, cyclopentyl, tetrahydrofuranyl, tetrahydrothiophenyl, pyrrolidinyl or benzyl; in another more specific embodiment, $R_2$ is 2-ethylbutyl, cyclopentyl, tetrahydrofuranyl or benzyl; in another more specific embodiment, $R_2$ is 2-ethylbutyl, cyclopentyl, cyclohexyl, tetrahydrofuranyl, tetrahydrothiophenyl, pyrrolidinyl, tetrahydropyranyl, tetrahydrothiopyranyl or tetrahydropyridinyl; in another more specific embodiment, $R_2$ is 2-ethylbutyl, cyclopentyl, tetrahydrofuranyl, tetrahydrothiophenyl or pyrrolidinyl; in another more specific embodiment, $R_2$ is 2-ethylbutyl, cyclopentyl or tetrahydrofuranyl.

In another more specific embodiment, $R_2$ is 3- to 12-membered cycloalkyl or 4- to 12-membered heterocyclyl, wherein the 3- to 12-membered cycloalkyl or the 4- to 12-membered heterocyclyl is optionally substituted with one or more substituents selected from halogen, $C_{1-6}$ alkyl, $C_{1-6}$ haloalkyl, $C_{1-6}$ hydroxyalkyl, $C_{2-6}$ alkenyl, $C_{2-6}$ alkynyl, 3- to 10-membered cycloalkyl, 4- to 10-membered heterocycloalkyl, $C_{6-10}$ aryl, 5- to 10-membered heteroaryl, -C(O)-$C_{1-6}$ alkyl, -C(O)-$C_{1-6}$ hydroxyalkyl, -C(O)-$C_{1-6}$ haloalkyl, -C(O)-$C_{2-6}$ alkenyl, -C(O)-$C_{2-6}$ alkynyl, -C(O)-3- to 10-membered cycloalkyl, -C(O)-4- to 10-membered heterocycloalkyl, -C(O)-$C_{6-10}$ aryl, -C(O)-5- to 10-membered heteroaryl, -CN and oxo group; alternatively, $R_2$ is 4- to 10-membered heterocyclyl optionally substituted with one or more substituents selected from halogen, $C_{1-6}$ alkyl, -C(O)-$C_{1-6}$ alkyl, $C_{1-6}$ haloalkyl, $C_{1-6}$ hydroxyalkyl, -C(O)-3- to 10-membered cycloalkyl, -CN and oxo group; and still alternatively, $R_2$ is 4- to 7-membered heterocyclyl, wherein the heteroatom is oxygen atom or nitrogen atom, and the 4- to 7-membered heterocyclyl is optionally substituted with one or more substituents selected from $C_{1-6}$ alkyl and oxo group; and still alternatively, $R_2$ is selected from:

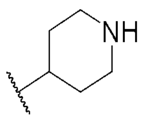, 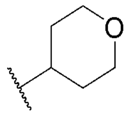,

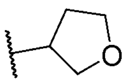, 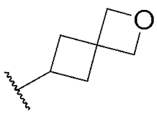, 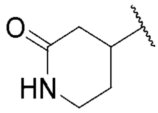, 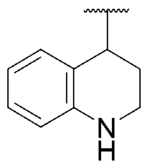, 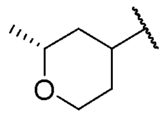, 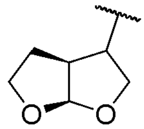, 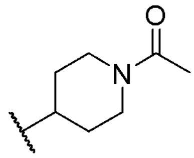,

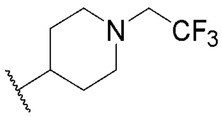, 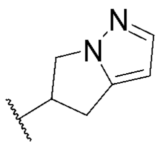, 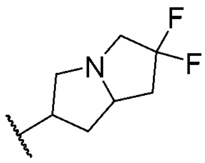, 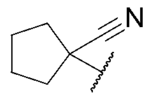, 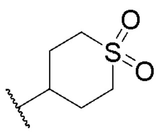, 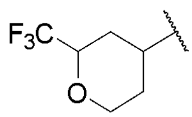,

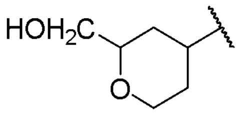 and 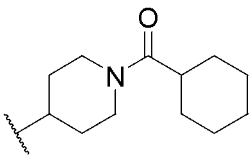.

$R_3$

In a specific embodiment, $R_3$ is $C_{1-6}$ alkyl, such as methyl or isopropyl; in another specific embodiment, $R_3$ is $C_{1-6}$ haloalkyl; in another specific embodiment, $R_3$ is $C_{2-6}$ alkenyl; in another specific embodiment, $R_3$ is $C_{2-6}$ alkynyl.

R and R'

In a specific embodiment, R and R' are H; In a specific embodiment, R and R' are halogen.

R"

In a specific embodiment, R" is $C_{1-6}$ alkyl; in another specific embodiment, R" is $C_{1-6}$ haloalkyl; in another specific embodiment, R" is $C_{0-6}$ alkylene-$C_{6-10}$ aryl; in another specific embodiment, R" is $C_{0-6}$ alkylene-5- to 10-membered heteroaryl.

Any technical solution in any one of the above specific embodiments, or any combination thereof, may be combined with any technical solution in other specific embodiments or any combination thereof. For example, any technical solution of X or any combination thereof may be combined with any technical solution of $R_1$ to $R_3$, R, R' and R", or any combination thereof. The present disclosure is intended to include all combinations of such technical solutions, which are not exhaustively listed here to save space.

In an alternative embodiment, the present disclosure relates to the following technical solutions:

**Technical Solution 1.** A compound of formula (I), or a pharmaceutically acceptable salt, an enantiomer, a diastereomer, a solvate, a hydrate, a polymorph or an isotopic variant thereof:

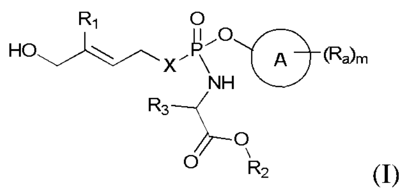 (I)

wherein,

ring A is $C_{6-10}$ aryl or 5- to 10-membered heteroaryl;

$R_a$ is selected from H, halogen, $C_{1-6}$ alkyl, $C_{1-6}$ haloalkyl, -OR", NR"R", $C_{2-6}$ alkenyl and $C_{2-6}$ alkynyl;

m=1, 2, 3, 4, or 5;

X is O or CRR';

$R_1$ is selected from H, F, Cl, CN and methyl, wherein the methyl is optionally substituted with 1 to 3 substituents independently selected from halogen, CN, OH and $NH_2$;

$R_2$ is selected from $C_{1-6}$ hydroxyalkyl, $C_{5-6}$ alkyl, $C_{5-6}$ haloalkyl, $C_{5-6}$ alkenyl, $C_{5-6}$ alkynyl, $C_{0-6}$ alkylene-$C_{3-12}$ cycloalkyl, $C_{0-6}$ alkylene-4- to 12-membered heterocyclyl and $C_{0-6}$ alkylene-5- to 10-membered heteroaryl, wherein the $C_{1-6}$ hydroxyalkyl, $C_{5-6}$ alkyl, $C_{5-6}$ haloalkyl, $C_{5-6}$ alkenyl, $C_{5-6}$ alkynyl, $C_{0-6}$ alkylene-$C_{3-12}$ cycloalkyl, $C_{0-6}$ alkylene-4- to 12-membered heterocyclyl and $C_{0-6}$ alkylene-5- to 10-membered heteroaryl are optionally substituted with one or more substituents selected from halogen, $C_{1-6}$ alkyl, $C_{1-6}$ haloalkyl, $C_{1-6}$ hydroxyalkyl, $C_{2-6}$ alkenyl, $C_{2-6}$ alkynyl, 3- to 10-membered cycloalkyl, 4- to 10-membered heterocycloalkyl, $C_{6-10}$ aryl, 5- to 10-membered heteroaryl, -C(O)-$C_{1-6}$ alkyl, -C(O)-$C_{1-6}$ hydroxyalkyl, -C(O)-$C_{1-6}$ haloalkyl, -C(O)-$C_{2-6}$ alkenyl, -C(O)-$C_{2-6}$ alkynyl, -C(O)-3- to 10-membered cycloalkyl, -C(O)-4-to 10-membered heterocycloalkyl, -C(O)-$C_{6-10}$ aryl, -C(O)-5- to 10-membered heteroaryl, -CN and oxo group;

$R_3$ is selected from $C_{1-6}$ alkyl, $C_{1-6}$ haloalkyl, $C_{2-6}$ alkenyl and $C_{2-6}$ alkynyl;

wherein,

R and R' are independently selected from H and halogen;

R" is independently selected from H, $C_{1-6}$ alkyl, $C_{1-6}$ haloalkyl, $C_{2-6}$ alkenyl, $C_{2-6}$ alkynyl, $C_{3-7}$ cycloalkyl, 3- to 7-membered heterocyclyl, $C_{6-10}$ aryl and 5- to 10-membered heteroaryl.

**Technical Solution 2.** The compound of formula (I) of Technical Solution 1, or a pharmaceutically acceptable salt, an enantiomer, a diastereomer, a solvate, a hydrate, a polymorph or an isotopic variant thereof:

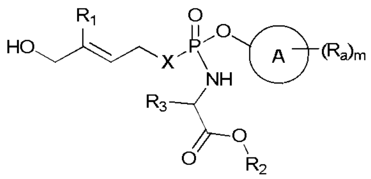

(I)

wherein,

ring A is $C_{6-10}$ aryl or 5- to 10-membered heteroaryl;

$R_a$ is selected from H, halogen, $C_{1-6}$ alkyl, $C_{1-6}$ haloalkyl, -OR", NR"R", $C_{2-6}$ alkenyl and $C_{2-6}$ alkynyl;

m=1, 2, 3, 4, or 5;

X is O or CRR';

$R_1$ is selected from H, F, Cl, CN and methyl, wherein the methyl is optionally substituted with 1 to 3 substituents independently selected from halogen, CN, OH and $NH_2$;

$R_2$ is selected from $C_{5-6}$ alkyl, $C_{5-6}$ haloalkyl, $C_{5-6}$ alkenyl, $C_{5-6}$ alkynyl, $C_{0-6}$ alkylene-$C_{3-7}$ cycloalkyl, $C_{0-6}$ alkylene-4- to 7-membered heterocyclyl and $C_{0-6}$ alkylene-5- to 10-membered heteroaryl;

$R_3$ is selected from $C_{1-6}$ alkyl, $C_{1-6}$ haloalkyl, $C_{2-6}$ alkenyl and $C_{2-6}$ alkynyl;

wherein,

R and R' are independently selected from H and halogen;

R" is independently selected from H, $C_{1-6}$ alkyl, $C_{1-6}$ haloalkyl, $C_{2-6}$ alkenyl, $C_{2-6}$ alkynyl, $C_{3-7}$ cycloalkyl, 3- to 7-membered heterocyclyl, $C_{6-10}$ aryl and 5- to 10-membered heteroaryl.

**Technical Solution 3.** The compound of Technical Solution 1, or a pharmaceutically acceptable salt, an enantiomer, a diastereomer, a solvate, a hydrate, a polymorph or an isotopic variant thereof, which is a compound of formula (II):

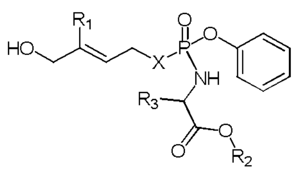

(II)

wherein,

X is O or CRR';
$R_1$ is selected from H, F, Cl, CN and methyl, wherein the methyl is optionally substituted with 1 to 3 substituents independently selected from halogen, CN, OH and $NH_2$;
$R_2$ is selected from $C_{1-6}$ hydroxyalkyl, $C_{5-6}$ alkyl, $C_{5-6}$ haloalkyl, $C_{5-6}$ alkenyl, $C_{5-6}$ alkynyl, $C_{0-6}$ alkylene-$C_{3-12}$ cycloalkyl and $C_{0-6}$ alkylene-4- to 12-membered heterocyclyl, wherein the $C_{1-6}$ hydroxyalkyl, $C_{5-6}$ alkyl, $C_{5-6}$ haloalkyl, $C_{5-6}$ alkenyl, $C_{5-6}$ alkynyl, $C_{0-6}$ alkylene-$C_{3-12}$ cycloalkyl and $C_{0-6}$ alkylene-4- to 12-membered heterocyclyl are optionally substituted with one or more substituents selected from halogen, $C_{1-6}$ alkyl, $C_{1-6}$ haloalkyl, $C_{1-6}$ hydroxyalkyl, $C_{2-6}$ alkenyl, $C_{2-6}$ alkynyl, 3- to 10-membered cycloalkyl, 4- to 10-membered heterocycloalkyl, $C_{6-10}$ aryl, 5- to 10-membered heteroaryl, -C(O)-$C_{1-6}$ alkyl, -C(O)-$C_{1-6}$ hydroxyalkyl, -C(O)-$C_{1-6}$ haloalkyl, -C(O)-$C_{2-6}$ alkenyl, -C(O)-$C_{2-6}$ alkynyl, -C(O)-3- to 10-membered cycloalkyl, -C(O)-4- to 10-membered heterocycloalkyl, -C(O)-$C_{6-10}$ aryl, -C(O)-5- to 10-membered heteroaryl, -CN and oxo group;
$R_3$ is selected from $C_{1-6}$ alkyl, $C_{1-6}$ haloalkyl, $C_{2-6}$ alkenyl and $C_{2-6}$ alkynyl;
wherein,
R and R' are independently selected from H and halogen.

**Technical Solution 4.** The compound of Technical Solution 1, or a pharmaceutically acceptable salt, an enantiomer, a diastereomer, a solvate, a hydrate, a polymorph or an isotopic variant thereof, which is a compound of formula (II):

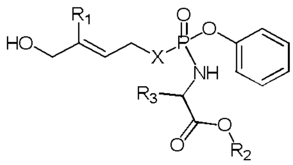
(II)

wherein,

X is O or CRR';
$R_1$ is selected from H, F, Cl, CN and methyl, wherein the methyl is optionally substituted with 1 to 3 substituents independently selected from halogen, CN, OH and $NH_2$;
$R_2$ is selected from $C_{5-6}$ alkyl, $C_{5-6}$ haloalkyl, $C_{5-6}$ alkenyl, $C_{5-6}$ alkynyl, $C_{0-6}$ alkylene-$C_{3-7}$ cycloalkyl and $C_{0-6}$ alkylene-4- to 7-membered heterocyclyl;
$R_3$ is selected from $C_{1-6}$ alkyl, $C_{1-6}$ haloalkyl, $C_{2-6}$ alkenyl and $C_{2-6}$ alkynyl;
wherein,
R and R' are independently selected from H and halogen.

**Technical Solution 5.** The compound of Technical Solution 1, or a pharmaceutically acceptable salt, an enantiomer, a diastereomer, a solvate, a hydrate, a polymorph or an isotopic variant thereof, which is a compound of formula (III):

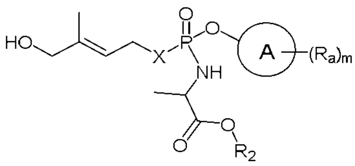
(III)

wherein,

X is O or CRR';
ring A is $C_{6-10}$ aryl or 5- to 10-membered heteroaryl;
$R_a$ is selected from H, halogen, $C_{1-6}$ alkyl, $C_{1-6}$ haloalkyl, -OR", NR"R", $C_{2-6}$ alkenyl and $C_{2-6}$ alkynyl;
$R_2$ is selected from $C_{1-6}$ hydroxyalkyl, $C_{5-6}$ alkyl, $C_{5-6}$ haloalkyl, $C_{5-6}$ alkenyl, $C_{5-6}$ alkynyl, $C_{0-6}$ alkylene-$C_{3-12}$ cycloalkyl and $C_{0-6}$ alkylene-4- to 12-membered heterocyclyl, wherein the $C_{1-6}$ hydroxyalkyl, $C_{5-6}$ alkyl, $C_{5-6}$ haloalkyl, $C_{5-6}$ alkenyl, $C_{5-6}$ alkynyl, $C_{0-6}$ alkylene-$C_{3-12}$ cycloalkyl and $C_{0-6}$ alkylene-4- to 12-membered heterocyclyl are optionally substituted with one or more substituents selected from halogen, $C_{1-6}$ alkyl, $C_{1-6}$ haloalkyl, $C_{1-6}$ hydroxyalkyl, $C_{2-6}$ alkenyl, $C_{2-6}$ alkynyl, 3- to 10-membered cycloalkyl, 4- to 10-membered heterocycloalkyl, $C_{6-10}$ aryl, 5- to 10-membered heteroaryl, -C(O)-$C_{1-6}$ alkyl, -C(O)-$C_{1-6}$ hydroxyalkyl, -C(O)-$C_{1-6}$ haloalkyl, -C(O)-$C_{2-6}$ alkenyl, -C(O)-$C_{2-6}$ alkynyl, -C(O)-3- to 10-membered cycloalkyl, -C(O)-4- to 10-membered heterocycloalkyl, -C(O)-$C_{6-10}$ aryl, -C(O)-5- to 10-membered heteroaryl, -CN and oxo group;

wherein,
R and R' are independently selected from H and halogen.

**Technical Solution 6.** The compound of any one of Technical Solutions 1-5, or a pharmaceutically acceptable salt, an enantiomer, a diastereomer, a solvate, a hydrate, a polymorph or an isotopic variant thereof, wherein X is CRR', alternatively $CH_2$ or $CF_2$.
**Technical Solution 7.** The compound of any one of Technical Solutions 1-4 and 6, or a pharmaceutically acceptable salt, an enantiomer, a diastereomer, a solvate, a hydrate, a polymorph or an isotopic variant thereof, wherein $R_1$ is selected from H, F, Cl, CN, $CH_3$, $CF_3$ and $CH_2OH$, and still alternatively $R_1$ is selected from H, $CH_3$, $CH_2OH$ and $CF_3$, and still alternatively $R_1$ is selected from $CH_3$ and $CF_3$, and still alternatively $R_1$ is $CH_3$.
**Technical Solution 8.** The compound of any one of Technical Solutions 1-7, or a pharmaceutically acceptable salt, an enantiomer, a diastereomer, a solvate, a hydrate, a polymorph or an isotopic variant thereof, wherein $R_2$ is 2-ethylbutyl, cyclopentyl, cyclohexyl, tetrahydrofuranyl, tetrahydrothiophenyl, pyrrolidinyl, tetrahydropyranyl, tetrahydrothiopyranyl or tetrahydropyridinyl, alternatively cyclopentyl, tetrahydrofuranyl, tetrahydrothiophenyl or pyrrolidinyl, and still alternatively cyclopentyl or tetrahydrofuranyl.
**Technical Solution 9.** The compound of any one of Technical Solutions 1-4 and 6-8, or a pharmaceutically acceptable salt, an enantiomer, a diastereomer, a solvate, a hydrate, a polymorph or an isotopic variant thereof, wherein $R_3$ is $C_{1-6}$ alkyl, alternatively methyl or isopropyl.
**Technical Solution 10.** The compound of Technical Solution 1, or a pharmaceutically acceptable salt, an enantiomer, a diastereomer, a solvate, a hydrate, a polymorph or an isotopic variant thereof, which is a compound of formula (IV):

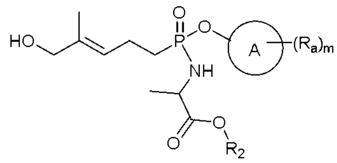
(IV)

wherein ring A is $C_{6-10}$ aryl or 5- to 10-membered heteroaryl, such as phenyl or naphthalene ring,
$R_a$ is selected from H, halogen, $C_{1-6}$ alkyl, $C_{1-6}$ haloalkyl, -OR", NR"R", $C_{2-6}$ alkenyl and $C_{2-6}$ alkynyl;
m=1, 2, 3, 4, or 5;
$R_2$ is 3- to 12-membered cycloalkyl or 4- to 12-membered heterocyclyl, wherein the 3- to 12-membered cycloalkyl or the 4- to 12-membered heterocyclyl is optionally substituted with one or more substituents selected from halogen, $C_{1-6}$ alkyl, $C_{1-6}$ haloalkyl, $C_{1-6}$ hydroxyalkyl, $C_{2-6}$ alkenyl, $C_{2-6}$ alkynyl, 3-to 10-membered cycloalkyl, 4- to 10-membered heterocycloalkyl, $C_{6-10}$ aryl, 5- to 10-membered heteroaryl, -C(O)-$C_{1-6}$ alkyl, -C(O)-$C_{1-6}$ hydroxyalkyl, -C(O)-$C_{1-6}$ haloalkyl, -C(O)-$C_{2-6}$ alkenyl, -C(O)-$C_{2-6}$ alkynyl, -C(O)-3- to 10-membered cycloalkyl, -C(O)-4- to 10-membered heterocycloalkyl, -C(O)-$C_{6-10}$ aryl, -C(O)-5-to 10-membered heteroaryl, -CN and oxo group.

**Technical Solution 11.** The compound of Technical Solution 10, or a pharmaceutically acceptable salt, an enantiomer, a diastereomer, a solvate, a hydrate, a polymorph or an isotopic variant thereof, which is a compound of formula (V):

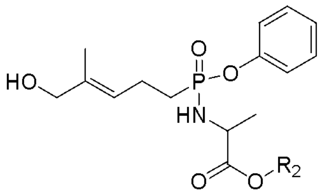
(V)

wherein $R_2$ is 4- to 12-membered heterocyclyl, wherein the 4- to 12-membered heterocyclyl is optionally substituted with one or more substituents selected from halogen, $C_{1-6}$ alkyl, $C_{1-6}$ haloalkyl, $C_{1-6}$ hydroxyalkyl, $C_{2-6}$ alkenyl, $C_{2-6}$ alkynyl, 3- to 10-membered cycloalkyl, 4- to 10-membered heterocycloalkyl, $C_{6-10}$ aryl, 5- to 10-membered heteroaryl, -C(O)-$C_{1-6}$ alkyl, -C(O)-$C_{1-6}$ hydroxyalkyl, -C(O)-$C_{1-6}$ haloalkyl, -C(O)-$C_{2-6}$ alkenyl, -C(O)-$C_{2-6}$ alkynyl, -C(O)-3- to 10-membered cycloalkyl, -C(O)-4- to 10-membered heterocycloalkyl, -C(O)-$C_{6-10}$ aryl, -C(O)-5- to 10-membered heteroaryl, -CN and oxo group.
**Technical Solution 12.** The compound of any one of Technical Solutions 1-11, or a pharmaceutically acceptable salt, an enantiomer, a diastereomer, a solvate, a hydrate, a polymorph or an isotopic variant thereof, wherein $R_2$ is 4- to 10-membered heterocyclyl optionally substituted with one or more substituents selected from halogen, $C_{1-6}$ alkyl, -C(O)-$C_{1-6}$ alkyl, $C_{1-6}$ haloalkyl, $C_{1-6}$ hydroxyalkyl, -C(O)-3- to 10-membered cycloalkyl, -CN and oxo group.

**Technical Solution 13.** The compound of any one of Technical Solutions 1-11, or a pharmaceutically acceptable salt, an enantiomer, a diastereomer, a solvate, a hydrate, a polymorph or an isotopic variant thereof, wherein $R_2$ is 4- to 7-membered heterocyclyl, the heteroatom is oxygen atom or nitrogen atom, and the 4- to 7-membered heterocyclyl is optionally substituted with one or more substituents selected from $C_{1-6}$ alkyl and oxo group.

**Technical Solution 14.** The compound of any one of Technical Solutions 1-11, or a pharmaceutically acceptable salt, an enantiomer, a diastereomer, a solvate, a hydrate, a polymorph or an isotopic variant thereof, wherein $R_2$ is selected from:

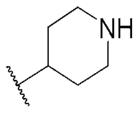, 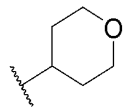, 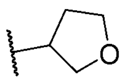, 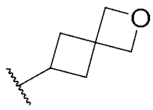, 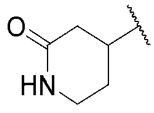, 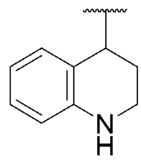, 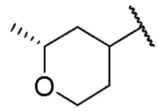,

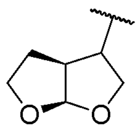, 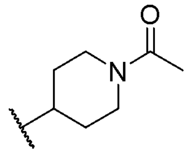, 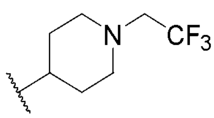, 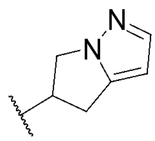, 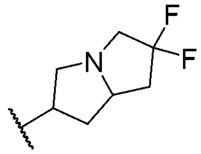, 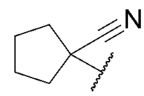,

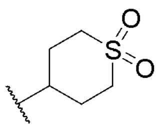, 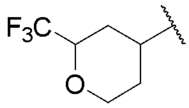, 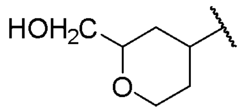 and 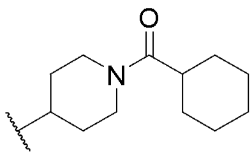.

**Technical Solution 15.** A compound of formula (I), or a pharmaceutically acceptable salt, an enantiomer, a diastereomer, a solvate, a hydrate, a polymorph or an isotopic variant thereof:

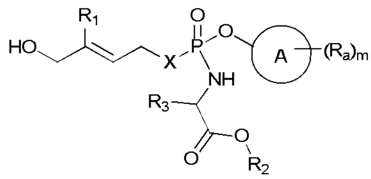

(I)

wherein,

ring A is $C_{6-10}$ aryl or 5- to 10-membered heteroaryl;
$R_a$ is selected from H, halogen, $C_{1-6}$ alkyl, $C_{1-6}$ haloalkyl, -OR", NR"R", $C_{2-6}$ alkenyl and $C_{2-6}$ alkynyl;
m=1, 2, 3, 4, or 5;
X is O or CRR';
$R_1$ is selected from H, F, Cl, CN and methyl, wherein the methyl is substituted with 1 to 3 substituents independently selected from halogen, CN, OH and $NH_2$;
$R_2$ is selected from $C_{1-6}$ hydroxyalkyl, $C_{5-6}$ alkyl, $C_{5-6}$ haloalkyl, $C_{5-6}$ alkenyl, $C_{5-6}$ alkynyl, $C_{0-6}$ alkylene-$C_{3-12}$ cycloalkyl and $C_{0-6}$ alkylene-4- to 12-membered heterocyclyl, wherein the $C_{1-6}$ hydroxyalkyl, $C_{5-6}$ alkyl, $C_{5-6}$ haloalkyl, $C_{5-6}$ alkenyl, $C_{5-6}$ alkynyl, $C_{0-6}$ alkylene-$C_{3-12}$ cycloalkyl and $C_{0-6}$ alkylene-4- to 12-membered heterocyclyl are optionally substituted with one or more substituents selected from halogen, $C_{1-6}$ alkyl, $C_{1-6}$ haloalkyl, $C_{1-6}$ hydroxyalkyl, $C_{2-6}$ alkenyl, $C_{2-6}$ alkynyl, 3- to 10-membered cycloalkyl, 4- to 10-membered heterocycloalkyl, $C_{6-10}$ aryl, 5- to 10-membered heteroaryl, -C(O)-$C_{1-6}$ alkyl, -C(O)-$C_{1-6}$ hydroxyalkyl, -C(O)-$C_{1-6}$ haloalkyl, -C(O)-$C_{2-6}$ alkenyl, -C(O)-$C_{2-6}$ alkynyl, -C(O)-3- to 10-membered cycloalkyl, -C(O)-4- to 10-membered heterocycloalkyl, -C(O)-$C_{6-10}$ aryl, -C(O)-5- to 10-membered heteroaryl, -CN and oxo group; alternatively, $R_2$ is selected from $C_{1-6}$ alkyl, $C_{1-6}$ haloalkyl, $C_{2-6}$ alkenyl, $C_{2-6}$ alkynyl, $C_{0-6}$ alkylene-$C_{3-7}$ cycloalkyl, $C_{0-6}$ alkylene-3- to 7-membered heterocyclyl, $C_{0-6}$ alkylene-$C_{6-10}$ aryl and $C_{0-6}$ alkylene-5- to 10-membered

heteroaryl;
$R_3$ is selected from $C_{1-6}$ alkyl, $C_{1-6}$ haloalkyl, $C_{2-6}$ alkenyl and $C_{2-6}$ alkynyl;
wherein,
R and R' are independently selected from H and halogen;
R" is independently selected from H, $C_{1-6}$ alkyl, $C_{1-6}$ haloalkyl, $C_{2-6}$ alkenyl, $C_{2-6}$ alkynyl, $C_{3-7}$ cycloalkyl, 3- to 7-membered heterocyclyl, $C_{6-10}$ aryl and 5- to 10-membered heteroaryl.

**Technical Solution 16.** The compound of Technical Solution 15, or a pharmaceutically acceptable salt, an enantiomer, a diastereomer, a solvate, a hydrate, a polymorph or an isotopic variant thereof, which is a compound of formula (II):

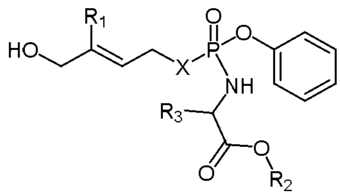
(II)

wherein,

X is O or CRR';
$R_1$ is selected from H, F, Cl, CN and methyl, wherein the methyl is substituted with 1 to 3 substituents independently selected from halogen, CN, OH and $NH_2$;
$R_2$ is selected from $C_{1-6}$ hydroxyalkyl, $C_{5-6}$ alkyl, $C_{5-6}$ haloalkyl, $C_{5-6}$ alkenyl, $C_{5-6}$ alkynyl, $C_{0-6}$ alkylene-$C_{3-12}$ cycloalkyl and $C_{0-6}$ alkylene-4- to 12-membered heterocyclyl, wherein the $C_{1-6}$ hydroxyalkyl, $C_{5-6}$ alkyl, $C_{5-6}$ haloalkyl, $C_{5-6}$ alkenyl, $C_{5-6}$ alkynyl, $C_{0-6}$ alkylene-$C_{3-12}$ cycloalkyl and $C_{0-6}$ alkylene-4- to 12-membered heterocyclyl are optionally substituted with one or more substituents selected from halogen, $C_{1-6}$ alkyl, $C_{1-6}$ haloalkyl, $C_{1-6}$ hydroxyalkyl, $C_{2-6}$ alkenyl, $C_{2-6}$ alkynyl, 3- to 10-membered cycloalkyl, 4- to 10-membered heterocycloalkyl, $C_{6-10}$ aryl, 5- to 10-membered heteroaryl, -C(O)-$C_{1-6}$ alkyl, -C(O)-$C_{1-6}$ hydroxyalkyl, -C(O)-$C_{1-6}$ haloalkyl, -C(O)-$C_{2-6}$ alkenyl, -C(O)-$C_{2-6}$ alkynyl, -C(O)-3- to 10-membered cycloalkyl, -C(O)-4- to 10-membered heterocycloalkyl, -C(O)-$C_{6-10}$ aryl, -C(O)-5- to 10-membered heteroaryl, -CN and oxo group; alternatively, $R_2$ is selected from $C_{1-6}$ alkyl, $C_{1-6}$ haloalkyl, $C_{2-6}$ alkenyl, $C_{2-6}$ alkynyl, $C_{0-6}$ alkylene-$C_{3-7}$ cycloalkyl, $C_{0-6}$ alkylene-3- to 7-membered heterocyclyl, $C_{0-6}$ alkylene-$C_{6-10}$ aryl and $C_{0-6}$ alkylene-5- to 10-membered heteroaryl;
$R_3$ is selected from $C_{1-6}$ alkyl, $C_{1-6}$ haloalkyl, $C_{2-6}$ alkenyl and $C_{2-6}$ alkynyl;
wherein,
R and R' are independently selected from H and halogen.

**Technical Solution 17.** The compound of Technical Solution 15 or 16, or a pharmaceutically acceptable salt, an enantiomer, a diastereomer, a solvate, a hydrate, a polymorph or an isotopic variant thereof, wherein X is CRR', alternatively $CH_2$ or $CF_2$.

**Technical Solution 18.** The compound of any one of Technical Solutions 15-17, or a pharmaceutically acceptable salt, an enantiomer, a diastereomer, a solvate, a hydrate, a polymorph or an isotopic variant thereof, wherein $R_1$ is selected from H, F, Cl, CN, $CF_3$ and $CH_2OH$, and still alternatively $R_1$ is selected from H and $CF_3$, and still alternatively $R_1$ is $CF_3$.

**Technical Solution 19.** The compound of any one of Technical Solutions 15-18, or a pharmaceutically acceptable salt, an enantiomer, a diastereomer, a solvate, a hydrate, a polymorph or an isotopic variant thereof, wherein $R_2$ is $C_{1-6}$ alkyl, $C_{0-6}$ alkylene-$C_{3-7}$ cycloalkyl, $C_{0-6}$ alkylene-3- to 7-membered heterocyclyl, $C_{0-6}$ alkylene-$C_{6-10}$ aryl or $C_{0-6}$ alkylene-5- to 10-membered heteroaryl, alternatively $R_2$ is isopropyl, 2-ethylbutyl, cyclopentyl, cyclohexyl, tetrahydrofuranyl, tetrahydrothiophenyl, pyrrolidinyl, tetrahydropyranyl, tetrahydrothiopyranyl, tetrahydropyridinyl or benzyl, and still alternatively $R_2$ is 2-ethylbutyl, cyclopentyl, tetrahydrofuranyl, tetrahydrothiophenyl, pyrrolidinyl or benzyl, and still alternatively $R_2$ is 2-ethylbutyl, cyclopentyl, tetrahydrofuranyl or benzyl.

**Technical Solution 20.** The compound of any one of Technical Solutions 15-19, or a pharmaceutically acceptable salt, an enantiomer, a diastereomer, a solvate, a hydrate, a polymorph or an isotopic variant thereof, wherein $R_3$ is $C_{1-6}$ alkyl, alternatively methyl or isopropyl.

**Technical Solution 21.** The compound of any one of Technical Solutions 15-20, or a pharmaceutically acceptable salt, an enantiomer, a diastereomer, a solvate, a hydrate, a polymorph or an isotopic variant thereof, wherein $R_2$ is 3- to

12-membered cycloalkyl or 4- to 12-membered heterocyclyl, wherein the 3- to 12-membered cycloalkyl or the 4- to 12-membered heterocyclyl is optionally substituted with one or more substituents selected from halogen, $C_{1-6}$ alkyl, $C_{1-6}$ haloalkyl, $C_{1-6}$ hydroxyalkyl, $C_{2-6}$ alkenyl, $C_{2-6}$ alkynyl, 3- to 10-membered cycloalkyl, 4- to 10-membered heterocycloalkyl, $C_{6-10}$ aryl, 5- to 10-membered heteroaryl, -C(O)-$C_{1-6}$ alkyl, -C(O)-$C_{1-6}$ hydroxyalkyl, -C(O)-$C_{1-6}$ haloalkyl, -C(O)-$C_{2-6}$ alkenyl, -C(O)-$C_{2-6}$ alkynyl, -C(O)-3- to 10-membered cycloalkyl, -C(O)-4- to 10-membered heterocycloalkyl, -C(O)-$C_{6-10}$ aryl, -C(O)-5- to 10-membered heteroaryl, -CN and oxo group; alternatively, $R_2$ is 4- to 10-membered heterocyclyl optionally substituted with one or more substituents selected from halogen, $C_{1-6}$ alkyl, -C(O)-$C_{1-6}$ alkyl, $C_{1-6}$ haloalkyl, $C_{1-6}$ hydroxyalkyl, -C(O)-3- to 10-membered cycloalkyl, -CN and oxo group; and still alternatively, $R_2$ is 4- to 7-membered heterocyclyl, wherein the heteroatom is oxygen atom or nitrogen atom, and the 4- to 7-membered heterocyclyl is optionally substituted with one or more substituents selected from $C_{1-6}$ alkyl and oxo group; and still alternatively, $R_2$ is selected from:

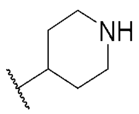, 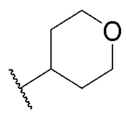, 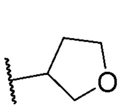, 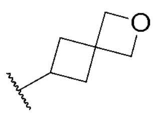, 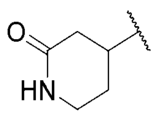, 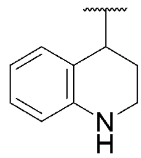, 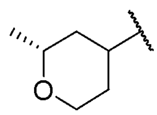,

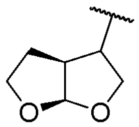, 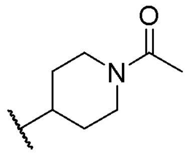, 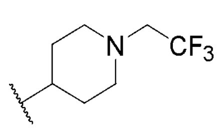, 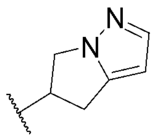, 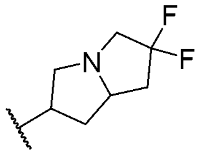, 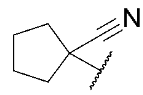,

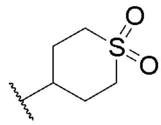, 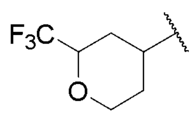, 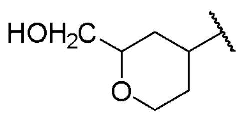 and 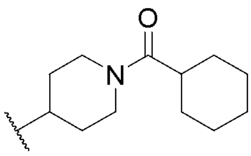.

**Technical Solution 22.** A compound of formula (VI), or a pharmaceutically acceptable salt, an enantiomer, a diastereomer, a solvate, a hydrate, a polymorph or an isotopic variant thereof:

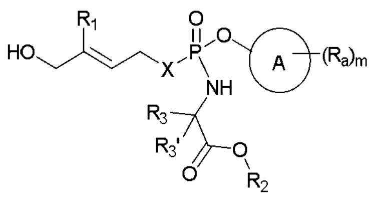

(VI)

wherein,

ring A is $C_{6-12}$ aryl or 5- to 12-membered heteroaryl;
$R_a$ is selected from halogen, $C_{1-6}$ alkyl, $C_{1-6}$ haloalkyl, -OR", NR"R", $C_{2-6}$ alkenyl and $C_{2-6}$ alkynyl;
m=1, 2, 3, 4, or 5;
X is O or CRR';
$R_1$ is selected from H, F, Cl, CN and methyl, wherein the methyl is optionally substituted with 1 to 3 substituents independently selected from halogen, CN, OH and $NH_2$;
$R_2$ is selected from $C_{1-6}$ hydroxyalkyl, $C_{5-6}$ alkyl, $C_{5-6}$ haloalkyl, $C_{5-6}$ alkenyl, $C_{5-6}$ alkynyl, $C_{0-6}$ alkylene-$C_{3-12}$ cycloalkyl, $C_{0-6}$ alkylene-4- to 12-membered heterocyclyl and $C_{0-6}$ alkylene-5- to 10-membered heteroaryl, wherein the $C_{1-6}$ hydroxyalkyl, $C_{5-6}$ alkyl, $C_{5-6}$ haloalkyl, $C_{5-6}$ alkenyl, $C_{5-6}$ alkynyl, $C_{0-6}$ alkylene-$C_{3-12}$ cycloalkyl, $C_{0-6}$ alkylene-4- to 12-membered heterocyclyl and $C_{0-6}$ alkylene-5- to 10-membered heteroaryl are optionally substituted with one or more substituents selected from halogen, $C_{1-6}$ alkyl, $C_{1-6}$ haloalkyl, $C_{1-6}$ hydroxyalkyl, $C_{2-6}$ alkenyl, $C_{2-6}$ alkynyl, 3- to 10-membered cycloalkyl, 4- to 10-membered heterocycloalkyl, $C_{6-10}$ aryl, 5- to 10-membered heteroaryl, -C(O)-$C_{1-6}$ alkyl, -C(O)-$C_{1-6}$ hydroxyalkyl, -C(O)-$C_{1-6}$ haloalkyl, -C(O)-$C_{2-6}$ alkenyl, -C(O)-$C_{2-6}$ alkynyl, -C(O)-3- to 10-membered cycloalkyl, -C(O)-4-to 10-membered heterocycloalkyl,

-C(O)-$C_{6-10}$ aryl, -C(O)-5- to 10-membered heteroaryl, -CN and oxo group;
$R_3$ and $R_3$' are each independently selected from $C_{1-6}$ alkyl, $C_{1-6}$ haloalkyl, $C_{2-6}$ alkenyl and $C_{2-6}$ alkynyl;
wherein,
R and R' are independently selected from H and halogen;
R" is independently selected from H, $C_{1-6}$ alkyl, $C_{1-6}$ haloalkyl, $C_{2-6}$ alkenyl, $C_{2-6}$ alkynyl, $C_{3-7}$ cycloalkyl, 3- to 7-membered heterocyclyl, $C_{6-10}$ aryl and 5- to 10-membered heteroaryl.

**Technical Solution 23.** The compound of Technical Solution 22, or a pharmaceutically acceptable salt, an enantiomer, a diastereomer, a solvate, a hydrate, a polymorph or an isotopic variant thereof, which is a compound of formula (VII):

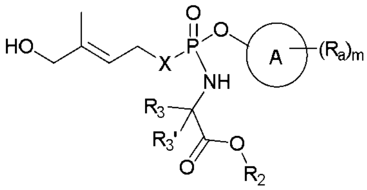

(VII)

wherein,

X is O or CRR';
Ring A is $C_{6-12}$ aryl or 5- to 12-membered heteroaryl;
$R_a$ is selected from halogen, $C_{1-6}$ alkyl, $C_{1-6}$ haloalkyl, -OR", NR"R", $C_{2-6}$ alkenyl and $C_{2-6}$ alkynyl;
m=1, 2, 3, 4, or 5;
X is O or CRR';
$R_2$ is selected from $C_{1-6}$ hydroxyalkyl, $C_{5-6}$ alkyl, $C_{5-6}$ haloalkyl, $C_{5-6}$ alkenyl, $C_{5-6}$ alkynyl, $C_{0-6}$ alkylene-$C_{3-12}$ cycloalkyl, $C_{0-6}$ alkylene-4- to 12-membered heterocyclyl and $C_{0-6}$ alkylene-5- to 10-membered heteroaryl, wherein the $C_{1-6}$ hydroxyalkyl, $C_{5-6}$ alkyl, $C_{5-6}$ haloalkyl, $C_{5-6}$ alkenyl, $C_{5-6}$ alkynyl, $C_{0-6}$ alkylene-$C_{3-12}$ cycloalkyl, $C_{0-6}$ alkylene-4- to 12-membered heterocyclyl and $C_{0-6}$ alkylene-5- to 10-membered heteroaryl are optionally substituted with one or more substituents selected from halogen, $C_{1-6}$ alkyl, $C_{1-6}$ haloalkyl, $C_{1-6}$ hydroxyalkyl, $C_{2-6}$ alkenyl, $C_{2-6}$ alkynyl, 3- to 10-membered cycloalkyl, 4- to 10-membered heterocycloalkyl, $C_{6-10}$ aryl, 5- to 10-membered heteroaryl, -C(O)-$C_{1-6}$ alkyl, -C(O)-$C_{1-6}$ hydroxyalkyl, -C(O)-$C_{1-6}$ haloalkyl, -C(O)-$C_{2-6}$ alkenyl, -C(O)-$C_{2-6}$ alkynyl, -C(O)-3- to 10-membered cycloalkyl, -C(O)-4-to 10-membered heterocycloalkyl, -C(O)-$C_{6-10}$ aryl, -C(O)-5- to 10-membered heteroaryl, -CN and oxo group;
$R_3$ and $R_3$' are each independently selected from $C_{1-6}$ alkyl, $C_{1-6}$ haloalkyl, $C_{2-6}$ alkenyl and $C_{2-6}$ alkynyl;
wherein,
R and R' are independently selected from H and halogen;
R" is independently selected from H, $C_{1-6}$ alkyl, $C_{1-6}$ haloalkyl, $C_{2-6}$ alkenyl, $C_{2-6}$ alkynyl, $C_{3-7}$ cycloalkyl, 3- to 7-membered heterocyclyl, $C_{6-10}$ aryl and 5- to 10-membered heteroaryl.

**Technical Solution 24.** The compound of Technical Solution 22 or 23, or a pharmaceutically acceptable salt, an enantiomer, a diastereomer, a solvate, a hydrate, a polymorph or an isotopic variant thereof, wherein X is CRR', alternatively $CH_2$ or $CF_2$.

**Technical Solution 25.** The compound of any one of Technical Solutions 22-24, or a pharmaceutically acceptable salt, an enantiomer, a diastereomer, a solvate, a hydrate, a polymorph or an isotopic variant thereof, wherein $R_2$ is $C_{1-6}$ alkyl, $C_{0-6}$ alkylene-$C_{3-7}$ cycloalkyl, $C_{0-6}$ alkylene-3- to 7-membered heterocyclyl, $C_{0-6}$ alkylene-$C_{6-10}$ aryl or $C_{0-6}$ alkylene-5- to 10-membered heteroaryl, alternatively $R_2$ is isopropyl, 2-ethylbutyl, cyclopentyl, cyclohexyl, tetrahydrofuranyl, tetrahydrothiophenyl, pyrrolidinyl, tetrahydropyranyl, tetrahydrothiopyranyl, tetrahydropyridinyl or benzyl, and still alternatively $R_2$ is 2-ethylbutyl, cyclopentyl, tetrahydrofuranyl, tetrahydrothiophenyl, pyrrolidinyl or benzyl, and still alternatively $R_2$ is 2-ethylbutyl, cyclopentyl, tetrahydrofuranyl or benzyl.

**Technical Solution 26.** The compound of any one of Technical Solutions 22-25, or a pharmaceutically acceptable salt, an enantiomer, a diastereomer, a solvate, a hydrate, a polymorph or an isotopic variant thereof, wherein $R_3$ is $C_{1-6}$ alkyl, alternatively methyl or isopropyl.

**Technical Solution 27.** The compound of any one of Technical Solutions 22-26, or a pharmaceutically acceptable salt, an enantiomer, a diastereomer, a solvate, a hydrate, a polymorph or an isotopic variant thereof, wherein $R_2$ is 3- to 12-membered cycloalkyl or 4- to 12-membered heterocyclyl, wherein the 3- to 12-membered cycloalkyl or the 4- to 12-membered heterocyclyl is optionally substituted with one or more substituents selected from halogen, $C_{1-6}$ alkyl, $C_{1-6}$

haloalkyl, $C_{1-6}$ hydroxyalkyl, $C_{2-6}$ alkenyl, $C_{2-6}$ alkynyl, 3- to 10-membered cycloalkyl, 4- to 10-membered heterocycloalkyl, $C_{6-10}$ aryl, 5- to 10-membered heteroaryl, -C(O)-$C_{1-6}$ alkyl, -C(O)-$C_{1-6}$ hydroxyalkyl, -C(O)-$C_{1-6}$ haloalkyl, -C(O)-$C_{2-6}$ alkenyl, -C(O)-$C_{2-6}$ alkynyl, -C(O)-3- to 10-membered cycloalkyl, -C(O)-4- to 10-membered heterocycloalkyl, -C(O)-$C_{6-10}$ aryl, -C(O)-5- to 10-membered heteroaryl, -CN and oxo group; alternatively, $R_2$ is 4- to 10-membered heterocyclyl optionally substituted with one or more substituents selected from halogen, $C_{1-6}$ alkyl, -C(O)-$C_{1-6}$ alkyl, $C_{1-6}$ haloalkyl, $C_{1-6}$ hydroxyalkyl, -C(O)-3- to 10-membered cycloalkyl, -CN and oxo group; and still alternatively, $R_2$ is 4- to 7-membered heterocyclyl, wherein the heteroatom is oxygen atom or nitrogen atom, and the 4- to 7-membered heterocyclyl is optionally substituted with one or more substituents selected from $C_{1-6}$ alkyl and oxo group; and still alternatively, $R_2$ is selected from:

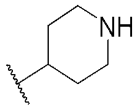, 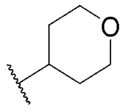, 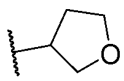, 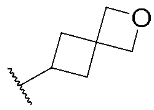, 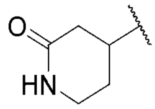, 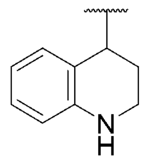, 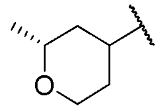,

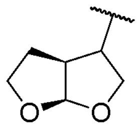, 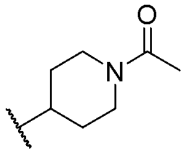, 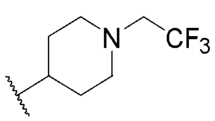, 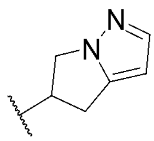, 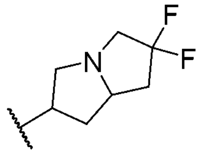, 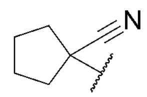,

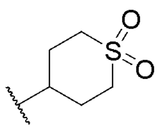, 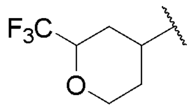, 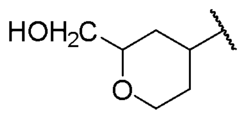 and 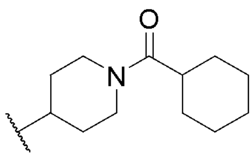.

**Technical Solution 28.** The compound of any one of Technical Solutions 1-27, or a pharmaceutically acceptable salt, an enantiomer, a diastereomer, a solvate, a hydrate, a polymorph or an isotopic variant thereof, wherein the compound is selected from:

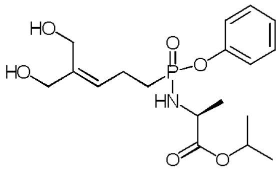, 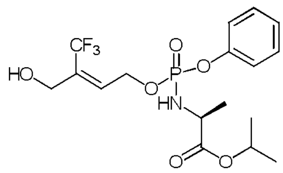, 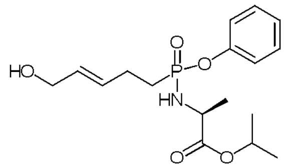,

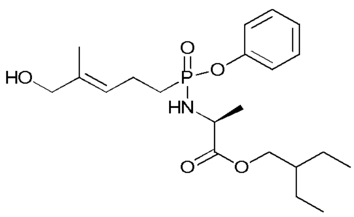, 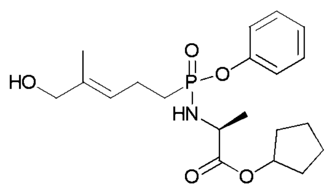, 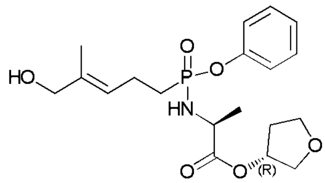,

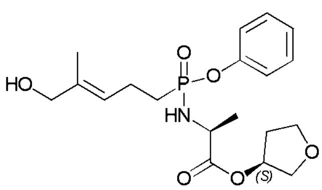, 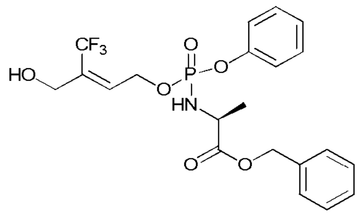, 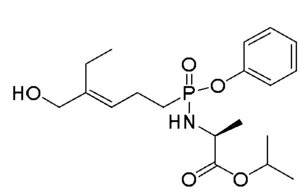,

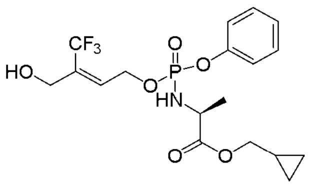,
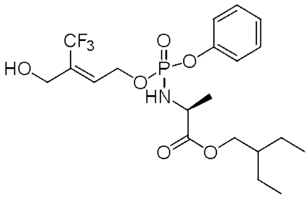,
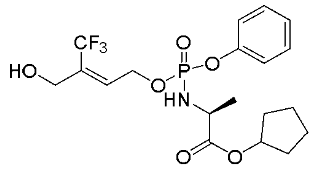,

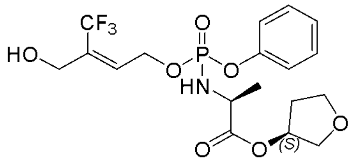,
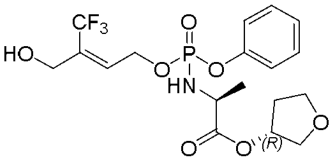,
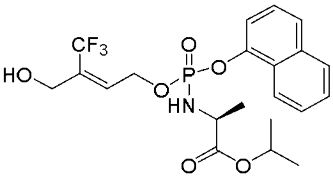,

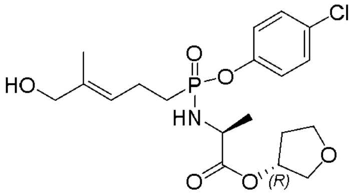,
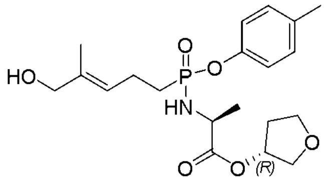,
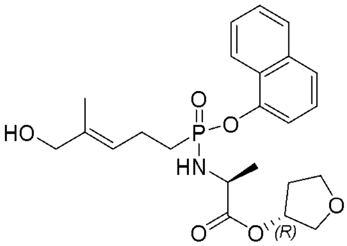,

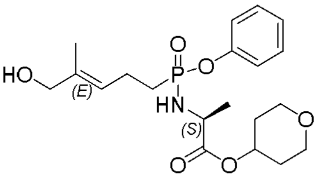,
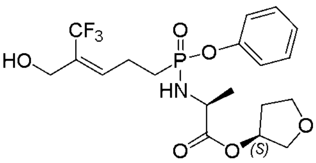,
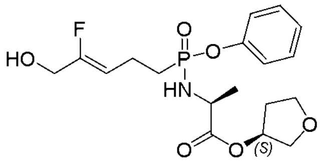,

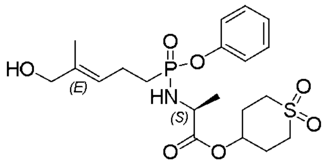,
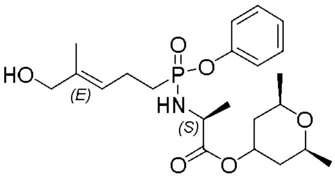,
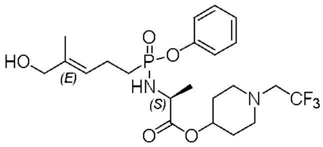,

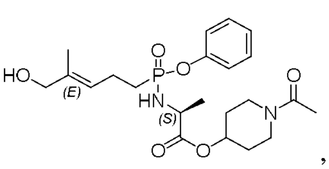,
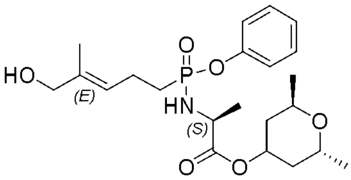,
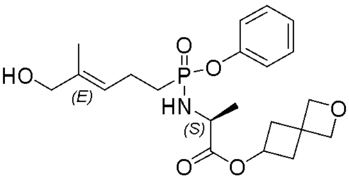,

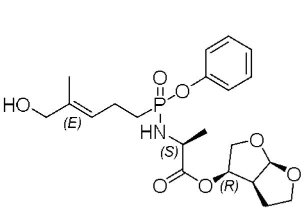,
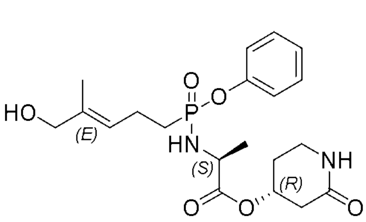,
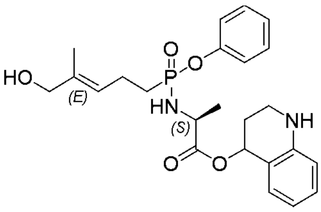,

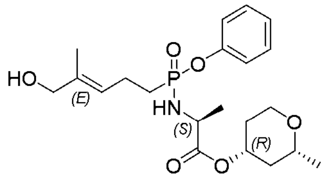,
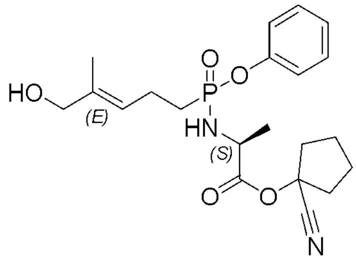,
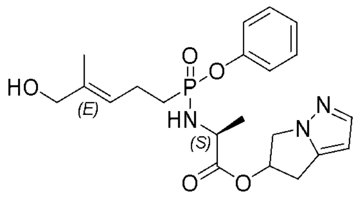,

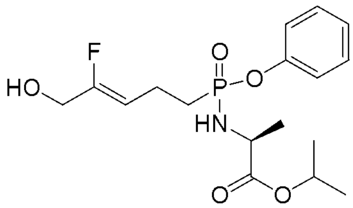,
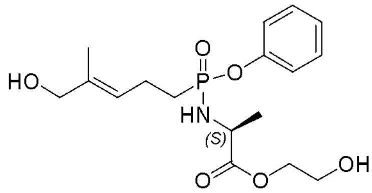,
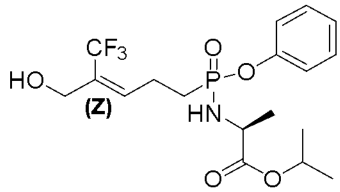,

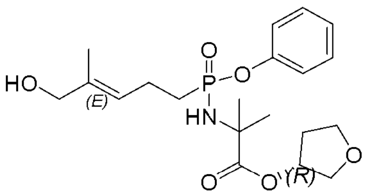,
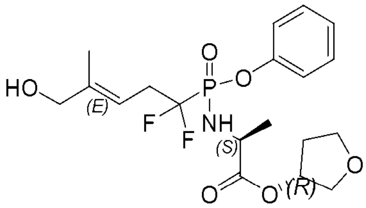,
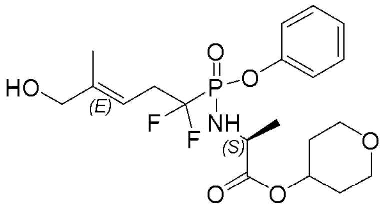,

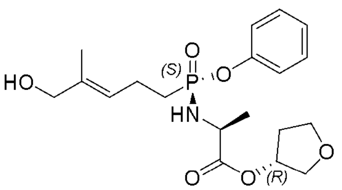,
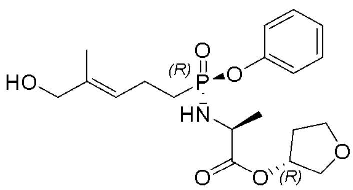,
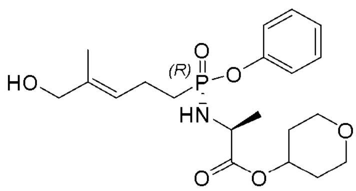

and 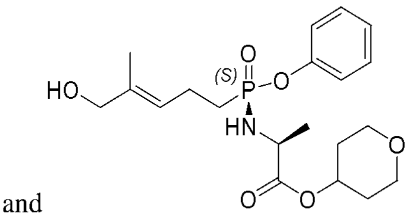.

**Technical Solution 29.** The compound of any one of Technical Solutions 1-28, or a pharmaceutically acceptable salt, an enantiomer, a diastereomer, a solvate, a hydrate, a polymorph or an isotopic variant thereof, wherein the compound is selected from:

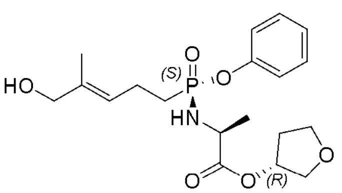,
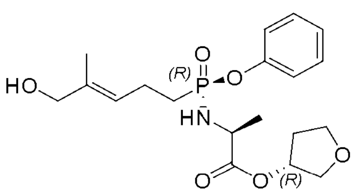,
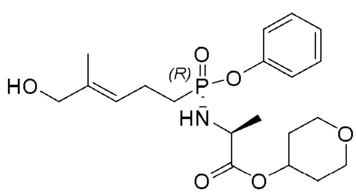

and 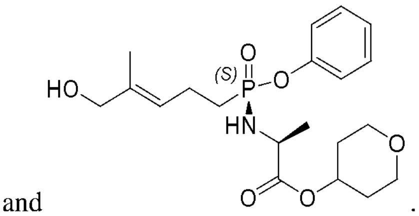 .

**Technical Solution 30.** A pharmaceutical composition comprising the compound of any one of Technical Solutions 1-29, or a pharmaceutically acceptable salt, an enantiomer, a diastereomer, a solvate, a hydrate, a polymorph or an isotopic variant thereof.

**Technical Solution 31.** Use of the compound of any one of Technical Solutions 1-29, or a pharmaceutically acceptable salt, an enantiomer, a diastereomer, a solvate, a hydrate, a polymorph or an isotopic variant thereof, or the pharmaceutical composition of Technical Solution 30 in the manufacture of a medicament for treating a proliferative disease.

**Technical Solution 32.** The compound of any one of Technical Solutions 1-29, or a pharmaceutically acceptable salt, an enantiomer, a diastereomer, a solvate, a hydrate, a polymorph or an isotopic variant thereof, or the pharmaceutical composition of Technical Solution 30, for use in treating a proliferative disease.

**Technical Solution 33.** A method for treating a proliferative disease in a subject, the method comprising administering to the subject the compound of any one of Technical Solutions 1-29, or a pharmaceutically acceptable salt, an enantiomer, a diastereomer, a solvate, a hydrate, a polymorph or an isotopic variant thereof, or the pharmaceutical composition of Technical Solution 14.

**Technical Solution 34.** The use of Technical Solution 31 or the use of the compound or pharmaceutical composition of Technical Solution 32 or the method of Technical Solution 33, wherein the proliferative disease is selected from cancer, cardiovascular disorders, infectious diseases, chronic inflammatory diseases, autoimmune disorders and other cell proliferative disorders; alternatively, wherein the cancer is selected from solid tumors and hematological malignancies, such as breast cancer, neuroblastoma, malignant rhabdomyomas, well-differentiated and dedifferentiated liposarcoma, glioma, lung cancer, colorectal cancer, gastric cancer, gastrointestinal stromal tumor (GIST), hepatocellular carcinoma, prostate tumor, sarcoma, ovarian cancer, cervical cancer, pancreatic cancer, melanoma, thyroid cancer, bile duct cancer, endometrial cancer, renal cancer, mesothelioma, lymphoma, leukemia, non-Hodgkin's lymphoma, mantle cell lymphoma, anaplastic large cell lymphoma, acute myeloid leukemia (AML), and multiple myeloma; alternatively, wherein the proliferative disease is selected from multiple myeloma, non-Hodgkin's lymphoma, lung cancer, renal cancer and prostate cancer.

**Technical Solution 35.** Use of a compound of formula (I), or a pharmaceutically acceptable salt, an enantiomer, a diastereomer, a solvate, a hydrate, a polymorph or an isotopic variant thereof in the manufacture of a medicament for promoting binding of butyrophilin 3A1/2A1:

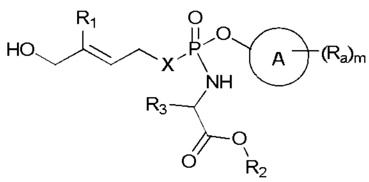

(I)

wherein,

ring A is $C_{6-10}$ aryl or 5- to 10-membered heteroaryl;
$R_a$ is selected from H, halogen, $C_{1-6}$ alkyl, $C_{1-6}$ haloalkyl, -OR", NR"R", $C_{2-6}$ alkenyl and $C_{2-6}$ alkynyl;
m=1, 2, 3, 4, or 5;
X is O or CRR';
$R_1$ is selected from H, F, Cl, CN and methyl, wherein the methyl is optionally substituted with 1 to 3 substituents independently selected from halogen, CN, OH and $NH_2$;
$R_2$ is selected from $C_{1-6}$ alkyl, $C_{1-6}$ haloalkyl, $C_{2-6}$ alkenyl, $C_{2-6}$ alkynyl, $C_{0-6}$ alkylene-$C_{3-7}$ cycloalkyl, $C_{0-6}$ alkylene-3- to 7-membered heterocyclyl, $C_{0-6}$ alkylene-$C_{6-10}$ aryl and $C_{0-6}$ alkylene-5- to 10-membered heteroaryl;
$R_3$ is selected from $C_{1-6}$ alkyl, $C_{1-6}$ haloalkyl, $C_{2-6}$ alkenyl and $C_{2-6}$ alkynyl;
wherein,
R and R' are independently selected from H and halogen;

R" is independently selected from H, $C_{1-6}$ alkyl, $C_{1-6}$ haloalkyl, $C_{2-6}$ alkenyl, $C_{2-6}$ alkynyl, $C_{3-7}$ cycloalkyl, 3- to 7-membered heterocyclyl, $C_{6-10}$ aryl and 5- to 10-membered heteroaryl.

**Technical Solution 36.** A compound of formula (I) or a pharmaceutically acceptable salt, an enantiomer, a diastereomer, a solvate, a hydrate, a polymorph or an isotopic variant thereof for use in promoting binding of butyrophilin 3A1/2A1:

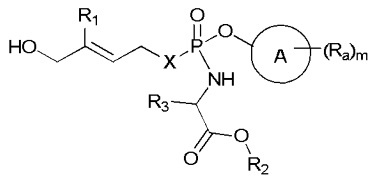

(I)

wherein,

ring A is $C_{6-10}$ aryl or 5- to 10-membered heteroaryl;
$R_a$ is selected from H, halogen, $C_{1-6}$ alkyl, $C_{1-6}$ haloalkyl, -OR", NR"R", $C_{2-6}$ alkenyl and $C_{2-6}$ alkynyl;
m=1, 2, 3, 4, or 5;
X is O or CRR';
$R_1$ is selected from H, F, Cl, CN and methyl, wherein the methyl is optionally substituted with 1 to 3 substituents independently selected from halogen, CN, OH and $NH_2$;
$R_2$ is selected from $C_{1-6}$ alkyl, $C_{1-6}$ haloalkyl, $C_{2-6}$ alkenyl, $C_{2-6}$ alkynyl, $C_{0-6}$ alkylene-$C_{3-7}$ cycloalkyl, $C_{0-6}$ alkylene-3- to 7-membered heterocyclyl, $C_{0-6}$ alkylene-$C_{6-10}$ aryl and $C_{0-6}$ alkylene-5- to 10-membered heteroaryl;
$R_3$ is selected from $C_{1-6}$ alkyl, $C_{1-6}$ haloalkyl, $C_{2-6}$ alkenyl and $C_{2-6}$ alkynyl;
wherein,
R and R' are independently selected from H and halogen;
R" is independently selected from H, $C_{1-6}$ alkyl, $C_{1-6}$ haloalkyl, $C_{2-6}$ alkenyl, $C_{2-6}$ alkynyl, $C_{3-7}$ cycloalkyl, 3- to 7-membered heterocyclyl, $C_{6-10}$ aryl and 5- to 10-membered heteroaryl.

**Technical Solution 37.** A method for promoting binding of butyrophilin 3A1/2A1 in a subject, the method comprising administering to the subject a compound of formula (I), or a pharmaceutically acceptable salt, an enantiomer, a diastereomer, a solvate, a hydrate, a polymorph or an isotopic variant thereof:

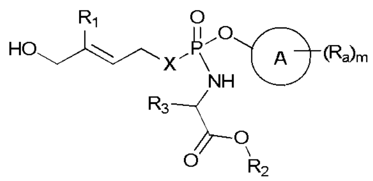

(I)

wherein,

ring A is $C_{6-10}$ aryl or 5- to 10-membered heteroaryl;
$R_a$ is selected from H, halogen, $C_{1-6}$ alkyl, $C_{1-6}$ haloalkyl, -OR", NR"R", $C_{2-6}$ alkenyl and $C_{2-6}$ alkynyl;
m=1, 2, 3, 4, or 5;
X is O or CRR';
$R_1$ is selected from H, F, Cl, CN and methyl, wherein the methyl is optionally substituted with 1 to 3 substituents independently selected from halogen, CN, OH and $NH_2$;
$R_2$ is selected from $C_{1-6}$ alkyl, $C_{1-6}$ haloalkyl, $C_{2-6}$ alkenyl, $C_{2-6}$ alkynyl, $C_{0-6}$ alkylene-$C_{3-7}$ cycloalkyl, $C_{0-6}$ alkylene-3- to 7-membered heterocyclyl, $C_{0-6}$ alkylene-$C_{6-10}$ aryl and $C_{0-6}$ alkylene-5- to 10-membered heteroaryl;
$R_3$ is selected from $C_{1-6}$ alkyl, $C_{1-6}$ haloalkyl, $C_{2-6}$ alkenyl and $C_{2-6}$ alkynyl;
wherein,
R and R' are independently selected from H and halogen;
R" is independently selected from H, $C_{1-6}$ alkyl, $C_{1-6}$ haloalkyl, $C_{2-6}$ alkenyl, $C_{2-6}$ alkynyl, $C_{3-7}$ cycloalkyl, 3- to 7-membered heterocyclyl, $C_{6-10}$ aryl and 5- to 10-membered heteroaryl.

**Technical Solution 38.** The use of Technical Solution 35 or the use of the compound or pharmaceutical composition of Technical Solution 36 or the method of Technical Solution 37, wherein the compound is selected from the compound of any one of Technical Solutions 1-29, or a pharmaceutically acceptable salt, an enantiomer, a diastereomer, a solvate, a hydrate, a polymorph or an isotopic variant thereof.

**Technical Solution 39.** The use of Technical Solution 35 or the use of the compound or pharmaceutical composition of Technical Solution 36 or the method of Technical Solution 37, wherein the compound is selected from the following compounds, or pharmaceutically acceptable salts, enantiomers, diastereomers, solvates, hydrates, polymorphs or isotopic variants thereof:

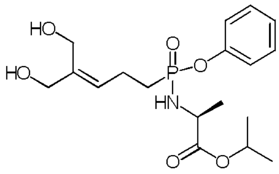 ,
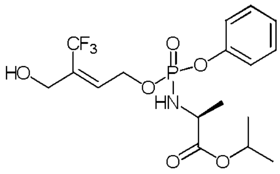 ,
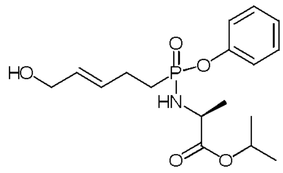 ,

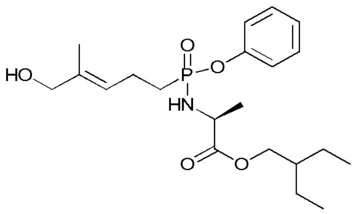 ,
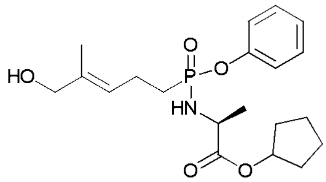 ,
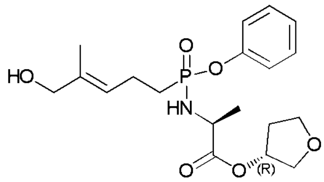 ,

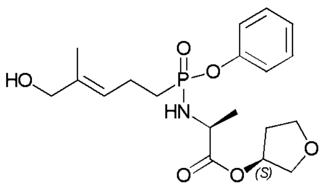 ,
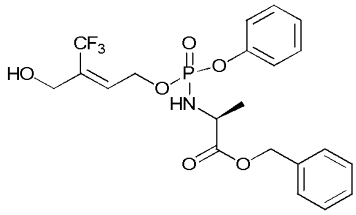 ,
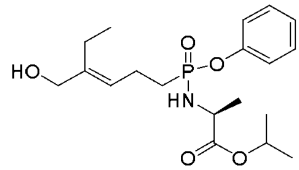 ,

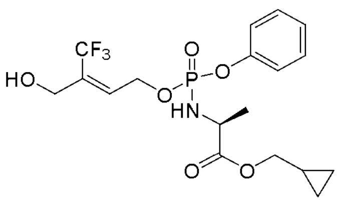 ,
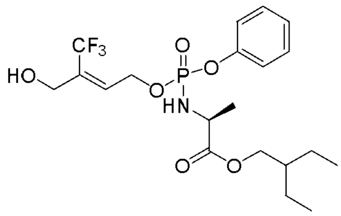 ,
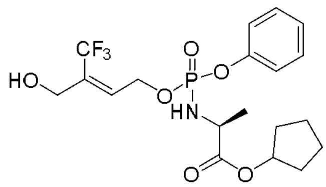 ,

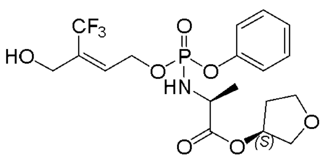 ,
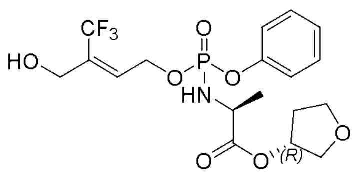 ,
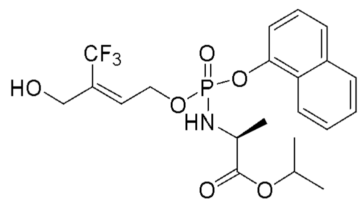 ,

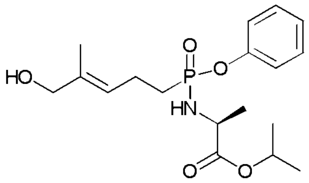 ,
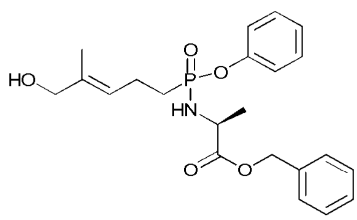 ,
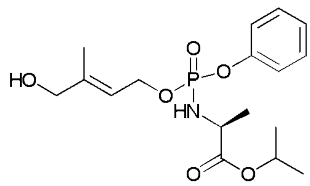 ,

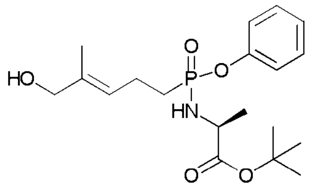 , 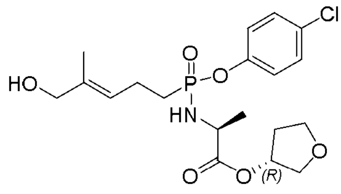 , 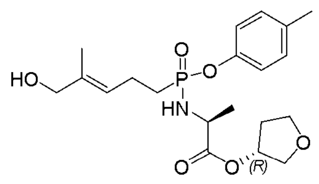 ,

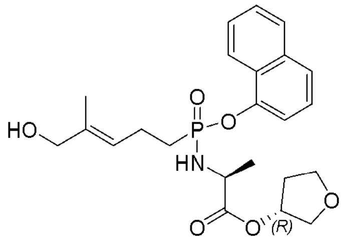 , 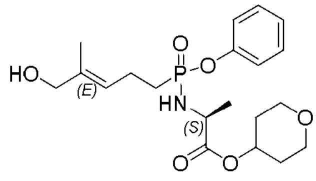 , 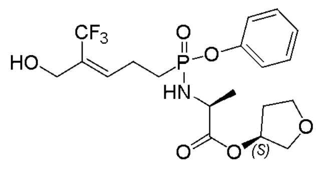 ,

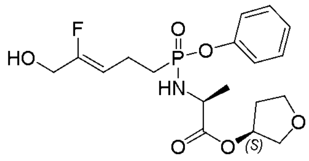 , 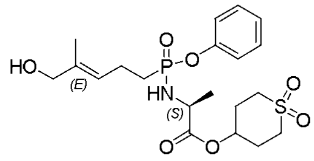 , 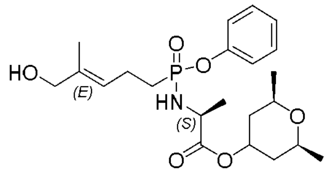 ,

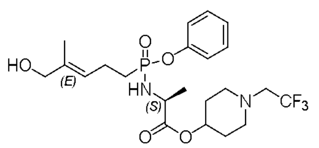 , 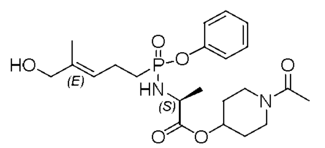 , 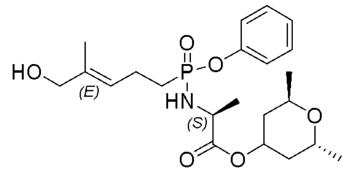 ,

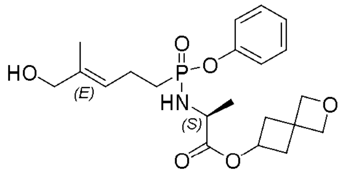 , 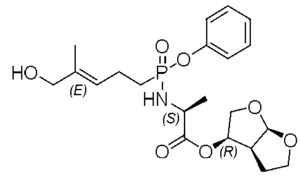 , 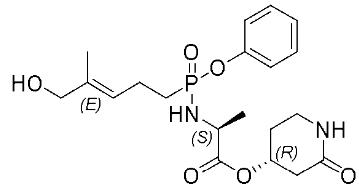 ,

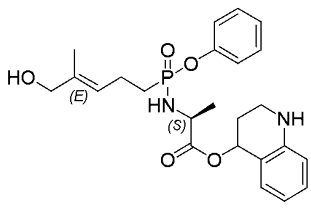 , 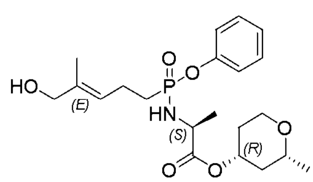 , 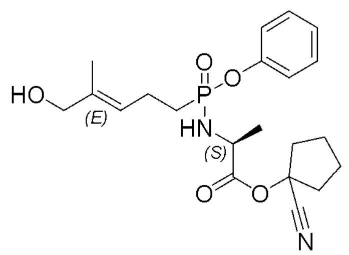 ,

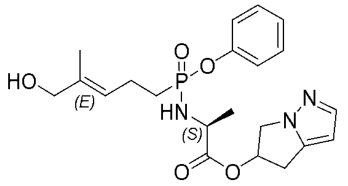 , 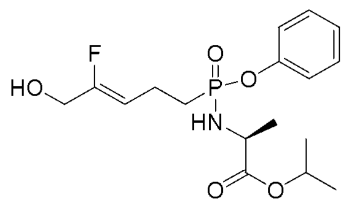 , 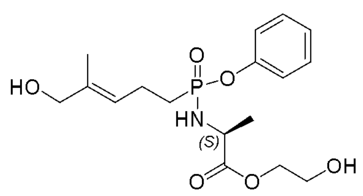 ,

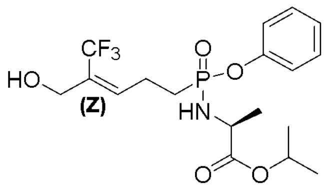, 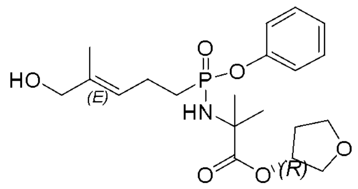, 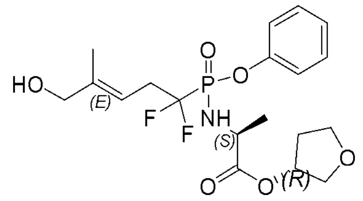,

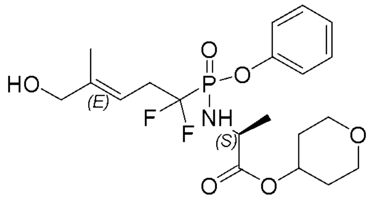, 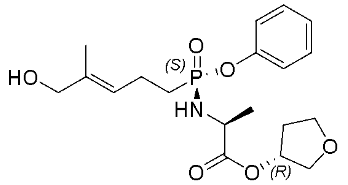, 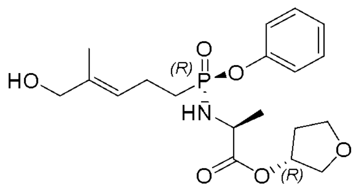,

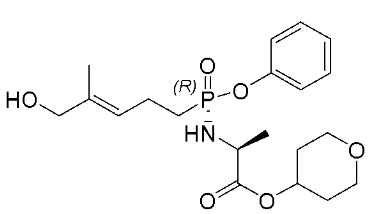 and 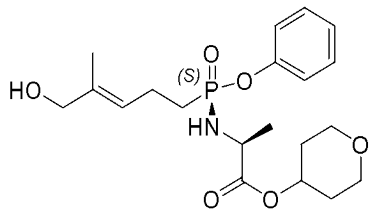.

The compounds of the present disclosure may include one or more asymmetric centers, and thus may exist in a variety of stereoisomeric forms, for example, enantiomers and/or diastereomers. For example, the compounds of the present disclosure may be in the form of an individual enantiomer, diastereomer or geometric isomer (e.g., cis- and trans-isomers), or may be in the form of a mixture of stereoisomers, including racemic mixture and a mixture enriched in one or more stereoisomers. The isomers can be separated from the mixture by the methods known to those skilled in the art, including chiral high-pressure liquid chromatography (HPLC) and the formation and crystallization of chiral salts; or alternative isomers can be prepared by asymmetric synthesis.

The present disclosure also includes all suitable isotopic derivatives of the compounds of this disclosure. The isotopic derivatives of the compounds of this disclosure are defined as derivatives in which at least one atom is replaced by an atom having the same atomic number but an atomic mass different from the atomic mass usually found in nature. Examples of isotopes that can be incorporated into compounds of the disclosure include isotopes of hydrogen, carbon, nitrogen, oxygen, fluorine and chlorine such as $^{2}H$, $^{3}H$, $^{13}C$, $^{14}C$, $^{15}N$, $^{17}O$, $^{18}O$, $^{18}F$, $^{31}P$, $^{32}P$, $^{35}S$ and $^{36}Cl$, respectively. Some isotopic derivatives of the compounds of this disclosure, for example, those in which a radioactive isotope such as $^{3}H$ or $^{14}C$ is incorporated, are useful in drug and/or substrate tissue distribution studies. Tritiated (i.e. $^{3}H$) and carbon-14 (i.e. $^{14}C$) isotopes are yet alternative for their ease of preparation and detectability. In addition, substitution with isotopes (e.g. deuterium, i.e. $^{2}H$) may afford certain therapeutic advantages resulting from greater metabolic stability, for example, increased *in vivo* half-life or reduced dosage requirements and hence may be alternative in some circumstances. Isotopic derivatives of the compounds of the disclosure can generally be prepared by conventional procedures such as by the illustrative methods or by the preparations described in the Examples hereafter using appropriate isotopic derivatives of suitable reagents.

The compounds of the present disclosure or a pharmaceutically acceptable salt thereof may be in an amorphous or a crystalline form. Furthermore, the compounds of the present disclosure may exist in one or more crystalline forms. Therefore, the present disclosure includes all amorphous or crystalline forms of the compounds of the present disclosure within its scope. The term "polymorph" refers to a crystalline form of a compound (or a salt, hydrate or solvate thereof) in a particular crystal packing arrangement. All polymorphs have the same elemental composition. Different crystalline forms generally have different X-ray diffraction patterns, infrared spectra, melting points, density, hardness, crystal shapes, optical and electrical properties, stability, and solubility. Recrystallization solvents, rate of crystallization, storage temperatures, and other factors may cause one crystalline form to dominate. Various polymorphs of a compound can be prepared by crystallization under different conditions.

It will be understood by those skilled in the art that many organic compounds can form complexes with solvents in which they are reacted or from which they are precipitated or crystallized. These complexes are known as "solvates." When the solvent is water, the complex is known as "hydrate." The present disclosure encompasses all solvates of the compounds of the present disclosure.

Pharmaceutical compositions, preparations and kits

In another aspect, the present disclosure provides a pharmaceutical composition comprising a compound of the present disclosure (also referred to as the "active ingredient") and a pharmaceutically acceptable excipient. In certain embodiments, the pharmaceutical composition comprises an effective amount of the active ingredient. In certain embodiments, the pharmaceutical composition comprises a therapeutically effective amount of the active ingredient. In certain embodiments, the pharmaceutical composition comprises a prophylactically effective amount of the active ingredient.

A pharmaceutically acceptable excipient for use in the present disclosure refers to a non-toxic carrier, adjuvant or vehicle which does not destroy the pharmacological activity of the compound formulated together. Pharmaceutically acceptable carriers, adjuvants, or vehicles that may be used in the compositions of the present disclosure include, but are not limited to, ion exchangers, alumina, aluminum stearate, lecithin, serum proteins (e.g., human serum albumin), buffer substances (such as phosphate), glycine, sorbic acid, potassium sorbate, a mixture of partial glycerides of saturated plant fatty acids, water, salt or electrolyte (such as protamine sulfate), disodium hydrogen phosphate, potassium hydrogen phosphate, sodium chloride, zinc salt, silica gel, magnesium trisilicate, polyvinyl pyrrolidone, cellulose-based materials, polyethylene glycol, sodium carboxymethyl cellulose, polyacrylate, wax, polyethylene-polyoxypropylene block polymers, polyethylene glycol and lanolin.

The present disclosure also includes kits (e.g., pharmaceutical packs). Kits provided may include a compound disclosed herein, other therapeutic agents, and a first and a second containers (e.g., vials, ampoules, bottles, syringes, and/or dispersible packages or other suitable containers) containing the compound disclosed herein or other therapeutic agents. In some embodiments, kits provided can also optionally include a third container containing a pharmaceutically acceptable excipient for diluting or suspending the compound disclosed herein and/or other therapeutic agent. In some embodiments, the compound disclosed herein provided in the first container and the other therapeutic agents provided in the second container is combined to form a unit dosage form.

## Examples

The following examples are provided so as to provide those of ordinary skill in the art with a complete disclosure and description of how to perform, prepare and evaluate the methods and compounds claimed herein, and are intended to illustrate the present disclosure only and not to limit the scope of the present disclosure. The present disclosure determines the structure of the compound by nuclear magnetic resonance (NMR) or/and mass spectrometry (MS). The NMR shift (δ) is given in units of $10^{-6}$ (ppm). The NMR was measured on a Bruker AVANCE - 400 NMR spectrometer using deuterated dimethyl sulfoxide (DMSO-$d_6$), deuterated chloroform ($CDCl_3$) and deuterated methanol ($CD_3OD$) as solvents, with tetramethylsilane (TMS) as an internal standard set to zero.

## Preparation of intermediates

### 4-Dichlorophosphorylbut-1-ene(Int-A)

The title compound was prepared according to the following scheme:

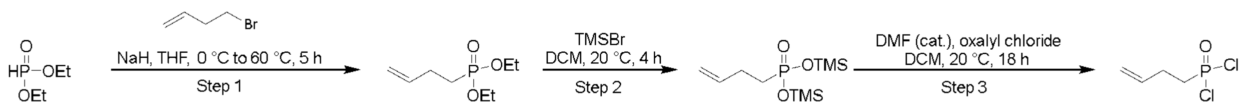


Int-A1 Int-A2 Int-A

### Experimental operation

### Step 1: Diethyl but-3-en-1-ylphosphonate (Int-A1)

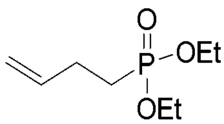

Int-A1

To a solution of sodium hydride (5.21 g, 130.34 mmol, 60% w/w, 1.2 eq) in anhydrous tetrahydrofuran (300 mL) was added diethyl phosphite (15 g, 108.62 mmol, 14.02 mL, 1 eq) dropwise at 0 °C and under the protection of nitrogen, and the mixture was stirred at 20 °C for 1 hour. Subsequently, 4-bromo-1-butene (17.60 g, 130.34 mmol, 13.23 mL, 1.2 eq) was added, and the mixture was stirred at 60 °C for 5 hours. After the reaction was completed, the mixture was cooled to room temperature and quenched by adding a saturated ammonium chloride solution (300 mL). The mixture was extracted with ethyl acetate (300 mL*3). The combined organic layer was dried over anhydrous sodium sulfate and concentrated under reduced pressure to obtain a residue. The residue was purified by column chromatography on silica gel eluting with 0-55% petroleum ether/ethyl acetate to give diethyl 3-butenylphosphonate (18.4 g, 95.74 mmol, 88.14% yield) as a colorless oil.

$^1$H NMR (400 MHz, $CDCl_3$) $\delta$ = 5.82 - 5.75 (m, 1H), 5.02 - 4.93 (m, 2H), 4.06 - 4.00(m, 4H), 2.28 - 2.21 (m, 2H), 1.80 - 1.71 (m, 2H), 1.27 (t, *J* = 7.2 Hz, 6H);
$^{31}$P NMR (162 MHz, $CDCl_3$) $\delta$ = 31.48.

**Step 2: bis(trimethylsilyl) but-3-en-1-ylphosphonate (Int-A2)**

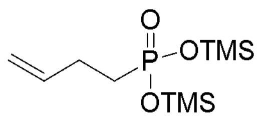

Int-A2

To a solution of diethyl but-3-en-1-ylphosphonate (18.4 g, 95.74 mmol, 1 eq) in dichloromethane (600 mL) was added trimethylsilyl bromide (146.48 g, 957.4 mmol, 10 eq) dropwise at 20 °C and under the protection of nitrogen, and the mixture was stirred at 20 °C for 4 hours. After the reaction was completed, the mixture was concentrated under reduced pressure to remove the solvent, to give bis(trimethylsilyl) but-3-en-1-ylphosphonate (25 g, 93% yield) as a yellow oil, which was used directly in the next step without further treatment.

$^1$H NMR (400 MHz, $CDCl_3$) $\delta$ = 5.82 - 5.75 (m, 1H), 5.04 - 4.95 (m, 2H), 2.30 - 2.25 (m, 2H), 1.86 - 1.80 (m, 2H), 0.27 (s, 18H);
$^{31}$P NMR (162 MHz, $CDCl_3$) $\delta$ = 14.94.

**Step 3: 4-dichlorophosphorylbut-1-ene (Int-A)**

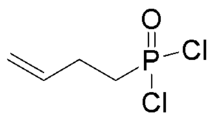

Int-A

Oxalyl chloride (34.0 g, 267.87 mmol, 3 eq) and anhydrous N,N-dimethylformamide (0.4 mL, 8.929 mmol) were added sequentially to a solution of bis(trimethylsilyl) but-3-en-1-ylphosphonate (25 g, 89.29 mmol) in dichloromethane (100 mL) at 0 °C and under the protection of nitrogen. The mixture was stirred at 20°C for 16 hours. After the reaction was completed, the reaction mixture was cooled to room temperature and the solvent was removed by concentration under reduced pressure to give 4-dichlorophosphorylbut-1-ene (14.5 g, 94% yield) as a yellow oil, which was used directly in the next step without further treatment.

**Isopropyl ((but-3-en-1-yl)(phenoxy)phosphoryl)-L-alaninate (Int-B)**

The title compound was prepared according to the following scheme:

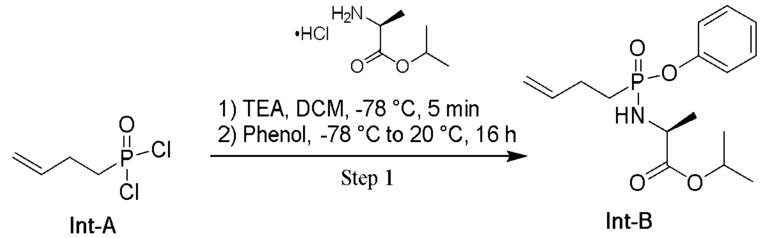


## Experimental operation

### Step 1: isopropyl ((but-3-en-1-yl)(phenoxy)phosphoryl)-L-alaninate (Int-B)

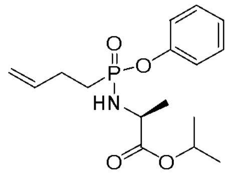

Int-B

To a solution of 4-dichlorophosphorylbut-1-ene (5.0 g, 28.9 mmol, 1 eq) in dichloromethane (200 mL) were added isopropyl L-alaninate hydrochloride (4.85 g, 28.9 mmol, 1 eq) and triethylamine (5.85 g, 57.8 mmol, 2 eq) at -78°C and under the protection of nitrogen. After stirring at -78°C for 5 minutes, phenol (2.72 g, 28.9 mmol, 1 eq) was added to the reaction solution. After stirring at -78°C for 0.5 hours, the mixture was stirred at 20°C for 16 hours. After the reaction was completed, the mixture was filtered, and the filtrate was concentrated under reduced pressure to obtain a residue. The residue was purified by column chromatography on silica gel eluting with 0-60% petroleum ether/ethyl acetate to give isopropyl ((but-3-en-1-yl)(phenoxy)phosphoryl)-L-alaninate (4.5 g, 13.83 mmol, 47.8% yield) as a colorless oil.

MS (ESI) m/z: calcd. 326.1 [M + H]$^+$, found 326.0 [M + H]$^+$.

### (Z)-3-(((tert-butyldimethylsilyl)oxy)methyl)-4,4,4-trifluorobut-2-en-1-ol (Int-C)

The title compound was prepared according to the following scheme:

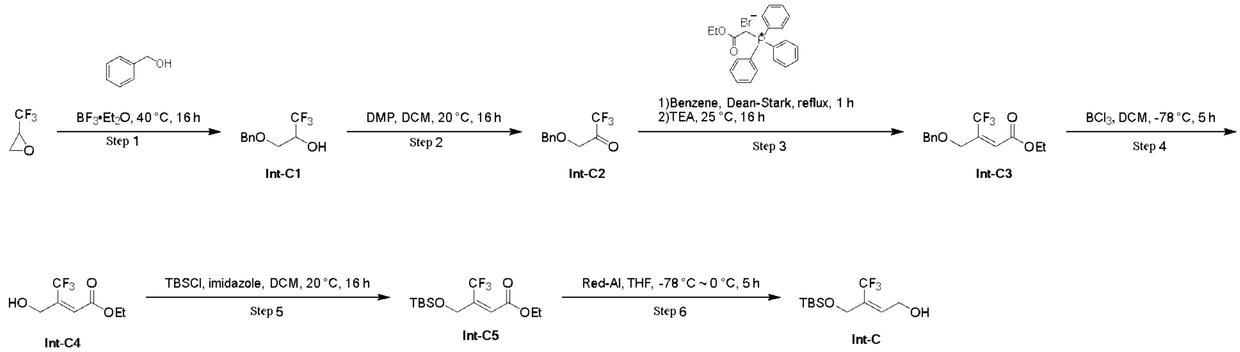


## Experimental operation

### Step 1: 3-(benzyloxy)-1,1,1-trifluoropropan-2-ol (Int-C1)

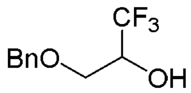

Int-C1

To a reaction solution of benzyl alcohol (19.11 g, 176.71 mmol, 1 eq) and boron trifluoride diethyl ether (501.63 mg, 3.53 mmol, 0.02 eq) was added 1,1,1-trifluoro-2,3-epoxypropane (19.8 g, 176.71 mmol, 1 eq) at room temperature, and the mixture was stirred at 40 °C for 16 hours. After the reaction was completed, the reaction mixture was cooled to room temperature and quenched by adding an appropriate amount of water. The mixture was extracted with dichloromethane (30 mL*3). The combined organic layer was dried over anhydrous sodium sulfate and concentrated under reduced pressure to obtain a residue. The residue was purified by column chromatography on silica gel eluting with 20~40% petroleum ether/ethyl acetate to give 3-(benzyloxy)-1,1,1-trifluoropropan-2-ol (30 g, 136.25 mmol, 77.1% yield) as a yellow oil.

$^1$H NMR (400 MHz, $CDCl_3$) $\delta$ = 7.42 - 7.37 (m, 5H), 4.63 (s, 2H), 4.18 -4.15 (m, 1H), 3.77 - 3.74 (m, 1H), 3.71 - 3.68 (m, 1H), 3.36 (d, *J* = 6.4 Hz, 1H);
$^{19}$F NMR (376 MHz, $CDCl_3$) $\delta$ = -77.61.

**Step 2: 3-(benzyloxy)-1,1,1-trifluoropropan-2-one (Int-C2)**

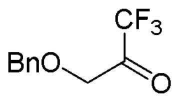

Int-C2

To a solution of 3-(benzyloxy)-1,1,1-trifluoropropan-2-ol (21 g, 95.37 mmol, 1.0 eq) in dichloromethane (50 mL) was added Dess-Martin periodinane DMP (56.63 g, 133.52 mmol, 1.4 eq) dropwise at 20 °C, and the mixture was stirred at 20 °C for 16 hours. After the reaction was completed, the mixture was diluted with dichloromethane, and the organic layer was washed with sodium thiosulfate and sodium bicarbonate solution, respectively, and then the organic layer was dried over anhydrous sodium sulfate and concentrated under reduced pressure to obtain a residue. The residue was purified by column chromatography on silica gel eluting with 20-40% petroleum ether/ethyl acetate to give the desired 3-(benzyloxy)-1,1,1-trifluoropropan-2-one (11.0 g, 50.42 mmol, 52.8% yield) as a colorless oil.

$^1$H NMR (400 MHz, CDCl3) $\delta$ = 7.42 - 7.32 (m, 5H), 4.70 (s, 2H), 3.69 (s, 2H);
$^{19}$F NMR (376 MHz, $CDCl_3$) $\delta$ = -85.13.

**Step 3: ethyl (Z)-3-((benzyloxy)methyl)-4,4,4-trifluorobut-2-enoate (Int-C3)**

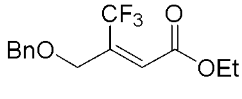

Int-C3

3-(Benzyloxy)-1,1,1-trifluoropropan-2-one (9.0 g, 41.26 mmol, 1 eq) was dissolved in an appropriate amount of benzene under the protection of nitrogen, and the water in the system was removed by reflux. The reaction solution was cooled to 25°C, and ethoxyformylmethyltriphenylphosphonium bromide (21.26 g, 49.51 mmol, 1.2 eq) and triethylamine (9.59 g, 94.90 mmol, 2.3 eq) were added to the reaction system and stirred at 25°C for 16 hours. After the reaction was completed, the reaction was quenched by adding an appropriate amount of water, and the mixture was extracted with ethyl acetate (30 mL*3). The combined organic layer was dried over anhydrous sodium sulfate and concentrated under reduced pressure to obtain a residue. The residue was purified by column chromatography on silica gel eluting with 20-30% petroleum ether/ethyl acetate to give ethyl (Z)-3-((benzyloxy)methyl)-4,4,4-trifluorobut-2-enoate (4.8 g, 16.65 mmol, 40.3% yield) as a colorless oil.

$^1H$ NMR (400 MHz, $CDCl_3$) δ = 7.36 - 7.34 (m, 5H), 6.53 (s, 1H), 4.62 (s, 2H), 4.57 (s, 2H), 4.23 (q, *J* = 7.2 Hz, 2H), 1.30 (t, *J* = 7.2 Hz, 3H);
$^{19}F$ NMR (376 MHz, $CDCl_3$) δ = -67.23.

**Step 4: ethyl (Z)-4,4,4-trifluoro-3-(hydroxymethyl)but-2-enoate (Int-C4)**

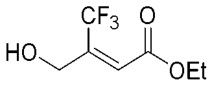

Int-C4

To a solution of ethyl (Z)-3-((benzyloxy)methyl)-4,4,4-trifluorobut-2-enoate (4.8 g, 16.64 mmol, 1 eq) in dichloromethane (80 mL) was added boron trichloride (1.0 M, 94.36 mL, 8.0 eq) dropwise at 0 °C and under the protection of nitrogen, and the mixture was stirred at -78°C for 5 hours. After the reaction was completed, the mixture was diluted with an appropriate amount of dichloromethane, the organic layer was washed with a saturated sodium chloride solution, and then the organic layer was dried over anhydrous sodium sulfate and concentrated under reduced pressure to obtain a residue. The residue was purified by column chromatography on silica gel eluting with 40-60% petroleum ether/ethyl acetate to give ethyl (Z)-4,4,4-trifluoro-3-(hydroxymethyl)but-2-enoate (2.1 g, 10.60 mmol, 63.70% yield) as a colorless oil.

$^1H$ NMR (400 MHz, $CDCl_3$) δ = 6.47 (t, *J* = 1.6 Hz, 1H), 5.01 (s, 1H), 4.35 (d, *J* = 0.8 Hz, 2H), 4.26 (q, *J* = 7.2 Hz, 2H), 1.31 (t, *J* = 7.2 Hz, 3H);
$^{19}F$ NMR (377 MHz, $CDCl_3$) δ = -63.15, -65.07.

**Step 5: ethyl (Z)-3-(((tert-butyldimethylsilyl)oxy)methyl)-4,4,4-trifluorobut-2-enoate (Int-C5)**

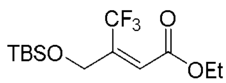

Int-C5

To a mixed solution of ethyl (Z)-4,4,4-trifluoro-3-(hydroxymethyl)but-2-enoate (2.1 g, 10.60 mmol, 1 eq) and imidazole (1.08 g, 15.90 mmol, 1.5 eq) in dichloromethane (25 mL) was added tert-butyldimethylsilyl chloride (2.08 g, 13.78 mmol, 1.3 eq) dropwise at 0°C and under the protection of nitrogen, and the mixture was stirred at 20°C for 16 hours. After the reaction was completed, the reaction mixture was diluted with an appropriate amount of dichloromethane, the organic layer was washed with a saturated sodium chloride solution, and then the organic layer was dried over anhydrous sodium sulfate and concentrated under reduced pressure to obtain a residue. The residue was purified by column chromatography on silica gel eluting with 40-60% petroleum ether/ethyl acetate to give ethyl (Z)-3-(((tert-butyldimethylsilyl)oxy)methyl)-4,4,4-trifluorobut-2-enoate (2.4 g, 7.69 mmol, 72.53% yield) as a colorless oil.

$^1H$ NMR (400 MHz, $CDCl_3$) δ = 6.44 (t, *J* = 2.4 Hz, 1H), 4.32 (s, 2H), 4.26 (q, *J* = 7.2 Hz, 2H), 1.32 (t, *J* = 7.2 Hz, 3H), 0.93 (s, 9H), 0.12 (s, 6H);
$^{19}F$ NMR (376 MHz, $CDCl_3$) δ = -63.16.

**Step 6: (Z)-3-(((tert-butyldimethylsilyl)oxy)methyl)-4,4,4-trifluorobut-2-en-1-ol (Int-C)**

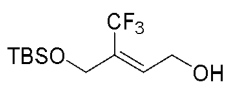

Int-C

To a solution of ethyl (Z)-3-(((tert-butyldimethylsilyl)oxy)methyl)-4,4,4-trifluorobut-2-enoate (2.1 g, 6.73 mmol, 1

eq) in tetrahydrofuran (30 mL) was added red-Al (1.0 M in toluene, 13.46 mL, 2.0 eq) dropwise at -78°C and under the protection of nitrogen, and the mixture was stirred at 0°C for 5 hours. After the reaction was completed, the mixture was diluted with an appropriate amount of dichloromethane, the organic layer was washed with a saturated sodium chloride solution, and then the organic layer was dried over anhydrous sodium sulfate and concentrated under reduced pressure to obtain a residue. The residue was purified by column chromatography on silica gel eluting with 10-20% petroleum ether/ethyl acetate to give (Z)-3-(((tert-butyldimethylsilyl)oxy)methyl)-4,4,4-trifluorobut-2-en-1-ol (0.65 g, 2.41 mmol, 35.81% yield) as a colorless oil.

$^1$H NMR (400 MHz, $CDCl_3$) $\delta$ = 6.23 - 6.20 (m, 1H), 4.46 - 4.45 (m, 2H), 4.25 (s, 2H), 0.94 - 0.93 (m, 9H), 0.11 - 0.10 (m, 6H);
$^{19}$F NMR (376 MHz, $CDCl_3$) $\delta$ = -60.92, -61.00.

**(trimethylsilyl) (E)-(4-methyl-5-((tetrahydro-2H-pyran-2-yl)oxy)pent-3-en-1-yl)phosphonate (Int-D)**

The title compound was prepared according to the following scheme:

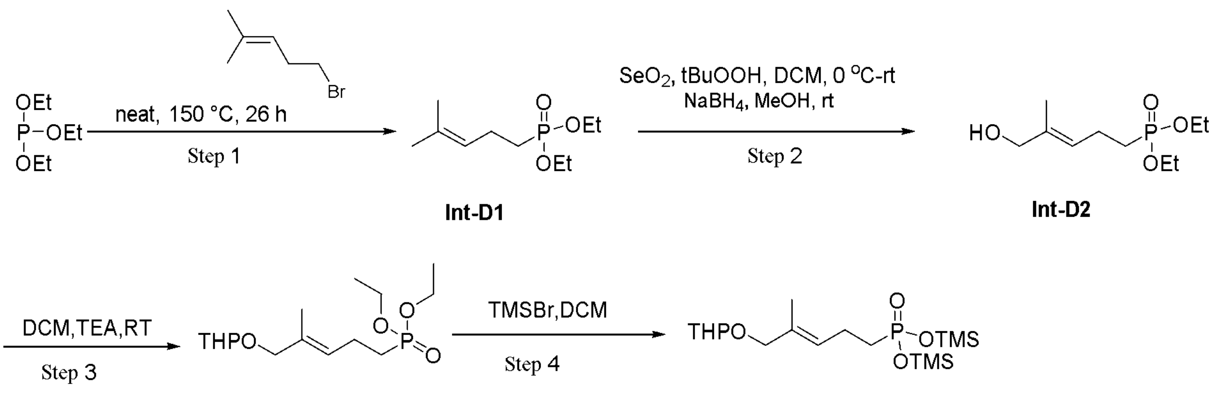


Int-D1 Int-D2 Int-D3 Int-D

**Experimental operation**

**Step 1-1: diethyl (4-methyl-3-en-1-yl)phosphonate (Int-D1)**

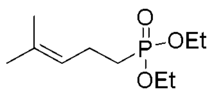

**Int-D1**

To triethyl phosphite (500 mL) was added 5-bromo-2-methyl-2-pentene (50 g, 0.31 mol) at room temperature, and the mixture was refluxed and stirred at 150 °C for 26 hours. After the reaction was completed, the mixture was distilled at 150 °C under atmospheric pressure, and then cooled to 100 °C and distilled under reduced pressure (0.1 Mp), and the temperature was gradually increased to 140 °C until no solvent was evaporated. The residue was obtained as a yellow oily product (38 g, 0.17 mol, 55.72% yield).

MS (ESI) m/z: calcd. 221.1 $[M + H]^+$, found 221.1 $[M + H]^+$.

**Step 2: diethyl (E)-(5-hydroxy-4-methylpent-3-en-1-yl)phosphonate (Int-D2)**

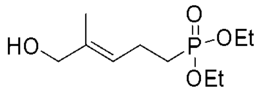

**Int-D2**

To a solution of diethyl (4-methyl-3-en-1-yl)phosphonate (10 g, 52.05 mmol, 1 eq) in dichloromethane (100 mL) were added selenium dioxide (2.89 g, 26.03 mmol, 0.5 eq), 4-hydroxybenzoic acid (0.72 g, 5.21 mmol, 0.1 eq), and tert-butyl hydroperoxide (5.5M aqueous solution, 37.82 mL, 208.20 mmol, 4 eq) at 20 °C and under nitrogen protection, and the mixture was stirred at 20 °C for 16 hours. After the reaction was completed, it was quenched by adding saturated aqueous sodium bicarbonate solution (50 mL) to the reaction system. The mixture was extracted with dichloromethane (100 mL*3), and the combined organic layer was dried over anhydrous sodium sulfate and concentrated under reduced pressure to obtain a residue (10 g, crude product). The crude product was dissolved in methanol (150 mL), and sodium borohydride (3.67 g, 104.10 mmol, 2 eq) was added in batches at 0 °C under nitrogen protection. The mixture was stirred at 0 °C for 2 hours. After the reaction was completed, saturated ammonium chloride solution (200 mL) was added to the mixture, and the mixture was extracted with ethyl acetate (200 mL*3). The combined organic layer was dried over anhydrous sodium sulfate and concentrated under reduced pressure to obtain a residue. The residue was purified by column chromatography on silica gel eluting with 0-10% dichloromethane/methanol, and concentrated under reduced pressure to obtain a crude product of diethyl (E)-(5-hydroxy-4-methylpent-3-en-1-yl)phosphonate (2.8 g, 11.86 mmol, 22.82% yield) as a yellow oil.

MS (ESI) m/z: calcd. 237.1 $[M + H]^+$, found 219.2 $[M + H-H_2O]^+$.

**Step 3: diethyl (E)-(4-methyl-5-((tetrahydro-2H-pyran-2-yl)oxy)pent-3-en-1-yl)phosphonate (Int-D3)**

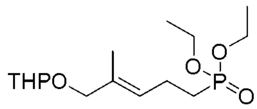

**Int-D3**

To a solution of diethyl (E)-(5-hydroxy-4-methylpent-3-en-1-yl)phosphonate (3 g, 12.70 mmol, 1 eq) and pyridine p-toluenesulfonate (0.34 g, 1.27 mmol, 0.1 eq) in tetrahydrofuran (30 mL) was added 3,4-dihydropyran (3.21 g, 38.10 mmol, 3 eq) at 20°C, and the reaction mixture was stirred at 20°C for 5 hours. After the reaction was completed by TLC detection, the reaction was quenched by adding water (20 ml), and the mixture was extracted with dichloromethane (20 mL*3). The combined organic layer was dried over anhydrous sodium sulfate and concentrated under reduced pressure to obtain a residue. The residue was purified by column chromatography on silica gel eluting with 10-20% petroleum ether/ethyl acetate to give diethyl (E)-(4-methyl-5-((tetrahydro-2H-pyran-2-yl)oxy)pent-3-en-1-yl)phosphonate (2.5 g, 7.81 mmol, 62.5% yield) as a light yellow oil.

MS (ESI) m/z: calcd. 321.2 $[M+H]^+$, found 219.1 $[M+H-102]^+$.

**Step 4: (trimethylsilyl) (E)-(4-methyl-5-((tetrahydro-2H-pyran-2-yl)oxy)pent-3-en-1-yl)phosphonate (Int-D)**

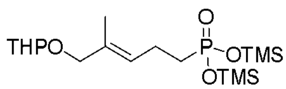

**Int-D**

To a solution of (E)-5-diethoxyphosphoryl-2-methyl-2-pent-1-yl octanoate (2.5 g, 7.81 mmol, 1 eq) in dichloromethane (25 mL) was added trimethylsilyl bromide (11.76 g, 78.10 mmol, 10 eq) dropwise at 0 °C and under nitrogen protection, and the mixture was stirred at room temperature for 16 hours. The reaction was monitored by TLC spot plate (PE:EA = 1:5). After the raw material was disappeared, the mixture was concentrated under reduced pressure to obtain (trimethylsilyl) (E)-(4-methyl-5-((tetrahydro-2H-pyran-2-yl)oxy)pent-3-en-1-yl)phosphonate (crude) (2.5 g, 6.12 mmol, 78.56% yield) as a yellow oil, and the crude product was directly used in the next step.

MS (ESI) m/z: calcd. 409.2 $[M + H]^+$, found 181.1 $[M + H-228]^+$.

**Control compound 1:**

**Isopropyl (((E)-5-hydroxy-4-methylpent-3-en-1-yl)(phenoxy)phosphoryl)-L-alaninate (control compound 1)**

The title compound was prepared according to the following scheme:

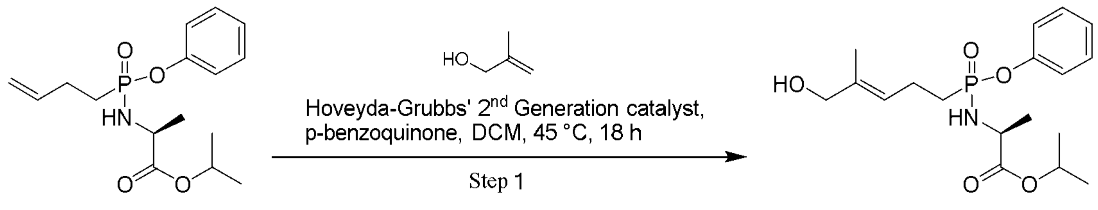


Int-B

Control compound 1

**Experimental operation**

**Step 1: isopropyl (((E)-5-hydroxy-4-methylpent-3-en-1-yl)(phenoxy)phosphoryl)-L-alaninate (control compound 1)**

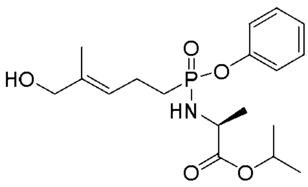

Control compound 1

To a solution of isopropyl ((but-3-en-1-yl)(phenoxy)phosphoryl)-L-alaninate (300.00 mg, 922.12 μmol, 1 eq), 2-methylallyl alcohol (134.97 mg, 1.87 mmol, 2 eq) and p-benzoquinone (12.00 mg, 111.01 μmol, 0.12 eq) in dichloromethane (20 mL) was added Hoveyda-Grubbs' 2nd Generation catalyst (43.35 mg, 69.15 μmol, 0.075 eq). The catalyst was added in three equal portions (14.45 mg, 23.05 μmol, 0.025 eq for each portion) at t = 0, 2, and 4 hours during the reaction. The solution was then heated to reflux for 18 hours at 45°C and under nitrogen atmosphere. After the reaction was completed, the mixture was filtered, and the filtrate was concentrated under reduced pressure to obtain a residue. The residue was purified by preparative HPLC (FA conditions) to give isopropyl (((E)-5-hydroxy-4-methylpent-3-en-1-yl)(phenoxy)phosphoryl)-L-alaninate (127.6 mg, 345.43 μmol, 37.4% yield), the target product, as a colorless oil.

$^1$H NMR (400 MHz, $CDCl_3$) δ = 7.31 - 7.29 (m, 2H), 7.28 - 7.27 (m, 2H), 7.21 - 7.19 (m, 1H), 5.50 - 5.46 (s, 1H), 5.00 - 4.96 (m, 1H), 4.05 - 3.94 (m, 3H), 3.50 - 3.29 (m, 1H), 2.48 - 2.44 (m, 2H), 2.00 - 1.93 (m, 2H), 1.70 (d, *J* = 4.4 Hz, 3H), 1.30 - 1.29 (m, 3H), 1.23 - 1.19 (m, 6H);
$^{31}$P NMR (162 MHz, $CDCl_3$) δ = 31.34, 30.98;
MS (ESI) *m/z:* calcd. 370.2 $[M+H]^+$, found 352.1 $[M+H-H_2O]^+$.

Example 1:

**Isopropyl ((5-hydroxy-4-(hydroxymethyl)pent-3-en-1-yl)(phenoxy)phosphoryl)-L-alaninate (compound 1)**

The title compound was prepared according to the following scheme:

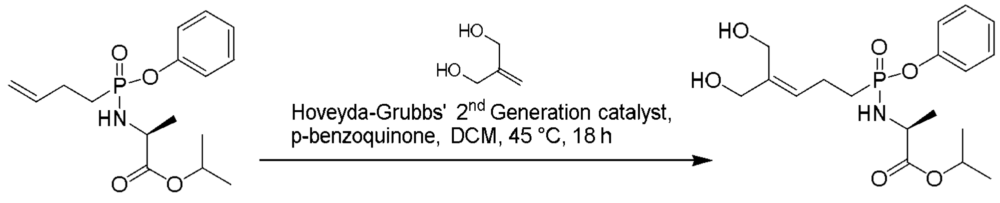


Int-B Compound 1

**Steps for operation:**

**Isopropyl ((5-hydroxy-4-(hydroxymethyl)pent-3-en-1-yl)(phenoxy)phosphoryl)-L-alaninate (compound 1)**

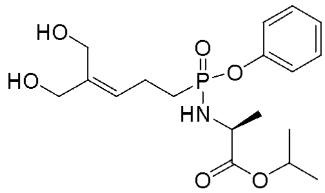

Compound 1

To a solution of isopropyl ((but-3-en-1-yl)(phenoxy)phosphoryl)-L-alaninate (1 g, 3.36 mmol, 1 eq), 2-methylene-1,3-propanediol (541.62 mg, 6.15 mmol, 2 eq) and p-benzoquinone (39.87 mg, 368.85 μmol, 0.12 eq) in dichloromethane (30 mL) was added Hoveyda-Grubbs' 2nd Generation catalyst (144.45 mg, 230.52 μmol, 0.075 eq). The catalyst was added in three equal portions (48.15 mg, 76.84 μmol, 0.025 eq for each portion) at t = 0, 2 and 4 hours during the reaction. The solution was then heated to reflux for 18 hours at 45°C under nitrogen. After the reaction was completed, the mixture was filtered, and the filtrate was concentrated under reduced pressure to obtain a residue. The residue was purified by preparative HPLC (FA conditions) to give isopropyl ((5-hydroxy-4-(hydroxymethyl)pent-3-en-1-yl)(phenoxy) phosphoryl)-L-alaninate (0.04 g, 117.19 μmol, 3.48% yield), the target product, as a colorless oil.

$^1$H NMR (400 MHz, $CDCl_3$) δ = 7.34 - 7.32 (m, 2H), 7.30 - 7.27 (m, 2H), 7.22 - 7.29 (m, 1H), 5.62 - 5.58 (m, 1H), 5.01 - 4.96 (m, 1H), 4.31 - 4.19 (m, 4H), 4.06 - 3.87 (m, 1H), 3.77 - 3.58 (m, 1H), 3.42 - 2.73 (m, 2H), 2.59 - 2.54 (m, 2H), 2.09 - 2.04 (m, 2H), 1.28 - 1.21 (m, 9H);
$^{31}$P NMR (162 MHz, $CDCl_3$) δ = 32.08, 31.86;
MS (ESI) m/z: calcd. 386.2 $[M + H]^+$, found 368.2 $[M + H - H_2O]^+$.

Example 2:

**2-Ethylbutyl (((E)-5-hydroxy-4-methylpent-3-en-1-yl)(phenoxy)phosphoryl)-L-alaninate (compound 2)**

The title compound was prepared according to the following scheme:

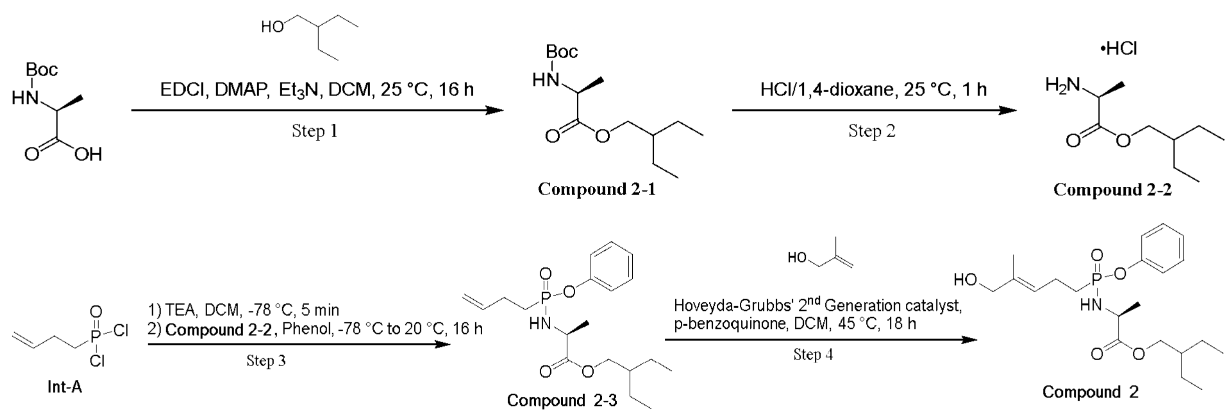


Compound 2-1 Compound 2-2 Int-A Compound 2-3 Compound 2

**Experimental operation**

**Step 1: 2-ethylbutyl (tert-butyloxycarbonyl)-L-alaninate (compound 2-1)**

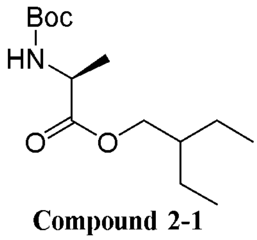

**Compound 2-1**

To a mixed solution of N-tert-butyloxycarbonyl-L-alanine (2.22 g, 11.74 mmol, 1.2 eq) and 2-ethyl-1-butanol (1.0 g, 9.79 mmol, 1 eq) in dichloromethane (50 mL) were added 4-dimethylaminopyridine (1.79 g, 14.68 mmol, 1.5 eq), triethylamine (1.19 g, 11.74 mmol, 1.2 eq) and 1-(3-dimethylaminopropyl)-3-ethylcarbodiimide hydrochloride EDCI (1.97 g, 10.28 mmol, 1.05 eq) at 25 °C, and the mixture was stirred at 25 °C for 16 hours. After the reaction was completed, the reaction was quenched by adding an appropriate amount of water. The mixture was extracted with dichloromethane (20 mL*3). The combined organic layer was dried over anhydrous sodium sulfate and concentrated under reduced pressure to give a residue. The residue was purified by column chromatography on silica gel eluting with 0-40% petroleum ether/ethyl acetate to give 2-ethylbutyl (tert-butyloxycarbonyl)-L-alaninate (1.6 g, 5.85 mmol, 59.80% yield) as a colorless oil.

MS (ESI) *m/z:* calcd. 274.2 $[M+H]^+$, found 296.0 $[M+Na]^+$.

**Step 2: 2-ethylbutyl L-alaninate hydrochloride (compound 2-2)**

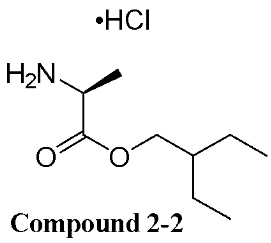

**Compound 2-2**

To a solution of hydrogen chloride in 1,4-dioxane (4 M, 15 mL) at 25 °C was added (tert-butyloxycarbonyl)-L-alanine 2-ethylbutyl ester (1.6 g, 5.85 mmol, 1.0 eq), and the mixture was stirred at 25 ° C for 1 hour. After the reaction was completed, the solvent was removed by concentration under reduced pressure to give 2-ethylbutyl L-alaninate hydrochloride (1.2 g, 98% yield) as a white solid, which was directly used in the next step without further treatment.

MS (ESI) *m/z:* calcd. 174.2 $[M+H]^+$, found 174.2 $[M+H]^+$.

**Step 3: 2-ethylbutyl ((but-3-en-1-yl)(phenoxy)phosphoryl)-L-alaninate (compound 2-3)**

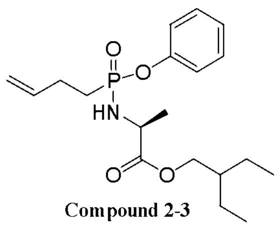

Compound 2-3

To a solution of 4-dichlorophosphorylbut-1-ene (1 g, 5.78 mmol, 1 eq) in dichloromethane (50 mL) was added triethylamine (1.17 g, 11.56 mmol, 2 eq) at -78 °C and under nitrogen protection, and the mixture was stirred at -78 °C for 5

minutes. Then, 2-ethylbutyl L-alaninate hydrochloride (1.20 g, 5.78 mmol, 1 eq) and phenol (0.54 g, 5.78 mmol, 1 eq) were added to the mixture at -78 °C and under nitrogen protection, and the mixture was stirred at 20 °C for 16 hours. After the reaction was completed, the reaction solution was filtered, and the filtrate was concentrated under reduced pressure to remove the solvent to obtain a residue. The residue was purified by column chromatography on silica gel eluting with 10-30% petroleum ether/ethyl acetate to give 2-ethylbutyl ((but-3-en-1-yl)(phenoxy)phosphoryl)-L-alaninate (210 mg, 0.57 mmol, 9.89% yield) as a colorless oil.

MS (ESI) m/z: calcd. 368.2 $[M + H]^+$, found 368.2 $[M + H]^+$.

**Step 4: 2-ethylbutyl (((E)-5-hydroxy-4-methylpent-3-en-1-yl)(phenoxy)phosphoryl)-L-alaninate (compound 2)**

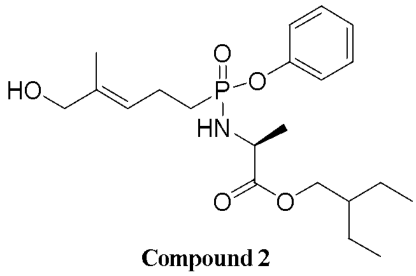

Compound 2

To a solution of 2-ethylbutyl ((but-3-en-1-yl)(phenoxy)phosphoryl)-L-alaninate (0.15 g, 408.25 μmol, 1 eq), 2-methylallyl alcohol (58.87 mg, 816.51 μmol, 2 eq) and p-benzoquinone (5.30 mg, 48.99 μmol, 0.12 eq) in dichloromethane (50 mL) was added Hoveyda-Grubbs' 2nd Generation catalyst (19.17 mg, 30.63 μmol, 0.075 eq). The catalyst was added in three equal portions (6.39 mg, 10.21 μmol, 0.025 eq for each portion) at t=0, 2 and 4 hours during the reaction. The solution was then heated to reflux at 45°C under nitrogen atmosphere for 18 hours. After the reaction was completed, the mixture was filtered and the filtrate was concentrated in vacuo to obtain a residue. The residue was purified by preparative HPLC (FA conditions) to give 2-ethylbutyl (((E)-5-hydroxy-4-methylpent-3-en-1-yl)(phenoxy)phosphoryl)-L-alaninate (8.8 mg, 21.40 μmol, 5.24% yield), the target product, as a colorless oil.

$^1H$ NMR (400 MHz, $CDCl_3$) δ = 7.33 - 7.31 (m, 2H), 7.30 - 7.29 (m, 2H), 7.22 - 7.14 (m, 1H), 5.48 (q, *J* = 7.2 Hz, 1H), 4.15 - 3.98 (m, 5H), 3.48 - 3.28 (m, 1H), 2.50 - 2.46 (m, 2H), 2.06 - 1.89 (m, 2H), 1.71 (d, *J* = 4.4 Hz, 3H), 1.54 - 1.44 (m, 1H), 1.35 - 1.20 (m, 7H), 0.88 (t, *J* = 7.2 Hz, 6H);
$^{31}P$ NMR (162 MHz, $CDCl_3$) δ = 31.53, 31.15;
MS (ESI) *m/z:* calcd. 412.2 $[M+H]^+$, found 412.2 $[M+H]^+$.

Example 3:

**Isopropyl**

**((phenoxy)(((Z)-4,4,4-trifluoro-3-(hydroxymethyl)but-2-en-1-yl)oxy)phosphoryl)-L-alaninate (compound** 3)

The title compound was prepared according to the following scheme:

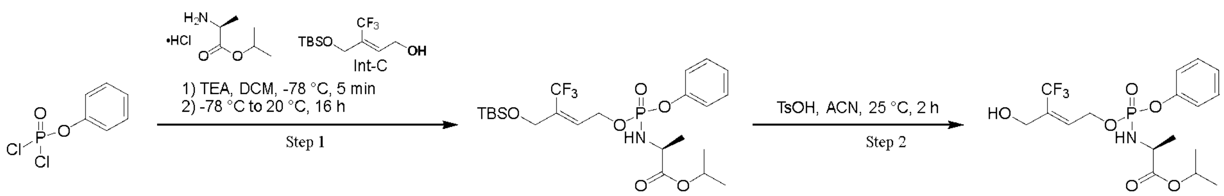


Compound 3-1

Compound 3

**Experimental operation**

**Step 1: isopropyl ((((Z)-3-(((tert-butyldimethylsilyl)oxy)methyl)-4,4,4-trifluorobut-2-en-1-yl)oxy)(phenoxy)phosphoryl)-L-alaninate (compound 3-1)**

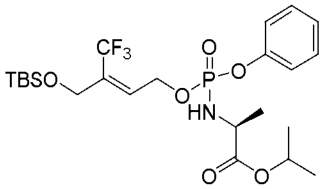

Compound 3-1

To a solution of phenyl dichlorophosphate (300 mg, 1.43 mmol, 1 eq) in dichloromethane (5 mL) was added triethylamine (289.20 mg, 2.86 mmol, 2 eq) at -78°C and under nitrogen protection, and the mixture was stirred at -78 °C for 5 minutes. Subsequently, isopropyl L-alaninate hydrochloride (238.91 mg, 1.43 mmol, 1 eq) and (Z)-3-(((tert-butyldimethylsilyl)oxy)methyl)-4,4,4-trifluorobut-2-en-1-ol (386.29 mg, 1.43 mmol, 1 eq) were added, and the mixture was stirred at 20°C for 16 hours. After the reaction was completed, the mixture was filtered, and the filtrate was concentrated under reduced pressure to obtain a residue. The residue was purified by preparative HPLC ($NH_4HCO_3$ conditions) to give isopropyl ((((Z)-3-(((tert-butyldimethylsilyl)oxy)methyl)-4,4,4-trifluorobut-2-en-1-yl)oxy)(phenoxy)phosphoryl)-L-alani nate (21.8 mg, 40.43 μmol, 2.83% yield) as a colorless oil.

$^1$H NMR (400 MHz, CDCl3) δ = 7.35 - 7.31 (m, 2H), 7.24 - 7.16 (m, 3H), 6.20 (q, J = 6.0 Hz, 1H), 5.05 - 4.98 (m, 1H), 4.91 - 4.90 (m, 2H), 4.24 (s, 2H), 4.01 - 3.96 (m, 1H), 3.60 (t, J = 10.4 Hz, 1H), 1.38 (dd, J = 6.8, 4.4 Hz, 3H), 1.26 - 1.21 (m, 6H), 0.92 (s, 9H), 0.09 (s, 6H);
$^{31}$P NMR (162 MHz, $CDCl_3$) δ = 2.38;
$^{19}$F NMR (376 MHz, $CDCl_3$) δ = -61.40;
MS (ESI) *m/z:* calcd. 540.1 $[M+H]^+$, found 540.1 $[M+H]^+$.

**Step 2: isopropyl ((phenoxy)(((Z)-4,4,4-trifluoro-3-(hydroxymethyl)but-2-en-1-yl)oxy)phosphoryl)-L-alaninate (compound 3)**

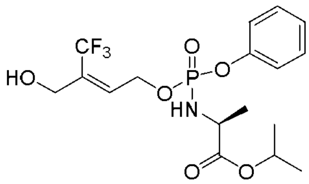

Compound 3

To a solution of isopropyl ((((Z)-3-(((tert-butyldimethylsilyl)oxy)methyl)-4,4,4-trifluorobut-2-en-1-yl)oxy)(phenoxy)phosphoryl)-L-alani nate (10.0 mg, 18.53 μmol, 1.0 eq) in acetonitrile (3 mL) was added p-toluenesulfonic acid (6.38 mg, 37.06 μmol, 2.0 eq), and the mixture was stirred at 25 ° C for 2 hours. After the reaction was completed, the mixture was filtered, and the filtrate was concentrated under reduced pressure to obtain a residue. The residue was purified by preparative HPLC ($NH_4HCO_3$ conditions) to give the desired product, isopropyl ((phenoxy)(((Z)-4,4,4-trifluoro-3-(hydroxymethyl)but-2-en-1-yl)oxy)phosphoryl)-L-alaninate (2.7 mg, 6.35 μmol, 34.27% yield) as a colorless oil.

$^1$H NMR (400 MHz, $CDCl_3$) δ = 7.36 - 7.32 (m, 2H), 7.23 - 7.18 (m, 3H), 6.27 - 6.21 (m, 1H), 5.05 - 4.99 (m, 1H), 4.91 - 4.87 (m, 2H), 4.24 (s, 2H), 4.01 - 3.95 (m, 1H), 3.68 - 3.60 (m, 1H), 2.63 (s, 1H), 1.37 (t, *J* = 7.2 Hz, 3H), 1.25 - 1.22 (m, 6H);
$^{31}$P NMR (162 MHz, $CDCl_3$) δ = 2.56, 2.38;
$^{19}$F NMR (376 MHz, $CDCl_3$) δ = 61.20;
MS (ESI) *m/z:* calcd. 426.1 $[M + H]^+$, found 426.0 $[M + H]^+$.

Example 4:

**Isopropyl (((E)-5-hydroxy-4-ethylpent-3-en-1-yl)(phenoxy)phosphoryl)-L-alaninate (compound 4)**

The title compound was prepared according to the following scheme:

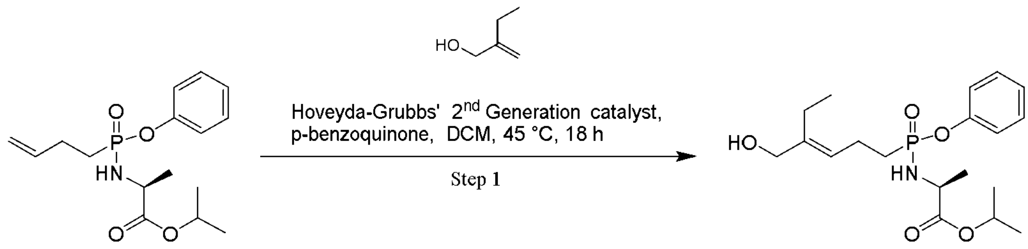


Int-B

Compound 4

**Experimental operation**

**Step 1: isopropyl (((E)-5-hydroxy-4-ethylpent-3-en-1-yl)(phenoxy)phosphoryl)-L-alaninate (compound 4)**

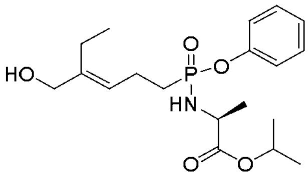

Compound 4

To a solution of isopropyl (but-3-en-1-yl(phenoxy)phosphoryl)-L-alaninate (300 mg, 0.92 mmol, 1 eq), 2 -methylenebutan-1-ol (237.72 mg, 2.76 mmol, 3 eq) and p-benzoquinone (11.89 mg, 0.11 mmol, 0.12 eq) in dichloromethane (10 mL) was added Hoveyda-Grubbs' 2nd Generation catalyst (43.2 mg, 69 μmol, 0.075 eq). The catalyst was added in three equal portions (14.4 mg, 23 μmol, 0.025 eq for each portion) at t=0, 2 and 4 hours during the reaction. The solution was then heated to reflux at 45 °C under nitrogen atmosphere for 18 hours. After the reaction was completed, the mixture was filtered and the filtrate was concentrated under reduced pressure to obtain a residue. The residue was purified by preparative HPLC ($NH_4HCO_3$ conditions) to give isopropyl (((E)-5-hydroxy-4-ethylpent-3-en-1-yl)(phenoxy)phosphoryl)-L-alaninate (2.8 mg, 7.30 μmol, 7.93% yield), the target product, as a colorless oil.

$^1$H NMR (400 MHz, $CDCl_3$) δ = 7.34 - 7.30 (m, 2H), 7.22 (d, *J* = 7.6 Hz, 2H), 7.16 - 7.14 (m, 1H), 5.45 - 5.42 (m, 1H), 5.02 - 4.96 (m, 1H), 4.07 (s, 2H), 3.44 - 3.27 (m, 1H), 2.50 - 2.45 (m, 2H), 2.18 - 2.14 (m, 2H), 2.00 - 1.89 (m, 2H), 1.32 - 1.20 (m, 3H), 1.25 - 1.23 (m, 6H), 1.04 - 1.00 (m, 3H);
$^{31}$P NMR (162 MHz, $CDCl_3$): δ 31.40, 30.99;
MS (ESI) m/z: calcd. 384.2 $[M+H]^+$, found 384.2 $[M+H]^+$.

Example 5:

**Isopropyl (((E)-5-hydroxypent-3-en-1-yl)(phenoxy)phosphoryl)-L-alaninate (compound 5)**

The title compound was prepared according to the following scheme:

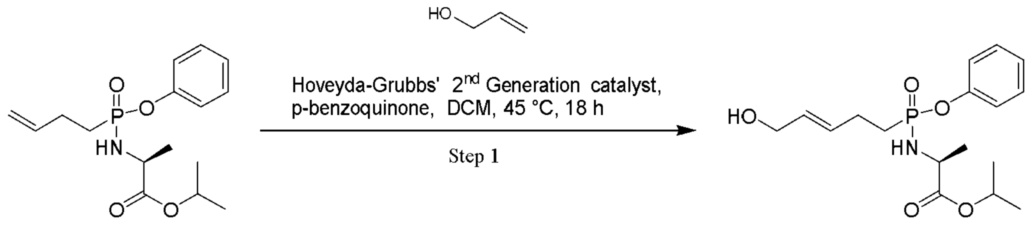


Int-B

Compound 5

## Experimental operation

**Step 1: isopropyl (((E)-5-hydroxypent-3-en-1-yl)(phenoxy)phosphoryl)-L-alaninate (compound 5)**

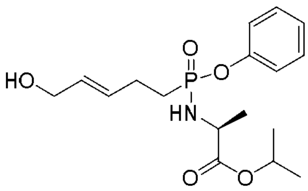

Compound 5

To a solution of isopropyl ((but-3-en-1-yl)(phenoxy)phosphoryl)-L-alaninate (200 mg, 0.62 mmol, 1 eq), allyl alcohol (83.90 mg, 1.45 mmol, 2.35 eq) and p-benzoquinone (8.04 mg, 0.74 mmol, 0.12 eq) in dichloromethane (10 mL) was added Hoveyda-Grubbs' 2nd Generation catalyst (29.16 mg, 46.5 μmol, 0.075 eq). The catalyst was added in three equal portions (9.72 mg, 15.5 μmol, 0.025 eq for each portion) at t=0, 2 and 4 hours during the reaction. The solution was then heated to reflux at 45 °C and under nitrogen atmosphere for 18 hours. After the reaction was completed, the mixture was filtered and the filtrate was concentrated under reduced pressure to obtain a residue. The residue was purified by preparative HPLC (FA conditions) to give isopropyl (((E)-5-hydroxypent-3-en-1-yl)(phenoxy)phosphoryl)-L-alaninate (16.8 mg, 47.30 μmol, 7.63% yield), the target product, as a colorless oil.

$^1$H NMR (400 MHz, $CDCl_3$) δ = 7.34 - 7.30 (m, 2H), 7.23 (d, *J* = 8.4 Hz, 2H), 7.28 - 7.14 (m, 1H), 5.77 - 5.75 (m, 2H), 4.98 (dt, *J* = 12.4, 6.4 Hz, 1H), 4.12 (d, *J* = 2.4 Hz, 2H), 4.00 - 3.93 (m, 1H), 3.41 - 3.36 (m, 1H), 2.49 - 2.45 (m, 2H), 2.01 - 1.97 (m, 2H), 1.30 (d, *J* = 7.2 Hz, 3H), 1.24 - 1.20 (m, 6H);
$^{31}$P NMR (162 MHz, $CDCl_3$) δ = 30.73;
MS (ESI) m/z: calcd. 356.1 $[M+H]^+$, found 338.1 $[M+H-H_2O]^+$.

Example 6:

**Cyclopentyl (((E)-5-hydroxy-4-methylpent-3-en-1-yl)(phenoxy)phosphoryl)-L-alaninate (compound 6)**

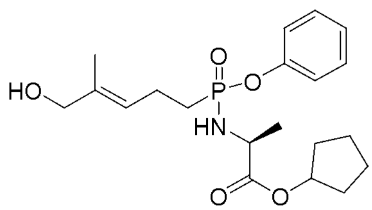

Compound 6

Example 6 was synthesized using benzyloxycarbonyl-L-alanine and cyclopentanol as raw materials by the same method as described in Example 2.

$^1$H NMR (400 MHz, $CDCl_3$) δ = 7.34 - 7.30 (m, 2H), 7.21 (d, *J* = 8.4 Hz, 2H), 7.16 - 7.12 (m, 1H), 5.47 (t, *J* = 7.2 Hz, 1H),

5.17 - 5.13 (m, 1H), 4.02 (s, 2H), 3.99 - 3.93 (m, 1H), 3.43 - 3.27 (m, 1H), 2.53 - 2.39 (m, 2H), 2.00 - 1.92 (m, 2H), 1.89 - 1.79 (m, 2H), 1.73 - 1.70 (m, 3H), 1.68 - 1.64 (m, 6H), 1.31-1.18 (m, 3H);
$^{31}$P NMR (162 MHz, $CDCl_3$) δ = 31.51, 31.11;
MS (ESI) *m/z:* calcd. 396.2 $[M+H]^+$, found 396.4 $[M+H]^+$.

Example 7:

**(R)-Tetrahydrofuran-3-yl**

**(((E)-5-hydroxy-4-methylpent-3-en-1-yl)(phenoxy)phosphoryl)-L-alaninate (compound 7)**

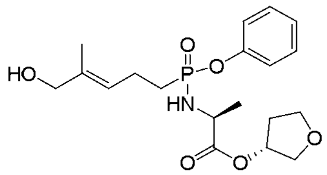

Compound 7

Example 7 was synthesized using benzyloxycarbonyl-L-alanine and (R)-tetrahydrofuran-3-ol as raw materials by the same method as described in Example 2.

$^1$H NMR (400 MHz, $CDCl_3$) δ = 7.32 - 7.29 (m, 2H), 7.22 - 7.20 (m, 2H), 7.19 - 7.14 (m, 1H), 5.53 - 5.42 (m, 1H), 5.33 - 5.20 (m, 1H), 4.14 - 4.02 (m, 1H), 4.01 (s, 2H), 3.92 - 3.79 (m, 3H), 3.76 - 3.67 (m, 1H), 3.44 - 3.22 (m, 1H), 2.54 - 2.39 (m, 2H), 2.22 - 2.10 (m, 1H), 2.04 - 1.89 (m, 3H), 1.71 - 1.68 (m, 3H), 1.33 - 1.20 (m, 3H);
$^{31}$P NMR (162 MHz, $CDCl_3$) δ = 31.40, 31.00;
MS (ESI) m/z: calcd. 398.1 $[M+H]^+$, found 380.1 $[M+H-H_2O]^+$.

Example 8:

**(S)-tetrahydrofuran-3-yl**

**(((E)-5-hydroxy-4-methylpent-3-en-1-yl)(phenoxy)phosphoryl)-L-alaninate (compound 8)**

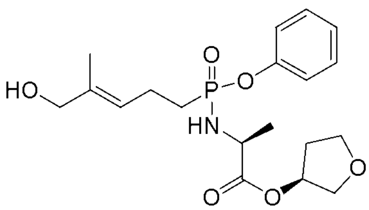

Compound 8

Example 8 was synthesized using benzyloxycarbonyl-L-alanine and (S)-tetrahydrofuran-3-ol as raw materials by the same method as described in Example 2.

$^1$H NMR (400 MHz, $CDCl_3$) δ = 7.34 - 7.29 (m, 2H), 7.21 - 7.19 (m, 2H), 7.16 - 7.14 (m, 1H), 5.50 - 5.45 (m, 1H), 5.25 (t, *J* = 5.2 Hz, 1H), 4.14 - 4.02 (m, 1H), 4.00 (s, 2H), 3.90 - 3.84 (m, 3H), 3.83 - 3.74 (m, 1H), 3.45 - 3.25 (m, 1H), 2.45 (*J* = 14.8, 7.6 Hz, 2H), 2.22 - 2.12 (m, 1H), 2.01 - 1.91 (m, 3H), 1.70 (d, *J* = 4.0 Hz, 3H), 1.33 - 1.21 (m, 3H);
$^{31}$P NMR (162 MHz, $CDCl_3$) δ = 31.40, 31.00;
MS (ESI) m/z: calcd. 398.1 $[M+H]^+$, found 380.1 $[M+H-H_2O]^+$.

Example 9:

**Cyclopropylmethyl**

**((phenoxy)(((Z)-4,4,4-trifluoro-3-(hydroxymethyl)but-2-en-1-yl)oxy)phosphoryl)-L-alaninate (compound 9)**

The title compound was prepared according to the following scheme:

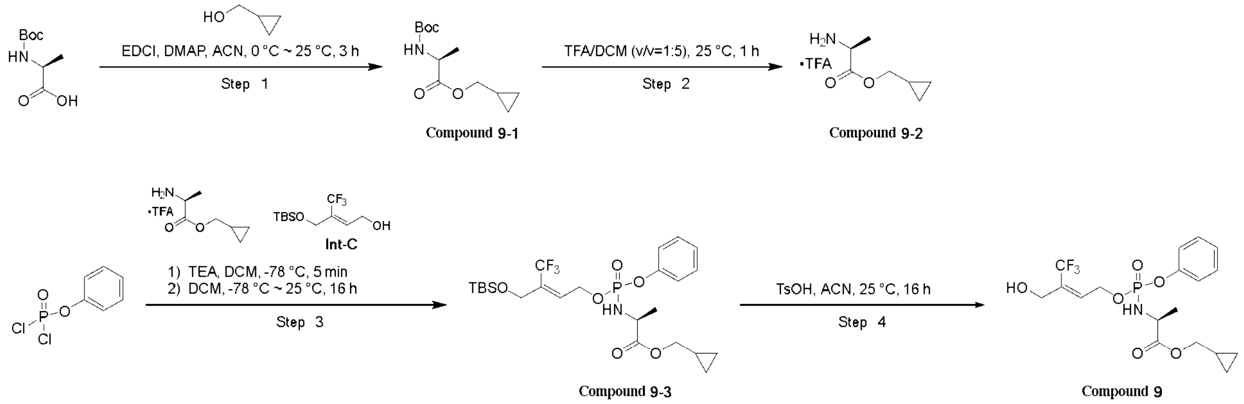

Compound 9-1
Compound 9-2
Compound 9-3
Compound 9

**Experimental operation**

**Step 1: cyclopropylmethyl (tert-butyloxycarbonyl)-L-alaninate (compound 9-1)**

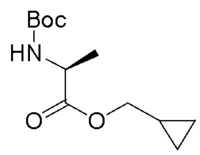

Compound 9-1

To a mixed solution of N-tert-butyloxycarbonyl-L-alanine (5.00 g, 26.43 mmol, 1 eq) and hydroxymethylcyclopropane (2.29 g, 31.72 mmol, 1.2 eq) in acetonitrile (50 mL) were added 4-dimethylaminopyridine (4.84 g, 39.65 mmol, 1.5 eq) and 1-(3-dimethylaminopropyl)-3-ethylcarbodiimide hydrochloride (5.32 g, 27.75 mmol, 1.05 eq) at 0 °C, and the mixture was stirred at 25 °C for 3 hours. After the reaction was completed, the reaction was quenched by adding an appropriate amount of water. The mixture was extracted with ethyl acetate (50 mL*3). The combined organic layer was dried over anhydrous sodium sulfate and concentrated under reduced pressure to obtain a residue. The residue was purified by column chromatography on silica gel eluting with 0-20% petroleum ether/ethyl acetate to give cyclopropylmethyl (tert-butoxycarbonyl)-L-alaninate (3.5 g, 14.39 mmol, 54.45% yield) as a colorless oil.

$^1$H NMR (400 MHz, CDCl3) δ = 5.07 (d, J = 2.4 Hz, 1H), 4.35 - 4.31 (m, 1H), 4.02 - 3.95 (m, 2H), 1.46 (s, 9H), 1.40 (d, J = 7.2 Hz, 3H), 1.16 - 1.12 (m, 1H), 0.59 - 0.57 (m, 2H), 0.31 - 0.27 (m, 2H).

**Step 2: cyclopropylmethyl L-alaninate trifluoroacetate (compound 9-2)**

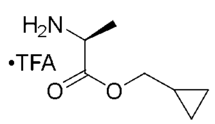

Compound 9-2

To a solution of cyclopropylmethyl (tert-butyloxycarbonyl)-L-alaninate (0.5 g, 2.06 mmol, 1 eq) in dichloromethane (5 mL) was added trifluoroacetic acid (1 mL) at 25 °C, and the mixture was stirred at 25 °C for 1 hour. After the reaction was completed, the solvent was removed by concentration under reduced pressure to give cyclopropylmethyl L-alaninate trifluoroacetate (0.46 g, 92.7% yield) as a bright yellow oil, which was directly used in the next step without further treatment.

1H NMR (400 MHz, CDCl3) δ = 9.85 (s, 2H), 4.15 - 4.13 (m, 1H), 4.07 - 4.01 (m, 2H), 1.63 (d, J = 7.2 Hz, 3H), 1.16 - 1.12 (m, 1H), 0.64 - 0.60 (m, 2H), 0.30 (q, J = 4.8 Hz, 2H).

**Step 3: cyclopropylmethyl ((((Z)-3-(((tert-butyldimethylsilyl)oxy)methyl)-4,4,4-trifluorobut-2-en-1-yl)oxy)(phenoxy)phosphoryl)-L-alaninate (compound 9-3)**

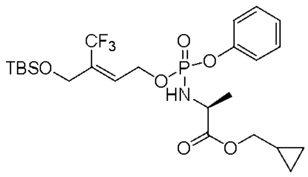

Compound 9-3

To a solution of phenyl dichlorophosphate (300 mg, 1.43 mmol, 1 eq) in dichloromethane (10 mL) was added triethylamine (718.2 mg, 7.1 mmol, 5 eq) at -78 °C and under nitrogen protection, and the mixture was stirred at -78 °C for 5 minutes. Cyclopropylmethyl L-alaninate trifluoroacetate (365.11 mg, 1.42 mmol, 1 eq) and (Z)-3-(((tert-butyldimethylsilyl) oxy)methyl)-4,4,4-trifluorobut-2-en-1-ol (383.78 mg, 1.42 mmol, 1 eq) were then added. The mixture was stirred at 25 °C for 16 hours. After the reaction was completed, the reaction was quenched by adding an appropriate amount of water. The mixture was extracted with dichloromethane (20 mL*3). The combined organic layer was dried over anhydrous sodium sulfate and concentrated under reduced pressure to obtain a residue. The residue was purified by column chromatography on silica gel eluting with 0-55% petroleum ether/ethyl acetate to give cyclopropylmethyl ((((Z)-3-(((tert-butyldimethylsilyl) oxy)methyl)-4,4,4-trifluorobut-2-en-1-yl)oxy)(phenoxy)phosphoryl)-L-alani nate (70 mg, 126.90 μmol, 8.94% yield) as a yellow oil.

MS (ESI) m/z: calcd. 552.2 $[M+H]^+$, found 552.2 $[M+H]^+$.

**Step 4: cyclopropylmethyl ((phenoxy)(((Z)-4,4,4-trifluoro-3-(hydroxymethyl)but-2-en-1-yl)oxy)phosphoryl)-L-alaninate (compound 9)**

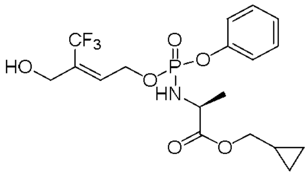

Compound 9

To a solution of cyclopropylmethyl ((((Z)-3-(((tert-butyldimethylsilyl)oxy)methyl)-4,4,4-trifluorobut-2-en-1-yl)oxy) (phenoxy)phosphoryl)-L-alani nate (70 mg, 126.90 μmol, 1 eq) in acetonitrile (3 mL) was added p-toluenesulfonic acid (43.69 mg, 253.8 μmol, 2 eq), and the mixture was stirred at 25° C for 16 hours. After the reaction was completed, the mixture was filtered, and the filtrate was concentrated under reduced pressure to obtain a residue. The residue was purified by preparative HPLC ($NH_4HCO_3$ conditions) to give cyclopropylmethyl ((phenoxy)(((Z)-4,4,4-trifluoro-3-(hydroxymethyl)but-2-en-1-yl)oxy)phosphoryl)-L-alaninate (0.5 mg, 1.14 μmol, 0.90% yield), the target product, as a colorless oil.

$^1H$ NMR (400 MHz, $CDCl_3$) δ = 7.36 - 7.33 (m, 2H), 7.24 - 7.18 (m, 3H), 6.23 (dt, *J* = 11.6, 5.6 Hz, 1H), 4.92 - 4.90 (m, 2H), 4.25 (s, 2H), 4.11 - 4.00 (m, 1H), 3.99 - 3.94 (m, 2H), 3.74 (q, *J* = 7.2 Hz, 1H), 3.72 - 3.57 (m, 1H), 1.41 (t, *J* = 7.2 Hz, 3H), 1.25 (t, *J* = 7.2 Hz, 1H) 1.13 - 1.11 (m, 1H), 0.60 - 0.56 (m, 2H), 0.30 - 0.27 (m, 2H);
$^{31}P$ NMR (162 MHz, $CDCl_3$) δ = 2.29;
$^{19}F$ NMR (376 MHz, $CDCl_3$) δ = -61.19;
MS (ESI) *m/z:* calcd. 438.1 $[M+H]^+$, found 438.1 $[M+H]^+$.

Example 10:

**2-Ethylbutyl**

**((phenoxy)(((Z)-4,4,4-trifluoro-3-(hydroxymethyl)but-2-en-1-yl)oxy)phosphoryl)-L-alaninate (compound 10)**

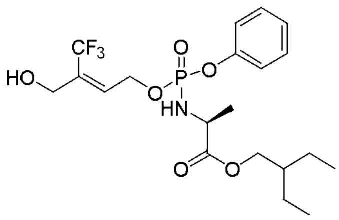

Compound 10

Example 10 was synthesized using N-tert-butyloxycarbonyl-L-alanine and 2-ethyl-1-butanol as raw materials by the same method as described in Example 9.

$^1$H NMR (400 MHz, $CDCl_3$) $\delta$ = 7.37 - 7.33 (m, 2H), 7.25 - 7.19 (m, 3H), 6.25 (dt, *J* = 11.6, 5.6 Hz, 1H), 4.91 (dd, *J* = 4.4, 2.0 Hz, 2H), 4.25 (s, 2H), 4.10 - 4.04 (m, 3H), 3.72 - 3.66 (m, 1H), 1.56 - 1.48 (m, 1H), 1.42 - 1.34 (m, 7H), 0.93 - 0.88 (m, 6H);
$^{31}$P NMR (162 MHz, $CDCl_3$) $\delta$ = 2.51;
$^{19}$F NMR (376 MHz, $CDCl_3$) $\delta$ = -61.24;
MS (ESI) *m/z:* calcd. 468.2 $[M+H]^+$, found 468.2 $[M+H]^+$.

Example 11:

**Cyclopentyl**

**((phenoxy)(((Z)-4,4,4-trifluoro-3-(hydroxymethyl)but-2-en-1-yl)oxy)phosphoryl)-L-alaninate (compound 11)**

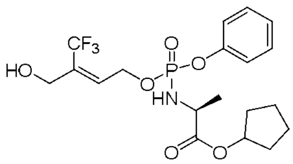

Compound 11

Example 11 was synthesized using N-benzyloxycarbonyl-L-alanine and cyclopentanol as raw materials by the same method as described in Example 9.

$^1$H NMR (400 MHz, $CDCl_3$) $\delta$ = 7.36 - 7.32 (m, 2H), 7.23 - 7.18 (m, 3H), 6.26 - 6.22 (m, 1H), 5.20 - 5.17 (m, 1H), 4.91 - 4.88 (m, 2H), 4.25 (s, 2H), 3.99 - 3.95 (m, 1H), 3.60 - 3.57 (m, 1H), 2.48 - 2.47 (m, 1H), 1.88 - 1.84 (m, 2H), 1.72 - 1.67 (m, 6H), 1.36 (t, *J* = 7.6 Hz, 3H);
$^{31}$P NMR (162 MHz, $CDCl_3$) $\delta$ = 2.35;
$^{19}$F NMR (376 MHz, $CDCl_3$) $\delta$ = -61.24;
MS (ESI) *m/z:* calcd. 452.1 $[M+H]^+$, found 452.1 $[M+H]^+$.

Example 12:

**(S)-tetrahydrofuran-3-yl**

**((phenoxy)(((Z)-4,4,4-trifluoro-3-(hydroxymethyl)but-2-en-1-yl)oxy)phosphoryl)-L-alaninate (compound 12)**

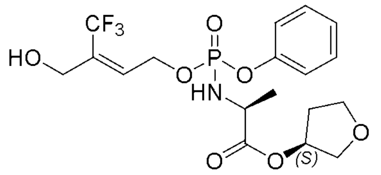

Compound 12

Example 12 was synthesized using N-benzyloxycarbonyl-L-alanine and (S)-3-hydroxytetrahydrofuran as raw materials by the same method as described in Example 9.

$^1$H NMR (400 MHz, $CDCl_3$) δ = 7.36 - 7.32 (m, 2H), 7.23 - 7.18 (m, 3H), 6.26 - 6.21 (m, 1H), 5.31 - 5.29 (m, 1H), 4.92 - 4.90 (m, 2H), 4.23 (s, 2H), 4.08 - 3.97 (m, 1H), 3.93 - 3.81 (m, 3H), 3.78 - 3.64 (m, 2H), 2.22 - 2.15 (m, 1H), 2.00 - 1.97 (m, 1H), 1.38 (dd, *J* = 6.8, 3.2 Hz, 3H);
$^{31}$P NMR (162 MHz, $CDCl_3$) δ = 2.38, 2.15;
$^{19}$F NMR (376 MHz, $CDCl_3$) δ = -61.18, -61.19;
MS (ESI) *m/z:* calcd. 454.1 $[M+H]^+$, found 454.0 $[M+H]^+$.

Example 13: Compound 13

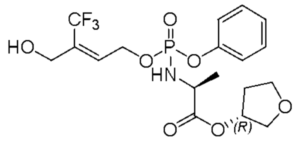

Compound 13

Example 13 was synthesized using N-benzyloxycarbonyl-L-alanine and (R)-3-hydroxytetrahydrofuran as raw materials by the same method as described in Example 9.

$^1$H NMR (400 MHz, $CDCl_3$) δ = 7.36 - 7.32 (m, 2H), 7.24 - 7.18 (m, 3H), 6.25 - 6.18 (m, 1H), 5.31 - 5.29 (m, 1H), 4.92 - 4.90 (m, 2H), 4.24 (s, 2H), 4.06 - 3.78 (m, 5H), 3.67 - 3.57 (m, 1H), 2.22 - 2.15 (m, 1H), 2.02 - 1.95 (m, 1H), 1.38 (dd, *J* = 7.2, 3.6 Hz, 3H);
$^{31}$P NMR (162 MHz, $CDCl_3$) δ = 2.40, 1.95;
$^{19}$F NMR (376 MHz, $CDCl_3$) δ = -61.19, -61.27;
MS (ESI) *m/z:* calcd. 454.1 $[M+H]^+$, found 454.2 $[M+H]^+$.

Example 14: Compound 14

The title compound was prepared according to the following scheme:

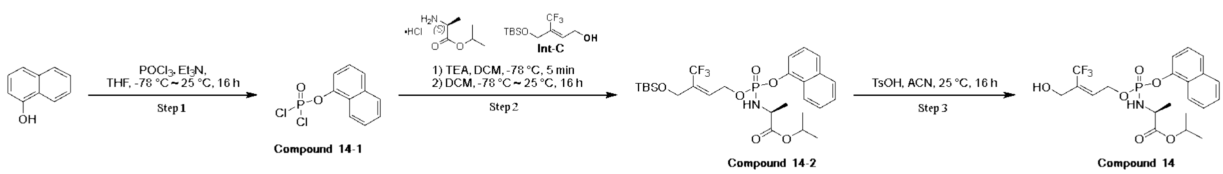


## Experimental operation

### Step 1: 1-naphthyl dichlorophosphate (compound 14-1)

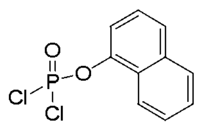

Compound 14-1

To a solution of 1-naphthol (1 g, 6.94 mmol, 1 eq) in tetrahydrofuran (40 mL) was added phosphorus oxychloride (1.06 g, 6.94 mmol, 1 eq) at -78 °C and under nitrogen protection. Triethylamine (701.87 mg, 6.94 mmol, 1 eq) was then added dropwise to the mixture at -78 °C and under nitrogen protection. The mixture was stirred at 25 °C for 16 hours. After the reaction was completed, the reaction mixture was cooled to room temperature and concentrated under reduced pressure to remove the solvent, to give 1-naphthyl dichlorophosphate (1.2 g, 66.7% yield) as a colorless oil, which was directly used in the next step without further treatment.

**Step 2: isopropyl ((((Z)-3-(((tert-butyldimethylsilyl)oxy)methyl)-4,4,4-trifluorobut-2-en-1-yl)oxy)(naphthyloxy) phosphoryl )-L-alaninate (compound 14-2)**

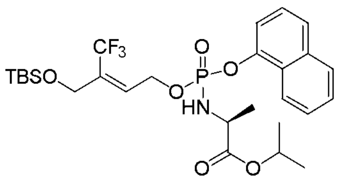

Compound 14-2

To a solution of 1-naphthyl dichlorophosphate (300 mg, 1.15 mmol, 1 eq) in dichloromethane (5 mL) was added triethylamine (465.31 mg, 4.6 mmol, 4 eq) at -78 °C and under nitrogen protection, and the mixture was stirred at -78 °C for 5 minutes. Subsequently, isopropyl L-alaninate hydrochloride (192.72 mg, 1.15 mmol, 1 eq) and (Z)-3-(((tert-butyldimethylsilyl)oxy)methyl)-4,4,4-trifluorobut-2-en-1-ol (310.81 mg, 1.15 mmol, 1 eq) were added. The mixture was stirred at 25 °C for 16 hours. After the reaction was completed, the reaction was quenched by adding an appropriate amount of water. The mixture was extracted with dichloromethane (20 mL*3). The combined organic layer was dried over anhydrous sodium sulfate and concentrated under reduced pressure to obtain a residue. The residue was purified by column chromatography on silica gel eluting with 0-50% petroleum ether/ethyl acetate to give isopropyl ((((Z)-3-(((tert-butyldimethylsilyl)oxy)methyl)-4,4,4-trifluorobut-2-en-1-yl)oxy)(naphthyloxy)phosphoryl)-L-a laninate (25 mg, 42.4 μmol, 3.69% yield) as a colorless oil.

MS (EI) *m/z:* calcd. 590.2 $[M+H]^+$, found 590.2 $[M+H]^+$.

**Step 3: isopropyl ((naphthyloxy)(((Z)-4,4,4-trifluoro-3-(hydroxymethyl)but-2-en-1-yl)oxy)phosphoryl)-L-alaninate (compound 14)**

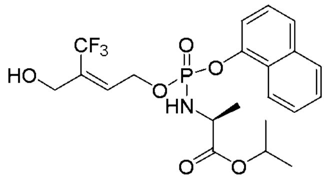

Compound 14

To a solution of isopropyl ((((Z)-3-(((tert-butyldimethylsilyl)oxy)methyl)-4,4,4-trifluorobut-2-en-1-yl)oxy)(naphthyloxy)phosphoryl)-L-a laninate (20 mg, 33.92 μmol, 1 eq) in acetonitrile (2 mL) was added p-toluenesulfonic acid (11.68 mg, 67.84 μmol, 2.0 eq), and the mixture was stirred at 25 °C for 16 hours. After the reaction was completed, the mixture was filtered, and the filtrate was concentrated under reduced pressure to obtain a residue. The residue was purified by preparative HPLC (NH3.H2O conditions) to give isopropyl ((naphthyloxy)(((Z)-4,4,4-trifluoro-3-(hydroxymethyl)but-2-en-1-yl)oxy)phosphoryl)-L-alaninate (1.4 mg, 2.94 μmol, 8.67% yield), the target product, as a colorless oil.

$^{1}H$ NMR (400 MHz, $CDCl_3$) δ = 8.11 (dd, J = 6.4, 3.2 Hz, 1H), 7.86 - 7.84 (m, 1H), 7.67 (dd, J = 8.4, 1.6 Hz, 1H), 7.54 - 7.52 (m, 3H), 7.41 (td, J = 8.0, 3.2 Hz, 1H), 6.23 - 6.18 (m, 1H), 4.98 - 4.93 (m, 3H), 4.21 (s, 2H), 4.06 - 4.04 (m, 1H), 3.69 - 3.62 (m, 1H), 2.10 - 2.04 (m, 1H), 1.33 (ddd, J = 12.8, 7.2, 3.2 Hz, 3H), 1.23 (dt, J = 6.4, 3.2 Hz, 3H), 1.18 (td, J = 6.4, 3.2 Hz, 3H);
$^{31}P$ NMR (162 MHz, $CDCl_3$) δ = 2.82;
$^{19}F$ NMR (376 MHz, $CDCl_3$) δ = -61.21;
MS (ESI) m/z: calcd. 476.1 $[M+H]^+$, found 476.1 $[M+H]^+$.

**Example 15:**

**(*R*)-tetrahydrofuran-3-yl((4-chlorophenoxy)(E)-5-hydroxy-4-methylpent-3-en-1-yl)phosphoryl)-L-alaninate (compound 15)**

The title compound was prepared according to the following scheme:

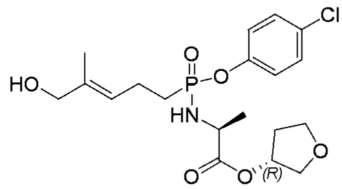

Compound 15

Example 15 was synthesized by the same method as described in Example 2 using benzyloxycarbonyl-L-alanine and (R)-3-hydroxytetrahydrofuran as raw materials, and using p-chlorophenol in place of phenol in step 3 of Example 2.

$^{1}H$ NMR (400 MHz, $CDCl_3$) δ = 7.29 - 7.28 (m, 2H), 7.18 - 7.16 (m, 2H), 5.49 - 5.47 (m, 1H), 5.28 - 5.24 (m, 1H), 4.12 - 4.02 (m, 3H), 3.90 - 3.85 (m, 3H), 3.77 - 3.71 (m, 1H), 3.36 - 3.24 (m, 1H), 2.48 - 2.45 (m, 2H), 2.23 - 2.13 (m, 1H), 1.99 - 1.92 (m, 3H), 1.71 (s, 3H), 1.35 - 1.23 (m, 3H);
$^{31}P$ NMR (162 MHz, $CDCl_3$) δ = 31.57, 31.49
MS (ESI) *m/z:* calcd. 432.1 $[M (^{35}Cl) + H]^+$, 434.1 $[M (^{37}Cl) + H]^+$, found 432.1 $[M (^{35}Cl) + H]^+$, 434.1 $[M (^{37}Cl) + H]^+$

**Example 16:**

**(*R*)-tetrahydrofuran-3-yl**

**((E)-5-hydroxy-4-methylpent-3-en-1-yl)(p-methoxy)phosphoryl)-L-alaninate (compound 16)**

The title compound was prepared according to the following scheme:

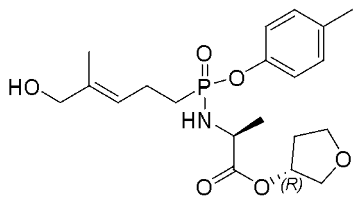

Compound 16

Example 16 was synthesized by the same method as described in Example 2 using benzyloxycarbonyl-L-alanine and (R)-3-hydroxytetrahydrofuran as raw materials, and using p-methylphenol in place of phenol in step 3 of Example 2.

$^{1}H$ NMR (400 MHz, $CDCl_3$) δ = 7.12 - 7.07 (m, 4H), 5.50 - 5.46 (m, 1H), 5.27 - 5.25 (m, 1H), 4.03 - 4.01 (m, 3H), 3.90 - 3.83 (m, 3H), 3.78 - 3.71 (m, 1H), 3.36 - 3.20 (m, 1H), 2.47 - 2.43 (m, 2H), 2.31 (s, 3H), 2.21 - 2.11 (m, 1H), 1.96 - 1.89 (m, 3H), 1.70 (s, 3H), 1.34 - 1.23 (m, 3H);
$^{31}P$ NMR (162 MHz, $CDCl_3$) δ = 30.86, 30.82
MS (ESI) *m/z:* calcd. 412.2 $[M + H]^+$, found 394.2 $[M + H - H_2O]^+$.

**Example 17:**

**(R)-tetrahydrofuran-3-yl**

**((E)-5-hydroxy-4-methylpent-3-en-1-yl)(naphthalen-1-yloxy)phosphoryl)-L-alaninate (compound 17)**

The title compound was prepared according to the following scheme:

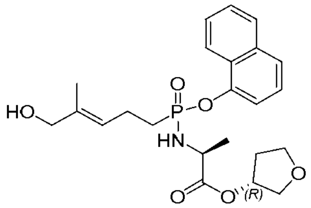

Compound 17

Example 17 was synthesized by the same method as described in Example 2 using benzyloxycarbonyl-L-alanine and (R)-3-hydroxytetrahydrofuran as raw materials, and using 1-naphthol in place of phenol in step 3 of Example 2.

$^1$H NMR (400 MHz, $CDCl_3$) δ = 8.08 - 8.06 (m, 1H), 7.86 - 7.84 (m, 1H), 7.64 (d, *J* = 8.0 Hz, 1H), 7.59 (d, *J* = 7.6 Hz, 1H), 7.60 - 7.52 (m, 2H), 7.43 - 7.39 (m, 1H), 5.51 - 5.48 (m, 1H), 5.17 - 5.14 (m, 1H), 4.10 - 4.04 (m, 1H), 4.01 (s, 2H), 3.83 - 3.78 (m, 3H), 3.67 - 3.62 (m, 1H), 3.47 - 3.28 (m, 1H), 2.55 - 2.50 (m, 2H), 2.12 - 2.05 (m, 3H), 1.83 - 1.80 (m, 1H), 1.71 - 1.69 (m, 3H), 1.29 - 1.26 (m, 3H);
$^{31}$P NMR (162 MHz, $CDCl_3$) δ = 31.44, 31.39;
MS (ESI) *m/z:* calcd. 448.2 $[M + H]^+$, found 470.0 $[M + Na]^+$.

**Example 18:**

**(R)-tetrahydrofuran-3-yl**

**((S)-((E)-5-hydroxy-4-methylpent-3-en-1-yl)(phenoxy)phosphoryl)-L-alaninate (compound 18) & (R)-tetrahydrofuran-3-yl ((S)-((R)-5-hydroxy-4-methylpent-3-en-1-yl)(phenoxy)phosphoryl)-L-alaninate (compound 19)**

The title compounds were prepared according to the following scheme:
The compound 7 of Example 7 was resolved by SFC.

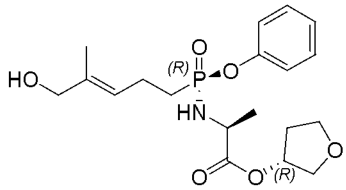

Compound 18

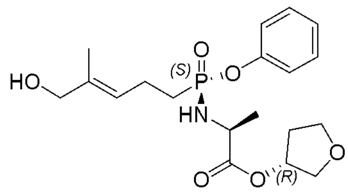

Compound 19

(R)-tetrahydrofuran-3-yl ((E)-5-hydroxy-4-methylpent-3-en-1-yl)(phenoxy)phosphoryl)-L-alaninate (600 mg, 1.51 mmol, 1 eq) was purified by supercritical fluid chromatography (SFC) and normal phase high pressure liquid chromatography (DAICEL CHIRALCEL OD-H (250 mm*30 mm, 5 μm); mobile phase: [$CO_2$-EtOH]; B%: 25%, isocratic elution). The first eluted product was compound 18 (305.56 mg, 768.90 μmol, 50.92% yield), and the second eluted product was compound 19 (137.6 mg, 346.25 μmol, 22.93% yield), both of which were target products as a colorless oil.
The characterization data of compound 18 is as follows:

$^1$H NMR (400 MHz, $CDCl_3$) δ = 7.34 - 7.30 (m, 2H), 7.21 (d, *J* = 8.0 Hz, 2H), 7.17 - 7.15 (m, 1H), 5.47 (t, *J* = 6.8 Hz, 1H), 5.27 - 5.24 (m, 1H), 4.07 - 4.05 (m, 1H), 4.02 (s, 2H), 3.90 - 3.84 (m, 3H), 3.73 (d, *J* = 10.4 Hz, 1H), 3.35 (t, *J* = 10.4 Hz, 1H), 2.48 - 2.43 (m, 2H), 2.22 - 2.12 (m, 1H), 2.04 - 1.86 (m, 3H), 1.71 - 1.69 (m, 3H), 1.33 (d, *J* = 7.2 Hz, 3H);
$^{31}$P NMR (162 MHz, $CDCl_3$) δ = 30.88;
MS (EI) *m/z:* calcd. 398.2 $[M + H]^+$, found 398.1 $[M + H]^+$.

The characterization data of compound 19 is as follows:

$^1$H NMR (400 MHz, $CDCl_3$) $\delta$ = 7.35 - 7.31 (m, 2H), 7.21 (d, $J$ = 8.4 Hz, 2H), 7.17 - 7.15 (m, 1H), 5.49 (t, $J$ = 6.8 Hz, 1H), 5.27 - 5.25 (m, 1H), 4.14 - 4.08 (m, 1H), 4.02 (s, 2H), 3.91 - 3.84 (m, 3H), 3.79 - 3.76 (m, 1H), 3.25 (t, $J$ = 10.4 Hz, 1H), 2.49 - 2.45 (m, 2H), 2.21 - 2.17 (m, 1H), 2.03 - 1.86 (m, 3H), 1.77 - 1.72 (m, 3H), 1.22 (d, $J$ = 7.2 Hz, 3H);
$^{31}$P NMR (162 MHz, $CDCl_3$) $\delta$ = 31.31;
MS (EI) *m/z:* calcd. 398.2 $[M + H]^+$, found 398.1 $[M + H]^+$.

Example 20:

**Tetrahydro-2H-pyran-4-yl**

**((E)-5-hydroxy-4-methylpent-3-en-1-yl)(phenoxy)phosphoryl)-L-alaninate (compound 20)**

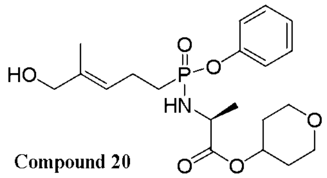

Compound 20

The product was synthesized by the same method as described in Example 2 using benzyloxycarbonyl-L-alanine and tetrahydro-2H-pyran-4-ol as raw materials.

$^1$H NMR (400 MHz, $CDCl_3$) $\delta$ = 7.33 - 7.31 (m, 2H), 7.23 - 7.20 (m, 2H), 7.15 - 7.13 (m, 1H), 5.51 - 5.43 (m, 1H), 4.96 - 4.88 (m, 1H), 4.05 - 4.03 (m, 1H), 4.01 (s, 2H), 3.91 - 3.83 (m, 2H), 3.55 - 3.49 (m, 2H), 3.42- 3.33 (m, 0.6H), 3.30 - 3.25 (m, 0.4H), 2.53 - 2.40 (m, 2H), 2.04 - 1.92 (m, 2H), 1.90 - 1.84 (m, 4H), 1.71 (s, 1.5H), 1.70 (s, 1.5H), 1.67 - 1.60 (m, 2H), 1.34 - 1.31 (m, 3H);
$^{31}$P NMR (162 MHz, $CDCl_3$) $\delta$ = 31.42, 31.04;
MS (ESI) *m/z:* calcd. 412.2 $[M + H]^+$, found 394.2 $[M+H-H_2O]^+$, 434.1 $[M + Na]^+$;

**Examples 21 and 22:**

**Tetrahydro-2H-pyran-4-yl**

**((S)-((E)-5-hydroxy-4-methylpent-3-en-1-yl)(phenoxy)phosphoryl)-L-alanine (compound 21) & Tetrahydro-2H-pyran-4-yl ((R)-((E)-5-hydroxy-4-methylpent-3-en-1-yl)(phenoxy)phosphoryl)-L-alanine (compound 22)**

The title compounds were prepared according to the following scheme:

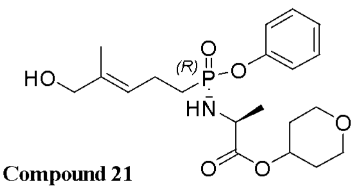

Compound 21

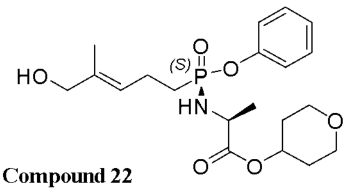

Compound 22

Compound 20 (90 mg, 218.75 µmol, 1 eq) was purified by supercritical fluid chromatography (DAICEL CHIRALPAK AD-H(250mm*30mm,5µm);mobile phase: [CO2-EtOH/ACN]; B%: 35%, isocratic elution mode) and preparative HPLC (Waters Xbridge C18 150*50mm* 10µm; mobile phase: [water(NH4HCO3)-ACN]; gradient:17%-47% B over 10 min). The firstly obtained compound was compound 21 (10 mg, 24.31 µmol, 11.11% yield) and the secondly obtained compound was compound 22 (5 mg, 12.15 µmol, 5.55% yield), both of which were target products as a colorless oil.

The characterization of compound 21 is as follows:

$^1$H NMR (400 MHz, $CDCl_3$) $\delta$ = 7.33 - 7.29 (m, 2H), 7.23 - 7.20 (m, 2H), 7.16 - 7.14 (m, 1H), 5.49 - 5.45 (m, 1H), 4.95 -

4.90 (m, 1H), 4.05 - 4.03 (m, 1H), 4.01 (s, 2H), 3.90 - 3.85 (m, 2H), 3.55 - 3.49 (m, 2H), 3.40 (t, *J* = 10.4 Hz, 1H), 2.46 (dq, *J* = 14.8, 7.2 Hz, 2H), 1.99 - 1.92 (m, 2H), 1.87 - 1.86 (m, 2H), 1.70 (s, 3H), 1.67 - 1.58 (m, 2H), 1.34 (d, *J* = 7.2 Hz, 3H);
$^{31}$P NMR (162 MHz, $CDCl_3$) δ = 31.02;
MS (ESI) *m/z:* calcd. 412.2 $[M + H]^+$, found 394.2 $[M + H\text{-}H_2O]^+$.

The characterization of compound 22 is as follows:

$^1$H NMR (400 MHz, $CDCl_3$) δ = 7.34 - 7.30 (m, 2H), 7.22 - 7.20 (m, 2H), 7.17 - 7.13 (m, 1H), 5.49 (t, *J* = 6.8 Hz, 1H), 4.94 - 4.90 (m, 1H), 4.13 - 4.10 (m, 1H), 4.01 (s, 2H), 3.90 - 3.86 (m, 2H), 3.55 - 3.50 (m, 2H), 3.29 (t, *J* = 10.8 Hz, 1H), 2.53 - 2.41 (m, 2H), 2.04 - 1.98 (m, 2H), 1.88 - 1.87 (m, 2H), 1.71 (s, 3H), 1.65 - 1.63 (m, 2H), 1.22 (d, *J* = 7.2 Hz, 3H);
$^{31}$P NMR (162 MHz, $CDCl_3$) δ = 31.42;
MS (ESI) *m/z:* calcd. 412.2 $[M + H]^+$, found 394.2 $[M + H\text{-}H_2O]^+$.

**Example 23:**

**Isopropyl (((*Z*)-4-fluoro-5-hydroxy-3-en-1-yl)(phenoxy)phosphoryl)-*L*-alaninate (compound 23)**

The title compound was prepared according to the following scheme:

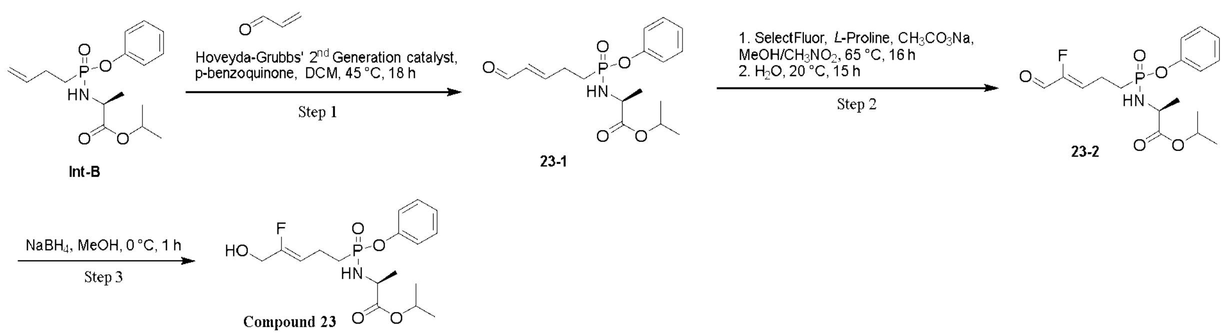

Int-B
23-1
23-2
Compound 23

**Experimental operation**

**Step 1: isopropyl (((*E*)-5-oxo-3-en-1-yl)(phenoxy)phosphoryl)-*L*-alaninate (compound 23-1)**

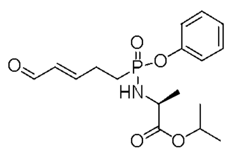

23-1

To a solution of isopropyl (but-3-en-1-yl(phenoxy)phosphoryl)-*L*-alaninate (13 g, 39.96 mmol, 1 eq), acrolein (9.81 g, 319.68 mmol, 8 eq) and 1,4-benzoquinone (367.54 mg, 3.40 mmol, 0.1 eq) in dichloromethane (150 mL) was added Hoveyda-Grubbs' 2nd Generation catalyst (2.13 g, 3.40 mmol, 0.1 eq). The catalyst was added in three equal portions (710 mg, 1.13 mmol for each portion) at t=0, 2 and 4 hours during the reaction. The solution was then heated to reflux for 18 hours at 45 °C and under nitrogen atmosphere. LCMS showed that the raw material was completely consumed and that the product was detected. The mixture was filtered, and the filtrate was concentrated in vacuo to obtain a residue. The residue was purified by column chromatography on silica gel eluting with 10% ethyl acetate/methanol to give isopropyl (((*E*)-5-oxo-3-en-1-yl)(phenoxy)phosphoryl)-*L*-alaninate (7.5 g, 21.23 mmol, 53.13% yield), the target product, as a brown oil.
MS (ESI) *m/z:* calcd. 354.2 $[M + H]^+$, found 354.1 $[M + H]^+$.

**Step 2: isopropyl (((*Z*)-4-fluoro-5-oxo-3-en-1-yl)(phenoxy)phosphoryl)-*L*-alaninate (compound 23-2)**

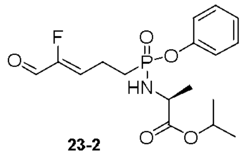

23-2

To a solution of isopropyl (((*E*)-5-oxo-3-en-1-yl)(phenoxy)phosphoryl)-*L*-alaninate (6 g, 16.98 mmol, 1 eq), *L*-proline (1.95 g, 16.98 mmol, 1 eq), sodium acetate (1.39 g, 16.98 mmol, 1 eq) and 1-chloromethyl-4-fluoro-1,4-diazabicyclo[2.2.2]octane di(tetrafluoroborate) salt (12.03 g, 33.96 mmol, 2 eq) in methanol (60 mL) was added nitromethane (57.23 g, 937.58 mmol, 55.2 eq) under nitrogen atmosphere. The solution was then stirred for 16 hours at 65 °C under nitrogen atmosphere. LCMS showed that the raw material was completely consumed and that the product was detected. The mixture was cooled to room temperature and quenched by adding saturated ammonium chloride solution (100 mL). The mixture was extracted with dichloromethane (80 mL*3). The combined organic layer was dried over anhydrous sodium sulfate and concentrated under reduced pressure to obtain a residue. The residue was purified by column chromatography on silica gel eluting with 10% ethyl acetate/methanol to give isopropyl (((*Z*)-4-fluoro-5-oxo-3-en-1-yl)(phenoxy)phosphoryl)-*L*-alaninate (720 mg, 1.94 mmol, 11.42% yield), the target product, as a brown oil.

MS (ESI) *m/z:* calcd. 372.1 $[M + H]^+$, found 372.1 $[M + H]^+$.

**Step 7: isopropyl (((*Z*)-4-fluoro-5-hydroxy-3-en-1-yl)(phenoxy)phosphoryl)-*L*-alaninate (compound 23)**

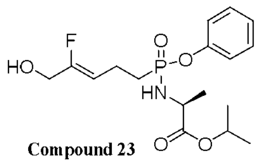

Compound 23

To a solution of isopropyl (((*Z*)-4-fluoro-5-oxo-3-en-1-yl)(phenoxy)phosphoryl)-*L*-alaninate (360 mg, 969.46 μmol, 1 eq) in methanol (5 mL) was slowly added sodium borohydride (73.39 mg, 1.94 mmol, 2 eq) at 0 °C and under nitrogen atmosphere. The solution was then stirred for 1 hour at 0 °C and under nitrogen atmosphere. LCMS showed that the raw material was completely consumed and that the product was detected. The mixture was quenched by adding saturated ammonium chloride solution (10 mL). The mixture was extracted with dichloromethane (50 mL*2). The combined organic layer was dried over anhydrous sodium sulfate and concentrated under reduced pressure to obtain a residue. The residue was purified by column chromatography on silica gel eluting with 10% ethyl acetate/methanol and then purified by preparative HPLC (Phenomenex Synergi C18 150*25mm* 10μm; mobile phase: [water(0.1%FA)-ACN]; B%: 40%-50%, 2 min) to give isopropyl (((*Z*)-4-fluoro-5-hydroxy-3-en-1-yl)(phenoxy)phosphoryl)-*L*-alaninate (3.08 mg, 8.25 μmol, 0.85% yield), the target product, as a colorless oil.

$^1H$ NMR (400 MHz, $CDCl_3$) δ = 7.34 - 7.30 (m, 2H), 7.22 - 7.20 (m, 2H), 7.15 - 7.14 (m, 1H), 5.04 - 4.95 (m, 2H), 4.14 - 4.04 (m, 2H), 3.97 - 3.95 (m, 1H), 3.41 - 3.27 (m, 1H), 2.55 - 2.51 (m, 2H), 2.06 - 2.00 (m, 2H), 1.32 - 1.20 (m, 9H); $^{31}P$ NMR (162 MHz, $CDCl_3$) δ = 30.64, 30.39; $^{19}F$ NMR (376 MHz, $CDCl_3$) δ = -118.43, -118.49; MS (ESI) *m/z:* calcd. 374.2 $[M + H]^+$, found 374.1 $[M + H]^+$.

**Example 24:**

**Isopropyl (((*Z*)-4-fluoro-5-hydroxy-3-en-1-yl)(phenoxy)phosphoryl)-*L*-alaninate (compound 24)**

The title compound was prepared according to the following scheme:

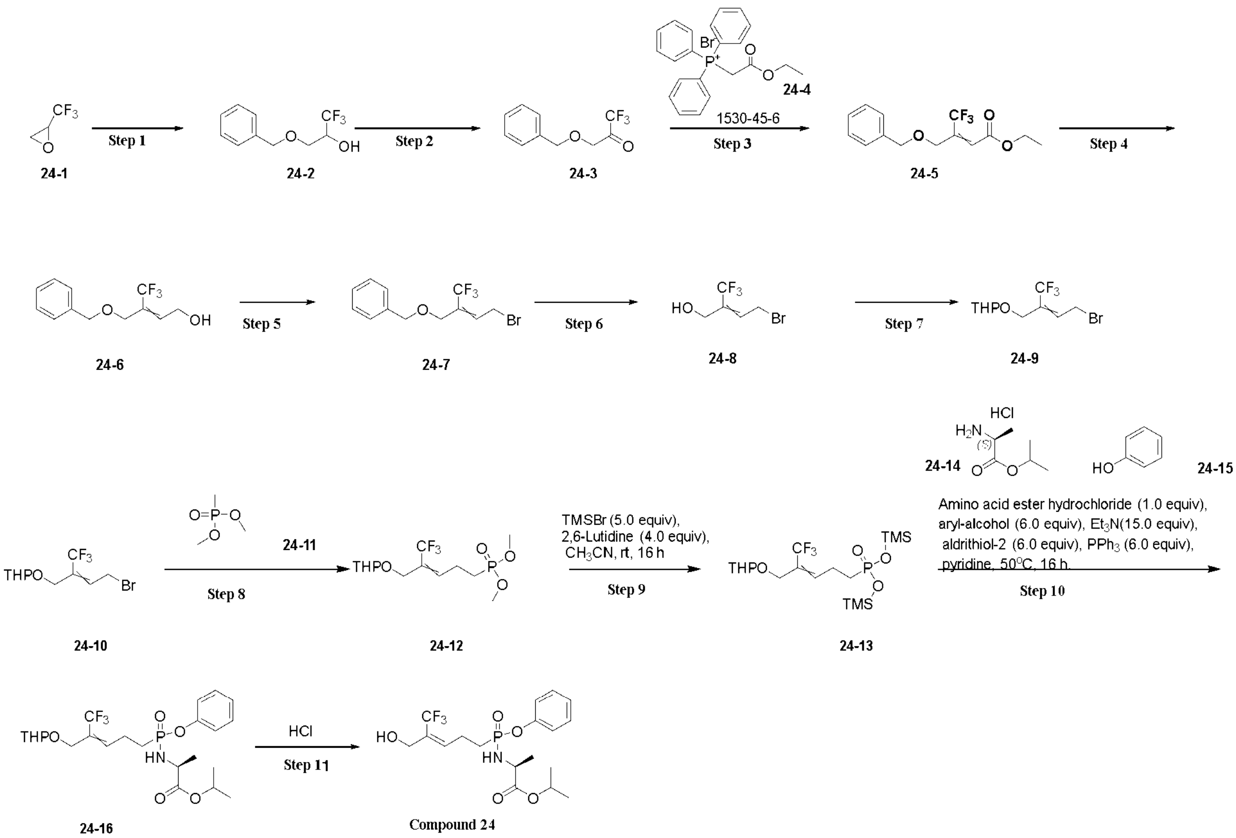


## Experimental operation

### Step 1: 3-(benzyloxy)-1,1,1-trifluoropropan-2-ol (compound 24-2)

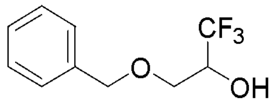

**24-2**

To a reaction solution of benzyl alcohol (30 g, 267.70 mmol, 1 eq) and boron trifluoride etherate (3.8 g, 26.77 mmol, 0.1 eq) was added 1,1,1-trifluoro-2,3-epoxypropane (29 g, 267.70 mmol, 1 eq) at room temperature, and the mixture was stirred at 40 °C for 16 hours. After the reaction was completed, the reaction mixture was cooled to room temperature and quenched by adding an appropriate amount of water. The mixture was extracted with dichloromethane (300 mL*3). The combined organic layer was dried over anhydrous sodium sulfate and concentrated under reduced pressure to obtain a residue. The residue was purified by column chromatography on silica gel eluting with 20~40% petroleum ether/ethyl acetate to give 3-(benzyloxy)-1,1,1-trifluoropropan-2-ol (35 g, 206.20 mmol, 57.08% yield) as a yellow oil.

$^1$H NMR (400 MHz, $CDCl_3$) δ = 7.40 - 7.32 (m, 5H), 4.61 (s, 2H), 4.18 - 4.14 (m, 1H), 3.76 - 3.65 (m, 2H), 2.88 (d, *J* = 4.0 Hz, 1H).

### Step 2: 3-(benzyloxy)-1,1,1-trifluoropropan-2-one (compound 24-3)

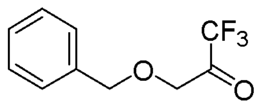

24-3

To a solution of 3-(benzyloxy)-1,1,1-trifluoropropan-2-ol (24 g, 116.40 mmol, 1.0 eq) in dichloromethane (500 mL) was added Dess-Martin periodinane DMP (64.18 g, 151.32 mmol, 1.3 eq) dropwise at 20 °C, and the mixture was stirred at 20 ° C for 16 hours. After the reaction was completed, the mixture was diluted with dichloromethane, and the organic layer was washed with sodium thiosulfate and sodium bicarbonate solution, respectively, and then the organic layer was dried over anhydrous sodium sulfate and concentrated under reduced pressure to obtain a residue. The residue was purified by column chromatography on silica gel eluting with 20-40% petroleum ether/ethyl acetate to give the desired 3-(benzyloxy)-1,1,1-trifluoropropan-2-one (15 g, 66.10 mmol, 56.79% yield) as a colorless oil.

$^1$H NMR (400 MHz, $CDCl_3$) $\delta$ = 7.39 - 7.37 (m, 5H), 4.70 (s, 2H), 3.68 (s, 2H).

**Step 3: ethyl 3-((benzyloxy)methyl)-4,4,4-trifluorobut-2-enoate (compound 24-5)**

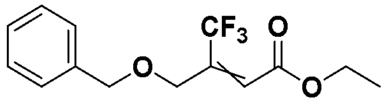

24-5

3-(benzyloxy)-1,1,1-trifluoropropan-2-one (20 g, 91.70 mmol, 1 eq) was dissolved in an appropriate amount of benzene under nitrogen protection, and the water in the system was removed by reflux. The reaction solution was cooled to 25 °C, and ethoxyformylmethyltriphenylphosphonium bromide (47.18 g, 109.92 mmol, 1.2 eq) and triethylamine (10.66 g, 210.68 mmol, 2.3 eq) were added to the reaction system and stirred at 25 °C for 16 hours. After the reaction was completed, the reaction was quenched by adding an appropriate amount of water, and the mixture was extracted with ethyl acetate (30 mL*3). The combined organic layer was dried over anhydrous sodium sulfate and concentrated under reduced pressure to obtain a residue. The residue was purified by column chromatography on silica gel eluting with 20-30% petroleum ether/ethyl acetate to give ethyl 3-((benzyloxy)methyl)-4,4,4-trifluorobut-2-enoate (15 g, 52.03 mmol, 51.09% yield) as a colorless oil.

$^1$H NMR (400 MHz, $CDCl_3$) $\delta$ = 7.39 - 7.32 (m, 5H), 6.52 - 6.48 (m, 1H), 4.60 (s, 2H), 4.28 - 4.21 (m, 2H), 4.18 (s, 2H), 1.33 -1.26 (m, 3H).

**Step 4: 3-((benzyloxy)methyl)-4,4,4-trifluorobut-2-en-1-ol (compound 24-6)**

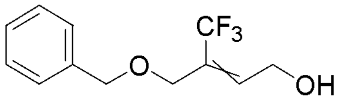

24-6

To a solution of ethyl 3-((benzyloxy)methyl)-4,4,4-trifluorobut-2-enoate (6 g, 20.80 mmol, 1 eq) in tetrahydrofuran (50 mL) was added DIBALH (1.0 M, 41.60 mL, 2.0 eq) dropwise at -60 °C and under nitrogen protection. After the addition was completed, the mixture was slowly warmed to room temperature and stirred at room temperature for 15 hours. After the reaction was completed, the mixture was diluted with dichloromethane (100 ml), and the organic layer was washed with saturated sodium chloride solution (20 ml *3), and then the organic layer was dried over anhydrous sodium sulfate and concentrated under reduced pressure to obtain a residue. The residue was purified by column chromatography on silica gel eluting with 10-20% petroleum ether/ethyl acetate to give 3-((benzyloxy)methyl)-4,4,4-trifluorobut-2-en-1-ol (3.8 g, 13.90 mmol, 66.8% yield) as a colorless oil.

$^1$H NMR (400 MHz, $CDCl_3$) $\delta$ = 7.37 -7.30 (m, 5H), 6.55-6.52(m, 0.6H), 6.25 - 6.22 (m, 0.4H), 4.54 (s, 0.7H), 4.53 (s, 1.3H), 4.46 (s, 0.7H), 4.34 (s, 1.3H), 4.17 (s, 1.3H), 4.14 - 4.09 (m, 0.7H).

**Step 5: (((4-bromo-2-(trifluoromethyl)but-2-en-1-yl)oxy)methyl)benzene (compound 24-7)**

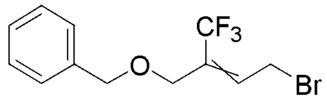

**24-7**

To a solution of 3-((benzyloxy)methyl)-4,4,4-trifluorobut-2-ene-1-ol (3.5 g, 14.20 mmol, 1 eq) in dichloromethane (20 mL) were added triphenylphosphine (4.47 g, 17.04 mmol, 1.2 eq) and carbon tetrabromide (5.18 g, 15.62 mmol, 1.1 eq) at 0 °C and under nitrogen protection, and the resulting mixture was stirred at 0 °C for 2 hours. After the reaction was completed, the reaction mixture was diluted with an appropriate amount of dichloromethane, the organic layer was washed with a saturated sodium chloride solution, and then the organic layer was dried over anhydrous sodium sulfate and concentrated under reduced pressure to obtain a residue. The residue was purified by column chromatography on silica gel eluting with 40-60% petroleum ether/ethyl acetate to give 24-7 (3.6 g, 32.65 mmol, 98.02% yield) as a colorless oil.

$^1$H NMR (400 MHz, $CDCl_3$) $\delta$ = 7.39 -7.32 (m, 5H), 6.58-6.56(m, 0.6H), 6.36-6.31 (m, 0.4H), 4.56 (s, 0.7H), 4.54(s, 1.3H)4.20 (s, 0.2H), 4.19(s, 1.1H), 4.14-4.11 (m, 1.4H),4.09-4.08 (m, 0.7H), 4.07- 4.06 (m, 0.6H).

$^{19}$F NMR (376 MHz, $CDCl_3$) $\delta$ = -60.38, -67.68.

**Step 6: 4-bromo-2-(trifluoromethyl)but-2-en-1-ol (compound 24-8)**

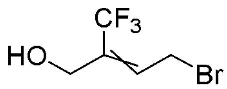

**24-8**

To a solution of (((4-bromo-2-(trifluoromethyl)but-2-en-1-yl)oxy)methyl)benzene (2.9 g, 52.03 mmol, 1 eq) in dichloromethane (150 mL) was added boron trichloride (1.0 M, 75.20 mL, 8.0 eq) dropwise at -78 °C and under nitrogen protection, and the resulting mixture was gradually heated to 20 °C and stirred for another 5 hours. After the reaction was completed, the mixture was diluted with dichloromethane (150 mL), the organic layer was washed with saturated sodium chloride solution (50 ml*3), and then the organic layer was dried over anhydrous sodium sulfate and concentrated under reduced pressure to obtain a residue. The residue was purified by column chromatography on silica gel eluting with 40-60% petroleum ether/ethyl acetate to give 24-8 (1.20 g, 4.90 mmol, 52.13% yield) as a colorless oil.

$^1$H NMR (400 MHz, $CDCl_3$) $\delta$ = 6.58-6.54(m, 0.7H), 6.36-6.531 (m, 0.3H), 4.38 (s, 1.5H), 4.31(s, 0.5H) 4.15 - 4.13 (m, 1.5H) 4.13-4.12(m, 0.5H).

**Step 7: 2-((4-bromo-2-(trifluoromethyl)but-2-en-1-yl)oxy)tetrahydro-2H-pyran (compound 24-9)**

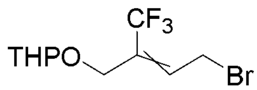

**24-9**

To a solution of 4-bromo-2-(trifluoromethyl)but-2-en-1-ol (1.50 g, 6.80 mmol, 1 eq) and pyridine p-toluenesulfonate (0.17 g, 0.06 mmol, 0.1 eq) in tetrahydrofuran (40 mL) was added 3,4-dihydropyran (1.71 g, 20.30 mmol, 3 eq) at 20 °C, and the reaction mixture was stirred at 20 °C for 2 hours. After the reaction was completed, the reaction was quenched by adding water (20 ml), and the mixture was extracted with dichloromethane (50 mL*3). The combined organic layer was dried over anhydrous sodium sulfate and concentrated under reduced pressure to obtain a residue. The residue was

purified by column chromatography on silica gel eluting with 10-20% petroleum ether/ethyl acetate to give 2-((4-bromo-2-(trifluoromethyl)but-2-en-1-yl)oxy)tetrahydro-2H-pyran (1.5 g, 4.97 mmol, 73.1% yield) as a colorless oil.

$^1$H NMR (400 MHz, $CDCl_3$) δ = 6.59-6.55 (m, 0.6H), 6.35-6.30 (m, 0.4H), 4.68 - 4.66 (m, 0.4H), 4.65-4.64 (m, 0.6H) , 4.42-4.39 (m, 0.6H), 4.38-4.34 (m, 0.4H), 4.22-4.19 (m, 0.6H), 4.15-4.10 (m, 2H), 4.10-4.06 (m, 0.4H), 3.87-3.80 (m, 1H), 3.58-3.53 (m, 1H), 1.82 -1.69 (m, 3H), 1.67-1.54 (m, 3H).

**Step 8: dimethyl (5,5,5-trifluoro-4-(((tetrahydro-2H-pyran-2-yl)oxy)methyl)pent-3-en-1-yl)phosphonate (compound 24-12)**

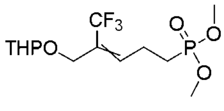

**24-12**

To a solution of dimethyl methylphosphonate (135 mg, 1.08 mmol, 1 eq) in tetrahydrofuran (15 mL) was added a solution of 2.4M n-butyllithium in n-hexane (0.50 mL, 1.30 mmol, 1.2 eq) dropwise at -78 °C and under nitrogen protection, and the reaction solution was stirred at -78 °C for 0.5 h. Then, a solution of 2-((4-bromo-2-(trifluoromethyl)but-2-en-1-yl) oxy)tetrahydro-2H-pyran (330 mg, 1.08 mmol, 1.0 eq) in tetrahydrofuran (1 mL) was added dropwise to the reaction solution, the reaction system was slowly heated, and stirred at 20 °C for 16 h. After the reaction was completed, it was quenched by adding a saturated aqueous ammonium chloride solution (20 mL). The mixture was extracted with ethyl acetate (30 mL*3). The combined organic layer was dried over anhydrous sodium sulfate and concentrated under reduced pressure to obtain a residue. The residue was purified by column chromatography on silica gel eluting with 0-10% ethyl acetate/methanol to give dimethyl (5,5,5-trifluoro-4-(((tetrahydro-2H-pyran-2-yl)oxy)methyl)pent-3-en-1-yl)phosphonate (94 mg, 0.29 mmol, 26.90% yield) as a yellow oil.

MS (ESI) *m/z:* calcd. 347.3 $[M+H]^+$, found 263.0 $[M-THP+H]^+$.

**Step** 9: **bis(trimethylsilyl) (5,5,5-trifluoro-4-(((tetrahydro-2H-pyran-2-yl)oxy)methyl)pent-3-en-1-yl)phosphonate (compound 24-13)**

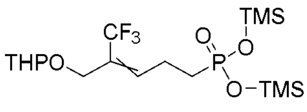

**24-13**

To a solution of (E)-5-diethoxyphosphoryl-2-methyl-2-pent-1-yl octanoate (1.13 g, 3.12 mmol, 1 eq) in dichloromethane (500 mL) was added TMSBr (4.77 g, 31.20 mmol, 10 eq) dropwise at 0 °C, and the mixture was stirred at room temperature for 16 hours. The reaction was monitored by TLC spot plate (PE:EA = 1:5), and after the reaction was completed, the mixture was concentrated under reduced pressure. Bis(trimethylsilyl) (5,5,5-trifluoro-4-(((tetrahydro-2H-pyran-2-yl)oxy)methyl)pent-3-en-1-yl)phosphonate (crude product) (90 mg, 0.21 mmol, 67.94% yield) was obtained as a yellow oil, which was used directly in the next step.

**Step 10: isopropyl (phenoxy(-5,5,5-trifluoro-4-(((tetrahydro-2H-pyran-2-yl)oxy)methyl)pent-3-en-1-yl)phosphoryl)-L-alani nate (compound 24-16)**

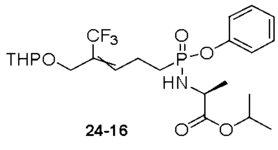

**24-16**

To a solution of bis(trimethylsilyl) (5,5,5-trifluoro-4-(((tetrahydro-2H-pyran-2-yl)oxy)methyl)pent-3-en-1-yl)phosphonate (90 mg, 0.19 mmol, 1 eq) in pyridine (5 mL) were added isopropyl (2S)-2-aminopropionate (26 mg, 0.19 mmol, 1 eq), phenol (110 mg, 1.16 mmol, 6 eq), triethylamine (295 mg, 2.92 mmol, 15 eq), triphenylphosphine (306 mg, 1.17 mmol, 6 eq) and 1,2-di(pyridin-2-yl)disulfane (257 mg, 1.17 mmol, 6 eq) under nitrogen protection, and the mixture was stirred at 50 °C for 16 hours. After the reaction was completed, the filtrate was concentrated under reduced pressure to give a residue. The residue was purified by preparative TLC to give isopropyl (phenoxy((Z)-5,5,5-trifluoro-4-(((tetrahydro-2H-pyran-2-yl)oxy)methyl)pent-3-en-1-yl)phosphoryl)-L-alaninat e (10 mg, 0.02 mmol, 8.65% yield), the target product, as a white solid.

MS (ESI) *m/z:* calcd. 508.2 $[M+H]^+$, found 508.1 $[M+H]^+$.

**Step 11: isopropyl (phenoxy((Z)-5,5,5-trifluoro-4-(hydroxymethyl)pent-3-en-1-yl)phosphoryl)-L-alaninate (compound 24)**

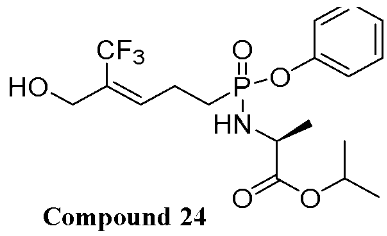

**Compound 24**

To a solution of isopropyl (phenoxy(-5,5,5-trifluoro-4-(hydroxymethyl)pent-3-en-1-yl)phosphoryl)-L-alaninate (10 mg, 0.02 mmol, 1 eq) in water (2 mL) was added 1N hydrochloric acid solution dropwise until pH = 4-5, and the mixture was stirred at 25 °C for 2 hours. After the reaction was completed, the reaction solution was purified by preparative HPLC (Colμmn: Gemini-C18 150 x 21.2 mm, 5 μm, mobile phase: ACN/$H_2O$ from 25% to 50% over 40 mins) to obtain isopropyl (phenoxy((Z)-5,5,5-trifluoro-4-(hydroxymethyl)pent-3-en-1-yl)phosphoryl)-L-alaninate (3.1 mg, 37.16% yield)

$^1$H NMR (400 MHz, $CDCl_3$) δ = 7.34 - 7.30 (m, 2H), 7.20 - 7.14 (m, 3H), 6.18 - 6.15 (m, 1H), 4.99 - 4.94 (m, 1H), 4.23 (s, 2H), 4.06 - 3.90 (m, 1H), 3.49 - 3.42 (m, 0.5H), 3.25 - 3.21 (m, 0.5H), 2.78 - 2.68 (m, 2H), 2.12 - 1.96 (m, 2H), 1.30 - 1.18 (m, 9H);
$^{31}$P NMR (162 MHz, $CDCl_3$) δ = 29.94, 29.75;
$^{19}$F NMR (376 MHz, $CDCl_3$) δ = -60.09, -59.98;
MS (ESI) *m/z:* calcd. 424.1 $[M+H]^+$, found 424.1 $[M+H]^+$.

**Example 25:**

**2-Hydroxyethyl (((E)-5-hydroxy-4-methylpent-3-en-1-yl)(phenoxy)phosphoryl)-L-alaninate (compound 25)**

The title compound was prepared according to the following scheme:

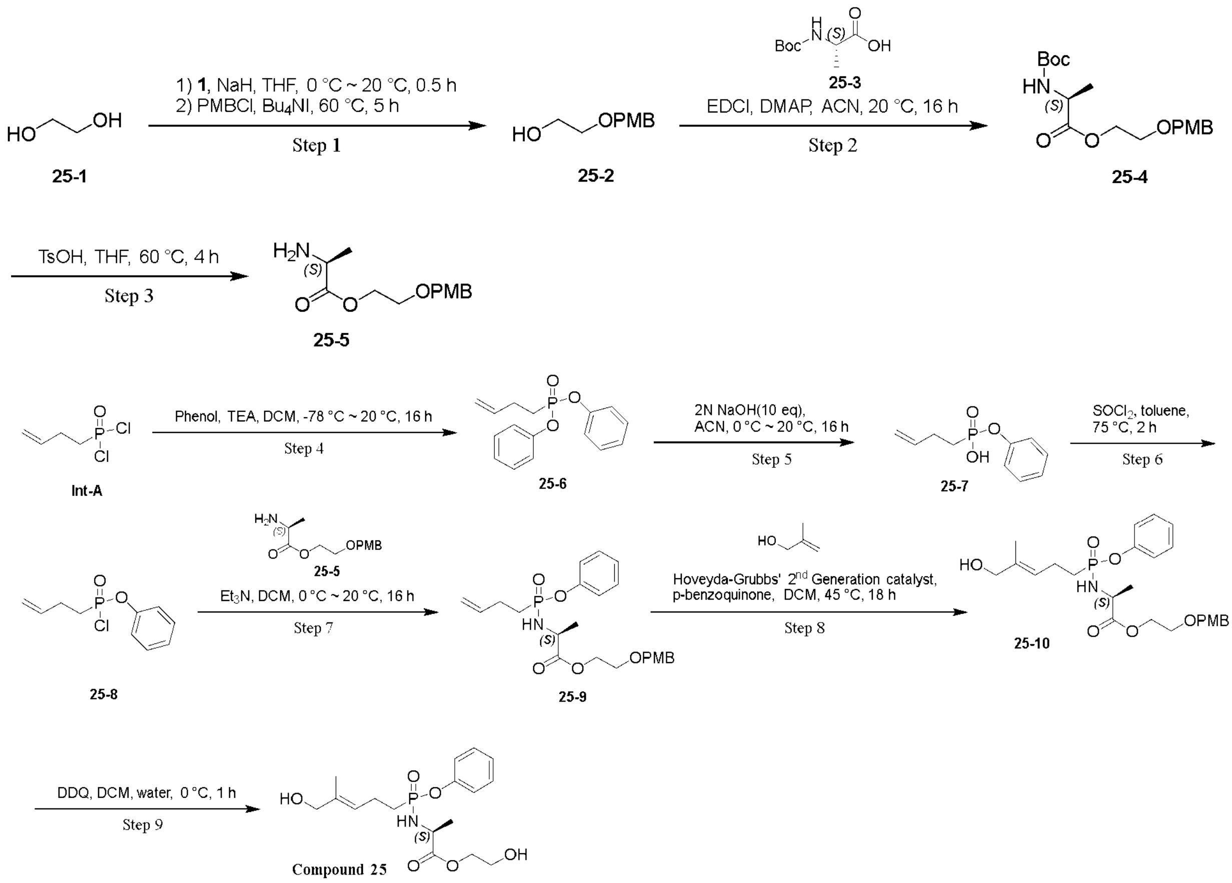


## Experimental operation

### Step 1: 2-((4-methoxybenzyl)oxy)ethan-1-ol (compound 25-2)

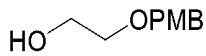

Compound 25-2

To a solution of ethylene glycol (5.94 g, 95.78 mmol, 1 eq) in tetrahydrofuran (60 mL) was added sodium hydride (3.84 g, 95.78 mmol, 60%w/w, 1 eq) in batches at 0 °C and under nitrogen protection, and the mixture was stirred at 20 °C for 0.5 hours. p-methoxybenzyl chloride (15.0 g, 95.78 mmol, 1 eq) and tetrabutylammonium iodide (3.54 g, 9.58 mmol, 0.1 eq) were added to the reaction solution at 20 °C and under nitrogen protection, and the mixture was stirred at 60 °C for 5 hours. TLC (PE:EA=1:1) showed that the reaction was completed and a new spot with less polarity was found. The mixture was cooled to room temperature and quenched by adding saturated ammonium chloride solution (100 mL). The mixture was extracted with ethyl acetate (100 mL*3). The combined organic layers were dried over anhydrous sodium sulfate and concentrated under reduced pressure to give a residue. The residue was purified by column chromatography on silica gel eluting with 0-50% petroleum ether/ethyl acetate to give 2-((4-methoxybenzyl)oxy)ethan-1-ol (13 g, 71.34 mmol, 74.48% yield) as a yellow oil.

$^1H$ NMR (400 MHz, $CDCl_3$) δ = 7.29 - 7.27 (m, 2H), 6.91 - 6.88 (m, 2H), 4.50 (s, 2H), 3.81 (s, 3H), 3.76 - 3.74 (m, 2H), 3.59 - 3.57 (m, 2H).

### Step 2: 2-((4-methoxybenzyl)oxy)ethyl (tert-butoxycarbonyl)-L-alaninate (compound 25-4)

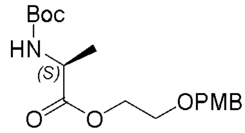

Compound 25-4

To a solution of 2-((4-methoxybenzyl)oxy)ethan-1-ol (12.0 g, 65.85 mmol, 1 eq) in acetonitrile (100 mL) were added BOC-L-alanine (14.95 g, 79.02 mmol, 1.2 eq), 1-(3-dimethylaminopropyl)-3-ethylcarbodiimide (13.89 g, 72.44 mmol, 1.1 eq) and 4-dimethylaminopyridine (8.85 g, 72.44 mmol, 1.1 eq) at 20 °C and under nitrogen protection, and the mixture was stirred at 20 °C for 16 hours. TLC (PE:EA=1:1) showed that the reaction was completed and a new spot with less polarity was found. The reaction mixture was diluted with ethyl acetate, washed with water and brine, dried over anhydrous sodium sulfate and concentrated under reduced pressure to obtain a residue. The residue was purified by column chromatography on silica gel eluting with 0-60% petroleum ether/ethyl acetate to give 2-((4-methoxybenzyl)oxy) ethyl (tert-butoxycarbonyl)-L-alaninate (12.4 g, 35.09 mmol, 53.29% yield) as a yellow oil.

$^1$H NMR (400 MHz, $CDCl_3$) δ = 7.20 - 7.18 (m, 2H), 6.83 - 6.80 (m, 2H), 4.42 (s, 2H), 4.29 - 4.23 (m, 3H), 3.74 - 3.73 (m, 3H), 3.59 (t, *J* = 4.8 Hz, 2H), 1.39 - 1.38 (m, 9H), 1.33 (d, *J* = 7.2 Hz, 3H).

**Step 3: 2-((4-methoxybenzyl)oxy)ethyl L-alaninate (compound 25-5)**

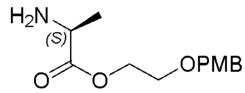

Compound 25-5

To a solution of 2-((4-methoxybenzyl)oxy)ethyl (tert-butyloxycarbonyl)-L-alaninate (5.0 g, 14.15 mmol, 1 eq) in tetrahydrofuran (40 mL) was added p-toluenesulfonic acid monohydrate (9.75 g, 56.60 mmol, 4 eq) at 20 °C and under nitrogen protection, and the mixture was stirred at 60 °C for 4 hours. LCMS showed that the raw material was completely consumed and that the product was detected. The reaction mixture was diluted with ethyl acetate, quenched with water, washed with brine, dried over anhydrous sodium sulfate and concentrated under reduced pressure to obtain a residue. The residue was purified by preparative HPLC (Waters Xbridge C18 150*50mm* 10μm; mobile phase: [water($NH_4HCO_3$)-ACN]; gradient: 30%-45% B over 5 min) to give 2-((4-methoxybenzyl)oxy)ethyl L-alaninate (0.6 g, 2.37 mmol, 16.75% yield) as a colorless oil.

MS (ESI) *m/z:* calcd. 254.1 $[M + H]^+$, found 254.1 $[M + H]^+$.

**Step 4: diphenyl butyl-3-en-1-ylphosphonate (compound 25-6)**

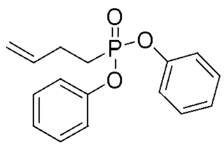

**25-6**

To a solution of 4-dichlorophosphorylbut-1-ene (20 g, 115.62 mmol, 1 eq) in dichloromethane (200 mL) was added triethylamine (23.40 g, 231.24 mmol, 2 eq) at -78 °C and under nitrogen protection, and the mixture was stirred at -78 °C for 5 minutes. A solution of phenol (23.94 g, 254.37 mmol, 2.2 eq) in dichloromethane (200 mL) was slowly added dropwise to the mixture at -78 °C and under nitrogen protection. The mixture was then stirred at 20 °C for 16 hours. LCMS showed that the raw material was completely consumed and that the product was detected. After the reaction was completed, the mixture was concentrated under reduced pressure to obtain a residue. The residue was purified by column chromatography on silica gel eluting with 0-20% petroleum ether/ethyl acetate to give diphenyl butyl-3-en-1-ylphosphonate (28.0 g, 97.13 mmol, 84.01% yield) as a colorless oil.

MS (ESI) *m/z:* calcd. 289.1 $[M + H]^+$, found 289.0 $[M + H]^+$.

**Step 5: phenyl hydrogen but-3-en-1-ylphosphonate (compound 25-7)**

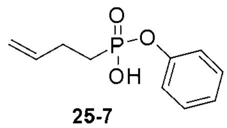

**25-7**

To a solution of diphenyl butyl-3-en-1-ylphosphonate (28.0 g, 97.13 mmol, 1 eq) in acetonitrile (300 mL) was added sodium hydroxide aqueous solution (2M, 485.65 mL, 10 eq) at 0 °C and under nitrogen protection, and the mixture was stirred at 20 °C for 16 hours. LCMS showed that the raw material was completely consumed and that the product was detected. The mixture was concentrated under reduced pressure to obtain a residue. The residue was purified by preparative HPLC (Waters Xbridge C18 150*50mm* 10μm; mobile phase: [water($NH_3$•$H_2O$)-ACN]; gradient: 20%-30% B over 5 min) to give phenyl hydrogen but-3-en-1-ylphosphonate (13.0 g, 61.27 mmol, 63.08% yield) as a colorless oil.
MS (ESI) *m/z:* calcd. 213.1 $[M + H]^+$, found 213.0 $[M + H]^+$.

**Step 6: phenyl but-3-en-1-ylphosphonochloridate (compound 25-8)**

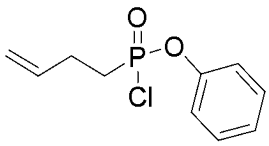

**25-8**

To a solution of phenyl hydrogen but-3-en-1-ylphosphonate (7.0 g, 32.99 mmol, 1 eq) in toluene (50 mL) was added thionyl chloride (19.62 g, 164.95 mmol, 5 eq) under nitrogen protection. The mixture was stirred at 75 °C for 2 hours. LCMS showed that the raw material was completely consumed and that the product was detected. The reaction mixture was cooled to room temperature and concentrated under reduced pressure to remove the solvent, to give phenyl but-3-en-1-ylphosphonochloridate (7.0 g, crude product) as a yellow oil, which was directly used in the next step without further treatment.

**Step 7: 2-((4-methoxybenzyl)oxy)ethyl (but-3-en-1-yl(phenoxy)phosphoryl)-L-alaninate (compound 25-9)**

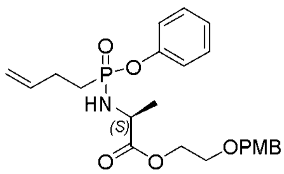

**25-9**

To a solution of phenyl but-3-en-1-ylphosphonochloridate (500 mg, 2.17 mmol, 1 eq) in dichloromethane (10 mL) was added triethylamine (438.76 mg, 4.34 mmol, 2 eq) at 0 °C and under nitrogen protection, and the mixture was stirred at 0 °C for 5 minutes. A solution of ethyl 2-((4-methoxybenzyl)oxy)L-alaninate (549.14 mg, 2.17 mmol, 1 eq) in dichloromethane (2 mL) was slowly added dropwise to the mixture at 0 °C and under nitrogen protection. The mixture was then stirred at 20 °C for 16 hours. LCMS showed that the raw material was completely consumed and that the product was detected. The mixture was concentrated under reduced pressure to obtain a residue. The residue was purified by column chromatography on silica gel eluting with 50-80% petroleum ether/ethyl acetate to give 2-((4-methoxybenzyl)oxy)ethyl (but-3-en-1-yl(phenoxy)phosphoryl)-L-alaninate (300 mg, 670.45 μmol, 30.92% yield) as a colorless oil.
MS (ESI) *m/z:* calcd. 448.2 $[M + H]^+$, found 448.2 $[M + H]^+$.

**Step** 8: **2-((4-methoxybenzyl)oxy)ethyl ((*E*)-5-hydroxy-4-methylpent-3-en-1-yl)(phenoxy)phosphoryl)-L-alaninate (compound 25-10)**

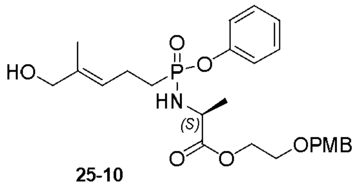

**25-10**

To a solution of 2-((4-methoxybenzyl)oxy)ethyl (but-3-en-1-yl(phenoxy)phosphoryl)-L-alaninate (300 mg, 670.45 μmol, 1 eq), 2-methylprop-2-en-1-ol (386.75 mg, 5.36 mmol, 8 eq) and 1,4-benzoquinone (7.25 mg, 67.05 μmol, 0.1 eq) in dichloromethane (6 mL) was added Hoveyda-Grubbs' 2nd Generation catalyst (84.02 mg, 134.09 μmol, 0.2 eq). The catalyst was added in three equal portions (28.01 mg, 44.70 μmol for each portion) at t = 0, 2 and 4 hours during the reaction. The solution was then heated to reflux for 18 hours at 45 °C and under nitrogen atmosphere. LCMS showed that the raw material was completely consumed and that the product was detected. The mixture was concentrated under reduced pressure to obtain a residue. The residue was purified by column chromatography on silica gel eluting with 0-10% ethyl acetate/methanol to give 2-((4-methoxybenzyl)oxy)ethyl ((E)-5-hydroxy-4-methylpent-3-en-1-yl)(phenoxy)phosphoryl)-L-alaninate (160 mg, 325.52 μmol, 48.55% yield), the target product, as a colorless oil.

MS (ESI) *m/z:* calcd. 492.2 $[M + H]^+$, found 492.2 $[M + H]^+$.

**Step** 9: **2-hydroxyethyl (((E)-5-hydroxy-4-methylpent-3-en-1-yl)(phenoxy)phosphoryl)-L-alaninate**

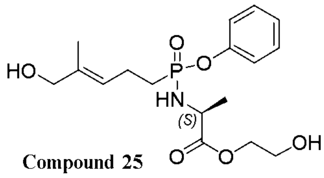

**Compound 25**

To a mixed solution of 2-((4-methoxybenzyl)oxy)ethyl ((E)-5-hydroxy-4-methylpent-3-en-1-yl)(phenoxy)phosphoryl)-L-alaninate (130 mg, 264.49 μmol, 1 eq) in dichloromethane (4 mL) and water (0.4 mL) was added dichlorodicyanobenzoquinone (72.05 mg, 317.39 μmol, 1.2 eq) at 0 °C and under nitrogen protection, and the mixture was stirred at 0 °C for 1 hour. LCMS showed that the raw material was completely consumed and that the product was detected. The reaction mixture was diluted with dichloromethane, quenched with water, washed with brine, dried over anhydrous sodium sulfate, filtered and concentrated under reduced pressure to obtain a residue. The residue was purified by preparative HPLC (Phenomenex Synergi C18 150*25mm* 10μm; mobile phase: [water(0.1%FA)-ACN]; B%: 30%-45%, 2 min) to give 2-hydroxyethyl (((E)-5-hydroxy-4-methylpent-3-en-1-yl)(phenoxy)phosphoryl)-L-alaninate (12.79 mg, 34.46 μmol, 13.03% yield), the target product, as a colorless oil.

$^1H$ NMR (400 MHz, $CDCl_3$) δ = 7.35 - 7.30 (m, 2H), 7.21 - 7.14 (m, 3H), 5.52 - 5.28 (m, 1H), 4.21 - 4.09 (m, 3H), 4.00 - 3.92 (m, 2H), 3.78 - 3.75 (m, 2H), 3.44 - 3.39 (m, 1H), 2.50 - 2.45 (m, 2H), 2.03 - 1.99 (m, 2H), 1.82 - 1.68 (m, 3H), 1.32 - 1.24 (m, 3H);
$^{31}P$ NMR (162 MHz, $CDCl_3$) δ = 32.89, 32.35;
MS (ESI) *m/z:* calcd. 372.2 $[M + H]^+$, found 394.1 $[M + Na]^+$;

**Example 26:**

**(R)-tetrahydrofuran-3-yl**

**2-((((E)-5-hydroxy-4-methylpent-3-en-1-yl)(phenoxy)phosphoryl)amino)-2-methylpropanoate (compound 26)**

The title compound was prepared according to the following scheme:

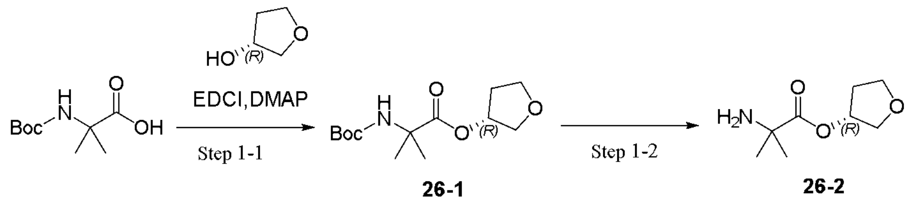


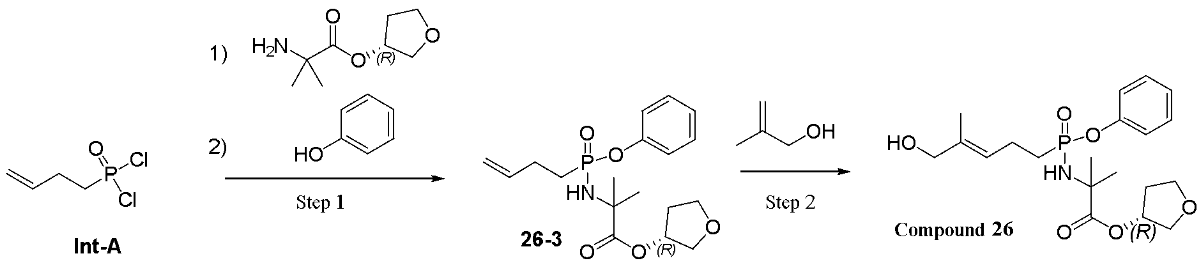


**Experimental operation**

**Step (R)-tetrahydrofuran-3-yl 2-((tert-butoxycarbonyl)amino)-2-methylpropanoate (26-1)**

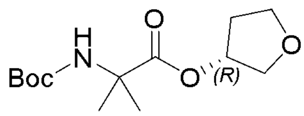

**26-1**

To a solution of 2-((tert-butoxycarbonyl)amino)-2-methylpropanoic acid (5 g, 22.5 mmol, 1.0 eq) in tetrahydrofuran (50 mL) were added 4-dimethylaminopyridine (3.01 g, 2.46 mmol 1.0 eq) and carbodiimide hydrochloride (9.35 g, 24.6 mmol, 1.0 eq) at 0 °C and under nitrogen protection. The resulting mixture was stirred under nitrogen protection at 0 °C for 0.5 hours, and (R)-tetrahydrofuran-3-ol (2.38 g, 27.0 mmol, 1.1 eq) was added to the reaction system. The reaction solution was stirred for 16 hours. After the reaction was completed, the reaction was quenched by adding water (100 mL). The mixture was extracted with dichloromethane (100 mL*3). The combined organic layer was washed with saturated brine, dried over anhydrous sodium sulfate, filtered and concentrated under reduced pressure to obtain a residue. The resulting residue was purified by silica gel chromatography eluting with 0-10% petroleum ether/ethyl acetate to give (R)-tetrahydrofuran-3-yl 2-((tert-butoxycarbonyl)amino)-2-methylpropanoate (5 g, 16.5 mmol, 67% yield) as a light yellow oil.

MS (ESI) m/z: calcd. 274.2 $[M+H]^+$, found 174.2 $[M+H-100]^+$.

**Step 1-2: (R)-tetrahydrofuran-3-yl 2-amino-2-methylpropanoate (26-2)**

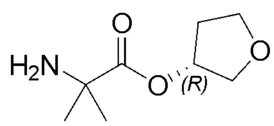

**26-2**

(R)-tetrahydrofuran-3-yl 2-((tert-butoxycarbonyl)amino)-2-methylpropanoate (5 g, 18.2 mmol) was dissolved in hydrochloride/ethyl acetate (2 mmol/mL, 10 mL) at 20 °C, and the resulting mixture was stirred at 20 °C for 1 hour. After the reaction was completed, it was quenched by adding a saturated sodium bicarbonate aqueous solution (10 mL) to the reaction system. The mixture was extracted with ethyl acetate (50 mL*2), and the combined organic layer was dried over anhydrous sodium sulfate and concentrated under reduced pressure to give (R)-tetrahydrofuran-3-yl 2-amino-2-methylpropanoate (4.2 g, crude product) as a white solid.

MS (ESI) m/z: calcd. 174.2 $[M + H]^+$, found 174.2 $[M + H]^+$.

**Step** 1: **(R)-tetrahydrofuran-3-yl 2-((but-3-en-1-yl(phenoxy)phosphoryl)amino)-2-methylpropanoate (26-3)**

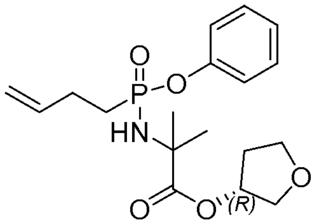

To a solution of but-3-en-1-ylphosphine dichloride (500 mg, 2.89 mmol, 1 eq) in dichloromethane (10 mL) were added (R)-tetrahydrofuran-3-yl 2-amino-2-methylpropanoate (500 mg, 2.89 mmol, 1 eq) and triethylamine (584 mg, 5.78 mmol, 2 eq) dropwise at -78 °C and under nitrogen protection. The resulting mixture was stirred for 6 hours at room temperature under nitrogen protection. The reaction solution was then cooled to -78 °C, followed by phenol (109 mg, 1.16 mmol, 1 eq) and triethylamine (584 mg, 5.78 mmol, 2 eq) were added, and the resulting mixture was stirred for 16 hours at room temperature under nitrogen protection. After the reaction was completed, it was quenched by adding water (20 mL) to the reaction solution. The mixture was extracted with dichloromethane (20 mL*2). The combined organic layer was dried over anhydrous sodium sulfate, filtered and concentrated under reduced pressure to give a residue. The resulting residue was purified by silica gel chromatography eluting with 20-40% petroleum ether/ethyl acetate to give (R)-tetrahydrofuran-3-yl 2-((but-3-en-1-yl(phenoxy)phosphoryl)amino)-2-methylpropanoate (150 mg, 0.41 mmol, 14.2% yield) as a yellow oil.

MS (ESI) *m/z:* calcd. 368.2 $[M + H]^+$, found 368.2 $[M + H]^+$.

**Step** 2: **(R)-tetrahydrofuran-3-yl 2-((((E)-5-hydroxy-4-methylpent-3-en-1-yl)(phenoxy)phosphoryl)amino)-2-methylpropanoate (compound 26)**

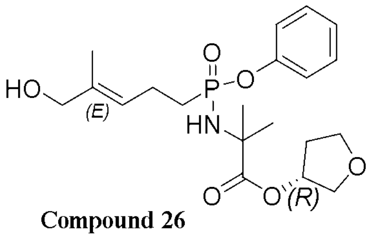

Compound 26

To a solution of (R)-tetrahydrofuran-3-yl 2-((but-3-en-1-yl(phenoxy)phosphoryl)amino)-2-methylpropanoate (150 mg, 0.41 mmol, 1 eq) in dichloromethane (5 mL) were added 2-methylprop-2-en-1-ol (58.9 mg, 0.82 mmol, 2 eq), HOVEYDA-GRUBBS catalyst (25.6 mg, 0.04 mmol, 0.1 eq) and p-benzoquinone (1 mg, 0.01 mmol, 0.1 eq) at 20 °C under nitrogen protection, and the resulting mixture was stirred at 40°C under nitrogen protection for 16 hours. After the reaction was completed, the mixture was concentrated under reduced pressure to obtain a residue. The residue was purified by HPLC: column: Gemini-C18 150 x 21.2 mm, 5 μm, mobile phase: ACN-$H_2O$, gradient: 27%-95%, flow rate: 20 ml/min, ACN (%): 45, retention time: 13 min to give the title **compound** (12.7 mg, 0.03 mmol, 7.5% yield) as a colorless oil.

$^1H$ NMR (400 MHz, $CDCl_3$) δ 7.34 - 7.30 (m, 2H), 7.23 - 7.21 (m, 2H), 7.16-7.12 (m, 1H), 5.50-5.46 (m, 1H), 5.31 - 5.29 (m, 1H), 4.01 (s, 2H), 3.90-3.86 (m, 3H), 3.81 - 3.77 (m, 1H), 3.66 - 3.64 (m, 1H), 2.46-2.39 (m, 2H), 2.19-2.13 (m, 1H), 1.98 - 1.91 (m, 3H), 1.70 (s, 3H), 1.55 (s, 3H), 1.45 (s, 3H).
$^{31}P$ NMR (162 MHz, $CDCl_3$) δ = 30.51;
MS (ESI) m/z: calcd. 412.2 $[M+H]^+$, found 412.1 $[M+H]^+$.

**Example 27:**

**(R)-tetrahydrofuran-3-yl**

**(phenoxy((Z)-5,5,5-trifluoro-4-(hydroxymethyl)pent-3-en-1-yl)phosphoryl)-L-alaninate (compound 27)**

Example 27 was synthesized using benzyloxycarbonyl-L-alanine and (R)-3-hydroxytetrahydrofuran as raw materials according to the method of Example 24.

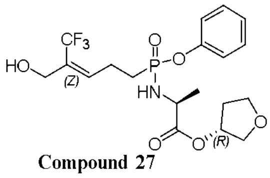

Compound 27

$^1H$ NMR (400 MHz, $CDCl_3$) δ 7.34-7.30 (m, 2H), 7.21 - 7.13 (m, 3H), 6.17 - 6.14 (m, 1H), 5.27 - 5.23 (m, 1H), 4.21 (s, 2H), 4.09 - 4.00 (m, 1H), 3.90 - 3.80 (m, 3H), 3.74 - 3.72 (m, 1H), 3.43 (t, *J* = 20Hz, 0.5H), 3.23 (t, *J* = 20Hz, 0.5H), 2.74 - 2.64 (m, 3H), 2.19-1.88 (m, 4H), 1.31 (d, *J* = 7.2Hz, 1.5H), 1.22 (d, *J* = 7.2Hz, 1.5H).
$^{31}P$ NMR (162 MHz, $CDCl_3$) δ = 29.72, 29.42;
$^{19}F$ NMR (376.5 MHz, $CDCl_3$) δ = -60.00, -60.10;
MS (ESI) m/z: calcd. 452.1 $[M+H]^+$, found 452.1 $[M+H]^+$.

**Example 28:**

**1,1-Dioxotetrahydro-2H-thiopyran-4-yl**

**(((E)-5-hydroxy-4-methylpent-3-en-1-yl)(phenoxy)phosphoryl)-L-alaninate (compound 28)**

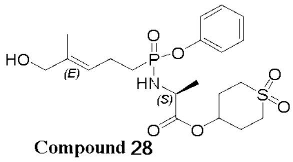

Compound 28

Example 28 was synthesized using benzyloxycarbonyl-L-alanine and 4-hydroxytetrahydro-2H-thiopyran-1,1-dioxide as raw materials according to the method of Example 2.

$^1H$ NMR (400 MHz, $CDCl_3$) δ 7.34-7.28 (m, 2H), 7.20 - 7.11 (m, 3H), 5.50 - 5.43 (m, 1H), 5.06 - 5.04 (m, 0.5H), 5.01 - 4.99 (m, 0.5H), 4.16 - 4.08 (m, 1H), 4.00 (s, 2H), 3.16 - 3.09 (m, 2H), 2.96 - 2.91 (m, 2H), 2.49 - 2.39 (m, 2H), 2.36 - 2.18 (m, 4H), 2.03-1.90 (m, 2H), 1.81 (brs, 1H), 1.70 (s, 1.5H), 1.69 (s, 1.5H), 1.35 (d, *J* = 6.8Hz, 1.5H), 1.22(d, *J* = 7.2Hz, 1.5H).
$^{31}P$ NMR (162 MHz, $CDCl_3$) δ = 32.03, 31.73;
MS (ESI) m/z: calcd. 460.2 $[M+H]^+$, found 460.2 $[M+H]^+$.

**Example 29:**

**Compound 29**

Example 29 was synthesized using benzyloxycarbonyl-L-alanine and 4-hydroxypiperidine as raw materials according to the method of Example 2.

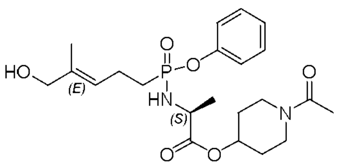

Compound 29

$^1$H NMR (400 MHz, $CDCl_3$) δ 7.32 - 7.27 (m, 2H), 7.19 - 7.10 (m, 3H), 5.48 - 5.42 (m, 1H), 4.98 - 4.89 (m, 1H), 4.14 - 4.01 (m, 1H), 3.99 (s, 3H), 3.82 - 3.75 (m, 1H), 3.60 - 3.53 (m, 2H), 3.44 - 3.25 (m, 3H), 2.51 - 2.38 (m, 2H), 2.07 (s, 3H), 1.86 - 1.78 (m, 2H), 1.69 (s, 1.5H), 1.68 (s, 1.5H), 1.61 - 1.55 (m, 2H), 1.33 - 1.30 (m, 1.5H), 1.21 - 1.18 (m, 1.5H).
$^{31}$P NMR (162 MHz, $CDCl_3$) δ = 32.05, 31.98, 31.66, 31.62;
MS (ESI) m/z: calcd. 453.2 $[M+H]^+$, found 453.2 $[M+H]^+$.

**Example 30:**

**2-Oxaspiro[3.3]heptan-6-yl**

**(((E)-5-hydroxy-4-methylpent-3-en-1-yl)(phenoxy)phosphoryl)-L-alaninate (compound 30)**

Example 30 was synthesized using benzyloxycarbonyl-L-alanine and 2-oxaspiro[3.3]heptan-6-ol as raw materials according to the method of Example 2.

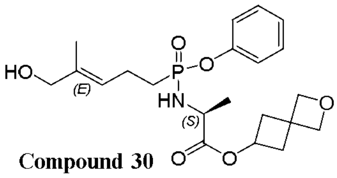

Compound 30

$^1$H NMR (400 MHz, $CDCl_3$) δ 7.32 - 7.27 (m, 2H), 7.19 - 7.11 (m, 3H), 5.45 (q, *J* = 8.0Hz, 1H), 4.81 - 4.76 (m, 1H), 4.67 (s, 2H), 4.64 - 4.60 (m, 2H), 4.08 - 3.95 (m, 3H), 3.31 (t, *J* = 10.4Hz, 0.4H), 3.20 (t, *J* = 10.8Hz, 0.6H), 2.69 - 2.64 (m, 2H), 2.48 - 2.40 (m, 2H), 2.23-2.13 (m, 2H), 2.01 - 1.83 (m, 2H), 1.69 (s, 1.8H), 1.68 (s, 1.2H), 1.31 (d, *J* = 6.8Hz, 1.2H), 1.22 (d, *J* = 6.8Hz, 1.8H).
$^{31}$P NMR (162 MHz, $CDCl_3$) δ = 31.98, 31.56;
MS (ESI) m/z: calcd. 424.2 $[M+H]^+$, found 424.0 $[M+H]^+$.

**Example 31:**

**Compound 31**

Example 31 was synthesized using benzyloxycarbonyl-L-alanine and 4-hydroxypiperidine as raw materials according to the method of Example 2.

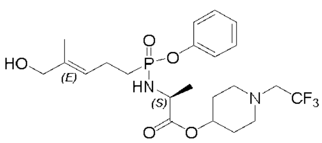

Compound 31

$^1$H NMR (400 MHz, $CDCl_3$) δ 7.34 - 7.29 (m, 2H), 7.21 - 7.12 (m, 3H), 5.47 (dd, *J1* = 7.6Hz, *J2* = 7.6Hz, 1H), 4.84 - 4.78 (m, 1H), 4.14 - 4.03 (m, 1H), 4.01 (s, 2H), 3.43 (t, *J* = 10Hz, 0.5H), 3.31 (t, *J* = 10.4 Hz, 0.5H), 3.08 - 3.06 (m, 2H), 2.88 (brs, 2H), 2.68 (brs, 2H), 2.55-2.40 (m, 2H), 2.03-1.86 (m, 4H), 1.80 - 1.74 (m, 2H),1.71 (s, 1.5H), 1.70 (s, 1.5H), 1.33 (d, *J* = 7.2Hz, 1.5H), 1.21 (d, *J* = 7.2Hz, 1.5H).
$^{31}$P NMR (162 MHz, $CDCl_3$) δ = 31.44, 31.10;
MS (ESI) m/z: calcd. 493.2 $[M+H]^+$, found 493.1 $[M+H]^+$.

**Example 32:**

**(3R,3aS,6aR)-hexahydrofuran[2,3-b]furan-3-yl**

**(((E)-5-hydroxy-4-methylpent-3-en-1-yl)(phenoxy)phosphoryl)-L-alaninate (compound 32)**

Example 32 was synthesized using benzyloxycarbonyl-L-alanine and (3R,3aS,6aR)-hexahydrofurano[2,3-b]furan-3-ol as raw materials according to the method of Example 2.

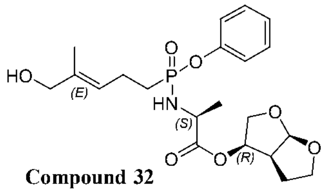

Compound 32

$^1H$ NMR (400 MHz, $CDCl_3$) δ 7.30 - 7.29 (m, 2H), 7.20 -7.19 (m, 2H), 7.16-7.14 (t, J = 6.7 Hz, 1H), 5.71 (d, *J* = 4.8 Hz, 1H), 5.52 - 5.35 (m, 1H), 5.13 (p, *J* = 6.6 Hz, 1H), 4.17 - 4.07 (m, 1H), 4.06 - 4.00 (m, 1H), 3.98 (s, 2H), 3.93 (m, 1H), 3.85 (p, *J* = 8.5, 7.7 Hz, 1H), 3.77 - 3.67 (m, 1H), 3.58 (d, J = 9.7 Hz, 0.5H), 3.43 (d, *J* = 9.8 Hz, 0.5H), 3.03 (m, 1H), 2.71-2.61 (m, 1H), 2.44-2.41 (m, 2H), 1.97 - 1.81 (m, 4H), 1.68 (s, 3H), 1.36 (d, *J* = 6.9 Hz, 1.5H), 1.24 (d, *J* = 6.6 Hz, 1.5H).
$^{31}P$ NMR (162 MHz, $CDCl_3$) δ = 31.48, 31.16;
MS (ESI) m/z: calcd. 440.2 $[M+H]^+$, found 422.2 $[M+H-H_2O]^+$.

**Example 33:**

**Compound 33**

Example 33 was synthesized using benzyloxycarbonyl-L-alanine and (2R,4R)-2-methyloxan-4-ol as raw materials according to the method of Example 2.

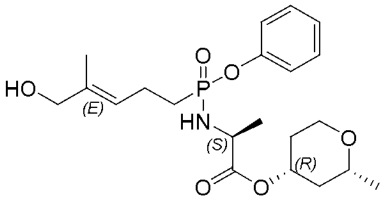

**Compound 33**

$^1H$ NMR (400 MHz, $CDCl_3$) δ 7.31 - 7.27 (m, 2H), 7.20-7.17 (m, 2H), 7.14 - 7.10 (m, 1H), 5.46 - 5.42 (m, 1H), 4.84 - 4.78 (m, 1H), 4.06-4.00 (m, 1H), 3.99 (s, 2H), 3.97-3.95 (m, 1H), 3.46 - 3.26 (m, 3H), 2.49 - 2.40 (m, 2H), 2.01 - 1.77 (m, 4H), 1.69 (s, 1.5H), 1.68 (s, 1.5H), 1.62 - 1.46 (m, 1H), 1.29 (d, J = 7.0 Hz, 1.5H), 1.28 - 1.21 (m, 1H), 1.20 - 1.16 (m, 4.5H).
$^{31}P$ NMR (162 MHz, $CDCl_3$) δ = 31.99, 31.59;
MS (ESI) m/z: calcd. 426.2 $[M+H]^+$, found 408.1 $[M+H-H_2O]^+$.

**Example 34:**

(2R,4r,6S)-2,6-dimethyltetrahydro-2H-pyran-4-yl

(((E)-5-hydroxy-4-methylpent-3-en-1-yl)(phenoxy)phosphoryl)-L-alaninate (compound 34)

The title compound was prepared according to the following scheme:

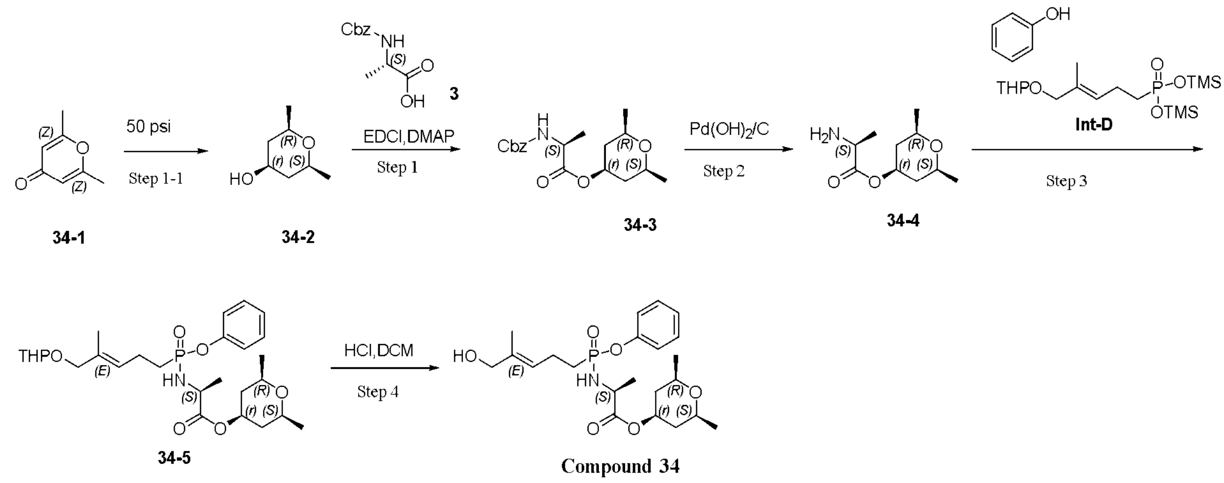


34-1 34-2 34-3 34-4 34-5 Compound 34

**Experimental operation**

**Step 1-1: (2R,4r,6S)-2,6-dimethyltetrahydro-2H-pyran-4-ol (34-2)**

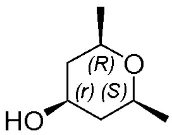

To a solution of 2,6-dimethyl-4H-pyran-4-one (4.00 g, 32.2 mmol, 1 eq) in ethanol (40 mL) was added 10% $Pd(OH)_2/C$ (3.39 g, 6.45 mmol, 0.1 eq) at room temperature, and the resulting mixed solution was placed in a 50 psi hydrogen reactor. The reaction system was stirred at 35 °C for 19 hours. After the reaction was completed, the mixture was filtered and concentrated under reduced pressure to give a crude product of (2R,4r,6S)-2,6-dimethyltetrahydro-2H-pyran-4-ol (3.2 g, 24.61 mmol, yield 76.92%) as a light yellow oil.

$^1H$ NMR (400 MHz, $CDCl_3$) $\delta$ 3.68 - 3.77 (m, 1H), 3.37-3.45 (m, 2H), 2.11 (s, 1H), 1.87 (dd, *J* = 12.1, *J*=4.7 Hz, 2H),1.18 (d, *J* = 6.2 Hz, 6H), 1.05 - 1.15 (m, 2H).

**Step 1: (2R,4r,6S)-2,6-dimethyltetrahydro-2H-pyran-4-yl ((benzyloxy)carbonyl)-L-alaninate (34-3)**

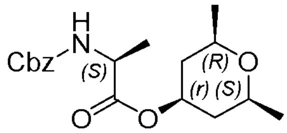

To a solution of ((benzyloxy)carbonyl)-L-alanine (2.00 g, 8.97 mmol, 1 eq) and (2R,4r,6S)-2,6-dimethyltetrahydro-2H-pyran-4-ol (1.28 g, 9.87 mmol, 1.1 eq) in dichloromethane (20 mL) were added 1-ethyl-(3-dimethylaminopropyl) carbodiimide hydrochloride (2.70 g, 13.46 mmol, 1.5 eq) and 4-dimethylaminopyridine (0.22 g, 1.79 mmol, 0.2 eq) at room temperature under nitrogen protection, and the resulting mixed solution was stirred for 16 hours at room temperature under nitrogen protection. After the reaction was completed, the mixture was concentrated under reduced pressure. The residue was purified by column chromatography on silica gel eluting with 10% petroleum ether/ethyl acetate to give (2R,4r,6S)-2,6-dimethyltetrahydro-2H-pyran-4-yl ((benzyloxy)carbonyl)-L-alaninate (1.80 g, 5.37 mmol, 60.37% yield) as a colorless oil.

MS (ESI) m/z: calcd. 336.2 $[M + H]^+$, found 336.0 $[M + H]^+$.

**Step 2: (2R,4R,6S)-2,6-dimethyltetrahydro-2H-pyran-4-yl L-alaninate (34-4)**

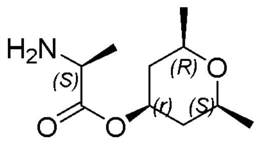

**34-4**

To a solution of (2R,4r,6S)-2,6-dimethyltetrahydro-2H-pyran-4-yl ((benzyloxy)carbonyl)-L-alaninate (1.80 g, 5.37 mmol, 1 eq) in tetrahydrofuran (20 mL) was added 20% palladium hydroxide on carbon (0.75 g, 1.34 mmol, 0.25 eq) at room temperature, and the system was purged three times with hydrogen gas. The resulting mixture was stirred for 3 hours at 35°C under hydrogen protection. After the reaction was completed, the mixture was filtered and concentrated under reduced pressure to give (2R,4r,6S)-2,6-dimethyltetrahydro-2H-pyran-4-yl L-alaninate (0.91 g, 4.50 mmol, yield 83.89%) as a colorless oil.

MS (ESI) m/z: calcd. 202.1 $[M + H]^+$, found 202.2 $[M + H]^+$.

**Step 3: (2R,4r,6S)-2,6-dimethyltetrahydro-2H-pyran-4-yl (((E)-4-methyl-5-((tetrahydro-2H-pyran-2-yl)oxy) pent-3-en-1-yl)(phenoxy)phosphoryl)-L-alaninate (34-5)**

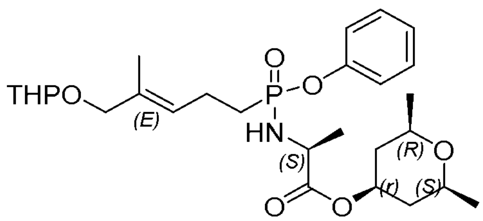

**34-5**

To a solution of (trimethylsilyl) (E)-(4-methyl-5-((tetrahydro-2H-pyran-2-yl)oxy)pent-3-en-1-yl)phosphonate (500 mg, 1.22 mmol, 1 eq) in pyridine (5 mL) were added (2R,4r,6S)-2,6-dimethyltetrahydro-2H-pyran-4-yl L-alaninate (248.46 mg, 1.23 mmol, 1 eq), phenol (691 mg, 7.35 mmol, 6 eq), triethylamine (1.86 g, 18.38 mmol, 15 eq), triphenylphosphine (1.93 mg, 7.35 mmol, 6 eq) and 1,2-di(pyridin-2-yl)disulfane (1.62 g, 7.35 mmol, 6 eq) under nitrogen protection, and the resulting mixture was stirred at 50 °C for 16 hours. After the reaction was completed, the reaction was filtered, and the filtrate was concentrated under reduced pressure. The resulting residue was purified by column chromatography on silica gel eluting with 50-100% petroleum ether/ethyl acetate to give a crude product, (2R,4r,6S)-2,6-dimethyltetrahydro-2H-pyran-4-yl (((E)-4-methyl-5-((tetrahydro-2H-pyran-2-yl)oxy)pent-3-en-1-yl) (phenoxy)phosphoryl)-L-alaninate (230 mg, 0.44 mmol, yield 35.98%) as a light yellow oil.

MS (ESI) m/z: calcd. 524.3 $[M+H]^+$, found 546.2 $[M+Na]^+$.

**Step 4: (2R,4r,6S)-2,6-dimethyltetrahydro-2H-pyran-4-yl (((E)-5-hydroxy-4-methylpent-3-en-1-yl)(phenoxy) phosphoryl)-L-alaninate (compound 34)**

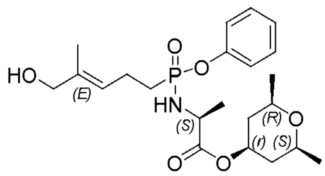

**Compound 34**

To a solution of (2R,4r,6S)-2,6-dimethyltetrahydro-2H-pyran-4-yl (((E)-4-methyl-5-((tetrahydro-2H-pyran-2-yl) oxy)pent-3-en-1-yl)(phenoxy)phosphoryl)-L-alaninate (230 mg, 0.44 mmol) in dichloromethane (5 mL) was added a solution of 2N hydrochloric acid in ethyl acetate (1 ml) dropwise at room temperature, and the resulting mixture was stirred at 25 °C for 2 hours. After the reaction was completed, the reaction mixture was concentrated under reduced pressure and

the crude product was purified by preparative HPLC (Colμmn: Gemini-C18 150 x 21.2 mm, Flow Rate: 20 ml/min, mobile phase: ACN/$H_2O$ from 0% to 60% over 7 mins) to give (2R,4r,6S)-2,6-dimethyltetrahydro-2H-pyran-4-yl (((E)-5-hydroxy-4-methylpent-3-en-1-yl)(phenoxy)phosphoryl)-L-alaninate (82.60 mg, 0.19 mmol, 43.18% yield) as a colorless oil.

$^1$H NMR (400 MHz, $CDCl_3$) $\delta$ 7.33 - 7.27 (m, 2H), 7.21 (d, $J$ = 8.7 Hz, 2H), 7.14 (td, $J$ = 7.5, J=3.2 Hz, 1H), 5.47 (m, 1H), 4.88-4.82 (m, 1H), 4.08 - 4.04 (m, 0.6H), 4.01 (s, 2H), 3.99-3.96 (m, , 0.4H), 3.53-3.46 (m, 2H), 3.37 (t, $J$ = 10.6 Hz, 0.5H), 3.27 (t, $J$ = 10.6 Hz, 0.5H), 2.51-2.40 (m, 2H), 2.01 - 1.93 (m, 2H), 1.91 - 1.85 (m, 2H), 1.71 (d, $J$ = 5.4 Hz, 3H), 1.31 (d, $J$ = 7.0 Hz, 1.5H), 1.23 - 1.14 (m, 9.5H).
$^{31}$P NMR (162 MHz, $CDCl_3$) $\delta$ = 31.35, 30.97;
MS (ESI) m/z: calcd. 440.2 $[M+H]^+$, found 440.3 $[M+H]^+$.

**Example 35:**

**(R)-2-piperidone-4-yl**

**(((E)-5-hydroxy-4-methylpent-3-en-1-yl)(phenoxy)phosphoryl)-L-alaninate (compound 35)**

Example 35 was synthesized using benzyloxycarbonyl-L-alanine and (R)-4-hydroxypiperidin-2-one as raw materials according to the method of Example 34.

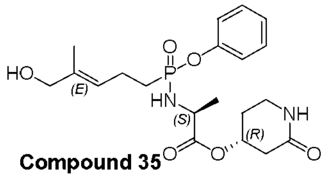

**Compound 35**

$^1$H NMR (400 MHz, $CDCl_3$) $\delta$ 7.32 (t, $J$ = 7.8 Hz, 2H), 7.25 - 7.18 (m, 2H), 7.15 (t, $J$ = 7.3 Hz, 1H), 6.64 (s, 1H), 5.52 - 5.39 (m, 1H), 5.17-5.15 (m, 1H), 4.15 - 3.95 (m, 3H), 3.67 (t, $J$ = 10.2 Hz, 0.6H), 3.51 - 3.45 (m, 0.4H), 3.44 - 3.41 (m, 1H), 3.35 - 3.24 (m, 1H), 2.67 (d, $J$ = 5.2 Hz, 0.4H), 2.63 (d, $J$ = 4.8 Hz, 0.4H), , 2.49 - 2.39 (m, 3H), 2.02 - 1.84 (m, 4H), 1.70 (s, 1.2H), 1.68 (s, 1.8H), 1.30 (d, $J$ = 7.0 Hz, 1.8H), 1.24 (d, $J$ = 7.1 Hz, 1.2H).
$^{31}$P NMR (162 MHz, $CDCl_3$) $\delta$ = 31.51, 31.12;
MS (ESI) m/z: calcd. 425.2 $[M+H]^+$, found 424.8 $[M+H]^+$.

**Example 36:**

**1-Cyanocyclopentyl**

**(((E)-5-hydroxy-4-methylpent-3-en-1-yl)(phenoxy)phosphoryl)-L-alaninate (compound 36)**

Example 36 was synthesized using benzyloxycarbonyl-L-alanine and 1-hydroxycyclopentane-1-carbonitrile as raw materials according to the method of Example 34.

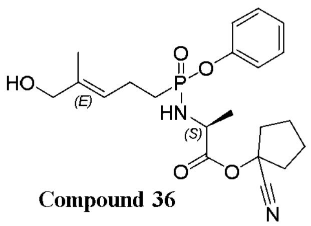

**Compound 36**

$^1$H NMR (400 MHz, $CDCl_3$) $\delta$ 7.32 - 7.27 (m, 2H), 7.19 - 7.17 (m, 2H), 7.14 - 7.10 (m, 1H), 5.45 (q, J = 7.8, 7.4 Hz, 1H), 4.15-4.03 (m, 1H), 3.992 (s, 1H), 3.985 (s, 1H), 3.44 - 3.37 (m, 0.5H), 3.28 - 3.22 (m, 0.5H), 2.48 - 2.38 (m, 2H), 2.36 - 2.23 (m, 2H), 2.21 - 2.04 (m, 2H), 2.01 - 1.87 (m, 3H), 1.83 - 1.71 (m, 3H), 1.68 (s, 1.5H), 1.67 (s, 1.5H), 1.32 (d, $J$ = 7.1 Hz, 1.5H), 1.20 (d, $J$ = 7.2 Hz, 1.5H).
$^{31}$P NMR (162 MHz, $CDCl_3$) $\delta$ = 32.00, 31.58;
MS (ESI) m/z: calcd. 421.2 $[M+H]^+$, found 403.1 $[M+H-H_2O]^+$.

**Example 37:**

**Tetrahydro-2H-pyran-4-yl**

**(((E)-1,1-difluoro-5-hydroxy-4-methylpent-3-en-1-yl)(phenoxy)phosphoryl)-L-alaninate (compound 37)**

The title compound was prepared according to the following scheme:

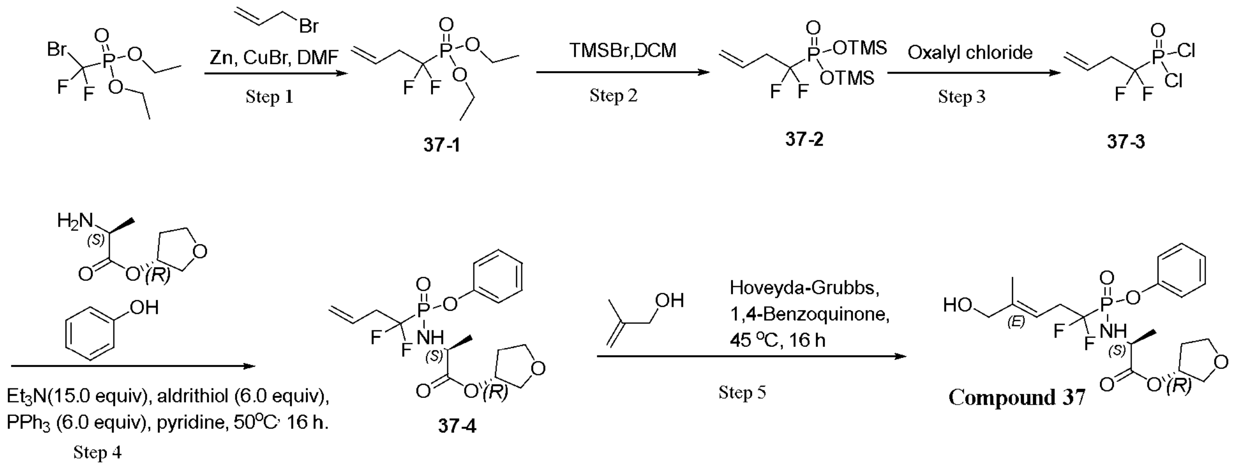


**Step 1: diethyl (1,1-difluorobut-3-en-1-yl)phosphonate (compound 37-1)**

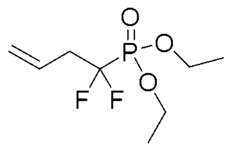

**37-1**

To a solution of activated zinc powder (1.22 g, 18.7 mmol) in anhydrous N,N-dimethylformamide (9.5 mL) was added diethyl bromofluoromethylphosphonate (3.3 mL, 18.6 mmol) dropwise under nitrogen protection. The resulting mixture was stirred for 3 hours under nitrogen protection at room temperature, and then cuprous bromide (2.66 g, 18.5 mmol) was added. After stirring at room temperature, allyl bromide (2.26 g, 18.5 mmol) was slowly added dropwise to the reaction system. The resulting mixture was stirred at room temperature for 16 hours, filtered, and the filtrate was evaporated to dryness with rotation. The crude product was purified by column chromatography on silica gel eluting with 60-70 % petroleum ether/ethyl acetate to give diethyl (1,1-difluorobut-3-en-1-yl)phosphonate (1.70 g, 7.46 mmol, 40.09% yield) as a yellow oil.

$^1$H NMR (400 MHz, $CDCl_3$) $\delta$ 5.86 - 5.8 (m, 1H), 5.3 - 5.26 (m, 2H), 4.27-4.24 (m, 4H), 2.87-2.78 (m, 2H), 1.38-1.24 (m, 6H).
$^{31}$P NMR (400 MHz, $CDCl_3$) $\delta$ = -111.12, -111.40;
MS (ESI) m/z: calcd. 229.1 $[M + H]^+$, found 229.1 $[M + H]^+$.

**Step 2 & 3: (1,1-difluorobut-3-en-1-yl)phosphonic dichloride (compound 37-3)**

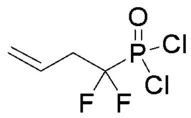

**37-3**

To a solution of diethyl (1,1-difluorobut-3-en-1-yl)phosphonate (4.00 g, 17.54 mmol, 1 eq) in dichloromethane (15 mL) was added TMSBr (26.84 g, 175.44 mmol, 10 eq) dropwise under nitrogen protection at 0 °C. The resulting mixture was stirred at room temperature for 16 hours. After the reaction was completed, the mixture was concentrated under reduced pressure. The mixture was dissolved in anhydrous dichloromethane (50 mL), the reaction solution was cooled to 0 °C, and then two drops of DMF and oxalyl chloride (6.68 g, 52.62 mmol, 3 eq) were added to the reaction system under nitrogen protection, and stirred at room temperature for 16 hours. After the reaction was completed, the mixture was concentrated under reduced pressure to give 3.6 g of crude (1,1-difluorobut-3-en-1-yl)phosphonic dichloride as a yellow-brown oil-solid mixture.

**Step 4: (R)-tetrahydrofuran-3-yl ((1,1-difluorobut-3-en-1-yl)(phenoxy)phosphoryl)-L-alaninate (compound 37-4)**

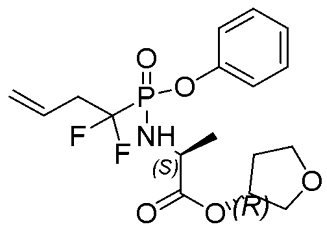

**37-4**

To a solution of (1,1-difluorobut-3-en-1-yl)phosphonic dichloride (2.30 g, 11.06 mmol, 1 eq) in dichloromethane (40 mL) was added (**R**)-tetrahydrofuran-3-yl L-alaninate (1.76 g, 11.06 mmol, 1 eq) and triethylamine (2.23 g, 22.12 mmol, 2 eq) dropwise at -78 °C and under nitrogen protection, and the resulting mixture was stirred at room temperature for 6 hours. The mixture was then cooled to -78 °C, followed by phenol (1.048 g, 11.06 mmol, 1 eq) and triethylamine (2.23 g, 22.12 mmol, 2 eq) were added, The resulting mixture was stirred for 16 hours at room temperature. After the reaction was completed, the reaction was quenched by adding water (30 mL). The mixture was extracted with dichloromethane (30 mL*3). The combined organic layer was dried over anhydrous sodium sulfate and concentrated under reduced pressure to obtain a residue. The residue was purified by column chromatography on silica gel eluting with 10-40% petroleum ether/ethyl acetate to give (R)-tetrahydrofuran-3-yl ((1,1-difluorobut-3-en-1-yl)(phenoxy)phosphoryl)-L-alaninate (1.30 g, 3.34 mmol, 30.20 % yield) as a yellow oil.

MS (ESI) m/z: calcd. 390.1 $[M+H]^+$, found 390.1 $[M+H]^+$.

**Step 5: (R)-tetrahydrofuran-3-yl (((E)-1,1-difluoro-5-hydroxy-4-methylpent-3-en-1-yl)(phenoxy)phosphoryl)-L-alaninate (compound 37)**

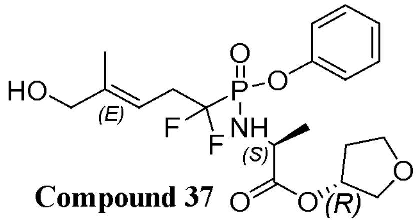

**Compound 37**

To a solution of (R)-tetrahydrofuran-3-yl ((1,1-difluorobut-3-en-1-yl)(phenoxy)phosphoryl)-L-alaninate (1.30 g, 3.34 mmol, 1 eq) and 2-methylprop-2-en-1-ol (387 mg, 6.68 mmol, 2 eq) in dichloromethane (40 mL) were added

HOVEYDA-GRUBBS catalyst (232.12 mg, 0.33 mmol, 0.1 eq) and p-benzoquinone (80.35 mg, 0.668 mmol, 0.2 eq) dropwise under nitrogen protection, and the resulting mixture was stirred for 16 hours at 45 °C. After the reaction was completed, the mixture was concentrated under reduced pressure to obtain a residue. The residue was purified by column chromatography on silica gel eluting with 40-100 % petroleum ether/ethyl acetate, and the obtained crude product was purified by preparative HPLC: chromatographic column: -Gemini-C18 150 x 21.2 mm, 5μm; mobile phase: ACN--$H_2O$; gradient: 30%-95%, flow rate: 20 ml/min, retention time: 10.5 min, to give (R)-tetrahydrofuran-3-yl (((E)-1,1-difluoro-5-hydroxy-4-methylpent-3-en-1-yl)(phenoxy)phosphoryl)-L-alaninate (680 mg, 1.57 mmol, 47.01% yield) as a colorless oil-solid mixture.

$^1$H NMR (400 MHz, DMSO-$d_6$) $\delta$ 7.39 - 7.34 (m, 2H), 7.25 - 7.17 (m, 3H), 6.64-6.57 (m, 1H), 5.45 (t, *J* = 7.1 Hz, 1H), 5.16-5.12 (m, 1H), 4.83 (td, *J* = 5.5, 2.0 Hz, 1H), 3.94-3.86 (m, 1H), 3.80 (d, *J* = 8.0 Hz, 2H), 3.78 - 3.62 (m, 3H), 3.55 (d, *J* = 10.4 Hz, 1H), 2.93 - 2.80 (m, 2H), 2.10 - 2.02 (m, 1H), 1.77 - 1.71 (m, 1H), 1.54 (s, 3H), 1.21 (d, *J* = 7.2 Hz, 1.5H), 1.18 (d, *J* = 7.2 Hz, 1.5H).
$^{31}$P NMR (400 MHz, DMSO-$d_6$) $\delta$ = 11.22, 10.76, 10.57, 10.13, 9.92, 9.47;
MS (ESI) m/z: calcd. 434.1 $[M+H]^+$, found 416.0 $[M+H-H_2O]^+$.

**Example 38: compound 38**

Example 38 was synthesized using benzyloxycarbonyl-L-alanine and 4-hydroxytetrahydropyran as raw materials according to the method of Example 37.

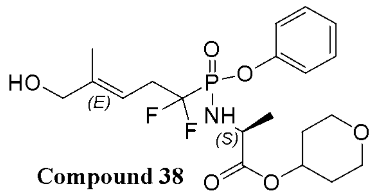

**Compound 38**

$^1$H NMR (400 MHz, $CDCl_3$) δ 7.35 - 7.31 (m, 2H), 7.27 - 7.18 (m, 3H), 5.56 - 5.54 (m, 1H), 4.96 - 4.92 (m, 1H), 4.19 - 4.11 (m, 1H), 4.02 (s, 2H), 3.91-3.87 (m, 3H), 3.54 - 3.48 (m, 2H), 3.03 - 2.86 (m, 2H), 2.36-2.29 (m, 1H), 1.92 - 1.84 (m, 2H), 1.69 (s, 3H), 1.66 - 1.59 (m, 2H), 1.35 (d, 7.2 Hz, 1.5H), 1.34 (d, J = 6.8, 1.5H).
$^{31}$P NMR (162 MHz, $CDCl_3$) δ 9.79, 9.21, 9.15, 9.09, 8.59, 8.51, 8.45, 7.89;
MS (ESI) m/z: calcd. 448.2 $[M+H]^+$, found 470.2 $[M+Na]^+$.

**Example 39: control compound 2**

Example 39 was synthesized using benzyloxycarbonyl-L-alanine and benzyl alcohol as raw materials according to the method of Example 2.

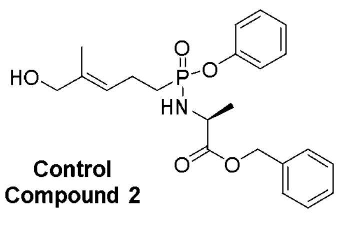

**Control Compound 2**

$^1$H NMR (400 MHz, $CDCl_3$) δ = 7.35 - 7.29 (m, 7H), 7.20 - 7.12 (m, 3H), 5.47 - 5.41 (m, 1H), 5.10 (s, 2H), 4.20 - 4.04 (m, 1H), 4.01 (s, 2H), 3.39 - 3.23 (m, 1H), 2.49 - 2.38 (m, 2H), 2.02 - 1.85 (m, 2H), 1.70 (s, 1.5H), 1.69 (s, 1.5H) 1.33 (d, *J* = 6.8 Hz, 1.5H), 1.21 (d, *J* = 7.2 Hz, 1.5H);
$^{31}$P NMR (162 MHz, $CDCl_3$) δ = 31.36, 30.94;
MS (ESI) *m/z:* calcd. 418.2 $[M+H]^+$, found 418.2 $[M+H]^+$

**Biological Examples**

Test Example 1: $EC_{50}$ assay of small molecule activated γδ T cells to kill tumor cells

γδT cells and MIApaca/FG2 tumor cell lines were expanded and cultured in vitro. The MIApaca/FG2 cells were digested, and the cells were counted and resuspended in DMEM complete medium, the density of the cell suspension was adjusted and added into a 96-well plate at $5\times10^3$ cells/50 μL/well for overnight incubation. On the next day, the test compound and the control compound were diluted at a 10-fold ratio with γδT complete medium. 50 μL of diluted compound was transferred to the corresponding wells which had been plated with tumor cells, and the actual detection concentration was in the range of $10^{-12}$-$10^{-5}$ M. yδT cells were resuspended in γδT complete medium, and the density of the cell suspension was adjusted according to the effector-target ratio of E:T=2:1, and then the cells were added to the corresponding wells at $1\times10^4$ cells/50μL/well. After co-culture at 37 °C, 5% $CO_2$ for 20 hours, the cells were centrifuged. The supernatant was removed, and 50 μL of cell lysate containing luciferin substrate was added to each well. After standing for 18 minutes in the dark, the signal of chemiluminescence was detected. The EC50 value was calculated by four-parameter fitting, with the corresponding signal value of the well with only tumor cells as the minimum killing value, and the corresponding signal value of the well without tumor cells as the maximum killing value. The killing data of MIApaca/FG2 cells is as follows:

| Compound | MIApaca killing (nM) |
|---|---|
| HMBPP control | 2.1 |
| Control compound 1 | 0.57 |
| Compound 1 | 57.1 |
| Compound 2 | 0.19 |
| Compound 3 | 8.0 |
| Compound 4 | 173.7 |
| Compound 5 | 11.6 |
| Compound 6 | 0.098 |
| Compound 7 | 0.078 |
| Compound 8 | 0.16 |
| Compound 9 | 9.9 |
| Compound 10 | 12.8 |
| Compound 11 | 35.4 |
| Compound 12 | 5.1 |
| Compound 13 | 6.6 |
| Compound 14 | 67.0 |

γδT cells and MIApaca/FG2 tumor cell lines were expanded and cultured in vitro. MIApaca/FG2 cells were digested, and the cells were counted and resuspended in DMEM complete medium, the density of the cell suspension was adjusted and added into a 96-well plate at $5\times10^3$ cells/100 μL/well for overnight incubation. On the next day, the test compound and the control compound were diluted at a 3-fold ratio with DMSO, and 4 μL of the diluted compound was added to 96 μL of culture medium for 25-fold dilution. 5 μL of 25-fold diluted compound was transferred to the corresponding well which had been plated with tumor cells, and the actual detection concentration was in the range of 0.05-100 nM. γδT cells were resuspended in yδT complete medium, and the density of the cell suspension was adjusted according to the effector-target ratio of E:T=2:1, and then the cell was added to the corresponding well at $1\times10^4$ cells/100 μL/well. After co-culture at 37 °C, 5% $CO_2$ for 20 hours, the cells were centrifuged. The supernatant was removed, and 50 μL of cell lysate containing fluorescein substrate was added to each well. After standing for 18 minutes in the dark, the signal of chemiluminescence was detected. The $EC_{50}$ value was calculated by four-parameter fitting, with the corresponding signal value of the well with only tumor cells as the minimum killing value, and the corresponding value of the well without tumor cells as the maximum killing value. The killing data of MIApaca/FG2 cells is as follows:

| Compound | MIApaca killing (nM) |
|---|---|
| Control compound 1 | 0.53 |
| Compound 15 | 0.20 |
| Compound 16 | 0.55 |
| Compound 17 | 0.12 |
| Compound 18 | 0.20 |
| Compound 19 | 0.28 |
| Compound 20 | 0.48 |
| Compound 21 | 0.14 |
| Compound 22 | 0.28 |
| Compound 23 | 3.96 |
| Compound 24 | 0.78 |
| Compound 25 | 0.17 |
| Compound 26 | 0.10 |
| Compound 27 | 0.23 |
| Compound 28 | 0.88 |
| Compound 29 | 1.63 |
| Compound 30 | 0.11 |
| Compound 31 | 0.13 |
| Compound 32 | 0.21 |
| Compound 33 | 0.65 |
| Compound 34 1145 | 1.03 |
| Compound 35 1152 | 2.15 |
| Compound 36 1155 | <0.05 |
| Compound 37 1156 | 0.20 |
| Compound 38 1157 | 0.65 |

γδT cells and Huh7/FG2 tumor cell lines were expanded and cultured in vitro. The Huh7/FG2 cells were digested, and the cells were counted and resuspended in DMEM complete medium, the density of the cell suspension was adjusted and added into a 96-well plate at $5\times10^3$ cells/100 μL/well for overnight incubation. On the next day, the test compound and the control compound were diluted at a 3-fold ratio with DMSO, and 4 μL of the diluted compound was added to 96 μL of culture medium for 25-fold dilution. 5 μL of 25-fold diluted compound was transferred to the corresponding well which had been plated with tumor cells, and the actual detection concentration was in the range of 0.05-100 nM. γδT cells were resuspended in γδT complete medium, and the density of the cell suspension was adjusted according to the effector-target ratio of E:T=5:1, and then the cell was added to the corresponding well at $2.5\times10^4$ cells/100 μL/well. After co-culture at 37 °C, 5% $CO_2$ for 20 hours, the cells were centrifuged. The supernatant was removed, and 50 μL of cell lysate containing fluorescein substrate was added to each well. After standing for 18 minutes in the dark, the signal of chemiluminescence was detected. The $EC_{50}$ value was calculated by four-parameter fitting, with the corresponding signal value of the well with only tumor cells as the minimum killing value, and the corresponding value of the well without tumor cells as the maximum killing value.

The killing data of Huh7 cells is as follows:

| Compound | Huh7 killing (nM) |
|---|---|
| Control compound 1 | 1.68 |
| Compound 19 | 0.42 |

γδT cells and SKOV3/ELP tumor cell lines were expanded and cultured in vitro. The SKOV3/ELP cells were digested, and the cells were counted and resuspended in MC5A complete medium, the density of the cell suspension was adjusted and added into a 96-well plate at 5×$10^3$ cells/100 μL/well for overnight incubation. On the next day, the test compound and the control compound were diluted at a 3-fold ratio with DMSO, and 4 μL of the diluted compound was added to 96 μL of culture medium for 25-fold dilution. 5 μL of 25-fold diluted compound was transferred to the corresponding well which had been plated with tumor cells, and the actual detection concentration was in the range of 0.05-100 nM. γδT cells were resuspended in yδT complete medium, and the density of the cell suspension was adjusted according to the effector-target ratio of E:T=2:1, and then the cell was added to the corresponding well at 1×$10^4$ cells/100 μL/well. After co-culture at 37 °C, 5% $CO_2$ for 20 hours, the cells were centrifuged. The supernatant was removed, and 50 μL of cell lysate containing fluorescein substrate was added to each well. After standing for 18 minutes in the dark, the signal of chemiluminescence was detected. The $EC_{50}$ value was calculated by four-parameter fitting, with the corresponding signal value of the well with only tumor cells as the minimum killing value, and the corresponding value of the well without tumor cells as the maximum killing value.

The killing data of SKOV3 cells is as follows:

| Compound | SKOV3 killing (nM) |
|---|---|
| Control compound 1 | 1.14 |
| Compound 19 | 0.47 |

γδT cells and MV4-11-luc tumor cell lines were expanded and cultured in vitro. The MV4-11-luc cells were digested, and the cells were counted and resuspended in IMDM complete medium, the density of the cell suspension was adjusted and added into a 96-well plate at 5×$10^3$ cells/100 μL/well for overnight incubation. On the next day, the test compound and the control compound were diluted at a 3-fold ratio with DMSO, and 4 μL of the diluted compound was added to 96 μL of culture medium for 25-fold dilution. 5 μL of 25-fold diluted compound was transferred to the corresponding well which had been plated with tumor cells, and the actual detection concentration was in the range of 0.05-100 nM. γδT cells were resuspended in yδT complete medium, and the density of the cell suspension was adjusted according to the effector-target ratio of E:T=2:1, and then the cell was added to the corresponding well at 1×$10^4$ cells/100 μL/well. After co-culture at 37 °C, 5% $CO_2$ for 20 hours, the cells were centrifuged. The supernatant was removed, and 50 μL of cell lysate containing fluorescein substrate was added to each well. After standing for 18 minutes in the dark, the signal of chemiluminescence was detected. The $EC_{50}$ value was calculated by four-parameter fitting, with the corresponding signal value of the well with only tumor cells as the minimum killing value, and the corresponding value of the well without tumor cells as the maximum killing value.

The killing data of MV4-11 cells is as follows:

| Compound | MV4-11 killing (nM) |
|---|---|
| Control compound 1 | 0.05 |
| Compound 19 | 0.004 |

Test Example 2: Stability experiment of compounds in human plasma

The human plasma stored in the refrigerator was thawed in a 37°C water bath. The solution was collected after centrifuged at 4000 rpm for 5 min. The plasma was added to a 96-well plate at 98 μL per well using the Apricot automated workstation with each sample in duplicate. Then 2 μL of 100 μM small molecule solution was added. After incubating for 0, 10, 30, 60, 120 minutes, 6 h, and 24 h, 500 μL of the quenching solution was added to the plate and the protein was precipitated. The plate was shaken for 20 minutes and centrifuged at 4000 rpm for 20 minutes at 4°C. The obtained supernatant was analyzed by LC-MS/MS.

Test Example 3: Stability experiment of compounds in human whole blood

(1) An appropriate amount of blank whole blood was added into an EP tube containing sodium heparin, then added the working solution of the test substance to a final concentration of 1 μM, and mixed by vortex;

(2) The sample was placed in a water bath at 37 °C with two parallel experiments; for one experiment, the time points were 0 h, 0.5 h, 1 h, 2 h, and 4 h; another experiment was performed to obtain the absolute zero point, which was compared with

the 0 h point.
(3) An appropriate amount of sample was taken out from the incubation system, to which 300 μL of internal standard precipitant was added for protein precipitation; (4) all samples were centrifuged at 6°C for 10 min (15700×g).
(5) 150 μL of the supernatant was taken out, to which 150 μL of water was added, which was mixed by vortex, and analyzed by LC-MS/MS. The stability data of compounds in human whole blood is as follows:

| Compound | $T_{1/2}$ (h) |
|---|---|
| Control compound 1 | >8 h |
| Control compound 2 | 2 h |
| Compound 19 | >8 h |

Test Example 4: Stability experiment of compounds in human liver microsomes

Compounds were prepared as a 10 mM stock solution with DMSO and then diluted with 80% acetonitrile-water to a 100 μM working solution for later use. Human liver microsomes were obtained from BIOIVT.
(1) The liver microsomes taken out from the refrigerator were placed in a 37 °C water bath thermostatic oscillator, pre-incubated for 5 min, and thawed for later use.
(2) A certain amount of NADPH was weighed and added to an appropriate amount of magnesium chloride solution to dissolve into a 2 mM solution for later use.
(3) A mixed solution of the warm incubation system (containing no β-NADPH) was prepared according to the proportions in the "composition of the experimental warm incubation system" above and dispensed at 165 μL/tubes (45 μL/tube for the negative control group and 120 μL/tube for the positive control group).
(4) For the sample at 0 min: 200 μL of internal standard working precipitant was added and then 30 μL of NADPH solution was added.
(5) For other samples: 135 μL NADPH solution was added to initiate the reaction (45 μL magnesium chloride solution was added for the negative control group), and incubated in a 37°C water bath for 5, 15, 30, and 60 min (incubated in a 37°C water bath for 60 min for the negative control group), then 60 μL was sampled and added to 200 μL of precipitant containing internal standard.
(6) For the positive control group: 90 μL NADPH solution was added to initiate the reaction, and incubated in a 37°C water bath for 5, 15 min, then 60 μL was sampled and added to 200 μL of precipitant containing internal standard.
(7) For neat sample: 297 μL of water was pipetted into a tube, 1000 μL of internal standard working solution was added, and 3 μL of test compound working solution was added.
(8) All samples were vortexed and centrifuged.
(9) 150 μL of the supernatant was sampled and added to 150 μL of water, mixed uniformly by vortex, and analyzed by LC-MS/MS. The half-life stability data of human liver microsomes is as follows:

| Compound | $T_{1/2}$ (min) |
|---|---|
| Control compound 1 | 8.7 |
| Control compound 2 | 1.4 |
| Compound 7 | 21.6 |
| Compound 18 | 20.9 |
| Compound 19 | 47.8 |
| Compound 20 | 24.0 |
| Compound 8 | 27.3 |
| Compound 27 | 10.5 |
| Compound 30 | 24.4 |
| Compound 31 | 3.91 |
| Compound 32 | 11.4 |
| Compound 33 | 19.9 |
| Compound 35 | 82.5 |

(continued)

| Compound | $T_{1/2}$ (min) |
|---|---|
| Compound 36 | 3.08 |
| Compound 37 | 8.11 |

Test Example 5: binding rate test of compounds and human plasma protein

(1) The dry dialysis membrane was soaked with ultrapure water for 20 min or more than 20 min, then soaked with 20% ethanol for 30 min to 1 h, and the membrane was rinsed with ultrapure water 2-3 times. Finally, the membrane was soaked with ultrapure water for 20 minutes for later use.

(2) The frozen plasma was thawed in a water bath at 37°C.

(3) The test compound was diluted into plasma preheated to 37°C at a final concentration of 1 μM, and the final concentration of the control compound warfarin in plasma was 2 μM.

(4) The pre-treated dialysis membrane was assembled into the dialysis plate according to the specification of the product, and 120 μL of the receiving solution (100 mM phosphate buffer solution added 0.002% Tween 80) was added to one side of the permeable membrane in each dialysis well.

(5) 20 μL of each of the final solutions of the test compound and the control compound were added into a 96-well sample plate in duplicate, and the T0 sample was obtained, which was stored in a refrigerator at -20°C. Another 20 μL of the above final solution was added to the other side of the membrane in the dialysis device in duplicate, and incubated at 37°C by shaking at constant temperature for 6 hours.

(6) After 6 hours of incubation, 20 μL of each of the dialyzed receiving solution and the administered plasma were weighed to obtain sample B and sample A in duplicate. The corresponding volume of corresponding blank plasma or receiving solution was added to sample B and sample A, respectively, so that the volume ratio of plasma to buffer in each sample well was 1:1. 300 μL of acetonitrile solution containing internal standard was added to all sample wells, and centrifuged after mixed uniformly.

(7) 200 μL of ultrapure water (150 μL for the control group) was added into the corresponding sample wells of the 96-well sample plate, and 300 μL of the supernatant (150 μL for the control group) was added into the sample well; the samples were analyzed after mixed uniformly. The free percentage and recovery rate of the compound in plasma were calculated using the following formula: Plasma protein binding rate (%) = 1 - free percentage, wherein free percentage (%) = CB/CA wherein $C_B$ is the concentration of the compound in the receiving solution after equilibrium dialysis, $C_A$ is the concentration of the compound in the drug-dosed plasma after equilibrium dialysis, and $CT_0$ is the initial concentration of the compound in plasma. The data of human plasma protein binding rate is as follows:

| Compound | % |
|---|---|
| Control compound 1 (1015) | 92.7 |
| Control compound 2 (1024) | 97.3 |
| Compound 7 | 77.8 |
| Compound 18 | 82.2 |
| Compound 19 | 64.6 |
| Compound 20 | 77.3 |
| Compound 8 | 83.2 |
| Compound 27 | 80.7 |
| Compound 30 | 64.1 |
| Compound 31 | 87.1 |
| Compound 32 | 78.6 |
| Compound 33 | 80.8 |
| Compound 35 | 57.1 |
| Compound 36 | 96.2 |

(continued)

| Compound | % |
|---|---|
| Compound 37 | 90.2 |

Test Example 6: test of inhibition rate of compound to CYPs

(1) 100 × specific inhibitor: the corresponding stock solution was diluted with 50% acetonitrile-water to formulate the inhibitor working solution with the corresponding concentration;
(2) 200 × compound: the compound stock solution was diluted with acetonitrile to a 2000 μM working solution;
(3) 200 × substrate: the corresponding stock solution was diluted with 50% acetonitrile-water to formulate the substrate working solution with the corresponding concentration;
(4) PB solution was used as solvent to formulate 4 mM NADPH solution;
(5) Liver microsome substrate solution: a certain amount of PB was added into a centrifuge tube, a certain amount of $MgCl_2$-PB solution was added, followed by human liver microsome solution, and then substrate working solution for each isoform was added respectively, mixed uniformly by vortex, and dispensed at 148 μL/tube.
(6) 1 μL of inhibitor/test compound working solution/50% acetonitrile-water was added to each tube, then placed in a 37 °C water bath for pre-incubation for 5 min, and NADPH was pre-incubate in a 37°C water bath for 5 min;
(7) 50 μL/well of NADPH working solution was added and incubated for 30 min (for 2C19), or 10 min (for other isoforms);
(8) 100 μL of the sample was sampled and 300 μL/well of ice internal standard working solution was added, the reaction was stopped after vortexed for 5 min, and then centrifuged;
(9) 100 μL of supernatant was sampled and added to 300 μL of water (for 1A2, 2C8), and 150 μL of supernatant was sampled and added to 150 μL of water (for other isoforms), mixed uniformly by vortex, and injected for LC-MS/MS analysis. Excel was used to calculate the enzyme inhibition rate of the metabolic rate of each specific probe substrate of the test substance and the control at the test concentration, and the calculation method was: enzyme inhibition rate% = 100 - average residual enzyme activity%. Residual enzyme activity% = peak area ratio of the metabolite of probe substrate after adding inhibitor/peak area ratio of the metabolite of probe substrate without adding inhibitor *100%

Test Example 7: Kinetic Solubility Assay of compounds

The test compound was formulated into a 10 mM stock solution with DMSO for later use.

(1) the test compound stock solution was added to a pH 7.4 buffer solution at a ratio of 1:49.
(2) The mixture was shaken thoroughly for 24 h.
(3) After centrifuging (15700 g, 20 min), the clear liquid was taken from the middle layer, diluted 2000 times with 80% acetonitrile-water, and injected for analysis after mixed uniformly.

Test Example 8: Caco 2 membrane permeability test

1. The test compound was diluted to 5 μM test solution with the corresponding transport buffer. The final content of organic reagents in the incubation system was less than 1%.
2. The transport rate of the compound from the apical to the basolateral of the substrate was determined. 75 μL of the dosing solution was added to each well of the upper compartment (apical) and 250 μL of the receiving solution was added to each well of the lower compartment (basolateral). The transport rate of the compound from the basolateral to the apical was determined. 75 μL of the receiving solution was added to each well of the upper compartment (apical) and 250 μL of the dosing solution was added to each well of the lower compartment (basolateral). After the plate was sealed, it was placed in a 37°C incubator and incubated for 120 min.
3. After the incubation was completed, the incubate was sampled, and finally 600 μL of the stop solution containing the internal standard was added after all samples were mixed.
4. It was mixed on a plate shaker and centrifuged; finally, 200 μL of the supernatant was taken from all samples, and 200 μL of water was added; the mixture was mixed by vortex, and injected for LC-MS/MS analysis.

5. The integrity of the cell monolayer membrane after 2 hours of incubation was evaluated using the leakage of fluorescent yellow, wherein the fluorescent yellow stock solution was diluted to a final concentration of 100 μM using the transport buffer solution. 75 μL of fluorescent yellow solution was added to each well of the upper Transwell insert plate, and 250 μL of transport buffer solution was added to each well of the lower receiving plate. After incubation for 120 minutes, 10 μL of solution aspirated from the upper layer of each well and 90 μL of transport buffer solution were added to a new 96-well plate, and 100 μL of solution aspirated from the lower layer was added to a new 96-well plate. Fluorescence assay was performed using a microplate reader under conditions of excitation wavelength of 428 nm and emission wavelength of 528 nm.

The apparent permeability coefficient (Papp, unit: cm/s 10-6) was calculated by the following formula:

$$Papp = (dCr/dt) \times Vr / (A \times C_0)$$

wherein dCr/dt is the cumulative concentration of the compound in the receiving compartment as a function of time (μM/s); Vr is the volume of the solution at the receiving end (0.075 mL at the apical and 0.25 mL at the basolateral); A is surface area, that is, the cell monolayer membrane area of 0.0804 cm2; $C_0$ is the initial concentration (μM) at the dosing end.

The efflux rate was calculated by the following formula:

$$\text{Efflux Ratio} = Papp\ (B\text{-}A) / Papp\ (A\text{-}B)$$

The recovery rate was calculated by the following formula:

$$\%\ \text{Recovery} = 100 \times [(Vr \times Cr) + (Vd \times Cd)] / (Vd \times C0)$$

wherein Vd is the volume of the dosing end (0.075 mL at the apical and 0.25 mL at the basolateral); Cd and Cr are the final concentrations of the transported compound at the dosing end and the receiving end, respectively.

Test Example 9: Pharmacokinetic Test in Cynomolgus Monkeys

The **control compound 2** and **compound 19** were formulated into 1 mg/mL solution in 10% DMSO/90% PBS. Cynomolgus monkeys were obtained from Suzhou Leo Bio Technology Co., Ltd. The control compound 2 and compound 19 were administered intravenously at 2 mg/kg, respectively. The blood collected before administration was recorded as zero point. Then 1 mL of blood was collected at eight time points: 0.083, 0.15, 0.5, 1.0 2.0, 4.0, 8.0, and 24.0, respectively. 1 mL of the collected whole blood was placed in a labeled EDTA-2K anticoagulant tube. After gently inverting up and down to fully mix the anticoagulant (EDTA-2K) with the blood, it was immediately placed in wet ice and centrifuged within 30 minutes to separate the plasma. The centrifugation conditions were set to 4°C, 2200 g, and 6 min. The plasma separated after centrifugation was placed in a labeled EP tube, and the content of compound 19 and control compound 2 in the plasma samples at different time points were detected and analyzed. There were 3 male cynomolgus monkeys in each group.

**Plasma drug concentration and PK parameters of the unchanged drug in cynomolgus monkeys after intravenous injection of 2 mg/kg**

| Dosage (mg/kg) | Route of administration | Time points of blood collection | Concentration (ng/mL) | |
|---|---|---|---|---|
| | | | Control compound 2 | Compound 19 |
| NA | IV | 5min | 299 | 1370 |
| | | 9min | 49 | 878 |
| | | 30min | 1 | 139 |
| | | 1h | 3 | 12 |

(continued)

| PK parameters | WinNonlin Parameters | Unit | Average | Average |
|---|---|---|---|---|
| Cl | Cl_obs | mL/min/kg | 417.7 | 76.3 |
| $AUC_{0-t}$ | AUClast | h*ng/mL | 194 | 426 |
| $AUC_{0-t}/AUC_{0-inf}$ | | / | 0.974 | 0.959 |

After being administered intravenously, the exposure of the unchanged drug of the compound of the present disclosure is more than 3 times that of the control compound, indicating that the compound of the present disclosure can provide better exposure to the active substance and therapeutic effect for the subjects.

Test Example 10: Molecular Binding Experiment

(A) Potential diagram of the BTN3A1 B30.2 - HMBPP crystal structure (PDB ID: 5ZXK). Basic regions were shown in blue and acidic regions in red. HMBPP and amino acids were shown as sphere models.
(B) Crystal structure of BTN2A1 B30.2 - HMBPP - BTN3A1 B30.2 (PDB ID: 7YGJ). BTN3A1 B30.2 was shown as a potential map. 2A1 B30.2 A chain and B chain were shown as cartoon models. HMBPP and amino acids were shown as sphere models.

Images were exported by Pymol.

## Claims

1. A compound of formula (I), or a pharmaceutically acceptable salt, an enantiomer, a diastereomer, a solvate, a hydrate, a polymorph or an isotopic variant thereof:

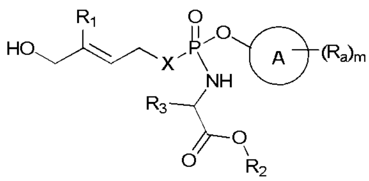

(I)

wherein,

ring A is $C_{6-10}$ aryl or 5- to 10-membered heteroaryl;
$R_a$ is selected from H, halogen, $C_{1-6}$ alkyl, $C_{1-6}$ haloalkyl, -OR", NR"R", $C_{2-6}$ alkenyl and $C_{2-6}$ alkynyl;
m=1, 2, 3, 4, or 5;
X is O or CRR';
$R_1$ is selected from H, F, Cl, CN and methyl, wherein the methyl is optionally substituted with 1 to 3 substituents independently selected from halogen, CN, OH and $NH_2$;
$R_2$ is selected from $C_{1-6}$ hydroxyalkyl, $C_{5-6}$ alkyl, $C_{5-6}$ haloalkyl, $C_{5-6}$ alkenyl, $C_{5-6}$ alkynyl, $C_{0-6}$ alkylene-$C_{3-12}$ cycloalkyl, $C_{0-6}$ alkylene-4- to 12-membered heterocyclyl and $C_{0-6}$ alkylene-5- to 10-membered heteroaryl, wherein the $C_{1-6}$ hydroxyalkyl, $C_{5-6}$ alkyl, $C_{5-6}$ haloalkyl, $C_{5-6}$ alkenyl, $C_{5-6}$ alkynyl, $C_{0-6}$ alkylene-$C_{3-12}$ cycloalkyl, $C_{0-6}$ alkylene-4- to 12-membered heterocyclyl and $C_{0-6}$ alkylene-5- to 10-membered heteroaryl are optionally substituted with one or more substituents selected from halogen, $C_{1-6}$ alkyl, $C_{1-6}$ haloalkyl, $C_{1-6}$ hydroxyalkyl, $C_{2-6}$ alkenyl, $C_{2-6}$ alkynyl, 3- to 10-membered cycloalkyl, 4- to 10-membered heterocycloalkyl, $C_{6-10}$ aryl, 5- to 10-membered heteroaryl, -C(O)-$C_{1-6}$ alkyl, -C(O)-$C_{1-6}$ hydroxyalkyl, -C(O)-$C_{1-6}$ haloalkyl, -C(O)-$C_{2-6}$ alkenyl, -C(O)-$C_{2-6}$ alkynyl, -C(O)-3- to 10-membered cycloalkyl, -C(O)-4-to 10-membered heterocycloalkyl, -C(O)-$C_{6-10}$ aryl, -C(O)-5- to 10-membered heteroaryl, -CN and oxo group;
$R_3$ is selected from $C_{1-6}$ alkyl, $C_{1-6}$ haloalkyl, $C_{2-6}$ alkenyl and $C_{2-6}$ alkynyl;
wherein,
R and R' are independently selected from H and halogen;

R" is independently selected from H, $C_{1-6}$ alkyl, $C_{1-6}$ haloalkyl, $C_{2-6}$ alkenyl, $C_{2-6}$ alkynyl, $C_{3-7}$ cycloalkyl, 3- to 7-membered heterocyclyl, $C_{6-10}$ aryl and 5- to 10-membered heteroaryl.

**2.** The compound of formula (I) of claim 1, or a pharmaceutically acceptable salt, an enantiomer, a diastereomer, a solvate, a hydrate, a polymorph or an isotopic variant thereof:

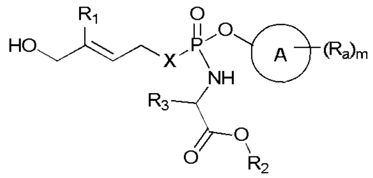
(I)

wherein,

ring A is $C_{6-10}$ aryl or 5- to 10-membered heteroaryl;
$R_a$ is selected from H, halogen, $C_{1-6}$ alkyl, $C_{1-6}$ haloalkyl, -OR", NR"R", $C_{2-6}$ alkenyl and $C_{2-6}$ alkynyl;
m=1, 2, 3, 4 or 5;
X is O or CRR';
$R_1$ is selected from H, F, Cl, CN and methyl, wherein the methyl is optionally substituted with 1 to 3 substituents independently selected from halogen, CN, OH and $NH_2$;
$R_2$ is selected from $C_{5-6}$ alkyl, $C_{5-6}$ haloalkyl, $C_{5-6}$ alkenyl, $C_{5-6}$ alkynyl, $C_{0-6}$ alkylene-$C_{3-7}$ cycloalkyl, $C_{0-6}$ alkylene-4- to 7-membered heterocyclyl and $C_{0-6}$ alkylene-5- to 10-membered heteroaryl;
$R_3$ is selected from $C_{1-6}$ alkyl, $C_{1-6}$ haloalkyl, $C_{2-6}$ alkenyl and $C_{2-6}$ alkynyl;
wherein,
R and R' are independently selected from H and halogen;
R" is independently selected from H, $C_{1-6}$ alkyl, $C_{1-6}$ haloalkyl, $C_{2-6}$ alkenyl, $C_{2-6}$ alkynyl, $C_{3-7}$ cycloalkyl, 3- to 7-membered heterocyclyl, $C_{6-10}$ aryl and 5- to 10-membered heteroaryl.

**3.** The compound of claim 1, or a pharmaceutically acceptable salt, an enantiomer, a diastereomer, a solvate, a hydrate, a polymorph or an isotopic variant thereof, which is a compound of formula (II):

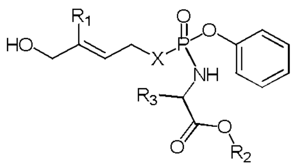
(II)

wherein,

X is O or CRR';
$R_1$ is selected from H, F, Cl, CN and methyl, wherein the methyl is optionally substituted with 1 to 3 substituents independently selected from halogen, CN, OH and $NH_2$;
$R_2$ is selected from $C_{1-6}$ hydroxyalkyl, $C_{5-6}$ alkyl, $C_{5-6}$ haloalkyl, $C_{5-6}$ alkenyl, $C_{5-6}$ alkynyl, $C_{0-6}$ alkylene-$C_{3-12}$ cycloalkyl and $C_{0-6}$ alkylene-4- to 12-membered heterocyclyl, wherein the $C_{1-6}$ hydroxyalkyl, $C_{5-6}$ alkyl, $C_{5-6}$ haloalkyl, $C_{5-6}$ alkenyl, $C_{5-6}$ alkynyl, $C_{0-6}$ alkylene-$C_{3-12}$ cycloalkyl and $C_{0-6}$ alkylene-4- to 12-membered heterocyclyl are optionally substituted with one or more substituents selected from halogen, $C_{1-6}$ alkyl, $C_{1-6}$ haloalkyl, $C_{1-6}$ hydroxyalkyl, $C_{2-6}$ alkenyl, $C_{2-6}$ alkynyl, 3- to 10-membered cycloalkyl, 4- to 10-membered heterocycloalkyl, $C_{6-10}$ aryl, 5- to 10-membered heteroaryl, -C(O)-$C_{1-6}$ alkyl, -C(O)-$C_{1-6}$ hydroxyalkyl, -C(O)-$C_{1-6}$ haloalkyl, -C(O)-$C_{2-6}$ alkenyl, -C(O)-$C_{2-6}$ alkynyl, -C(O)-3- to 10-membered cycloalkyl, -C(O)-4- to 10-membered heterocycloalkyl, -C(O)-$C_{6-10}$ aryl, -C(O)-5- to 10-membered heteroaryl, -CN and oxo group;
$R_3$ is selected from $C_{1-6}$ alkyl, $C_{1-6}$ haloalkyl, $C_{2-6}$ alkenyl and $C_{2-6}$ alkynyl;
wherein,
R and R' are independently selected from H and halogen.

**4.** The compound of claim 1, or a pharmaceutically acceptable salt, an enantiomer, a diastereomer, a solvate, a hydrate, a polymorph or an isotopic variant thereof, which is a compound of formula (II):

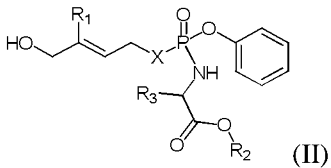

(II)

wherein,

X is O or CRR';
$R_1$ is selected from H, F, Cl, CN and methyl, wherein the methyl is optionally substituted with 1 to 3 substituents independently selected from halogen, CN, OH and $NH_2$;
$R_2$ is selected from $C_{5-6}$ alkyl, $C_{5-6}$ haloalkyl, $C_{5-6}$ alkenyl, $C_{5-6}$ alkynyl, $C_{0-6}$ alkylene-$C_{3-7}$ cycloalkyl and $C_{0-6}$ alkylene-4- to 7-membered heterocyclyl;
$R_3$ is selected from $C_{1-6}$ alkyl, $C_{1-6}$ haloalkyl, $C_{2-6}$ alkenyl and $C_{2-6}$ alkynyl;
wherein,
R and R' are independently selected from H and halogen.

**5.** The compound of claim 1, or a pharmaceutically acceptable salt, an enantiomer, a diastereomer, a solvate, a hydrate, a polymorph or an isotopic variant thereof, which is a compound of formula (III):

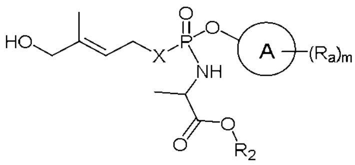

(III)

wherein,

X is O or CRR';
ring A is $C_{6-10}$ aryl or 5- to 10-membered heteroaryl;
$R_a$ is selected from H, halogen, $C_{1-6}$ alkyl, $C_{1-6}$ haloalkyl, -OR", NR"R", $C_{2-6}$ alkenyl and $C_{2-6}$ alkynyl;
$R_2$ is selected from $C_{1-6}$ hydroxyalkyl, $C_{5-6}$ alkyl, $C_{5-6}$ haloalkyl, $C_{5-6}$ alkenyl, $C_{5-6}$ alkynyl, $C_{0-6}$ alkylene-$C_{3-12}$ cycloalkyl and $C_{0-6}$ alkylene-4- to 12-membered heterocyclyl, wherein the $C_{1-6}$ hydroxyalkyl, $C_{5-6}$ alkyl, $C_{5-6}$ haloalkyl, $C_{5-6}$ alkenyl, $C_{5-6}$ alkynyl, $C_{0-6}$ alkylene-$C_{3-12}$ cycloalkyl and $C_{0-6}$ alkylene-4- to 12-membered heterocyclyl are optionally substituted with one or more substituents selected from halogen, $C_{1-6}$ alkyl, $C_{1-6}$ haloalkyl, $C_{1-6}$ hydroxyalkyl, $C_{2-6}$ alkenyl, $C_{2-6}$ alkynyl, 3- to 10-membered cycloalkyl, 4- to 10-membered heterocycloalkyl, $C_{6-10}$ aryl, 5- to 10-membered heteroaryl, -C(O)-$C_{1-6}$ alkyl, -C(O)-$C_{1-6}$ hydroxyalkyl, -C(O)-$C_{1-6}$ haloalkyl, -C(O)-$C_{2-6}$ alkenyl, -C(O)-$C_{2-6}$ alkynyl, -C(O)-3- to 10-membered cycloalkyl, -C(O)-4- to 10-membered heterocycloalkyl, -C(O)-$C_{6-10}$ aryl, -C(O)-5- to 10-membered heteroaryl, -CN and oxo group;
wherein,
R and R' are independently selected from H and halogen.

**6.** The compound of any one of claims 1-5, or a pharmaceutically acceptable salt, an enantiomer, a diastereomer, a solvate, a hydrate, a polymorph or an isotopic variant thereof, wherein X is CRR', alternatively $CH_2$ or $CF_2$.

**7.** The compound of any one of claims 1-4 and 6, or a pharmaceutically acceptable salt, an enantiomer, a diastereomer, a solvate, a hydrate, a polymorph or an isotopic variant thereof, wherein $R_1$ is selected from H, F, Cl, CN, $CH_3$, $CF_3$ and $CH_2OH$, and still alternatively $R_1$ is selected from H, $CH_3$, $CH_2OH$ and $CF_3$, and still alternatively $R_1$ is selected from $CH_3$ and $CF_3$, and still alternatively $R_1$ is $CH_3$.

**8.** The compound of any one of claims 1-7, or a pharmaceutically acceptable salt, an enantiomer, a diastereomer, a solvate, a hydrate, a polymorph or an isotopic variant thereof, wherein $R_2$ is 2-ethylbutyl, cyclopentyl, cyclohexyl, tetrahydrofuranyl, tetrahydrothiophenyl, pyrrolidinyl, tetrahydropyranyl, tetrahydrothiopyranyl or tetrahydropyridinyl, alternatively $R_2$ is cyclopentyl, tetrahydrofuranyl, tetrahydrothiophenyl or pyrrolidinyl, and still alternatively $R_2$ is cyclopentyl or tetrahydrofuranyl.

**9.** The compound of any one of claims 1-4 and 6-8, or a pharmaceutically acceptable salt, an enantiomer, a diastereomer, a solvate, a hydrate, a polymorph or an isotopic variant thereof, wherein $R_3$ is $C_{1-6}$ alkyl, alternatively methyl or isopropyl.

**10.** The compound of claim 1, or a pharmaceutically acceptable salt, an enantiomer, a diastereomer, a solvate, a hydrate, a polymorph or an isotopic variant thereof, which is a compound of formula (IV):

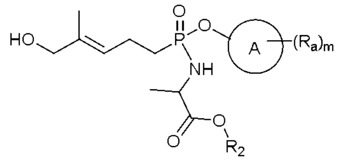
(IV)

wherein ring A is $C_{6-10}$ aryl or 5- to 10-membered heteroaryl, such as phenyl or naphthalene ring,
$R_a$ is selected from H, halogen, $C_{1-6}$ alkyl, $C_{1-6}$ haloalkyl, -OR", NR"R", $C_{2-6}$ alkenyl and $C_{2-6}$ alkynyl;
m=1, 2, 3, 4, or 5;
$R_2$ is 3- to 12-membered cycloalkyl or 4- to 12-membered heterocyclyl, wherein the 3- to 12-membered cycloalkyl or the 4- to 12-membered heterocyclyl is optionally substituted with one or more substituents selected from halogen, $C_{1-6}$ alkyl, $C_{1-6}$ haloalkyl, $C_{1-6}$ hydroxyalkyl, $C_{2-6}$ alkenyl, $C_{2-6}$ alkynyl, 3- to 10-membered cycloalkyl, 4- to 10-membered heterocycloalkyl, $C_{6-10}$ aryl, 5- to 10-membered heteroaryl, -C(O)-$C_{1-6}$ alkyl, -C(O)-$C_{1-6}$ hydroxyalkyl, -C(O)-$C_{1-6}$ haloalkyl, -C(O)-$C_{2-6}$ alkenyl, -C(O)-$C_{2-6}$ alkynyl, -C(O)-3- to 10-membered cycloalkyl, -C(O)-4- to 10-membered heterocycloalkyl, -C(O)-$C_{6-10}$ aryl, -C(O)-5-to 10-membered heteroaryl, -CN and oxo group.

**11.** The compound of claim 10, or a pharmaceutically acceptable salt, an enantiomer, a diastereomer, a solvate, a hydrate, a polymorph or an isotopic variant thereof, which is a compound of formula (V):

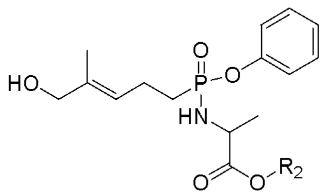
(V)

wherein $R_2$ is 4- to 12-membered heterocyclyl, wherein the 4- to 12-membered heterocyclyl is optionally substituted with one or more substituents selected from halogen, $C_{1-6}$ alkyl, $C_{1-6}$ haloalkyl, $C_{1-6}$ hydroxyalkyl, $C_{2-6}$ alkenyl, $C_{2-6}$ alkynyl, 3- to 10-membered cycloalkyl, 4- to 10-membered heterocycloalkyl, $C_{6-10}$ aryl, 5- to 10-membered heteroaryl, -C(O)-$C_{1-6}$ alkyl, -C(O)-$C_{1-6}$ hydroxyalkyl, -C(O)-$C_{1-6}$ haloalkyl, -C(O)-$C_{2-6}$ alkenyl, -C(O)-$C_{2-6}$ alkynyl, -C(O)-3- to 10-membered cycloalkyl, -C(O)-4- to 10-membered heterocycloalkyl, -C(O)-$C_{6-10}$ aryl, -C(O)-5- to 10-membered heteroaryl, -CN and oxo group.

**12.** The compound of any one of claims 1-11, or a pharmaceutically acceptable salt, an enantiomer, a diastereomer, a solvate, a hydrate, a polymorph or an isotopic variant thereof, wherein $R_2$ is 4- to 10-membered heterocyclyl optionally substituted with one or more substituents selected from halogen, $C_{1-6}$ alkyl, -C(O)-$C_{1-6}$ alkyl, $C_{1-6}$ haloalkyl, $C_{1-6}$ hydroxyalkyl, -C(O)-3- to 10-membered cycloalkyl, -CN and oxo group.

**13.** The compound of any one of claims 1-11, or a pharmaceutically acceptable salt, an enantiomer, a diastereomer, a solvate, a hydrate, a polymorph or an isotopic variant thereof, wherein $R_2$ is 4- to 7-membered heterocyclyl, wherein the heteroatom is oxygen atom or nitrogen atom, and the 4- to 7-membered heterocyclyl is optionally substituted with one or more substituents selected from $C_{1-6}$ alkyl and oxo group.

**14.** The compound of any one of claims 1-11, or a pharmaceutically acceptable salt, an enantiomer, a diastereomer, a solvate, a hydrate, a polymorph or an isotopic variant thereof, wherein $R_2$ is selected from:

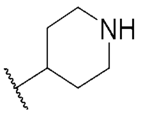, 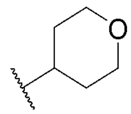, 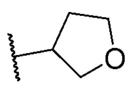, 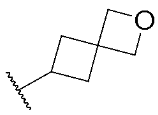, 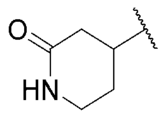, 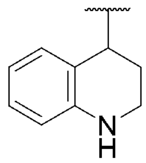, 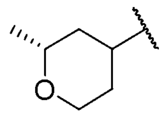,

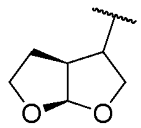, 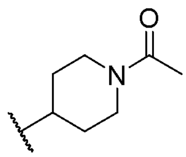, 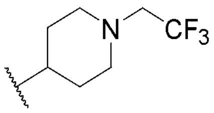, 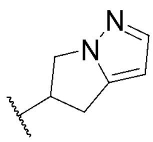, 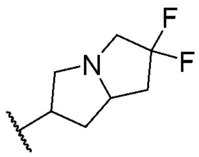, 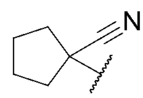,

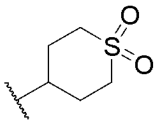, 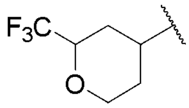, 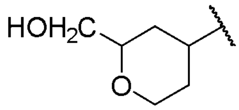 and 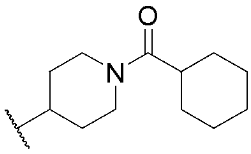.

**15.** A compound of formula (I), or a pharmaceutically acceptable salt, an enantiomer, a diastereomer, a solvate, a hydrate, a polymorph or an isotopic variant thereof:

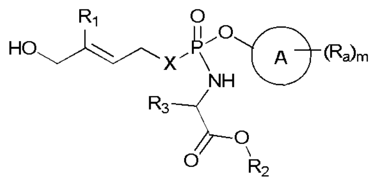

(I)

wherein,

ring A is $C_{6-10}$ aryl or 5- to 10-membered heteroaryl;
$R_a$ is selected from H, halogen, $C_{1-6}$ alkyl, $C_{1-6}$ haloalkyl, -OR", NR"R", $C_{2-6}$ alkenyl and $C_{2-6}$ alkynyl;
m=1, 2, 3, 4, or 5;
X is O or CRR';
$R_1$ is selected from H, F, Cl, CN and methyl, wherein the methyl is substituted with 1 to 3 substituents independently selected from halogen, CN, OH and $NH_2$;
$R_2$ is selected from $C_{1-6}$ hydroxyalkyl, $C_{5-6}$ alkyl, $C_{5-6}$ haloalkyl, $C_{5-6}$ alkenyl, $C_{5-6}$ alkynyl, $C_{0-6}$ alkylene-$C_{3-12}$ cycloalkyl and $C_{0-6}$ alkylene-4- to 12-membered heterocyclyl, wherein the $C_{1-6}$ hydroxyalkyl, $C_{5-6}$ alkyl, $C_{5-6}$ haloalkyl, $C_{5-6}$ alkenyl, $C_{5-6}$ alkynyl, $C_{0-6}$ alkylene-$C_{3-12}$ cycloalkyl and $C_{0-6}$ alkylene-4- to 12-membered heterocyclyl are optionally substituted with one or more substituents selected from halogen, $C_{1-6}$ alkyl, $C_{1-6}$ haloalkyl, $C_{1-6}$ hydroxyalkyl, $C_{2-6}$ alkenyl, $C_{2-6}$ alkynyl, 3- to 10-membered cycloalkyl, 4- to 10-membered heterocycloalkyl, $C_{6-10}$ aryl, 5- to 10-membered heteroaryl, -C(O)-$C_{1-6}$ alkyl, -C(O)-$C_{1-6}$ hydroxyalkyl, -C(O)-$C_{1-6}$ haloalkyl, -C(O)-$C_{2-6}$ alkenyl, -C(O)-$C_{2-6}$ alkynyl, -C(O)-3- to 10-membered cycloalkyl, -C(O)-4- to 10-membered heterocycloalkyl, -C(O)-$C_{6-10}$ aryl, -C(O)-5- to 10-membered heteroaryl, -CN and oxo group;
$R_3$ is selected from $C_{1-6}$ alkyl, $C_{1-6}$ haloalkyl, $C_{2-6}$ alkenyl and $C_{2-6}$ alkynyl;
wherein,
R and R' are independently selected from H and halogen;
R" is independently selected from H, $C_{1-6}$ alkyl, $C_{1-6}$ haloalkyl, $C_{2-6}$ alkenyl, $C_{2-6}$ alkynyl, $C_{3-7}$ cycloalkyl, 3- to 7-membered heterocyclyl, $C_{6-10}$ aryl and 5- to 10-membered heteroaryl.

**16.** The compound of claim 15, or a pharmaceutically acceptable salt, an enantiomer, a diastereomer, a solvate, a hydrate, a polymorph or an isotopic variant thereof, which is a compound of formula (II):

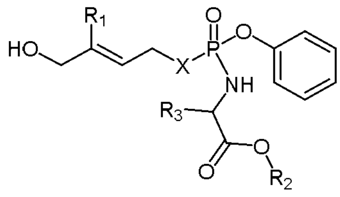

(II)

wherein,

X is O or CRR';

$R_1$ is selected from H, F, Cl, CN and methyl, wherein the methyl is substituted with 1 to 3 substituents independently selected from halogen, CN, OH and $NH_2$;

$R_2$ is selected from $C_{1-6}$ hydroxyalkyl, $C_{5-6}$ alkyl, $C_{5-6}$ haloalkyl, $C_{5-6}$ alkenyl, $C_{5-6}$ alkynyl, $C_{0-6}$ alkylene-$C_{3-12}$ cycloalkyl and $C_{0-6}$ alkylene-4- to 12-membered heterocyclyl, wherein the $C_{1-6}$ hydroxyalkyl, $C_{5-6}$ alkyl, $C_{5-6}$ haloalkyl, $C_{5-6}$ alkenyl, $C_{5-6}$ alkynyl, $C_{0-6}$ alkylene-$C_{3-12}$ cycloalkyl and $C_{0-6}$ alkylene-4- to 12-membered heterocyclyl are optionally substituted with one or more substituents selected from halogen, $C_{1-6}$ alkyl, $C_{1-6}$ haloalkyl, $C_{1-6}$ hydroxyalkyl, $C_{2-6}$ alkenyl, $C_{2-6}$ alkynyl, 3- to 10-membered cycloalkyl, 4- to 10-membered heterocycloalkyl, $C_{6-10}$ aryl, 5- to 10-membered heteroaryl, -C(O)-$C_{1-6}$ alkyl, -C(O)-$C_{1-6}$ hydroxyalkyl, -C(O)-$C_{1-6}$ haloalkyl, -C(O)-$C_{2-6}$ alkenyl, -C(O)-$C_{2-6}$ alkynyl, -C(O)-3- to 10-membered cycloalkyl, -C(O)-4- to 10-membered heterocycloalkyl, -C(O)-$C_{6-10}$ aryl, -C(O)-5- to 10-membered heteroaryl, -CN and oxo group;

$R_3$ is selected from $C_{1-6}$ alkyl, $C_{1-6}$ haloalkyl, $C_{2-6}$ alkenyl and $C_{2-6}$ alkynyl;

wherein,

R and R' are independently selected from H and halogen.

**17.** The compound of claim 15 or 16, or a pharmaceutically acceptable salt, an enantiomer, a diastereomer, a solvate, a hydrate, a polymorph or an isotopic variant thereof, wherein X is CRR', alternatively $CH_2$ or $CF_2$.

**18.** The compound of any one of claims 15-17, or a pharmaceutically acceptable salt, an enantiomer, a diastereomer, a solvate, a hydrate, a polymorph or an isotopic variant thereof, wherein $R_1$ is selected from H, F, Cl, CN, $CF_3$ and $CH_2OH$, and still alternatively $R_1$ is selected from H and $CF_3$, and still alternatively $R_1$ is $CF_3$.

**19.** The compound of any one of claims 15 to 18, or a pharmaceutically acceptable salt, an enantiomer, a diastereomer, a solvate, a hydrate, a polymorph or an isotopic variant thereof, wherein $R_2$ is $C_{1-6}$ alkyl, $C_{0-6}$ alkylene-$C_{3-7}$ cycloalkyl, $C_{0-6}$ alkylene-3- to 7-membered heterocyclyl, $C_{0-6}$ alkylene-$C_{6-10}$ aryl or $C_{0-6}$ alkylene-5- to 10-membered heteroaryl, alternatively $R_2$ is isopropyl, 2-ethylbutyl, cyclopentyl, cyclohexyl, tetrahydrofuranyl, tetrahydrothiophenyl, pyrrolidinyl, tetrahydropyranyl, tetrahydrothiopyranyl, tetrahydropyridinyl or benzyl, and still alternatively $R_2$ is 2-ethylbutyl, cyclopentyl, tetrahydrofuranyl, tetrahydrothiophenyl, pyrrolidinyl or benzyl, and still alternatively $R_2$ is cyclopentyl, tetrahydrofuranyl or benzyl.

**20.** The compound of any one of claims 15 to 19, or a pharmaceutically acceptable salt, an enantiomer, a diastereomer, a solvate, a hydrate, a polymorph or an isotopic variant thereof, wherein $R_3$ is $C_{1-6}$ alkyl, alternatively methyl or isopropyl.

**21.** The compound of any one of claims 15 to 20, or a pharmaceutically acceptable salt, an enantiomer, a diastereomer, a solvate, a hydrate, a polymorph or an isotopic variant thereof, wherein $R_2$ is 3- to 12-membered cycloalkyl or 4- to 12-membered heterocyclyl, wherein the 3- to 12-membered cycloalkyl or the 4- to 12-membered heterocyclyl is optionally substituted with one or more substituents selected from halogen, $C_{1-6}$ alkyl, $C_{1-6}$ haloalkyl, $C_{1-6}$ hydroxyalkyl, $C_{2-6}$ alkenyl, $C_{2-6}$ alkynyl, 3- to 10-membered cycloalkyl, 4- to 10-membered heterocycloalkyl, $C_{6-10}$ aryl, 5- to 10-membered heteroaryl, -C(O)-$C_{1-6}$ alkyl, -C(O)-$C_{1-6}$ hydroxyalkyl, -C(O)-$C_{1-6}$ haloalkyl, -C(O)-$C_{2-6}$ alkenyl, -C(O)-$C_{2-6}$ alkynyl, -C(O)-3- to 10-membered cycloalkyl, -C(O)-4- to 10-membered heterocycloalkyl, -C(O)-$C_{6-10}$ aryl, -C(O)-5- to 10-membered heteroaryl, -CN and oxo group; alternatively, $R_2$ is 4- to 10-membered heterocyclyl optionally substituted with one or more substituents selected from halogen, $C_{1-6}$ alkyl, -C(O)-$C_{1-6}$ alkyl, $C_{1-6}$ haloalkyl, $C_{1-6}$ hydroxyalkyl, -C(O)-3- to 10-membered cycloalkyl, -CN and oxo group; and still alternatively, $R_2$ is 4- to 7-membered heterocyclyl, wherein the heteroatom is oxygen atom or nitrogen atom, and the 4- to 7-membered heterocyclyl is optionally substituted with one or more substituents selected from $C_{1-6}$ alkyl and oxo group; and still alternatively, $R_2$ is selected from:

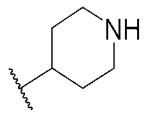, 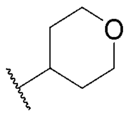, 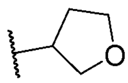, 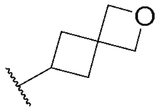, 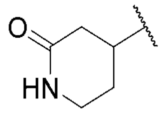, 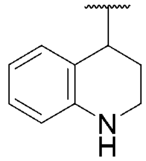, 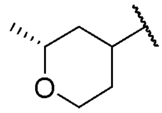,

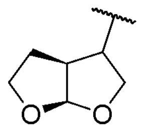, 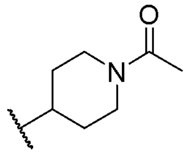, 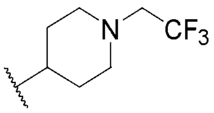, 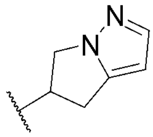, 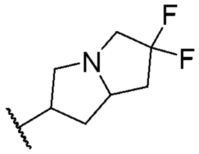, 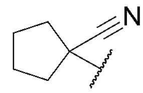,

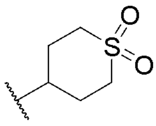, 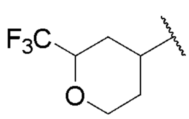, 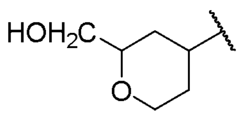 and 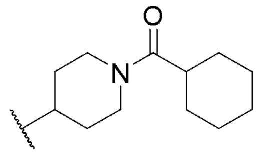.

**22.** A compound of formula (VI), or a pharmaceutically acceptable salt, an enantiomer, a diastereomer, a solvate, a hydrate, a polymorph or an isotopic variant thereof:

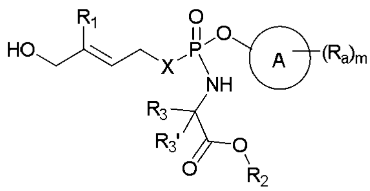

(VI)

wherein,

ring A is $C_{6-12}$ aryl or 5- to 12-membered heteroaryl;
$R_a$ is selected from halogen, $C_{1-6}$ alkyl, $C_{1-6}$ haloalkyl, -OR", NR"R", $C_{2-6}$ alkenyl and $C_{2-6}$ alkynyl;
m=1, 2, 3, 4, or 5;
X is O or CRR';
$R_1$ is selected from H, F, Cl, CN and methyl, wherein the methyl is optionally substituted with 1 to 3 substituents independently selected from halogen, CN, OH and $NH_2$;
$R_2$ is selected from $C_{1-6}$ hydroxyalkyl, $C_{5-6}$ alkyl, $C_{5-6}$ haloalkyl, $C_{5-6}$ alkenyl, $C_{5-6}$ alkynyl, $C_{0-6}$ alkylene-$C_{3-12}$ cycloalkyl, $C_{0-6}$ alkylene-4- to 12-membered heterocyclyl and $C_{0-6}$ alkylene-5- to 10-membered heteroaryl, wherein the $C_{1-6}$ hydroxyalkyl, $C_{5-6}$ alkyl, $C_{5-6}$ haloalkyl, $C_{5-6}$ alkenyl, $C_{5-6}$ alkynyl, $C_{0-6}$ alkylene-$C_{3-12}$ cycloalkyl, $C_{0-6}$ alkylene-4- to 12-membered heterocyclyl and $C_{0-6}$ alkylene-5- to 10-membered heteroaryl are optionally substituted with one or more substituents selected from halogen, $C_{1-6}$ alkyl, $C_{1-6}$ haloalkyl, $C_{1-6}$ hydroxyalkyl, $C_{2-6}$ alkenyl, $C_{2-6}$ alkynyl, 3- to 10-membered cycloalkyl, 4- to 10-membered heterocycloalkyl, $C_{6-10}$ aryl, 5- to 10-membered heteroaryl, -C(O)-$C_{1-6}$ alkyl, -C(O)-$C_{1-6}$ hydroxyalkyl, -C(O)-$C_{1-6}$ haloalkyl, -C(O)-$C_{2-6}$ alkenyl, -C(O)-$C_{2-6}$ alkynyl, -C(O)-3- to 10-membered cycloalkyl, -C(O)-4-to 10-membered heterocycloalkyl, -C(O)-$C_{6-10}$ aryl, -C(O)-5- to 10-membered heteroaryl, -CN and oxo group;
$R_3$ and $R_3$' are each independently selected from $C_{1-6}$ alkyl, $C_{1-6}$ haloalkyl, $C_{2-6}$ alkenyl and $C_{2-6}$ alkynyl;
wherein,
R and R' are independently selected from H and halogen;
R" is independently selected from H, $C_{1-6}$ alkyl, $C_{1-6}$ haloalkyl, $C_{2-6}$ alkenyl, $C_{2-6}$ alkynyl, $C_{3-7}$ cycloalkyl, 3- to 7-membered heterocyclyl, $C_{6-10}$ aryl and 5- to 10-membered heteroaryl.

**23.** The compound of claim 22, or a pharmaceutically acceptable salt, an enantiomer, a diastereomer, a solvate, a hydrate, a polymorph or an isotopic variant thereof, which is a compound of formula (VII):

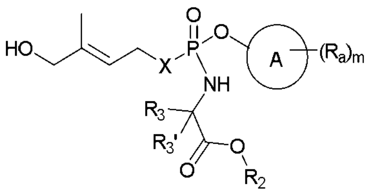

(VII)

wherein,

X is O or CRR';
ring A is $C_{6-12}$ aryl or 5- to 12-membered heteroaryl;
$R_a$ is selected from halogen, $C_{1-6}$ alkyl, $C_{1-6}$ haloalkyl, -OR", NR"R", $C_{2-6}$ alkenyl and $C_{2-6}$ alkynyl;
m=1, 2, 3, 4, or 5;
X is O or CRR';
$R_2$ is selected from $C_{1-6}$ hydroxyalkyl, $C_{5-6}$ alkyl, $C_{5-6}$ haloalkyl, $C_{5-6}$ alkenyl, $C_{5-6}$ alkynyl, $C_{0-6}$ alkylene-$C_{3-12}$ cycloalkyl, $C_{0-6}$ alkylene-4- to 12-membered heterocyclyl and $C_{0-6}$ alkylene-5- to 10-membered heteroaryl, wherein the $C_{1-6}$ hydroxyalkyl, $C_{5-6}$ alkyl, $C_{5-6}$ haloalkyl, $C_{5-6}$ alkenyl, $C_{5-6}$ alkynyl, $C_{0-6}$ alkylene-$C_{3-12}$ cycloalkyl, $C_{0-6}$ alkylene-4- to 12-membered heterocyclyl and $C_{0-6}$ alkylene-5- to 10-membered heteroaryl are optionally substituted with one or more substituents selected from halogen, $C_{1-6}$ alkyl, $C_{1-6}$ haloalkyl, $C_{1-6}$ hydroxyalkyl, $C_{2-6}$ alkenyl, $C_{2-6}$ alkynyl, 3- to 10-membered cycloalkyl, 4- to 10-membered heterocycloalkyl, $C_{6-10}$ aryl, 5- to 10-membered heteroaryl, -C(O)-$C_{1-6}$ alkyl, -C(O)-$C_{1-6}$ hydroxyalkyl, -C(O)-$C_{1-6}$ haloalkyl, -C(O)-$C_{2-6}$ alkenyl, -C(O)-$C_{2-6}$ alkynyl, -C(O)-3- to 10-membered cycloalkyl, -C(O)-4-to 10-membered heterocycloalkyl, -C(O)-$C_{6-10}$ aryl, -C(O)-5- to 10-membered heteroaryl, -CN and oxo group;
$R_3$ and $R_3$' are each independently selected from $C_{1-6}$ alkyl, $C_{1-6}$ haloalkyl, $C_{2-6}$ alkenyl and $C_{2-6}$ alkynyl;
wherein,
R and R' are independently selected from H and halogen;
R" is independently selected from H, $C_{1-6}$ alkyl, $C_{1-6}$ haloalkyl, $C_{2-6}$ alkenyl, $C_{2-6}$ alkynyl, $C_{3-7}$ cycloalkyl, 3- to 7-membered heterocyclyl, $C_{6-10}$ aryl and 5- to 10-membered heteroaryl.

**24.** The compound of claim 22 or 23, or a pharmaceutically acceptable salt, an enantiomer, a diastereomer, a solvate, a hydrate, a polymorph or an isotopic variant thereof, wherein X is CRR', alternatively $CH_2$ or $CF_2$.

**25.** The compound of any one of claims 22 to 24, or a pharmaceutically acceptable salt, an enantiomer, a diastereomer, a solvate, a hydrate, a polymorph or an isotopic variant thereof, wherein $R_2$ is $C_{1-6}$ alkyl, $C_{0-6}$ alkylene-$C_{3-7}$ cycloalkyl, $C_{0-6}$ alkylene-3- to 7-membered heterocyclyl, $C_{0-6}$ alkylene-$C_{6-10}$ aryl or $C_{0-6}$ alkylene-5- to 10-membered heteroaryl, alternatively $R_2$ is isopropyl, 2-ethylbutyl, cyclopentyl, cyclohexyl, tetrahydrofuranyl, tetrahydrothiophenyl, pyrrolidinyl, tetrahydropyranyl, tetrahydrothiopyranyl, tetrahydropyridinyl or benzyl, and still alternatively $R_2$ is 2-ethylbutyl, cyclopentyl, tetrahydrofuranyl, tetrahydrothiophenyl, pyrrolidinyl or benzyl, and still alternatively $R_2$ is cyclopentyl, tetrahydrofuranyl or benzyl.

**26.** The compound of any one of claims 22 to 25, or a pharmaceutically acceptable salt, an enantiomer, a diastereomer, a solvate, a hydrate, a polymorph or an isotopic variant thereof, wherein $R_3$ is $C_{1-6}$ alkyl, alternatively methyl or isopropyl.

**27.** The compound of any one of claims 22 to 26, or a pharmaceutically acceptable salt, an enantiomer, a diastereomer, a solvate, a hydrate, a polymorph or an isotopic variant thereof, wherein $R_2$ is 3- to 12-membered cycloalkyl or 4- to 12-membered heterocyclyl, wherein the 3- to 12-membered cycloalkyl or the 4- to 12-membered heterocyclyl is optionally substituted with one or more substituents selected from halogen, $C_{1-6}$ alkyl, $C_{1-6}$ haloalkyl, $C_{1-6}$ hydroxyalkyl, $C_{2-6}$ alkenyl, $C_{2-6}$ alkynyl, 3- to 10-membered cycloalkyl, 4- to 10-membered heterocycloalkyl, $C_{6-10}$ aryl, 5- to 10-membered heteroaryl, -C(O)-$C_{1-6}$ alkyl, -C(O)-$C_{1-6}$ hydroxyalkyl, -C(O)-$C_{1-6}$ haloalkyl, -C(O)-$C_{2-6}$ alkenyl, -C(O)-$C_{2-6}$ alkynyl, -C(O)-3- to 10-membered cycloalkyl, -C(O)-4- to 10-membered heterocycloalkyl, -C(O)-$C_{6-10}$ aryl, -C(O)-5- to 10-membered heteroaryl, -CN and oxo group; alternatively, $R_2$ is 4- to 10-membered heterocyclyl optionally substituted with one or more substituents selected from halogen, $C_{1-6}$ alkyl, -C(O)-$C_{1-6}$ alkyl, $C_{1-6}$ haloalkyl, $C_{1-6}$ hydroxyalkyl, -C(O)-3- to 10-membered cycloalkyl, -CN and oxo group; and still alternatively, $R_2$ is 4- to 7-membered heterocyclyl, wherein the heteroatom is oxygen atom or nitrogen atom, and the 4- to 7-membered heterocyclyl is optionally substituted with one or more substituents selected from $C_{1-6}$ alkyl and oxo group; and still alternatively, $R_2$ is selected from:

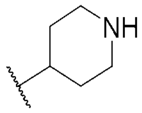, 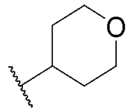, 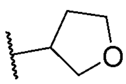, 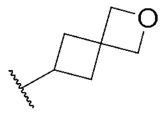, 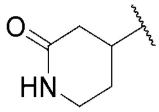, 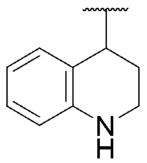, 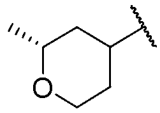,

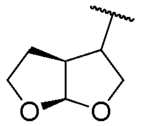, 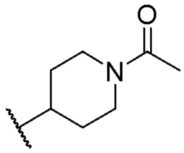, 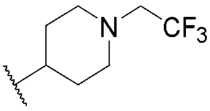, 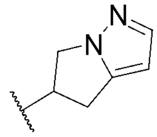, 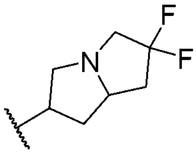, 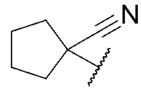,

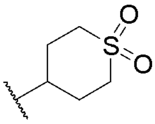, 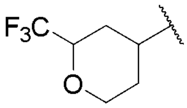, 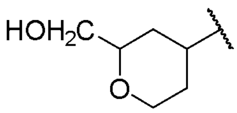 and 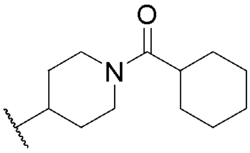.

**28.** The compound of any one of claims 1 to 27, or a pharmaceutically acceptable salt, an enantiomer, a diastereomer, a solvate, a hydrate, a polymorph or an isotopic variant thereof, wherein the compound is selected from:

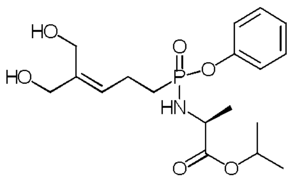, 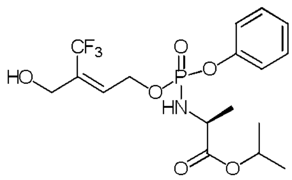, 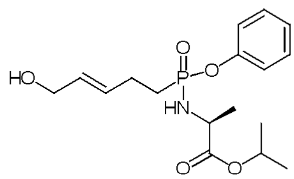,

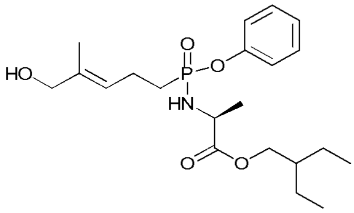, 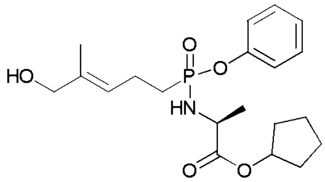, 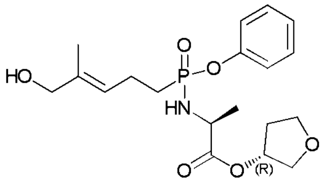,

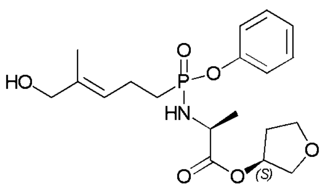, 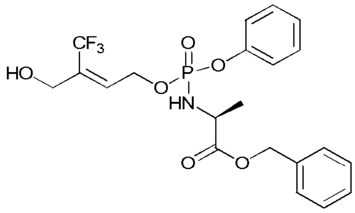, 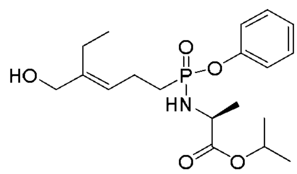,

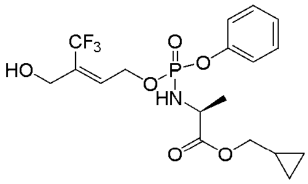, 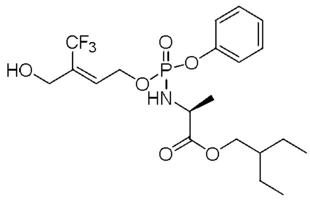, 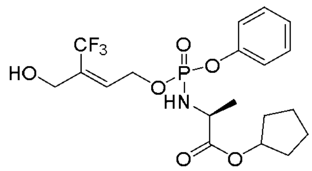,

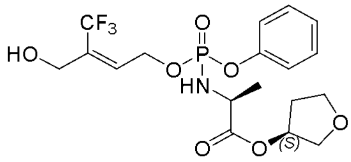, 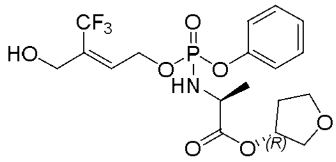, 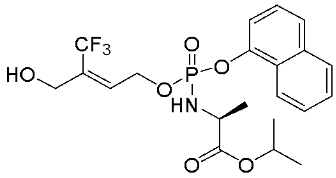,

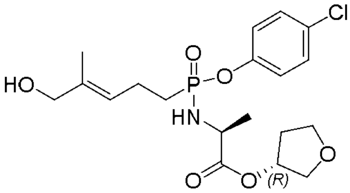, 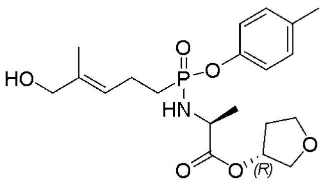, 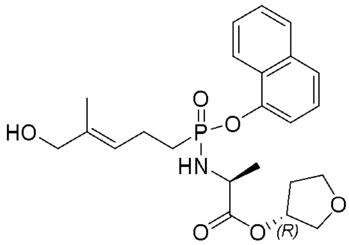,

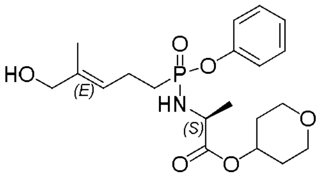, 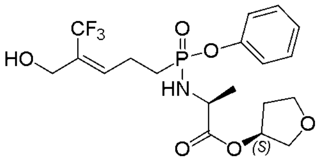, 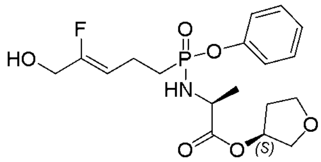,

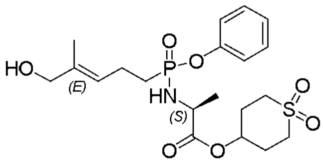, 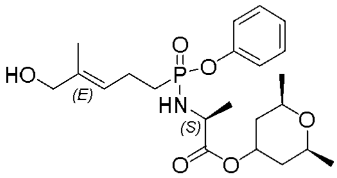, 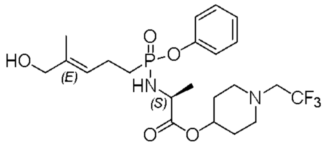,

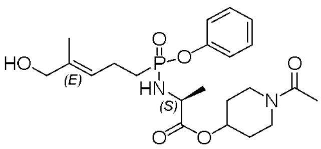, 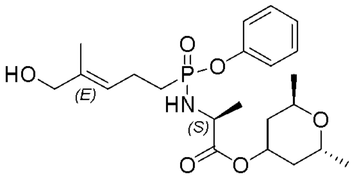, 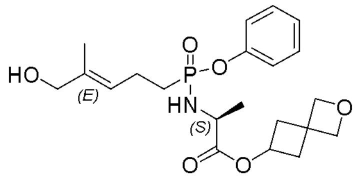,

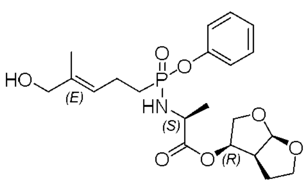, 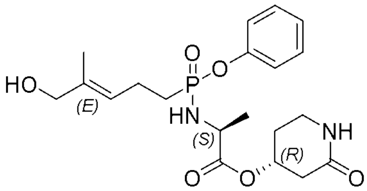, 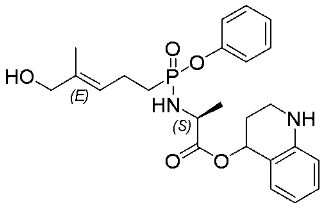,

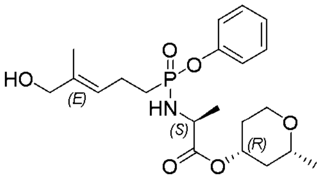, 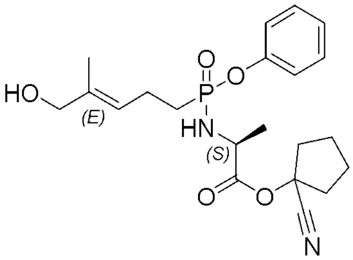, 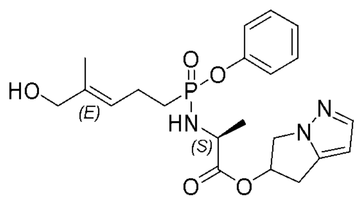,

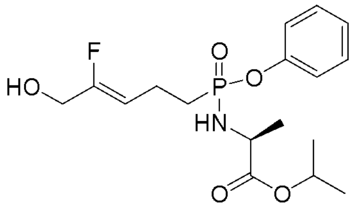, 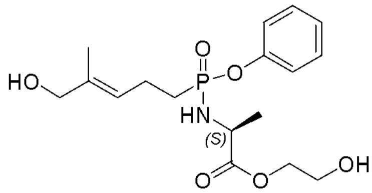, 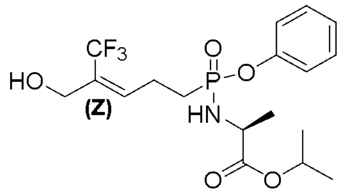,

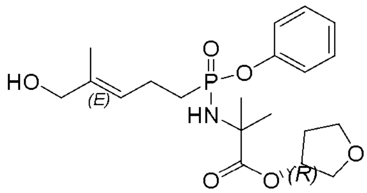, 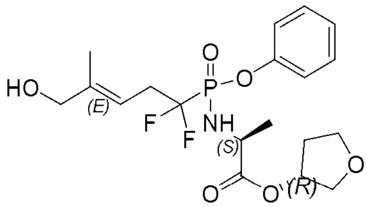, 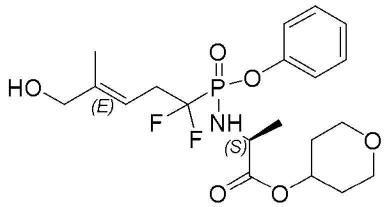,

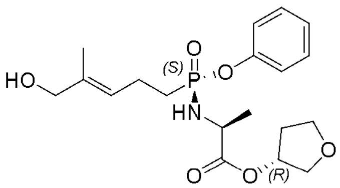, 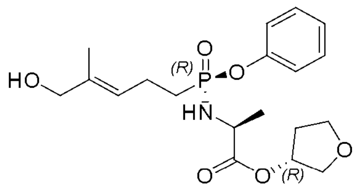, 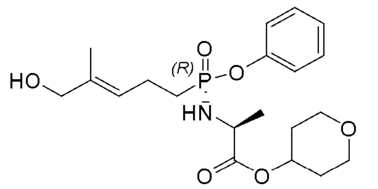

and 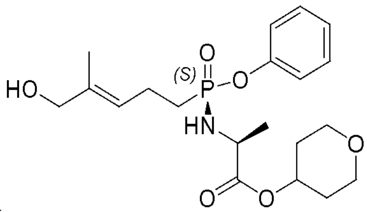.

**29.** The compound of any one of claims 1 to 28, or a pharmaceutically acceptable salt, an enantiomer, a diastereomer, a solvate, a hydrate, a polymorph or an isotopic variant thereof, wherein the compound is selected from:

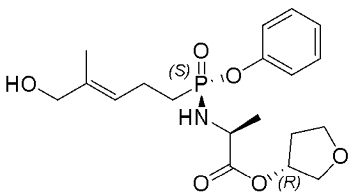, 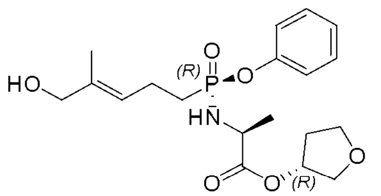, 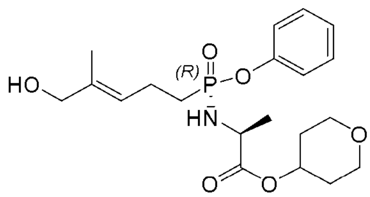

and 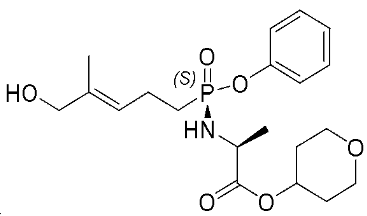.

**30.** A pharmaceutical composition comprising a compound of any one of claims 1-29, or a pharmaceutically acceptable salt, an enantiomer, a diastereomer, a solvate, a hydrate, a polymorph or an isotopic variant thereof.

**31.** Use of a compound of any one of claims 1-29, or a pharmaceutically acceptable salt, an enantiomer, a diastereomer, a solvate, a hydrate, a polymorph or an isotopic variant thereof, or a pharmaceutical composition of claim 30 in the manufacture of a medicament for treating a proliferative disease.

**32.** A compound of any one of claims 1-29 or a pharmaceutically acceptable salt, an enantiomer, a diastereomer, a solvate, a hydrate, a polymorph or an isotopic variant thereof, or a pharmaceutical composition of claim 30 for use in treating a proliferative disease.

**33.** A method for treating a proliferative disease in a subject, the method comprising administering to the subject a compound of any one of claims 1-29, or a pharmaceutically acceptable salt, an enantiomer, a diastereomer, a solvate, a hydrate, a polymorph or an isotopic variant thereof, or a pharmaceutical composition of claim 14.

**34.** The use of claim 31 or the use of the compound or pharmaceutical composition of claim 32 or the method of claim 33, wherein the proliferative disease is selected from cancer, cardiovascular disorders, infectious diseases, chronic inflammatory diseases, autoimmune disorders and other cell proliferative disorders; optionally, wherein the cancer is selected from solid tumors and hematological malignancies, such as breast cancer, neuroblastoma, malignant rhabdomyomas, well-differentiated and dedifferentiated liposarcoma, glioma, lung cancer, colorectal cancer, gastric cancer, gastrointestinal stromal tumor (GIST), hepatocellular carcinoma, prostate tumor, sarcoma, ovarian cancer, cervical cancer, pancreatic cancer, melanoma, thyroid cancer, bile duct cancer, endometrial cancer, renal cancer, mesothelioma, lymphoma, leukemia, non-Hodgkin's lymphoma, mantle cell lymphoma, anaplastic large cell lymphoma, acute myeloid leukemia (AML), and multiple myeloma; alternatively, wherein the proliferative disease is selected from multiple myeloma, non-Hodgkin's lymphoma, lung cancer, renal cancer and prostate cancer.

**35.** Use of a compound of formula (I), or a pharmaceutically acceptable salt, an enantiomer, a diastereomer, a solvate, a hydrate, a polymorph or an isotopic variant thereof in the manufacture of a medicament for promoting binding of butyrophilin 3A1/2A1:

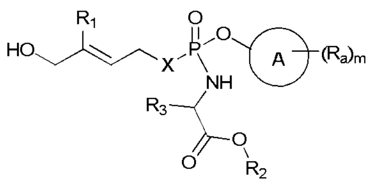

(I)

wherein,

ring A is $C_{6-10}$ aryl or 5- to 10-membered heteroaryl;
$R_a$ is selected from H, halogen, $C_{1-6}$ alkyl, $C_{1-6}$ haloalkyl, -OR", NR"R", $C_{2-6}$ alkenyl and $C_{2-6}$ alkynyl;
m=1, 2, 3, 4 or 5;
X is O or CRR';
$R_1$ is selected from H, F, Cl, CN and methyl, wherein the methyl is optionally substituted with 1 to 3 substituents independently selected from halogen, CN, OH and $NH_2$;
$R_2$ is selected from $C_{1-6}$ alkyl, $C_{1-6}$ haloalkyl, $C_{2-6}$ alkenyl, $C_{2-6}$ alkynyl, $C_{0-6}$ alkylene-$C_{3-7}$ cycloalkyl, $C_{0-6}$ alkylene-3- to 7-membered heterocyclyl, $C_{0-6}$ alkylene-$C_{6-10}$ aryl and $C_{0-6}$ alkylene-5- to 10-membered heteroaryl;
$R_3$ is selected from $C_{1-6}$ alkyl, $C_{1-6}$ haloalkyl, $C_{2-6}$ alkenyl and $C_{2-6}$ alkynyl;
wherein,
R and R' are independently selected from H and halogen;
R" is independently selected from H, $C_{1-6}$ alkyl, $C_{1-6}$ haloalkyl, $C_{2-6}$ alkenyl, $C_{2-6}$ alkynyl, $C_{3-7}$ cycloalkyl, 3- to 7-membered heterocyclyl, $C_{6-10}$ aryl and 5- to 10-membered heteroaryl.

**36.** A compound of formula (I), or a pharmaceutically acceptable salt, an enantiomer, a diastereomer, a solvate, a hydrate, a polymorph or an isotopic variant thereof for use in promoting binding of butyrophilin 3A1/2A1:

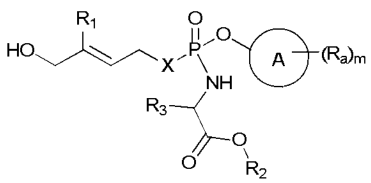

(I)

wherein,

ring A is $C_{6-10}$ aryl or 5- to 10-membered heteroaryl;
$R_a$ is selected from H, halogen, $C_{1-6}$ alkyl, $C_{1-6}$ haloalkyl, -OR", NR"R", $C_{2-6}$ alkenyl and $C_{2-6}$ alkynyl;
m=1, 2, 3, 4, or 5;
X is O or CRR';
$R_1$ is selected from H, F, Cl, CN and methyl, wherein the methyl is optionally substituted with 1 to 3 substituents independently selected from halogen, CN, OH and $NH_2$;
$R_2$ is selected from $C_{1-6}$ alkyl, $C_{1-6}$ haloalkyl, $C_{2-6}$ alkenyl, $C_{2-6}$ alkynyl, $C_{0-6}$ alkylene-$C_{3-7}$ cycloalkyl, $C_{0-6}$ alkylene-3- to 7-membered heterocyclyl, $C_{0-6}$ alkylene-$C_{6-10}$ aryl and $C_{0-6}$ alkylene-5- to 10-membered heteroaryl;
$R_3$ is selected from $C_{1-6}$ alkyl, $C_{1-6}$ haloalkyl, $C_{2-6}$ alkenyl and $C_{2-6}$ alkynyl;
wherein,
R and R' are independently selected from H and halogen;
R" is independently selected from H, $C_{1-6}$ alkyl, $C_{1-6}$ haloalkyl, $C_{2-6}$ alkenyl, $C_{2-6}$ alkynyl, $C_{3-7}$ cycloalkyl, 3- to 7-membered heterocyclyl, $C_{6-10}$ aryl and 5- to 10-membered heteroaryl.

**37.** A method for promoting binding of butyrophilin 3A1/2A1 in a subject, the method comprising administering to the subject a compound of formula (I), or a pharmaceutically acceptable salt, an enantiomer, a diastereomer, a solvate, a hydrate, a polymorph or an isotopic variant thereof:

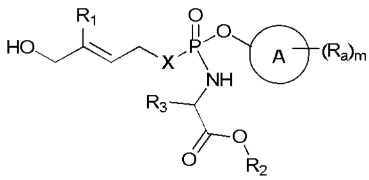

(I)

wherein,

ring A is $C_{6-10}$ aryl or 5- to 10-membered heteroaryl;
$R_a$ is selected from H, halogen, $C_{1-6}$ alkyl, $C_{1-6}$ haloalkyl, -OR", NR"R", $C_{2-6}$ alkenyl and $C_{2-6}$ alkynyl;
m=1, 2, 3, 4, or 5;
X is O or CRR';
$R_1$ is selected from H, F, Cl, CN and methyl, wherein the methyl is optionally substituted with 1 to 3 substituents independently selected from halogen, CN, OH and $NH_2$;
$R_2$ is selected from $C_{1-6}$ alkyl, $C_{1-6}$ haloalkyl, $C_{2-6}$ alkenyl, $C_{2-6}$ alkynyl, $C_{0-6}$ alkylene-$C_{3-7}$ cycloalkyl, $C_{0-6}$ alkylene-3- to 7-membered heterocyclyl, $C_{0-6}$ alkylene-$C_{6-10}$ aryl and $C_{0-6}$ alkylene-5- to 10-membered heteroaryl;
$R_3$ is selected from $C_{1-6}$ alkyl, $C_{1-6}$ haloalkyl, $C_{2-6}$ alkenyl and $C_{2-6}$ alkynyl;
wherein,
R and R' are independently selected from H and halogen;
R" is independently selected from H, $C_{1-6}$ alkyl, $C_{1-6}$ haloalkyl, $C_{2-6}$ alkenyl, $C_{2-6}$ alkynyl, $C_{3-7}$ cycloalkyl, 3- to 7-membered heterocyclyl, $C_{6-10}$ aryl and 5- to 10-membered heteroaryl.

**38.** The use of claim 35 or the use of the compound or pharmaceutical composition of claim 36 or the method of claim 37, wherein the compound is selected from the compound of any one of claims 1-29, or a pharmaceutically acceptable salt, an enantiomer, a diastereomer, a solvate, a hydrate, a polymorph or an isotopic variant thereof.

**39.** The use of claim 35 or the use of the compound or pharmaceutical composition of claim 36 or the method of claim 37, wherein the compound is selected from the following compounds or pharmaceutically acceptable salts, enantiomers, diastereomers, solvates, hydrates, polymorphs or isotopic variants thereof:

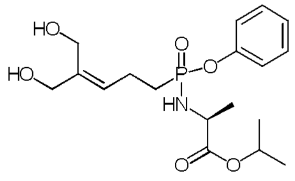,
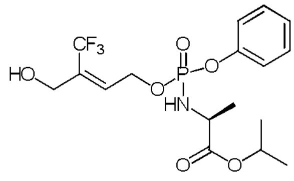,
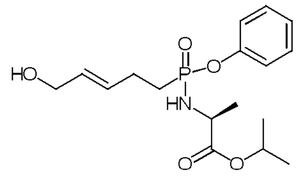,

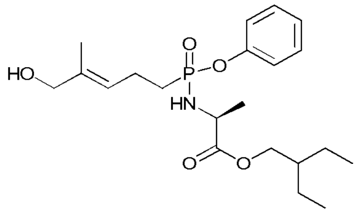,
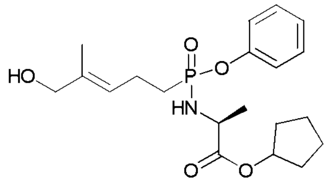,
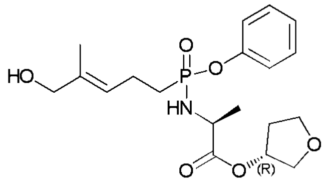,

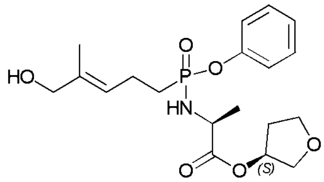,
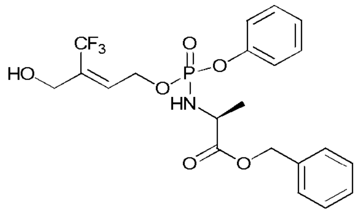,
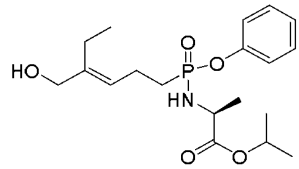,

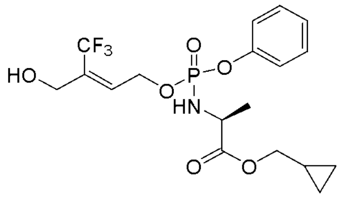,
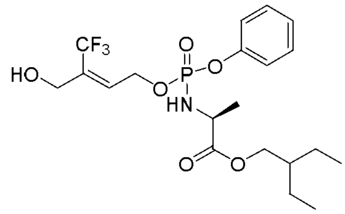,
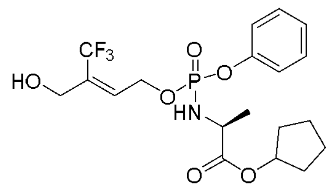,

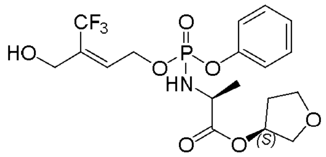,
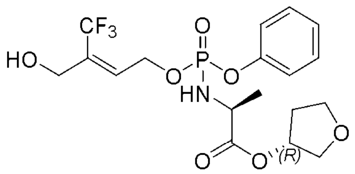,
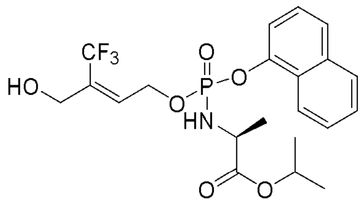,

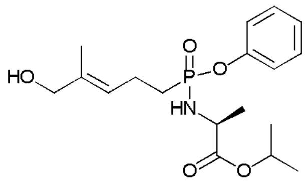,
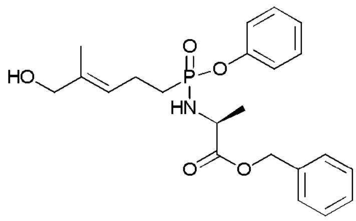,
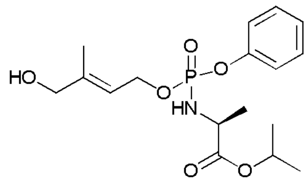,

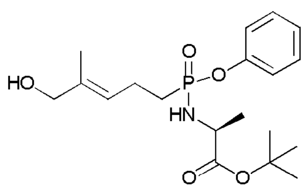,
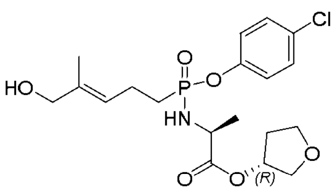,
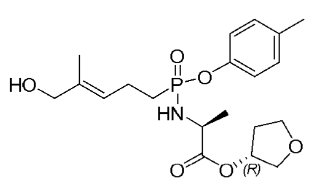,

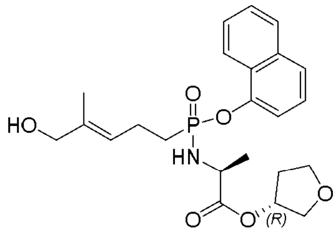 , 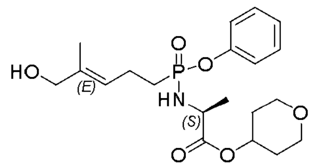 , 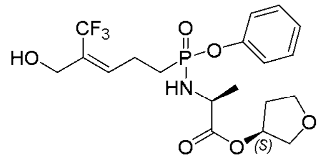 ,

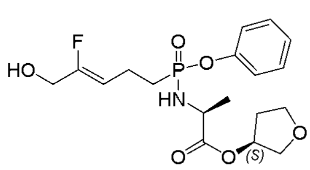 , 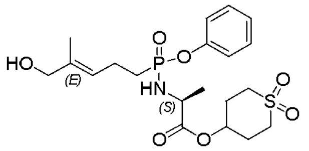 , 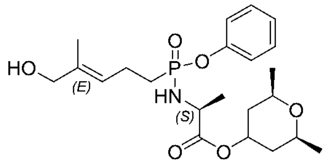 ,

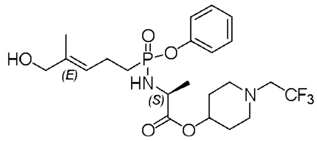 , 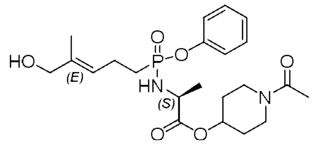 , 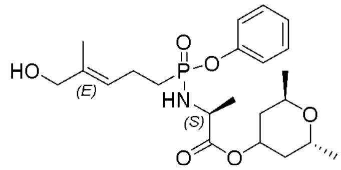 ,

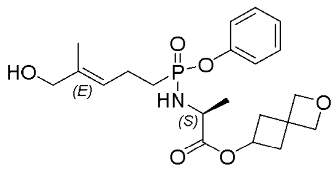 , 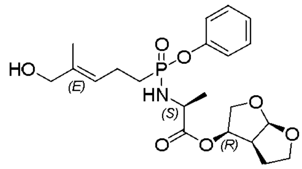 , 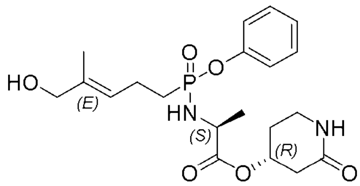 ,

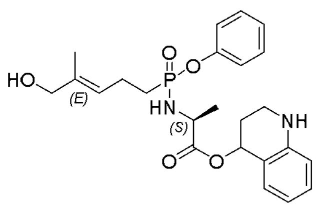 , 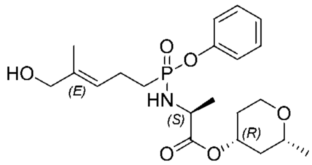 , 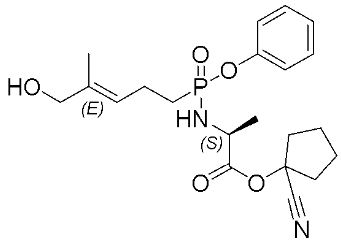 ,

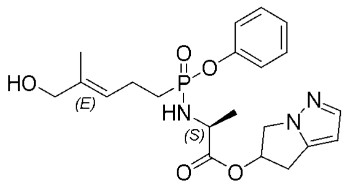 , 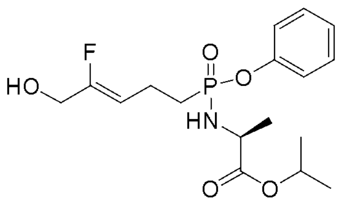 , 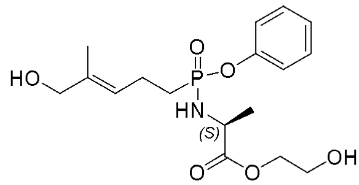 ,

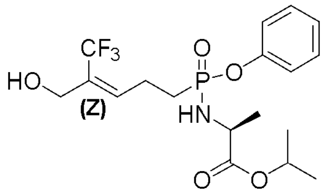 , 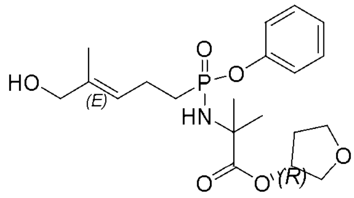 , 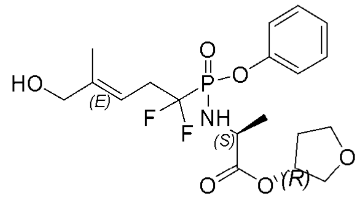 ,

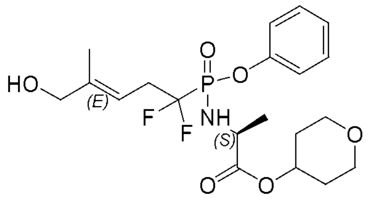
,
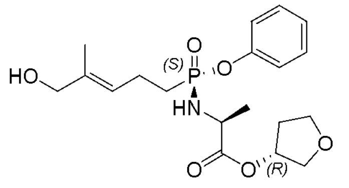
,
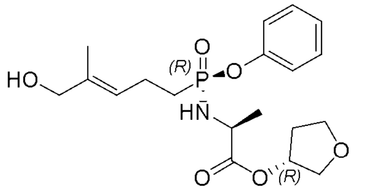
,
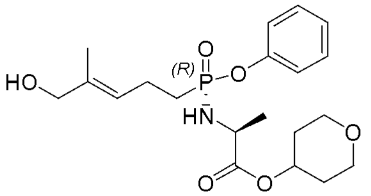
and
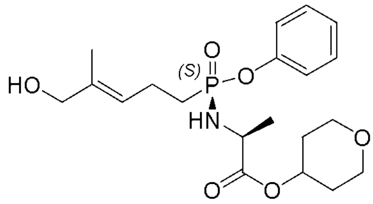
.

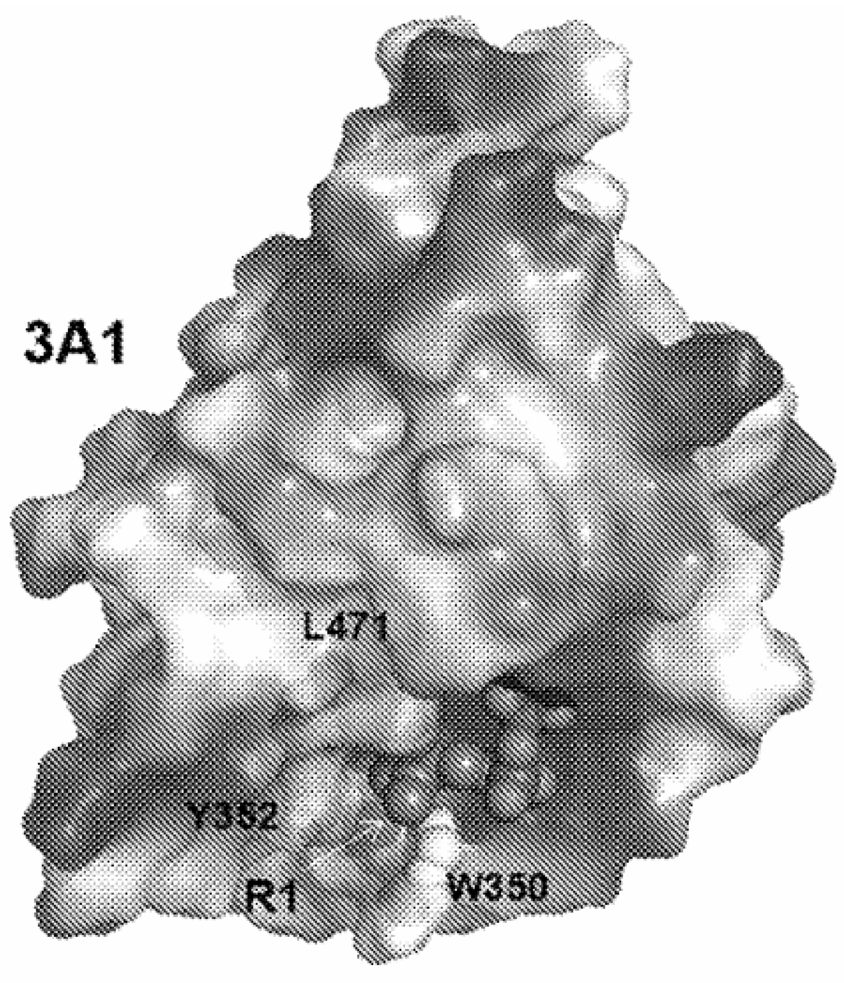


Fig.1

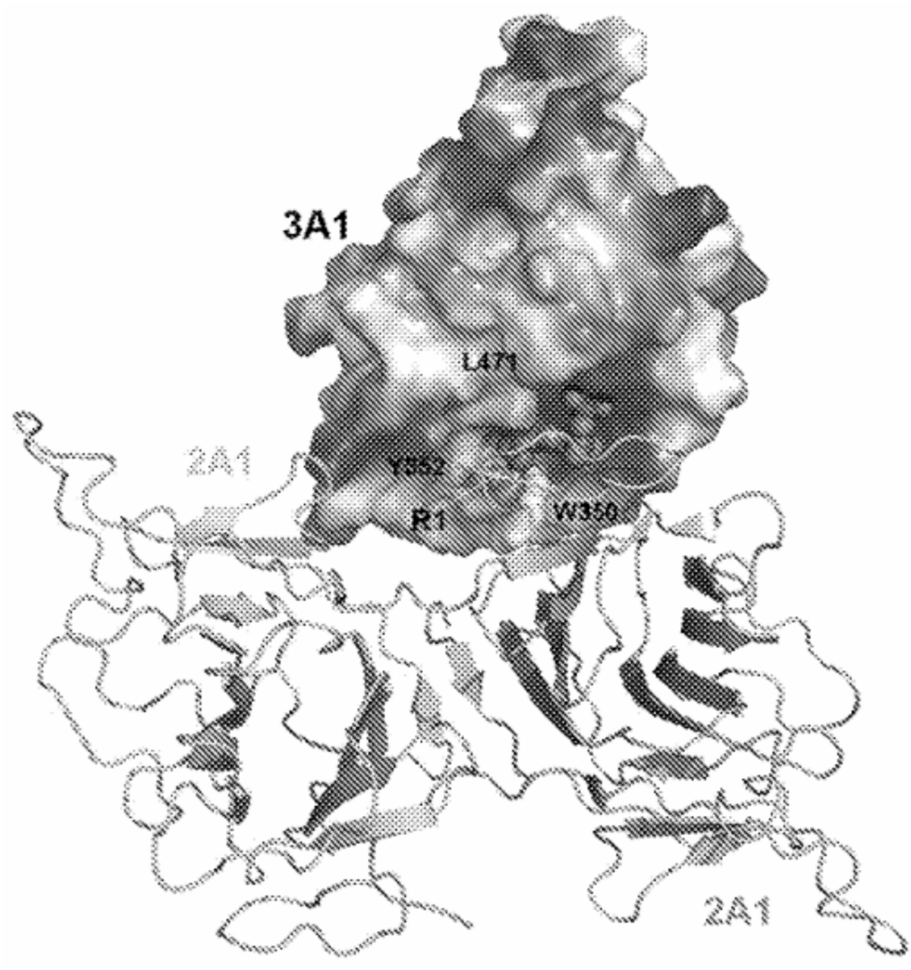


Fig.2

## INTERNATIONAL SEARCH REPORT

International application No.
**PCT/CN2024/070780**

**A. CLASSIFICATION OF SUBJECT MATTER**

C07F9/44(2006.01)i; A61K31/664(2006.01)i; A61P35/00(2006.01)i; A61P31/00(2006.01)i

According to International Patent Classification (IPC) or to both national classification and IPC

**B. FIELDS SEARCHED**

Minimum documentation searched (classification system followed by classification symbols)

IPC: C07F, A61K, A61P

Documentation searched other than minimum documentation to the extent that such documents are included in the fields searched

Electronic data base consulted during the international search (name of data base and, where practicable, search terms used)

CNABS; EPTXT; USTXT; WOTXT; JPTXT; CNTXT; CNKI, Registry(STN), Caplus(STN), Marpat(STN): 清辉联诺, 膦, 嗜乳脂蛋白, phos+, BTN3A1, BTN2A1, BTN, 癌症, 肿瘤, 感染, cancer, tumo?r, infection, 结构式检索, structural formula search

**C. DOCUMENTS CONSIDERED TO BE RELEVANT**

| Category* | Citation of document, with indication, where appropriate, of the relevant passages | Relevant to claim No. |
|---|---|---|
| X | WO 2020008189 A1 (UNIVERSITY COLLEGE CARDIFF CONSULTANTS LTD. et al.) 09 January 2020 (2020-01-09)<br>description, pages 1-5 and 8-9 | 1-39 |
| X | WO 2019182904 A1 (UNIVERSITY OF IOWA RESEARCH FOUNDATION et al.) 26 September 2019 (2019-09-26)<br>description, pages 1-2, and claim 13 | 1-39 |
| X | KADRI Hachemi et al. "Aryloxy Diester Phosphonamidate Prodrugs of Phosphoantigens (ProPAgens) as Potent Activators of Vγ9/Vδ2 T-Cell Immune Responses"<br>*Journal of Medicinal Chemistry*, Vol. vol. 63, 15 September 2020 (2020-09-15),<br>pages 11258-11270 | 36 |
| X | LENTINI Nicholas A. et al. "Synthesis and Bioactivity of the Alanyl Phosphonamidate Stereoisomers Derived from a Butyrophilin Ligand"<br>*ACS Medicinal Chemistry Letters*, Vol. vol. 10, 06 August 2019 (2019-08-06),<br>pages 1284-1289 | 36 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **23 March 2024** | **28 March 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

INTERNATIONAL SEARCH REPORT

International application No.
**PCT/CN2024/070780**

**C. DOCUMENTS CONSIDERED TO BE RELEVANT**

| Category* | Citation of document, with indication, where appropriate, of the relevant passages | Relevant to claim No. |
|---|---|---|
| X | KADRI Hachemi et al. "Aryloxy Triester Phosphonamidates of Phosphoantigens Exhibit Favorable Stability and Potent Activation of Vγ9/Vδ2 T-Cells" *ChemRxiv,* 31 December 2018 (2018-12-31), pp. 1-15 | 36 |
| A | LENTINI Nicholas A. et al. "Phosphonamidate Prodrugs of a Butyrophilin Ligand Display Plasma Stability and Potent Vγ9 Vδ2 T Cell Stimulation" *Journal of Medicinal Chemistry,* Vol. vol. 61, 10 September 2018 (2018-09-10), pages 8658-8669 | 1-39 |
| A | LENTINI Nicholas A. et al. "Synthesis and Metabolism of BTN3A1 Ligands: Studies on Modifications of the Allylic Alcohol" *ACS Medicinal Chemistry Letters,* Vol. vol. 12, 04 December 2020 (2020-12-04), pp. 136-142 | 1-39 |

Form PCT/ISA/210 (second sheet) (July 2022)

**INTERNATIONAL SEARCH REPORT**

International application No.
**PCT/CN2024/070780**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **33-34, 37-39**
because they relate to subject matter not required to be searched by this Authority, namely:

The subject matter of claims 33-34 and 37-39 relates to a method for treatment of diseases of the human or animal body, which falls within the cases set out in PCT Rule 39.1(iv). The search is carried out on the basis of the use of said compound or a pharmaceutically acceptable salt or pharmaceutical composition thereof in the preparation of a drug for corresponding diseases.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/070780**

| Patent document cited in search report | Publication date (day/month/year) | Patent family member(s) | Publication date (day/month/year) |
|---|---|---|---|
| WO 2020008189 A1 | 09 January 2020 | GB 201810965 D0 | 15 August 2018 |
| | | US 2021171552 A1 | 10 June 2021 |
| | | EP 3818064 A1 | 12 May 2021 |
| | | JP 2021529786 A | 04 November 2021 |
| | | CA 3137835 A1 | 09 January 2020 |
| WO 2019182904 A1 | 26 September 2019 | US 2021115074 A1 | 22 April 2021 |
| | | WO 2019182904 A8 | 07 May 2020 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2020008189 A
- WO 2019182904 A


### Non-patent literature cited in the description


- **BERGE et al.** *J. Pharmaceutical Sciences*, 1977, vol. 66, 1-19